# EUROPEAN PATENT SPECIFICATION

(11) **EP 2 007 733 B1**
(45) Date of publication and mention of the grant of the patent: **25.05.2016**
(21) Application number: 07733600.6
(22) Date of filing: 02.04.2007
(51) Int. Cl.: C07D 231/56, A61K 31/416, A61P 35/00, C07D 403/12, C07D 401/14, C07D 491/08, C07D 249/20

(54) **AMIDE SUBSTITUTED INDAZOLE AND BENZOTRIAZOLE DERIVATIVES AS POLY(ADP-RIBOSE)POLYMERASE (PARP) INHIBITORS**
AMIDSUBSTITUIERTE INDAZOL- UND BENZOTRIAZOLDERIVATE ALS INHIBITOREN DER POLY(ADP-RIBOSE)POLYMERASE (PARP)
UTILISATION DE DÉRIVÉS DE BENZOTRIAZOLE ET D'INDAZOLE AMIDE SUBSTITUÉ COMME INHIBITEURS DE LA POLY(ADP-RIBOSE)POLYMÉRASE (PARP)

(30) Priority: 03.04.2006 GB 0606663; 11.08.2006 GB 0615907; 10.01.2007 GB 0700432
(43) Date of publication of application: 31.12.2008
(73) Proprietor: MSD Italia S.r.l., 00189 Rome (IT)
(72) Inventor: BOUERES, Julia Kay, 00040 Pomezia (Rome) (IT); BRANCA, Danila, 00040 Pomezia (Rome) (IT); FERRIGNO, Federica, 00040 Pomezia (Rome) (IT); JONES, Philip, 00040 Pomezia (Rome) (IT); MURAGLIA, Ester, 00040 Pomezia (Rome) (IT); ONTORIA ONTORIA, Jesus Maria, 00040 Pomezia (Rome) (IT); ORVIETO, Federica, 00040 Pomezia (Rome) (IT); SCARPELLI, Rita, 00040 Pomezia (Rome) (IT); SCHULTZ-FADEMRECHT, Carsten, 00040 Pomezia (Rome) (IT)
(74) Representative: Hussain, Deeba
(86) International application number: PCT/GB2007/050177
(87) International publication number: WO 2007/113596

(56) References cited:
- WO-A-00/26192
- WO-A-2005/066136
- ISHIDA ET AL: "4-Phenyl-1,2,3,6-tetrahydropyridine, an excellent fragment to improve the potency of PARP-1 inhibitors" BIOORGANIC & MEDICINAL CHEMISTRY LETTERS, OXFORD, GB, vol. 15, no. 19, 1 October 2005 (2005-10-01), pages 4221-4225, XP005038878 ISSN: 0960-894X
- PARVEEN I ET AL: "2-nitroimidazol-5-ylmethyl as a potential bioreductively activated prodrug system: reductively triggered release of the PARP inhibitor 5-bromoisoquinolinone" BIOORGANIC & MEDICINAL CHEMISTRY LETTERS, OXFORD, GB, vol. 9, no. 14, 19 July 1999 (1999-07-19), pages 2031-2036, XP004171631 ISSN: 0960-894X

## Description

The present invention relates to amide substituted indazoles and benzotriazoles which are inhibitors of the enzyme poly(ADP-ribose)polymerase (PARP), previously known as poly(ADP-ribose)synthase and poly(ADP-ribosyl)transferase. The compounds of the present invention are useful as mono-therapies in tumors with specific defects in DNA-repair pathways and as enhancers of certain DNA-damaging agents such as anticancer agents and radiotherapy. Further, the compounds of the present invention are useful for reducing cell necrosis (in stroke and myocardial infarction), down regulating inflammation and tissue injury, treating retroviral infections and protecting against the toxicity of chemotherapy.

Poly(ADP-ribose) polymerase (PARP) constitute a super family of eighteen proteins containing PARP catalytic domains (Bioessays (2004) 26:1148). These proteins include PARP-1, PARP-2, PARP-3, tankyrase-1, tankyrase-2, vaultPARP and TiPARP. PARP-1, the founding member, consists of three main domains: an amino (N)-terminal DNA-binding domain (DBD) containing two zinc fingers, the automodification domain, and a carboxy (C)-terminal catalytic domain.

PARP are nuclear and cytoplasmic enzymes that cleave NAD⁺ to nicotinamide and ADP-ribose to form long and branched ADP-ribose polymers on target proteins, including topoisomerases, histones and PARP itself *(*Biochem. Biophys. Res. Commun. (1998) 245:1-10).

Poly(ADP-ribosyl)ation has been implicated in several biological processes, including DNA repair, gene transcription, cell cycle progression, cell death, chromatin functions and genomic stability.

The catalytic activity of PARP-1 and PARP-2 has been shown to be promptly stimulated by DNA strand breakages (see Pharmacological Research (2005) 52:25-33). In response to DNA damage, PARP-1 binds to single and double DNA nicks. Under normal physiological conditions there is minimal PARP activity, however, upon DNA damage an immediate activation of PARP activity of up to 500-fold occurs. Both PARP-1 and PARP-2 detect DNA strand interruptions acting as nick sensors, providing rapid signals to halt transcription and recruiting the enzymes required for DNA repair at the site of damage. Since radiotherapy and many chemotherapeutic approaches to cancer therapy act by inducing DNA damage, PARP inhibitors are useful as chemo- and radiosensitizers for cancer treatment. PARP inhibitors have been reported to be effective in radio sensitizing hypoxic tumor cells (US 5,032,617, US 5,215,738 and US 5,041,653).

Most of the biological effects of PARP relate to this poly (ADP-ribosyl)ation process which influences the properties and function of the target proteins; to the PAR oligomers that, when cleaved from poly(ADP-ribosyl)ated proteins, confer distinct cellular effects; the physical association of PARP with nuclear proteins to form functional complexes; and the lowering of the cellular level of its substrate NAD⁺ (Nature Review (2005) 4:421-440).

Besides being involved in DNA repair, PARP may also act as a mediator of cell death. Its excessive activation in pathological conditions such as ischemia and reperfusion injury can result in substantial depletion of the intercellular NAD⁺, which can lead to the impairment of several NAD⁺ dependent metabolic pathways and result in cell death (see *Pharmacological Research* (2005) **52**:44-59). As a result of PARP activation, NAD⁺ levels significantly decline. Extensive PARP activation leads to severe depletion of NAD⁺ in cells suffering from massive DNA damage. The short half-life of poly(ADP-ribose) results in a rapid turnover rate, as once poly(ADP-ribose) is formed, it is quickly degraded by the constitutively active poly(ADP-ribose) glycohydrolase (PARG). PARP and PARG form a cycle that converts a large amount of NAD⁺ to ADP-ribose, causing a drop of NAD⁺ and ATP to less than 20% of the normal level. Such a scenario is especially detrimental during ischemia when deprivation of oxygen has already drastically compromised cellular energy output. Subsequent free radical production during reperfusion is assumed to be a major cause of tissue damage. Part of the ATP drop, which is typical in many organs during ischemia and reperfusion, could be linked to NAD⁺ depletion due to poly(ADP-ribose) turnover. Thus, PARP inhibition is expected to preserve the cellular energy level thereby potentiating the survival of ischemic tissues after insult. Compounds which are inhibitors of PARP are therefore useful for treating conditions which result from PARP mediated cell death, including neurological conditions such as stroke, trauma and Parkinson's disease.

PARP inhibitors have been demonstrated as being useful for the specific killing of BRCA-1 and BRCA-2 deficient tumors (Nature (2005) 434:913-916 and 917-921; and Cancel Biology & Therapy (2005) 4:934-936).

PARP inhibitors have been shown to enhance the efficacy of anticancer drugs (Pharmacological Research (2005) 52:25-33), including platinum compounds such as cisplatin and carboplatin (Cancer Chemother Pharmacol (1993) 33:157-162 and Mol Cancer Ther (2003) 2:371-382). PARP inhibitors have been shown to increase the antitumor activity of topoisomerase I inhibitors such as Irinotecan and Topotecan (Mol Cancer Ther (2003) 2:371-382; and Clin Cancer Res (2000) 6:2860-2867) and this has been demonstrated in *in vivo* models (J Natl Cancer Inst (2004) 96:56-67).

PARP inhibitors have been shown to restore susceptibility to the cytotoxic and antiproliferative effects of temozolomide (TMZ) (see Curr Med Chem (2002) 9:1285-1301 and Med Chem Rev Online (2004) 1:144-150). This has been demonstrated in a number *of in vitro* models *(*Br J Cancer (1995) 72:849-856; Br J Cancer (1996) 74:1030-1036; Mol Pharmacol (1997) 52:249-258; Leukemia (1999) 13:901-909; Glia (2002) 40:44-54; and Clin Cancer Res (2000) 6:2860-2867 and (2004) 10:881-889) and *in vivo* models *(*Blood (2002) 99:2241-2244; Clin Cancer Res (2003) 9:5370-5379 and JNatl Cancer Inst (2004) 96:56-67). PAPR inhibitors have also been shown to prevent the appearance of necrosis induced by selective *N*3-adenine methylating agents such as MeOSO₂(CH₂)-lexitropsin (Me-Lex) (Pharmacological Research (2005) 52:25-33).

PARP inhibitors have been shown to act as radiation sensitizers. PARP inhibitors have been reported to be effective in radiosensitizing (hypoxic) tumor cells and effective in preventing tumor cells from recovering from potentially lethal *(*Br. J. Cancer (1984) 49(Suppl. VI):34-42; and Int. J. Radiat. Bioi. (1999) 75:91-100) and sub-lethal (Clin. Oncol. (2004) 16(1):29-39) damage of DNA after radiation therapy, presumably by their ability to prevent DNA strand break rejoining and by affecting several DNA damage signaling pathways.

PARP inhibitors have also been shown to be useful for treating acute and chronic myocardial diseases (see Pharmacological Research (2005) 52:34-43). For instance, it has been demonstrated that single injections of PARP inhibitors have reduced the infarct size caused by ischemia and reperfusion of the heart or skeletal muscle in rabbits. In these studies, a single injection of 3-amino-benzamide (10 mg/kg), either one minute before occlusion or one minute before reperfusion, caused similar reductions in infarct size in the heart (32-42%) while 1,5-dihydroxyisoquinoline (1 mg/kg), another PARP inhibitor, reduced infarct size by a comparable degree (38-48%). These results make it reasonable to assume that PARP inhibitors could salvage previously ischemic heart or reperfusion injury of skeletal muscle tissue *(*PNAS (1997) 94:679-683). Similar findings have also been reported in pigs (Eur. J. Pharmacol. (1998) 359:143-150 and Ann. Thorac. Surg. (2002) 73:575-581) and in dogs (Shock. (2004) 21:426-32).

PARP inhibitors have been demonstrated as being useful for treating certain vascular diseases, septic shock, ischemic injury and neurotoxicity *(*Biochim. Biophys. Acta (1989) 1014:1-7; J. Clin. Invest. (1997) 100: 723-735). Oxygen radical DNA damage that leads to strand breaks in DNA, which are subsequently recognized by PARP, is a major contributing factor to such disease states as shown by PARP inhibitor studies *(*J. Neurosci. Res. (1994) 39:38-46 and PNAS (1996) 93:4688-4692). PARP has also been demonstrated to play a role in the pathogenesis of hemorrhagic shock *(*PNAS (2000) 97:10203-10208).

PARP inhibitors have been demonstrated as being useful for treatment of inflammation diseases (see Pharmacological Research (2005) 52:72-82 and 83-92).

It has also been demonstrated that efficient retroviral infection of mammalian cells is blocked by the inhibition of PARP activity. Such inhibition of recombinant retroviral vector infections has been shown to occur in various different cell types *(*J. Virology, (1996) 70(6):3992-4000). Inhibitors of PARP have thus been developed for use in anti-viral therapies and in cancer treatment (WO 91/18591).

*In vitro* and *in vivo* experiments have demonstrated that PARP inhibitors can be used for the treatment or prevention of autoimmune diseases such as Type I diabetes and diabetic complications *(*Pharmacological Research (2005) 52:60-71).

PARP inhibition has been speculated as delaying the onset of aging characteristics in human fibroblasts *(*Biochem. Biophys. Res. Comm. (1994) 201(2):665-672 and Pharmacological Research (2005) 52:93-99). This may be related to the role that PARP plays in controlling telomere function (Nature Gen., (1999) 23(1):76-80).

The vast majority of PARP inhibitors to date interact with the nicotinamide binding domain of the enzyme and behave as competitive inhibitors with respect to NAD⁺ (Expert Opin. Ther. Patents (2004) 14:1531-1551). Structural analogues of nicotinamide, such as benzamide and derivatives were among the first compounds to be investigated as PARP inhibitors. However, these molecules have a weak inhibitory activity and possess other effects unrelated to PARP inhibition. Thus, there is a need to provide potent inhibitors of the PARP enzyme.

Structurally related PARP inhibitors have previously been described. WO 1999/59973 discloses amide substituted benzene rings fused to 5 membered heteroaromatic rings; WO2001/85687 discloses amide substituted indoles; WO 1997/04771, WO 2000/26192, WO 2000/32579, WO 2000/64878, WO 2000/68206, WO 2001/21615, WO 2002/068407, WO 2003/106430 and WO 2004/096793 disclose amide substituted benzoimidazoles; WO 2000/29384 discloses amide substituted benzoimidazoles and indoles; and EP 0879820 discloses amide substituted benzoxazoles.

It has now surprisingly been discovered that amide substituted indazoles and benzotriazoles of the present invention exhibit high levels of inibition of the activity of PARP.

The compounds of this invention are useful in the inhibition of poly(ADP-ribose)polymerase (PARP). They are particularly useful as inhibitors of PARP-1 and/or PARP-2. The present invention provides compounds of formula I: 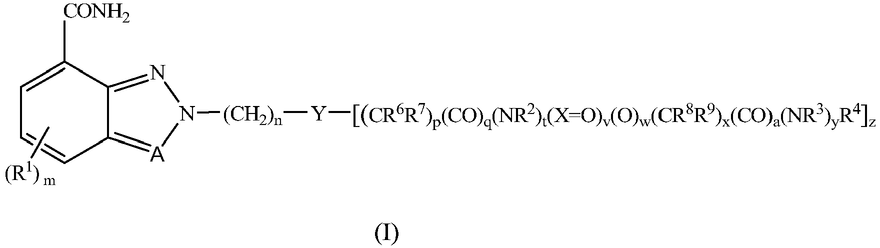 wherein:
a is 0 or 1;
m is 0, 1, 2 or 3;
n is 0, 1, 2, 3, 4, 5 or 6;
each p is independently 0, 1, 2, 3, 4, 5 or 6;
each q is independently 0 or 1;
each t is independently 0 or 1;
each v is independently 0 or 1;
each w is independently 0 or 1;
each x is independently 0, 1, 2, 3, 4, 5 or 6;
each y is independently 0 or 1;
z is 1, 2 or 3;
A is CH or N;
each R¹ is independently hydroxy, halogen, cyano, C₁₋₆alkyl, haloC₁₋₆alkyl, C₁₋₆alkoxy or haloC₁₋₆alkoxy;
Y is a direct bond or a ring which is: a C₃₋₅cycloalkyl, a 4 membered saturated heterocycle containing one N atom, a 5, 6 or 7 membered saturated or partially saturated heterocycle containing 1, 2 or 3 heteroatoms independently selected from N, O and S, a 5 membered unsaturated heterocycle containing 1, 2, 3 or 4 heteroatoms independently selected from O, N and S, but not more than one of which is O or S, a 6 membered unsaturated heterocycle containing 1, 2 or 3 nitrogen atoms, a 6-13 membered saturated, partially saturated or unsaturated hydrocarbon ring or a 8-13 membered unsaturated or partially saturated heterocyclic ring containing 1, 2, 3 or 4 heteroatoms independently selected from N, O and S;
each R² is independently hydrogen, C₁₋₆alkyl or C₃₋₁₀cycloalkyl;
each X is independently C or SO;
each R³ is independently hydrogen or C₁₋₆alkyl;
each R⁴ is independently hydrogen, hydroxy, cyano, halogen, C₁₋₆alkyl, C₂₋₁₀alkenyl, haloC₁₋₆alkyl, hydroxyC₁₋₆alkyl, C₁₋₆alkylcarbonyl, C₁₋₆alkoxy, haloC₁₋₆alkoxy, C₁₋₆alkoxycarbonyl, carboxy, nitro or a ring which is: C₆₋₁₀aryl; C₆₋₁₀aryloxy; C₆₋₁₀arylcarbonyl; C₃₋₁₀cycloalkyl; a 4 membered saturated heterocyclic ring containing one N atom; a 5 or 6 membered saturated or partially saturated heterocyclic ring containing one, two or three atoms independently selected from N, O and S; a 5 membered heteroaromatic ring containing 1, 2, 3 or 4 heteroatoms independently selected from N, O and S, not more than one heteroatom of which is O or S; a 6 membered heteroaromatic ring containing 1, 2 or 3 nitrogen atoms; or a 7-15 membered unsaturated, partially saturated or saturated heterocyclic ring containing 1, 2, 3 or 4 heteroatoms independently selected from N, O and S; any of which rings being optionally substituted by one or more groups independently selected from (CH₂)_{b}R⁵;
each b is independently 0, 1, 2, 3, 4, 5 or 6;
each R⁵ is independently hydroxy, oxo, cyano, halogen, C₁₋₆alkyl, C₂₋₁₀alkenyl, haloC₁₋₆alkyl, C₁₋₆alkylcarbonyl, C₁₋₆alkoxy, haloC₁₋₆alkoxy, hydroxyC₁₋₆alkyl, C₁₋₆alkoxycarbonyl, carboxy, NR^{a}R^{b}, CONR^{a}R^{b}, S(O)ᵣR^{c} or a ring which is: C₆₋₁₀aryl; C₆₋₁₀arylC₁₋₆alkyl; a 4 membered saturated heterocyclic ring containing one N atom; a 5, 6 or 7 membered saturated or partially saturated heterocyclic ring containing one, two or three atoms independently selected from N, O and S; a 5 membered heteroaromatic ring containing 1, 2, 3 or 4 heteroatoms independently selected from N, O and S, not more than one heteroatom of which is O or S; a 6 membered heteroaromatic ring containing 1, 2 or 3 nitrogen atoms; or a 7-10 membered unsaturated or partially saturated heterocyclic ring containing 1, 2, 3 or 4 heteroatoms independently selected from N, O and S; any of which rings being optionally substituted by one or more groups independently selected from hydroxy, cyano, halogen, C₁₋₆alkyl, C₁₋₆alkoxy, C₂₋₁₀alkenyl, haloC₁₋₆alkyl, amino, C₁₋₆alkylamino and di(C₁₋₆alkyl)amino;
each of R^{a} and R^{b} is independently hydrogen, C₁₋₆alkyl, C₁₋₆alkylcarbonyl, haloC₁₋₆alkyl, hydroxyC₁₋₆alkyl, S(O)ᵣR^{c} or S(O)ᵣN(R^{d})₂; or
R^{a} and R^{b} together with the N atom to which they are attached form a 4 membered saturated heterocycle containing one N atom or a 5, 6 or 7 membered saturated or partially saturated heterocycle containing one, two or three N atoms and zero or one O atom, the ring being optionally substituted by one or more groups independently selected from hydroxy, cyano, halogen, C₁₋₆alkyl, C₁₋₆alkoxy, C₂₋₁₀alkenyl and haloC₁₋₆alkyl;
r is 0, 1 or 2;
R^{c} is C₁₋₆alkyl, C₆₋₁₀aryl, a 5 membered heteroaromatic ring containing 1, 2, 3 or 4 heteroatoms independently selected from N, O and S, not more than one heteroatom of which is O or S; a 6 membered heteroaromatic ring containing 1, 2 or 3 nitrogen atoms; or a 7-10 membered unsaturated or partially saturated heterocyclic ring containing 1, 2, 3 or 4 heteroatoms independently selected from N, O and S; any of which rings being optionally substituted by one or more groups independently selected from hydroxy, cyano, halogen, C₁₋₆alkyl, C₂₋₁₀alkenyl and haloC₁₋₆alkyl;
each R^{d} is independently hydrogen or C₁₋₆alkyl;
each of R⁶ and R⁷ is independently hydrogen or C₁₋₆alkyl;
each of R⁸ and R⁹ is independently hydrogen, C₁₋₆alkyl, hydroxy, haloC₁₋₆alkyl, hydroxyC₁₋₆alkyl, amino, C₁₋₆alkylamino or di(C₁₋₆alkyl)amino;
or a pharmaceutically acceptable salt or tautomer thereof,
with the proviso that the following compound is excluded:

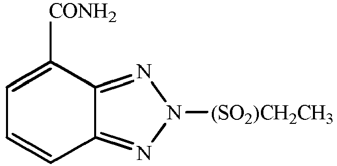

In an embodiment of the preceding embodiment R² is hydrogen or C₁₋₆alkyl.

In an embodiment of the preceding embodiment:
each R⁴ is independently hydrogen, hydroxy, cyano, halogen, C₁₋₆alkyl, C₂₋₁₀alkenyl, haloC₁₋₆alkyl, hydroxyC₁₋₆alkyl, C₁₋₆alkylcarbonyl, C₁₋₆alkoxy, haloC₁₋₆alkoxy, C₁₋₆alkoxycarbonyl, carboxy, nitro or a ring which is: C₆₋₁₀aryl; C₆₋₁₀aryloxy; C₆₋₁₀arylcarbonyl; C₃₋₁₀cycloalkyl; a 4 membered saturated heterocyclic ring containing one N atom; a 5, 6 or 7 membered saturated or partially saturated heterocyclic ring containing one, two or three N atoms and zero or one O atom; a 5 membered heteroaromatic ring containing 1, 2, 3 or 4 heteroatoms independently selected from N, O and S, not more than one heteroatom of which is O or S; a 6 membered heteroaromatic ring containing 1, 2 or 3 nitrogen atoms; or a 7-10 membered unsaturated or partially saturated heterocyclic ring containing 1, 2, 3 or 4 heteroatoms independently selected from N, O and S; any of which rings being optionally substituted by one or more groups independently selected from R⁵;
each R⁵ is independently hydroxy, oxo, cyano, halogen, C₁₋₆alkyl, C₂₋₁₀alkenyl, haloC₁₋₆alkyl, C₁₋₆alkylcarbonyl, C₁₋₆alkoxy, haloC₁₋₆alkoxy, C₁₋₆alkoxycarbonyl, carboxy, NR^{a}R^{b}, CONR^{a}R^{b} or a ring which is: C₆₋₁₀aryl; C₆₋₁₀arylC₁₋₆alkyl; a 4 membered saturated heterocyclic ring containing one N atom; a 5, 6 or 7 membered saturated or partially saturated heterocyclic ring containing one, two or three N atoms and zero or one O atom; a 5 membered heteroaromatic ring containing 1, 2, 3 or 4 heteroatoms independently selected from N, O and S, not more than one heteroatom of which is O or S; a 6 membered heteroaromatic ring containing 1, 2 or 3 nitrogen atoms; or a 7-10 membered unsaturated or partially saturated heterocyclic ring containing 1, 2, 3 or 4 heteroatoms independently selected from N, O and S; any of which rings being optionally substituted by one or more groups independently selected from hydroxy, cyano, halogen, C₁₋₆alkyl, C₁₋₆alkoxy, C₂₋₁₀alkenyl and haloC₁₋₆alkyl;
each of R⁸ and R⁹ is hydrogen;
or a pharmaceutically acceptable salt, stereoisomer or tautomer thereof.

In an embodiment of the preceding embodiment:
a is 0;
each of R⁶ and R⁷ is hydrogen; and
each R⁵ is independently hydroxy, oxo, cyano, halogen, C₁₋₆alkyl, C₂₋₁₀alkenyl, haloC₁₋₆alkyl, C₁₋₆alkylcarbonyl, C₁₋₆alkoxy, haloC₁₋₆alkoxy, C₁₋₆alkoxycarbonyl, carboxy, NR^{a}R^{b}, CONR^{a}R^{b} or a ring which is: C₆₋₁₀aryl; a 4 membered saturated heterocyclic ring containing one N atom; a 5, 6 or 7 membered saturated or partially saturated heterocyclic ring containing one, two or three N atoms and zero or one O atom; a 5 membered heteroaromatic ring containing 1, 2, 3 or 4 heteroatoms independently selected from N, O and S, not more than one heteroatom of which is O or S; a 6 membered heteroaromatic ring containing 1, 2 or 3 nitrogen atoms; or a 7-10 membered unsaturated or partially saturated heterocyclic ring containing 1, 2, 3 or 4 heteroatoms independently selected from N, O and S; any of which rings being optionally substituted by one or more groups independently selected from hydroxy, cyano, halogen, C₁₋₆alkyl, C₁₋₆alkoxy, C₂₋₁₀alkenyl and haloC₁₋₆alkyl.

In an embodiment a is 0. In another embodiment a is 1.

In an embodiment m is 0, 1 or 2.

In an embodiment m is 0 or 1.

In another embodiment m is 0.

In an embodiment R¹ is C₁₋₆alkyl, halogen or haloC₁₋₆alkyl.

In another embodiment R¹ is halogen, preferably chlorine or fluorine. A further R¹ group is hydroxy.

In an embodiment A is CH. In another embodiment A is N.

In another embodiment n is 0, 1 or 2.

In another embodiment n is 0 or 1. In another embodiment n is 0.

In an embodiment Y is a 6 membered unsaturated heterocycle containing 1, 2 or 3 nitrogen atoms, a 6-13 membered saturated, partially saturated or unsaturated hydrocarbon ring or a 8-13 membered unsaturated or partially saturated heterocyclic ring containing 1, 2, 3 or 4 heteroatoms independently selected from N, O and S.

In another embodiment Y is a 6-13 membered saturated, partially saturated or unsaturated hydrocarbon ring.

In another embodiment Y is a 4 membered saturated heterocycle containing one N atom or a 5, 6 or 7 membered saturated or partially saturated heterocycle containing 1, 2 or 3 heteroatoms independently selected from N, O and S.

Preferably, Y is phenyl. Further preferred Y groups are tetrahydroisoquinolinyl, pyridinyl and pyridazinyl. Further preferred Y groups are pyrrolidinyl, piperidinyl, tetrahydronaphthyridinyl and a direct bond.

Specific Y groups include phenyl, 1,2,3,4-tetrahydroisoquinolin-7-yl, 1,2,3,4-tetrahydroisoquinolin-6-yl, 1,2,3,4-tetrahydroisoquinolin-5-yl, pyridin-2-yl and pyridazin-3-yl. Further specific Y groups include pyrrolidin-3-yl, piperidin-4-yl, 5,6,7,8-tetrahydro-1,7-naphthyridin-3-yl, piperidin-3-yl and a direct bond.

In an embodiment Y is not a direct bond.

In an embodiment p is 0, 1 or 2. In another embodiment p is 0 or 1.

In an embodiment q is 0. In another embodiment q is 1.

In an embodiment t is 0 or 1.

In an embodiment R² is hydrogen or C₁₋₄alkyl, preferably hydrogen or methyl.

Particular R² groups include hydrogen, methyl, ethyl, propyl and cyclobutyl.

In another embodiment R² is hydrogen.

In an embodiment X is C. In another embodiment X is SO.

In an embodiment v is 0 or 1.

In an embodiment w is 0. In another embodiment w is 1.

In an embodiment x is 0, 1, or 2.

In another embodiment x is 0 or 1.

In an embodiment y is 0 or 1.

In an embodiment R³ is hydrogen or methyl.

In an embodiment z is 1 or 2.

In another embodiment z is 1.

In an embodiment each of p, q, t, v, w, x, a and y is zero.

In an embodiment R⁴ is hydrogen, hydroxy, halogen, C₁₋₄alkyl, haloC₁₋₄alkyl, C₁₋₄alkoxy or a ring selected from cyclopropyl, cyclobutyl, cyclopentyl, cyclohexyl, azetidinyl, pyrrolidinyl, piperidinyl, piperazinyl, diazepanyl, oxazolyl, pyridinyl, quinoxalinyl, phenyl, azoniaspiro[5.5]undecanyl, quinolinyl, isoquinolinyl, azepanyl, tetrahydroisoquinolinyl, octahydroindolizinyl, morpholinyl, 1'2'-dihydrospirocyclohexane-1,3 -indolyl, octahydroisoindolyl, azoniabicyclo[3.1.0]hexanyl, diazoniaspiro[4.4]nonanyl, hexahydropyrrolo[3,4-b]pyrrolyl, oxaazoniabicyclo[2.2.1]heptanyl, diazoniaspriro[5.5]undecanyl, diazoniaspiro[3.3]heptanyl, diazoniaspiro[3.5]nonanyl, diazoniaspiro[4.5]decanyl, octahydropyrrolo[3,4-c]pyrrolyl, octahydropyrrolo[3,4-b]pyrrolyl, octahydrocyclopenta[c]pyrrolyl, dihydroindolyl, benzothiazolyl, azoniaspiro[4.5]decanyl, diazoniabicyclo[2.2.2]octanyl, diazoniabicyclo[2.2.1]heptanyl, diazoniabicyclo[3.2.1]octanyl, diazoniabicyclo[2.2.1]heptanyl, azoniabicyclo[3.1.0]hexanyl, tetrahydrothiophenyl, tetrahydronaphthyridinyl, oxaazoniaspiro[4.5]decanyl and oxazepanyl, the ring being optionally substituted by one or more independently selected (CH₂)_{b}R⁵ groups. Further R⁴ groups are cyano or a ring selected from tetrazolyl, cyclobutyl, dihydroimidazolyl and pyrazolyl, the ring being optionally substituted by one or more independently selected (CH₂)_{b}R⁵ groups.

In an embodiment b is 0 or 1. In another embodiment b is 0.

In an embodiment R⁴ is a 5 or 6 membered saturated or partially saturated heterocyclic ring containing one, two or three atoms independently selected from N, O and S.

In an embodiment R⁴ is hydrogen, halogen, C₁₋₄alkyl, haloC₁₋₄alkyl, C₃₋₆cycloalkyl or a ring selected from azetidinyl, pyrrolidinyl, piperidinyl, piperazinyl, diazepanyl, oxazolyl, pyridinyl and quinoxalinyl, the ring being optionally substituted by one or more groups independently selected from R⁵.

In another embodiment R⁴ is hydrogen, halogen, C₁₋₆alkyl, a 4 membered saturated heterocyclic ring containing one N atom; or a 5, 6 or 7 membered saturated or partially saturated heterocyclic ring containing one, two or three N atoms and zero or one O atom; any of which rings being optionally substituted by one or more groups independently selected from R⁵.

In another embodiment R⁴ is hydrogen, halogen, C₁₋₆alkyl, or a 5 or 6 membered saturated or partially saturated heterocyclic ring containing one, two or three N atoms and zero or one O atom; any of which rings being optionally substituted by one or more groups independently selected from R⁵.

In an embodiment, when R⁴ is a ring it is optionally substituted by one, two or three R⁵ groups. More particularly, when R⁴ is a ring it is unsubstituted or monosubstituted.

In an embodiment, when R⁵ is a ring it is optionally substituted by one, two or three independently selected groups. More particularly, when R⁵ is a ring it is unsubstituted or monosubstituted.

In an embodiment R⁵ is C₁₋₆alkyl, NR^{a}R^{b}, oxo, S(O)ᵣR^{c}, C₁₋₆alkoxycarbonyl, halogen, hydroxy, C₁₋₆alkylcarbonyl, hydroxyC₁₋₆alkyl, CONR^{a}R^{b}, haloC₁₋₆alkyl or an optionally substituted ring selected from C₆₋₁₀aryl, C₆₋₁₀arylC₁₋₆alkyl, pyrrolidinyl, piperazinyl, pyrimidinyl, pyridinyl, piperidinyl, thiomorpholinyl, imidazolyl and benzimidazolyl.

Preferred optional substituents on a R⁵ ring are C₁₋₆alkyl, halogen, amino, C₁₋₆alkylamino and di(C₁₋₆alkyl)amino.

Particular optional substituents on a R⁵ ring are dimethylamino, methyl and chlorine.

In an embodiment R⁵ is C₁₋₆alkyl, C₆₋₁₀aryl, C₆₋₁₀arylC₁₋₆alkyl or pyrrolidinyl.

Particular R⁵ groups include methyl, phenyl, benzyl, pyrrolidinyl, amino, piperazinyl, pyrimidinyl, oxo, pyridinyl, methylsulfonyl, methoxycarbonyl, piperidinyl, fluorine, (dimethylamino)phenyl, hydroxy, propylcarbonyl, methylpyridinyl, hydroxymethyl, dimethylamino, thiomorpholinyl, (methylsulfonyl)amino, imidazolyl, (hydroxy)(methyl)ethyl, ethyl, chlorobenzyl, (methylamino)carbonyl, methylbenzimidazolyl and fluoromethyl.

Specific R⁵ groups are methyl, phenyl, benzyl and pyrrolidine-1-yl. Further specific R⁵ groups are amino, piperazin-1-yl, pyrimidin-2-yl, oxo, pyridin-2-yl, methylsulfonyl, methoxycarbonyl, piperidin-4-yl, pyridin-4-yl, pyridin-3-yl, fluorine, 4-(dimethylamino)phenyl, hydroxy, isopropylcarbonyl, 3-methylpyridin-2-yl, hydroxymethyl, dimethylamino, thiomorpholin-4-yl, (methylsulfonyl)amino, IH-imidazol-1-yl, 1-(hydroxy)-1-(methyl)ethyl, ethyl, 4-chlorobenzyl, 3-chlorobenzyl, (methylamino)carbonyl, 5-methyl-1H-benzimidazol-2-yl and fluoromethyl.

In another embodiment R⁵ is C₁₋₆alkyl, preferably methyl.

Thus, particular R⁴ groups include hydrogen, chlorine, methyl, methylpiperazine, morpholinyl, pyrrolidinyl and piperidinyl. Further particular R⁴ groups are pyridinyl, cyclohexyl, pyrrolidinylpiperidinyl, propyl, fluoroethyl, difluoroethyl, cyclopropyl, diazepanyl, azetidinyl, methylpyrrolidinyl, methylpiperidinyl, benzylpiperidinyl, phenylpiperazinyl, quinoxalinyl, trifluoromethyl, butyl, oxazolyl and fluorine. Further particular R⁴ groups include phenyl, methoxy, hydroxy, phenylpyrrolidinyl, aminocyclopentyl, methylazetidinyl, azoniaspiro[5.5]undecanyl, phenylpiperidinyl, (piperazinyl)pyridinyl, quinolinyl, isoquinolinyl, methylazepanyl, methyltetrahydroisoquinolinyl, (pyrimidinyl)piperidinyl, (amino)(oxo)octahydroindolizinyl, (pyridinyl)piperidinyl, methylmorpholinyl, [(methylsulfonyl)-1,2-dihydrospiro]cyclohexane-1,3-indolyl, octahydroisoindolyl, benzylmorpholinyl, (methoxycarbonyl)pyrrolidinyl, (piperidinyl)pyrrolidinyl, (pyridinyl)pyrrolidinyl, difluoropyrrolidinyl, (amino)azoniabicyclo[3.1.0]hexanyl, (methyl)diazoniaspiro[4.4]nonanyl, [(dimethylamino)phenyl]pyrrolidinyl, (methyl)hexahydropyrrolo[3,4-b]pyrrolyl, oxaazoniabicyclo[2.2.1]heptanyl, hydroxypropanyl, difluorocyclobutyl, fluorodiazepanyl, (pyrimidinyl)diazepanyl, benzylpyrrolidinyl, benzyldiazoniaspiro[4.4]nonanyl, benzyldiazoniaspiro[5.5]undecanyl, diazoniaspiro[3.3]heptanyl, diazoniaspiro[3.5]nonanyl, diazoniaspiro[5.5]undecanyl, diazoniaspiro[4.5]decanyl, octahydropyrrolo[3,4-c]pyrrolyl, octahydropyrrolo[3,4-b]pyrrolyl, octahydrocyclopenta[c]pyrrolyl, dihydroindolyl, benzothiazolyl, azoniaspiro[4.5]decanyl, fluoropiperidinyl, (benzyl)(methyl)piperidinyl, (isopropylcarbonyl)piperidinyl,(methylpyridinyl)piperidinyl, diazoniabicyclo[2.2.2]octanyl, (methyl)diazoniabicyclo[2.2.1]heptanyl, (pyridinylmethyl)piperazinyl, (benzyl)azaazoniabicyclo[2.2.2]octanyl, (benzyl)azaazoniabicyclo[3.2.1]octanyl, (benzyl)azaazoniabicyclo[2.2.1]heptanyl, azoniabicyclo[3.1.0]hexanyl, dioxohydroxytetrahydrothiophenyl, (hydroxy)(methyl)piperidinyl, (hydroxymethyl)cyclopentyl, tetrahydronaphthyridinyl, [(dimethylamino)methyl]piperidinyl, (thiomorpholinyl)piperidinyl, [(methylsulfonyl)amino]piperidinyl, (imidazolylmethyl)piperidinyl, oxaazoniaspiro[4.5]decanyl, [(hydroxy)(methyl)ethyl]piperidinyl, ethylpiperidinyl, bromine, (chlorobenzyl)azaazoniabicyclo[2.2.1]heptanyl, [(methylamino)carbonyl]piperazinyl, (hydroxymethyl)oxazepanyl, (hydroxymethyl)cyclobutyl, (hydroxymethyl)cyclohexyl, (methylbenzimidazolyl)piperidinyl, (hydroxy)(pyridinyl)piperidinyl, difluoropyrrolidinyl, (fluoromethyl)pyrrolidinyl and oxopyrrolidinyl. Further particular R⁴ groups include cyano, tetrazolyl, cyclobutyl, dihydroimidazolyl, pyrazolyl and piperazinyl.

Specific R⁴ groups include hydrogen, chlorine, methyl, 4-methylpiperazin-1-yl, morpholin-4-yl, pyrrolidin-1-yl and piperidin-1-yl. Further specific R⁴ groups are pyridin-4-yl, cyclohexyl, piperidin-4-yl, 4-pyrrolidin-1-ylpiperidin-1-yl, *iso*-propyl, 2-fluoroethyl, 2,2-difluoroethyl, cyclopropyl, 1,4-diazepan-1-yl, azetidin-3-yl, 1-methylpyrrolidin-2-yl, 1-methylpiperidin-3-yl, 1-methylpiperidin-4-yl, 1-benzylpiperidin-4-yl, 4-phenylpiperazin-1-yl, quinoxalin-6-yl, trifluoromethyl, pyridin-2-yl, *tert-*butyl*,* 1,3-oxazol-2-yl and fluorine. Further specific R⁴ groups are pyridin-3-yl, azetidin-2-yl, phenyl, methoxy, hydroxy, (2S)-pyrrolidin-2-yl, pyrrolidin-3-yl, (2R)-pyrrolidin-2-yl, (3R)-1-methylpyrrolidin-3-yl, 1-methylpiperidin-2-yl, (3S)-1-methylpyrrolidin-3-yl, (3R)-1-methylpiperidin-3-yl, (3S)-1-methylpiperidin-3-yl, 4-phenylpyrrolidin-2-yl, (1S,3R)-3-aminocyclopentyl, (1R,3R)-3-aminocyclopentyl, (1R,3S)-3-aminocyclopentyl, 2-methylazetidin-2-yl, 3-azoniaspiro[5.5]undecan-9-yl, 4-phenylpiperidin-4-yl, 2-(piperazin-1-yl)pyridin-3-yl, quinolin-4-yl, isoquinolin-4-yl, 1-methylazepan-2-yl, 2-methyl-1,2,3,4-tetrahydroisoquinolin-3-yl, 1-(pyrimidin-2-yl)piperidin-4-yl, 2-amino-3-oxooctahydroindolizin-5-yl, 1-(pyridin-2-yl)piperidin-3-yl, 4-methylmorpholin-2-yl, (1R,4R)-4-[1'-(methylsulfonyl)-1',2'-dihydrospiro]cyclohexane-1,3-indol-4-yl, octahydro-1H-isoindol-1-yl, 4-benzylmorpholin-2-yl, (3S,4R)-4-(methoxycarbonyl)pyrrolidin-3-yl, (2S)-1-(piperidin-4-yl)pyrrolidin-2-yl, (1S,3S)-3-aminocyclopentyl, 1-methylpyrrolidin-3-yl, 3-(pyridin-4-yl)pyrrolidin-1-yl, 3-(pyridin-2-yl)pyrrolidin-1-yl, 3-(pyridin-3-yl)pyrrolidin-1-yl, (3S,4S)-3,4-difluoropyrrolidin-1-yl, 6-amino-3-azoniabicyclo[3.1.0]hexan-3-yl, 7-methyl-2,7-diazoniaspiro[4,4]nonan-2-yl, 3-[4-(dimethylamino)phenyl]pyrrolidin-1-yl, 1-methyl-1,2,4,5,6,6a-hexahydropyrrolo[3,4-b]pyrrol-5-yl, (1R,4S)-2-oxa-5-azoniabicyclo[2.2.1]heptan-5-yl, 2-hydroxypropan-2-yl, 3,3-difluorocyclobutyl, 6-fluoro-1,4-diazepan-1-yl, 4-(pyrimidin-2-yl)-1,4-diazepan-1-yl, 1-benzylpyrrolidin-3-yl, 7-benzyl-2,7-diazoniaspiro[4.4]nonan-2-yl, 8-benzyl-2,8-diazoniaspiro[5.5]undecan-2-yl, 2,6-diazoniaspiro[3.3]heptan-2-yl, 2,7-diazoniaspiro[3.5]nonan-7-yl, 2,6-diazoniaspiro[3.5]nonan-2-yl, 2,8-diazoniaspiro[5.5]undecan-2-yl, 2,8-diazoniaspiro[4.5]decan-2-yl, 2,7-diazoniaspiro[4.5]decan-2-yl, 2,8-diazoniaspiro[4.5]decan-8-yl, 3,9-diazoniaspiro[5.5]undecan-3-yl, octahydropyrrolo[3,4-c]pyrrol-2-yl, octahydropyrrolo[3,4-b]pyrrol-5-yl, octahydrocyclopenta[c]pyrrol-4-yl, 2,3-dihydro-1H-indol-1-yl, 1,3-benzothiazol-5-yl, 8-azoniaspiro[4.5]decan-1-yl, (3R,4R)-3-fluoropiperidin-4-yl, (3R,4R)-3-benzyl-1-methylpiperidin-4-yl, 1-(isopropylcarbonyl)piperidin-4-yl, 1-(3-methylpyridin-2-y)piperidin-4-yl, 1-benzylpiperidin-3-yl, 5-aza-2-azoniabicyclo[2.2.2]octan-5-yl, (1 S,4S)-5-methyl-2,5-diazoniabicyclo[2.2.1 ]heptan-2-yl, 4-(pyridin-2-ylmethyl)piperazin-1-yl, 2-benzyl-5-aza-2-azoniabicyclo[2.2.2]octan-5-yl, 3-benzyl-8-aza-3-azoniabicyclo[3.2.1]octan-8-yl, (1S,4S)-2-benzyl-5-aza-2-azoniabicyclo[2.2.1]heptan-5-yl, 3-azoniabicyclo[3.1.0]hexan-6-yl, (3S,4S)-1,1-dioxo-4-hydroxytetrahydrothiophen-3-yl, 1-methyl-4-hydroxypiperidin-4-yl, 1-(hydroxymethyl)cyclopentyl, 1,2,3,4-tetrahydro-2,7-naphthyridin-2-yl, 3-[(dimethylamino)methyl]piperidin-1-yl, 4-(thiomorpholin-4-yl)piperidin-1-yl, 4-[(methylsulfonyl)amino]piperidin-1-yl, 4-[(1H-imidazol-1-yl)methyl]piperidin-1-yl, 1-oxa-7-azoniaspiro[4.5]decan-7-yl, 4-[1-(hydroxy)-1-(methyl)ethyl]piperidin-1-yl, 1-benzylpiperidin-2-yl, 1-ethylpiperidin-2-yl, 1-ethylpiperidin-3-yl, pyrrolidin-2-yl, bromine, (R)-3-[(dimethyaminol)methyl]piperidin-1-yl, (S)-3-[(dimethylamino)methyl]piperidin-1-yl, (1S,4S)-2-(4-chlorobenzyl)-5-aza-2-azoniabicyclo[2.2.1]heptan-5-yl, (1S,4S)-2-(3-chlorobenzyl)-5-aza-2-azoniabicyclo[2.2.1]heptan-5-yl, 4-[(methylamino)carbonyl]piperazin-1-yl, 6-(hydroxymethyl)-1,4-oxazepan-4-yl, 1-(hydroxymethyl)cyclobutyl, 1-(hydroxymethyl)cyclohexyl, 4-(5-methyl-1H-benzimidazol-2-yl)piperidin-1-yl, 4-hydroxy-4-(pyridin-2-yl)piperidin-1-yl, 3,3-difluoropyrrolidin-1-yl, (2R)-2-(fluoromethyl)pyrrolidin-1-yl, 2-oxopyrrolidin-3-yl, 1-methylazetidin-3-yl and (3R)-1-methylpiperidin-3-yl. Further specific R⁴ groups include cyano, 1H-tetrazol-5-yl, cyclobutyl, 4,5-dihydro-1H-imidazol-2-yl, piperidin-2-yl, 1H-pyrazol-1-yl and piperazin-2-yl.

R⁴ may be piperidin-3-yl, provided that:
when A is CH; m is 0 or 1; R¹ is fluorine substituted at the 5 position of the indazole ring; and n is 0; then Y-[(CR⁶R⁷)ₚ(CO)_{q}(NR²)ₜ(X=O)ᵥ(O)_{w}(CR⁸R⁹)ₓ(CO)ₐ(NR³)_{y}R⁴]_{z} is not 4-(piperidin-3-yl)phenyl or (fluoro)-4-(piperidin-3-yl)phenyl.

In an embodiment, when y is 1 then R⁴ is piperidin-3-yl.

In an embodiment R⁴ is piperidinyl.

In an embodiment R⁶ is hydrogen or C₁₋₄alkyl and R⁷ is hydrogen.

Particular R⁶ groups are hydrogen and methyl.

A particular R⁷ group is hydrogen. A further particular R⁷ group is methyl.

In an embodiment each of R⁶ and R⁷ is hydrogen.

In an embodiment R⁸ is hydrogen, C₁₋₄alkyl, hydroxy, haloC₁₋₄alkyl, hydroxyC₁₋₄alkyl, amino, C₁₋₆alkylamino or di(C₁₋₆alkyl)amino and R⁹ is hydrogen.

Particular R⁸ groups are hydrogen, dimethylamino, hydroxymethyl, chloromethyl and hydroxy.

A particular R⁹ group is hydrogen.

In an embodiment each of R⁸ and R⁹ is hydrogen.

In an embodiment when R^{a} and R^{b} together form a ring or R^{c} is a ring, the ring is optionally substituted by one, two or three optionally selected groups.

In an embodiment each of R^{a} and R^{b} is independently selected from hydrogen, C₁₋₆alkyl and SO₂R^{c}.

Particular R^{a} groups are hydrogen, methyl and methylsulfonyl.

Particular R^{b} groups are hydrogen and methyl.

A particular R^{c} group is C₁₋₆alkyl, especially methyl.

The present invention also provides compounds of formula II: 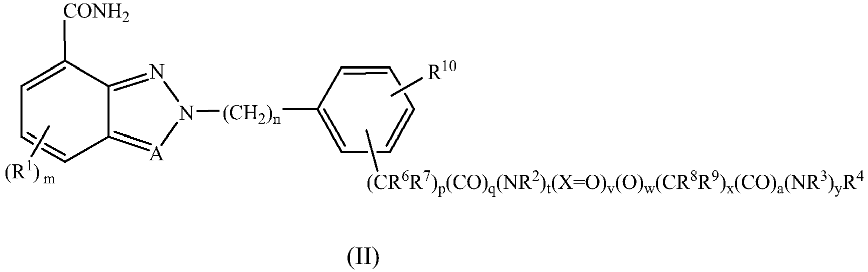 wherein:
a, m, n, p, q, t, v, w, x, y, A, R¹, R², R³, R⁴, R⁶, R⁷, R⁸, R⁹ and X are as defined above;
R¹⁰ is hydrogen, halogen, C₁₋₆alkyl or haloC₁₋₆alkyl;
or a pharmaceutically acceptable salt, stereoisomer or tautomer thereof.

In an embodiment of formula II, each of R⁸ and R⁹ is hydrogen.

The present invention also provides compounds of formula III: 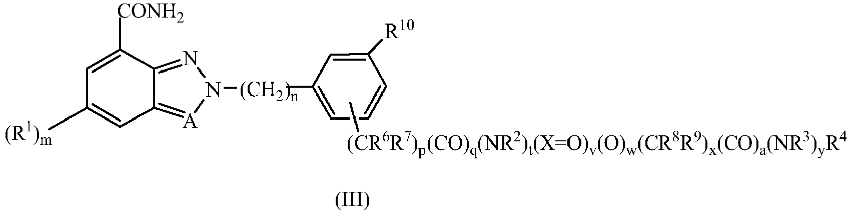 wherein:
a, m, n, p, q, t, v, w, x, y, A, R¹, R², R³, R⁴, R⁶, R⁷, R⁸, R⁹ and X are as defined above;
R¹⁰ is hydrogen, halogen, C₁₋₆alkyl or haloC₁₋₆alkyl;
or a pharmaceutically acceptable salt, stereoisomer or tautomer thereof.

In another embodiment of any one of formulae II or III, n is 0.

The present invention also provides compounds of formula IV: 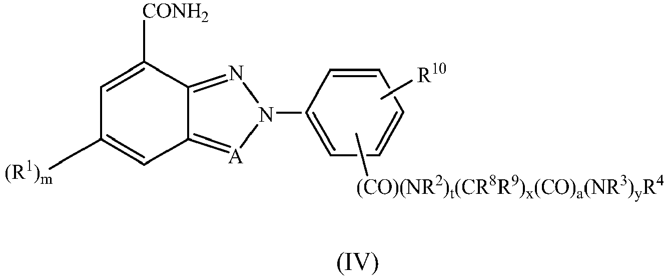 wherein:
m, A, R¹, R², R³, R⁴, R⁸and R⁹ are as defined above;
a is 0 or 1;
t is 0 or 1;
x is 0, 1 or 2;
y is 0 or 1;
R¹⁰ is hydrogen, halogen, C₁₋₆alkyl or haloC₁₋₆alkyl;
or a pharmaceutically acceptable salt, stereoisomer or tautomer thereof

The present invention also provides compounds of formula V: 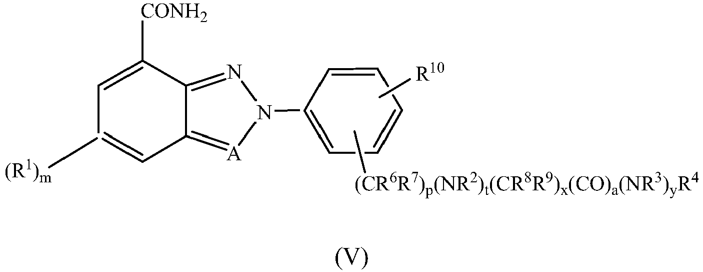 wherein:
a, m, t, y, A, R¹, R², R³, R⁴, R⁶, R⁷, R⁸and R⁹ are as defined above;
p is 1 or 2;
x is 0, 1 or 2;
R¹⁰ is hydrogen, halogen, C₁₋₆alkyl or haloC₁₋₆alkyl;
or a pharmaceutically acceptable salt, stereoisomer or tautomer thereof

In an embodiment p is 1.

The present invention also provides compounds of formula VI: 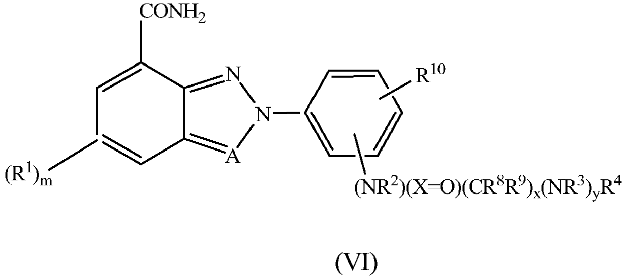 wherein:
m, y, A, R¹, R², R³, R⁴, R⁸, R⁹ and X are as defined above;
x is 0, 1 or 2;
R¹⁰ is hydrogen, halogen, C₁₋₆alkyl or haloC₁₋₆alkyl;
or a pharmaceutically acceptable salt, stereoisomer or tautomer thereof

The present invention also provides compounds of formula VII: 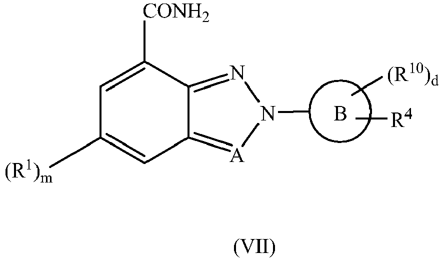 wherein:
m, A, R¹ and R⁴ are as defined above;
d is 0, 1 or 2;
B is a 6 membered unsaturated heterocycle containing 1, 2 or 3 nitrogen atoms, 6-13 membered saturated, partially saturated or unsaturated hydrocarbon ring or a 8-13 membered unsaturated or partially saturated heterocyclic ring containing 1, 2, 3 or 4 heteroatoms independently selected from N, O and S;
R¹⁰ is halogen or C₁₋₆alkyl;
or a pharmaceutically acceptable salt, stereoisomer or tautomer thereof.

In an embodiment of formula VII, R¹ may be substituted at any substitutable position of the fused benzene ring.

The preferred identities with reference to any one of formulae II, III, IV, V, VI and VII are as defined previously for formula I *mutatis mutandis.*

In an embodiment of any one of formulae II, III, IV, V, VI or VII, A is CH.

Particular R¹⁰ groups are methyl, chlorine and fluorine, especially chlorine and fluorine.

In an embodiment d is 0 or 1. In another embodiment d is 0.

Particular B groups are phenyl, tetrahydroisoquinolinyl, pyridinyl and pyridazinyl. A further particular B group is tetrahydronaphthyridinyl.

More particular B groups are phenyl and tetrahydroisoquinolinyl.

Specific B groups are phenyl, 1,2,3,4-tetrahydroisoquinolin-7-yl, 1,2,3,4-tetrahydroisoquinolin-6-yl, 1,2,3,4-tetrahydroisoquinolin-5-yl, pyridin-2-yl and pyridazin-3-yl. A further specific B group is 5,6,7,8-tetrahydro-1,7-naphthyridin-3-yl.

In an embodiment B is phenyl, 1,2,3,4-tetrahydroisoquinolin-7-yl, 1,2,3,4-tetrahydroisoquinolin-6-yl or 1,2,3,4-tetrahydroisoquinolin-5-yl.

In an embodiment the R⁴ ring is unsubstituted, monosubstituted or disubstituted. In another embodiment the ring is unsubstituted.

Particular R⁴ rings are azetidinyl, pyrrolidinyl, piperidinyl, piperazinyl, morpholinyl, tetrahydrofuran, thiomorpholinyl, diazepanyl, azepanyl and oxazepanyl. A further particular R⁴ ring is dihydroimidazolyl.

The present invention also provides compounds of formula VIII: 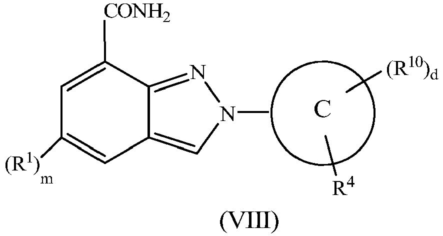
wherein m, R¹ and R⁴ are as defined above;
d is 0, 1 or 2;
C is a 4 membered saturated heterocycle containing one N atom or a 5, 6 or 7 membered saturated or partially saturated heterocycle containing 1, 2 or 3 heteroatoms independently selected from N, O and S;
R¹⁰ is halogen or C₁₋₆alkyl;
or a pharmaceutically acceptable salt, stereoisomer or tautomer thereof.

The preferred identities with reference to formula VIII are as defined previously for formulae I, II, III, IV, V, VI and VII *mutatis mutandis*.

In an embodiment C is a 5 or 6 membered saturated or partially saturated heterocycle containing 1, 2 or 3 heteroatoms independently selected from N, O and S.

Particular C rings are pyrrolidinyl, piperidinyl, piperazinyl, morpholinyl, tetrahydrofuran, thiomorpholinyl, diazepanyl, azepanyl and oxazepanyl.

More particularly, C is pyrrolidinyl or piperidinyl.

Specific C rings include pyrrolidin-3-yl, piperidin-4-yl and piperidin-3-yl.

The present invention also includes within its scope N-oxides of the compounds of formula I above. In general, such N-oxides may be formed on any available nitrogen atom. The N-oxides may be formed by conventional means, such as reacting the compound of formula I with oxone in the presence of wet alumina.

Also disclosed are prodrugs of the compounds of formula I above. In general, such prodrugs will be functional derivatives of the compounds of formula I which are readily convertible *in vivo* into the required compound of formula I. Conventional procedures for the selection and preparation of suitable prodrug derivatives are described, for example, in "Design of Prodrugs", ed. H. Bundgaard, Elsevier, 1985.

A prodrug may be a pharmacologically inactive derivative of a biologically active substance (the "parent drug" or "parent molecule") that requires transformation within the body in order to release the active drug, and that has improved delivery properties over the parent drug molecule. The transformation *in vivo* may be, for example, as the result of some metabolic process, such as chemical or enzymatic hydrolysis of a carboxylic, phosphoric or sulphate ester, or reduction or oxidation of a susceptible functionality.

The present invention includes within its scope solvates of the compounds of formula I and salts thereof, for example, hydrates.

The compounds of the present invention may have asymmetric centers, chiral axes, and chiral planes (as described in: E.L. Eliel and S.H. Wilen, Stereochemistry of Carbon Compounds, John Wiley & Sons, New York, 1994, pages 1119-1190), and occur as racemates, racemic mixtures, and as individual diastereomers, with all possible isomers and mixtures thereof, including optical isomers, all such stereoisomers being included in the present invention. In addition, the compounds disclosed herein may exist as tautomers and both tautomeric forms are intended to be encompassed by the scope of the invention, even though only one tautomeric structure is depicted.

The compounds may exist in different isomeric forms, all of which are encompassed by the present invention.

The compounds may exist in a number of different polymorphic forms.

When any variable (e.g. R¹ and R², etc.) occurs more than one time in any constituent, its definition on each occurrence is independent at every other occurrence. Also, combinations of substituents and variables are permissible only if such combinations result in stable compounds. Lines drawn into the ring systems from substituents represent that the indicated bond may be attached to any of the substitutable ring atoms.

It is understood that substituents and substitution patterns on the compounds of the instant invention can be selected by one of ordinary skill in the art to provide compounds that are chemically stable and that can be readily synthesized by techniques known in the art, as well as those methods set forth below, from readily available starting materials. If a substituent is itself substituted with more than one group, it is understood that these multiple groups may be on the same carbon or on different carbons, so long as a stable structure results. The phrase "optionally substituted" should be taken to be equivalent to the phrase "unsubstituted or substituted with one or more substituents" and in such cases the preferred embodiment will have from zero to three substituents. More particularly, there are zero to two substituents. A substituent on a saturated, partially saturated or unsaturated heterocycle can be attached at any substitutable position.

As used herein, "alkyl" is intended to include both branched, straight-chain and cyclic saturated aliphatic hydrocarbon groups having the specified number of carbon atoms. For example,"C₁₋₆alkyl" is defined to include groups having 1, 2, 3, 4, 5 or 6 carbons in a linear, branched or cyclic arrangement. For example,"C₁₋₆alkyl" specifically includes methyl, ethyl, n-propyl, i-propyl, n-butyl, t-butyl, i-butyl, pentyl, hexyl, cyclopropyl, cyclobutyl, cyclopentyl and cyclohexyl and so on. Preferred alkyl groups are methyl and ethyl. The term "cycloalkyl" means a monocyclic, bicyclic or polycyclic saturated aliphatic hydrocarbon group having the specified number of carbon atoms. For example, "C₃₋₇cycloalkyl" includes cyclopropyl, methyl-cyclopropyl, 2,2-dimethyl-cyclobutyl, 2-ethyl-cyclopentyl, cyclohexyl, and so on. In an embodiment of the invention the term "cycloalkyl" includes the groups described immediately above and further includes monocyclic unsaturated aliphatic hydrocarbon groups. For example, "cycloalkyl" as defined in this embodiment includes cyclopropyl, methyl-cyclopropyl, 2,2-dimethyl-cyclobutyl, 2-ethyl-cyclopentyl, cyclohexyl, cyclopentenyl, cyclobutenyl, 7,7-dimethylbicyclo[2.2.1]heptyl and so on. Preferred cycloalkyl groups are cyclopropyl, cyclobutyl, cyclopentyl and cyclohexyl.

As used herein, the term "C₂₋₆alkenyl" refers to a non-aromatic hydrocarbon radical, straight or branched, containing from 2 to 6 carbon atoms and at least one carbon to carbon double bond. Preferably one carbon to carbon double bond is present, and up to four non-aromatic carbon-carbon double bonds may be present. Alkenyl groups include ethenyl, propenyl, butenyl and 2-methylbutenyl. Preferred alkenyl groups include ethenyl and propenyl.

As used herein, the term "C₂₋₆alkynyl" refers to a hydrocarbon radical straight or branched, containing from 2 to 6 carbon atoms and at least one carbon to carbon triple bond. Up to three carbon-carbon triple bonds may be present. Alkynyl groups include ethynyl, propynyl, butynyl, 3-methylbutynyl and so on. Preferred alkynyl groups include ethynyl and propynyl

"Alkoxy" represents an alkyl group of indicated number of carbon atoms attached through an oxygen bridge. "Alkoxy" therefore encompasses the definitions of alkyl above. Examples of suitable alkoxy groups include methoxy, ethoxy, n-propoxy, i-propoxy, n-butoxy, s-butoxy and t-butoxy. The preferred alkoxy groups are methoxy and ethoxy. The term 'C₆₋₁₀aryloxy' can be construed analogously, and an example of this group is phenoxy.

The terms "haloC₁₋₆alkyl" and "haloC₁₋₆alkoxy" mean a C₁₋₆alkyl or C₁₋₆alkoxy group in which one or more (in particular, 1 to 3) hydrogen atoms have been replaced by halogen atoms, especially fluorine or chlorine atoms. Preferred are fluoroC₁₋₆alkyl and fluoroC₁₋₆alkoxy groups, in particular fluoroC₁₋₃alkyl and fluoroC₁₋₃alkoxy groups, for example, CF₃, CHF₂, CH₂F, CH₂CH₂F, CH₂CHF₂, CH₂CF₃, OCF₃, OCHF₂, OCH₂F, OCH₂CH₂F, OCH₂CHF₂ or OCH₂CF₃, and most especially CF₃, OCF₃ and OCHF₂.

As used herein, the term "hydroxyC₁₋₆alkyl" means a C₁₋₆alkyl group in which one or more (in particular, 1 to 3) hydrogen atoms have been replaced by hydroxy groups. Preferred are CH₂OH, CH₂CHOH and CHOHCH₃.

The term "C₁₋₆alkylcarbonyl" or "C₁₋₆alkoxycarbonyl" denotes a C₁₋₆alkyl or C₁₋₆alkoxy radical, respectively, attached via a carbonyl (C=O) radical. Suitable examples of C₁₋₆alkylcarbonyl groups include methylcarbonyl, ethylcarbonyl, propylcarbonyl, isopropylcarbonyl and *tert*-butylcarbonyl. Examples of C₁₋₆alkoxycarbonyl include methoxycarbonyl, ethoxycarbonyl, propoxycarbonyl, isopropoxycarbonyl and *tert*-butoxycarbonyl. The term 'C₆₋₁₀arylcarbonyl' can be construed analogously, and an example of this group is benzoyl.

The rings present in the compounds of this invention may be monocyclic or multicyclic, particularly bicyclic. The multicyclic rings may be fused or spiro linked.

As used herein, "C₆₋₁₀aryl" is intended to mean any stable monocyclic or bicyclic carbon ring of 6 to 10 atoms, wherein at least one ring is aromatic. Examples of such aryl elements include phenyl, naphthyl, tetrahydronaphthyl, indanyl and tetrahydrobenzo[7]annulene. The preferred aryl group is phenyl or naphthyl, especially phenyl.

Examples of particular heterocycles of this invention are benzimidazolyl, benzofurandionyl, benzofuranyl, benzofurazanyl, benzopyrazolyl, benzotriazolyl, benzothienyl, benzoxazolyl, benzoxazolonyl, benzothiazolyl, benzothiadiazolyl, benzodioxolyl, benzoxadiazolyl, benzoisoxazolyl, benzoisothiazolyl, chromenyl, chromanyl, isochromanyl, carbazolyl, carbolinyl, cinnolinyl, epoxidyl, furyl, furazanyl, imidazolyl, indolinyl, indolyl, indolizinyl, indolinyl, isoindolinyl, indazolyl, isobenzofuranyl, isoindolyl, isoquinolyl, isothiazolyl, isoxazolyl, naphthpyridinyl, oxadiazolyl, oxazolyl, oxazolinyl, isoxazolinyl, oxetanyl, purinyl, pyranyl, pyrazinyl, pyrazolyl, pyridazinyl, pyridopyridinyl, pyridazinyl, pyridinyl, pyrimidinyl, triazinyl, tetrazinyl, pyrrolyl, quinazolinyl, quinolinyl, quinoxalinyl, quinolizinyl, tetrahydropyranyl, tetrahydrothiopyranyl, tetrahydroisoquinolinyl, tetrazolyl, tetrazolopyridyl, thiadiazolyl, thiazolyl, thienyl, triazolyl, azetidinyl, 1,4-dioxanyl, hexahydroazepinyl, piperazinyl, piperidyl, pyridin-2-onyl, pyrrolidinyl, imidazolinyl, imidazolidinyl, pyrazolinyl, pyrrolinyl, morpholinyl, thiomorpholinyl, dihydrobenzoimidazolyl, dihydrobenzofuranyl, dihydrobenzothiophenyl, dihydrobenzoxazolyl, dihydrofuranyl, dihydroimidazolyl, dihydroindolyl, dihydroisooxazolyl, dihydroisothiazolyl, dihydrooxadiazolyl, dihydrooxazolyl, dihydropyrazinyl, dihydropyrazolyl, dihydropyridinyl, dihydropyrimidinyl, dihydropyrrolyl, dihydroquinolinyl, dihydroisoquinolinyl, dihydrotetrazolyl, dihydrothiadiazolyl, dihydrothiazolyl, dihydrothienyl, dihydrotriazolyl, dihydroazetidinyl, dihydroisochromenyl, dihydrochromenyl, dihydroimidazolonyl, dihydrotriazolonyl, dihydrobenzodioxinyl, dihydrothiazolopyrimidinyl, dihydroimidazopyrazinyl, methylenedioxybenzoyl, tetrahydrofuranyl, tetrahydrothienyl, tetrahydroquinolinyl, thiazolidinonyl, imidazolonyl, isoindolinonyl, octahydroquinolizinyl, octahydroisoindolyl, imidazopyridinyl, azabicycloheptanyl, chromenonyl, triazolopyrimidinyl, dihydrobenzoxazinyl, thiazolotriazolyl, azoniabicycloheptanyl, azoniabicyclooctanyl, phthalazinyl, naphthyridinyl, quinazolinyl, pteridinyl, dihydroquinazolinyl, dihydrophthalazinyl, benzisoxazolyl, tetrahydronaphthyridinyl, dibenzo[*b,d*]furanyl, dihydrobenzothiazolyl, imidazothiazolyl, tetrahydroindazolyl, tetrahydrobenzothienyl, hexahydronaphthyridinyl, tetrahydroimidazopyridinyl, tetrahydroimidazopyrazinyl, pyrrolopyridinyl and N-oxides thereof. Further examples include azoniaspiro[5.5]undecanyl, azepanyl, octahydroindolizinyl, 12-dihydrospirocyclohexane-1,3-indolyl, azoniabicyclo[3.1.0]hexanyl, diazoniaspiro[4.4]nonanyl, hexahydropyrrolo[3,4-b]pyrrolyl, oxaazoniabicyclo[2.2.1]heptanyl, diazoniaspriro[5.5]undecanyl, diazoniaspiro[3.3]heptanyl, diazoniaspiro[3.5]nonanyl, diazoniaspiro[4.5]decanyl, octahydropyrrolo[3,4-c]pyrrolyl, octahydropyrrolo[3,4-b]pyrrolyl, octahydrocyclopenta[c]pyrrolyl, dihydroindolyl, azoniaspiro[4.5]decanyl, diazoniabicyclo[2.2.2]octanyl, diazoniabicyclo[2.2.1]heptanyl, diazoniabicyclo[3.2.1]octanyl, diazoniabicyclo[2.2.1]heptanyl, azoniabicyclo[3.1.0]hexanyl, tetrahydrothiophenyl, oxaazoniaspiro[4.5]decanyl and oxazepanyl. Attachment of a heterocyclyl substituent can occur via a carbon atom or via a heteroatom.

A preferred 4 membered saturated heterocycle is azetidinyl.

Preferred 5 or 6 membered saturated or partially saturated hetereocycles are pyrrolidinyl, piperidinyl, piperazinyl, morpholinyl, tetrahydrofuran and thiomorpholinyl. A further preferred heterocycle is dihydroimidazolyl.

A preferred 7 membered saturated heterocycle is diazepanyl. Further preferred rings are azepanyl and oxazepanyl.

Preferred 5 membered heteroaromatic rings are thienyl, thiazolyl, pyrazolyl, isoxazolyl, isothiazolyl, imidazolyl, thiadiazolyl, oxazolyl, oxadiazolyl, triazolyl, tetrazolyl, furyl and pyrrolyl.

Preferred 6 membered heteraromatic rings are pyridinyl, pyrimidinyl, pyridazinyl and pyrazinyl.

Preferred 6-13 membered saturated, partially saturated or unsaturated hydrocarbon rings are cyclohexyl, cyclohexadienyl, cyclohepyl, cyclooctyl, phenyl, naphthyl, tetrahydronaphthalenyl, dihydroindenyl, fluorenyl, adamantly, tetrahydrobenzo[7]annulenyl, indanyl, tetrahydroindenyl and tetrahydrobenzo[7]annulene.

Preferred 7-13 membered partially saturated or unsaturated heterocyclic rings are tetrahydroquinolinyl, quinolinyl, indolyl, imidazopyridinyl, benzothiazolyl, quinoxalinyl, benzothiadiazolyl, benzoxazolyl, dihydrobenzodioxinyl, benzotriazolyl, benzodioxolyl, dihydroisoindolyl, dihydroindolyl, tetrahydroisoquinolinyl, isoquinolinyl, benzoisothiazolyl, dihydroimidazopyrazinyl, benzothienyl, benzoxadiazolyl, thiazolotriazolyl, dihydrothiazolopyrimidinyl, dihydrobenzoxazinyl, dihydrobenzofuranyl, benzimidazolyl, benzofuranyl, dihydrobenzoxazolyl, dihydroquinazolinyl, dihydrophthalazinyl, indazolyl, benzisoxazolyl, tetrahydronaphthyridinyl, triazolopyrimidinyl, dibenzo[*b,d*]furanyl, naphthyridinyl, dihydroquinolinyl, dihydroisochromenyl, dihydrochromenyl, dihydrobenzothiazolyl, imidazothiazolyl, tetrahydroindazolyl, tetrahydrobenzothienyl, hexahydronaphthyridinyl, tetrahydroimidazopyridinyl, tetrahydroimidazopyrazinyl and pyrrolopyridinyl. Further preferred rings are quinazolinyl and indolizinyl. Further preferred 7-15 menbered saturated, partially saturated or unsaturated heterocycles include azoniaspiro[5.5]undecanyl, azepanyl, octahydroindolizinyl, 1'2'-dihydrospirocyclohexane-1,3'-indolyl, octahydroisoindolyl, azoniabicyclo[3.1.0]hexanyl, diazoniaspiro[4.4]nonanyl, hexahydropyrrolo[3,4-b]pyrrolyl, oxaazoniabicyclo[2.2.1]heptanyl, diazoniaspriro[5.5]undecanyl, diazoniaspiro[3.3]heptanyl, diazoniaspiro[3.5]nonanyl, diazoniaspiro[4.5]decanyl, octahydropyrrolo[3,4-c]pyrrolyl, octahydropyrrolo[3,4-b]pyrrolyl, octahydrocyclopenta[c]pyrrolyl, dihydroindolyl, azoniaspiro[4.5]decanyl, diazoniabicyclo[2.2.2]octanyl, diazoniabicyclo[2.2.1]heptanyl, diazoniabicyclo[3.2.1]octanyl, diazoniabicyclo[2.2.1]heptanyl, azoniabicyclo[3.1.0]hexanyl, tetrahydrothiophenyl, oxaazoniaspiro[4.5]decanyl and oxazepanyl.

As used herein, the term "halogen" refers to fluorine, chlorine, bromine and iodine, of which fluorine and chlorine are preferred.

Particular compounds within the scope of the present invention are:
2-phenyl-2*H-*indazole-7-carboxamide;
2-(3-chlorophenyl)-2*H-*indazole-7-carboxamide; and
2-{4-[(dimethylamino)methyl]phenyl}-2*H-*indazole-7-carboxamide;
2-{4-[(N,N-dimethylglycyl)amino]phenyl}-2H-indazole-7-carboxamide;
2-benzyl-2*H-*indazole-7-carboxamide;
2-(4-chlorophenyl)-2H-indazole-7-carboxamide;
2-(2-chlorophenyl)-2H-indazole-7-carboxamide;
2-{4-[(4-methylpiperazin-1-yl)methyl]phenyl}-2H-indazole-7-carboxamide;
2-[4-(morpholin-4-ylmethyl)phenyl]-2H-indazole-7-carboxamide;
2-{4-[(methylamino)methyl]phenyl}-2H-indazole-7-carboxamide;
2-[4-(pyrrolidin-1-ylmethyl)phenyl]-2*H*-indazole-7-carboxamide;
2-[4-(piperidin-1-ylmethyl)phenyl]-2*H*-indazole-7-carboxamide;
and pharmaceutically acceptable salts or tautomers thereof.

A particular salt of the present invention is:
{4-[7-(aminocarbonyl)-2H-indazol-2-yl]phenyl}-N,N-dimethylmethanaminium chloride;
or a tautomer thereof.

Further particular compounds within the scope of the present invention are:
4-[({4-[7-(aminocarbonyl)-2*H-*indazol-2-yl]benzoyl}amino)methyl]pyridinium trifluoroacetate;
2-{4-[1-(methylamino)ethyl]phenyl}-2*H-*indazole-7-carboxamide;
*N*-{4-[7-(aminocarbonyl)-2*H*-indazol-2-yl]benzyl}cyclohexanaminium trifluoroacetate;
{4-[7-(aminocarbonyl)-4-chloro-2*H-*indazol-2-yl]phenyl}-*N*-methylmethanaminium trifluoroacetate;
2-phenyl-2*H-*1,2,3-benzotriazole-4-carboxamide;
2-benzyl-2*H-*1,2,3-benzotriazole-4-carboxamide;
2-{3-[(methylamino)methyl]phenyl}-2H-indazole-7-carboxamide;
4-({3-[7-(aminocarbonyl)-2*H*-indazol-2-yl]benzoyl}amino)piperidinium trifluoroacetate;
{4-[7-(aminocarbonyl)-2H-indazol-2-yl]phenyl}-N-methylmethanaminium chloride;
2-{3-chloro-4-[(dimethylamino)methyl]phenyl}-2H-indazole-7-carboxamide;
1-[2-({4-[7-(aminocarbonyl)-2H-indazol-2-yl]phenyl}amino)-2-oxoethyl]-4-methylpiperazin-1-ium trifluoroacetate;
2-(4-{[(4-pyrrolidin-1-ylpiperidin-1-yl)acetyl]amino}phenyl)-2H*-*indazole-7-carboxamide;
2-{4-[(pyrrolidin-1-ylacetyl)amino]phenyl}-2H-indazole-7-carboxamide;
2-{4-[(piperidin-1-ylacetyl)amino]phenyl}-2H-indazole-7-carboxamide;
2-{4-[(morpholin-4-ylacetyl)amino]phenyl}-2H-indazole-7-carboxamide;
4-[2-({4-[7-(aminocarbonyl)-2H-indazol-2-yl]phenyl}amino)-2-oxoethyl]morpholin-4-ium chloride;
2-{4-[(ethylamino)methyl]phenyl}-2H-indazole-7-carboxamide;
2-{4-[(isopropylamino)methyl]phenyl}-2H-indazole-7-carboxamide;
N-{4-[7-(aminocarbonyl)-2H-indazol-2-yl]benzyl}propan-2-aminium chloride;
2-(4-{[(2-fluoroethyl)amino]methyl}phenyl)-2H-indazole-7-carboxamide;
2-(4-{[(2,2-difluoroethyl)amino]methyl}phenyl)-2H-indazole-7-carboxamide;
2-{4-[(cyclopropylamino)methyl]phenyl}-2H-indazole-7-carboxamide;
4-{4-[7-(aminocarbonyl)-2H-indazol-2-yl]benzoyl}-1,4-diazepan-1-ium trifluoroacetate;
2-({4-[7-(aminocarbonyl)-2H-indazol-2-yl]benzoyl}amino)-N,N-dimethylethanaminium trifluoroacetate;
4-[({4-[7-(aminocarbonyl)-2H-indazol-2-yl]benzoyl}amino)methyl]piperidinium trifluoroacetate;
N-{4-[7-(aminocarbonyl)-2H-indazol-2-yl]benzyl}-N,N',N'-trimethylethane-1,2-diaminium dichloride;
4-{4-[7-(aminocarbonyl)-2H-indazol-2-yl]benzoyl}-1-methylpiperazin-1-ium trifluoroacetate;
3-[({4-[7-(aminocarbonyl)-2H-indazol-2-yl]phenyl}amino)carbonyl]azetidinium trifluoroacetate;
(2S)-2-[({4-[7-(aminocarbonyl)-2H-indazol-2-yl]phenyl}amino)carbonyl]-1-methylpyrrolidinium trifluoroacetate;
3-[({4-[7-(aminocarbonyl)-2H-indazol-2-yl]phenyl}amino)carbonyl]-1-methylpiperidinium trifluoroacetate;
4-[({4-[7-(aminocarbonyl)-2H-indazol-2-yl]phenyl}amino)carbonyl]-1-methylpiperidinium trifluoroacetate;
4-({4-[7-(aminocarbonyl)-2H-indazol-2-yl]benzoyl}amino)-1-benzylpiperidinium trifluoroacetate;
2-{4-[(pyridin-4-ylamino)carbonyl]phenyl}-2H-indazole-7-carboxamide;
2-{4-[(4-phenylpiperazin-1-yl)carbonyl]phenyl}-2H-indazole-7-carboxamide;
2-(4-{[methyl(quinoxalin-6-ylmethyl)amino]carbonyl}phenyl)-2H-indazole-7-carboxamide;
2-(4-formylphenyl)-2H-indazole-7-carboxamide;
1-[2-({4-[7-(aminocarbonyl)-2H-indazol-2-yl]benzoyl}amino)ethyl]pyrrolidinium trifluoroacetate;
1-[2-({4-[7-(aminocarbonyl)-2H-indazol-2-yl]benzoyl}amino)ethyl]piperidinium trifluoroacetate;
4-[2-({4-[7-(aminocarbonyl)-2H-indazol-2-yl]benzoyl}amino)ethyl]morpholin-4-ium trifluoroacetate;
4-({4-[7-(aminocarbonyl)-2H-indazol-2-yl]benzoyl}amino)-1-methylpiperidinium trifluoroacetate;
2-[4-[(4-methylpiperazin-1-yl)methyl]-3-(trifluoromethyl)phenyl]-2H-indazole-7-carboxamide;
2-[4-[(methylamino)methyl]-3-(trifluoromethyl)phenyl]-2H-indazole-7-carboxamide;
1-{4-[7-(aminocarbonyl)-2*H-*indazol-2-yl]phenyl}-*N-*methylethanaminium chloride;
2-[4-(pyrrolidin-1-ylmethyl)-3-(trifluoromethyl)phenyl]-2H-indazole-7-carboxamide;
2-[4-(piperidin-1-ylmethyl)-3-(trifluoromethyl)phenyl]-2H-indazole-7-carboxamide;
2-[4-[(ethylamino)methyl]-3-(trifluoromethyl)phenyl]-2H-indazole-7-carboxamide;
4-{4-[7-(aminocarbonyl)-4-chloro-2H-indazol-2-yl]benzyl}-1-methylpiperazin-1-ium trifluoroacetate;
1-[2-({4-[7-(aminocarbonyl)-2*H*-indazol-2-yl]benzyl}ammonio)ethyl]piperidinium bis(trifluoroacetate);
4-[2-({4-[7-(aminocarbonyl)-2*H-*indazol-2-yl]benzyl}ammonio)ethyl]morpholin-4-ium bis(trifluoroacetate);
1-[2-({4-[7-(aminocarbonyl)-2*H*-indazol-2-yl]benzyl}ammonio)ethyl]pyrrolidinium bis(trifluoroacetate);
4-({4-[7-(aminocarbonyl)-2*H*-indazol-2-yl]benzyl}ammonio)-1-methylpiperidinium bis(trifluoroacetate);
4-({4-[7-(aminocarbonyl)-2*H*-indazol-2-yl]benzyl}ammonio)-1-benzylpiperidinium bis(trifluoroacetate);
1-{4-[7-(aminocarbonyl)-2*H*-indazol-2-yl]benzyl}-4-phenylpiperazinediium bis(trifluoroacetate);
*N*-{4-[7-(aminocarbonyl)-2*H*-indazol-2-yl]benzyl}-2-(dimethylamino)-2-oxoethanaminium trifluoroacetate;
2-[({4-[7-(aminocarbonyl)-2*H*-indazol-2-yl]benzyl}ammonio)methyl]pyridinium bis(trifluoroacetate);
4-[({4-[7-(aminocarbonyl)-2*H*-indazol-2-yl]benzyl}ammonio)methyl]pyridinium bis(trifluoroacetate);
*N-*{4-[7-(aminocarbonyl)-2*H-*indazol-2-yl]benzyl}-2-methylpropan-2-aminium trifluoroacetate;
*N*-{4-[7-(aminocarbonyl)-2*H*-indazol-2-yl]benzyl}-*N,N-*dimethylethane-1,2-diaminium bis(trifluoroacetate);
{4-[7-(aminocarbonyl)-2*H*-indazol-2-yl]phenyl}-*N-*(1,3-oxazol-2-ylmethyl)methanaminium trifluoroacetate;
and pharmaceutically acceptable salts or tautomers thereof.

Further particular compounds within the scope of the present invention are:
7-[7-(Aminocarbonyl)-2*H-*indazol-2-yl]-1,2,3,4-tetrahydroisoquinolinium chloride;
6-[7-(Aminocarbonyl)-2*H-*indazol-2-yl]-1,2,3,4-tetrahydroisoquinolinium chloride;
5-[7-(Aminocarbonyl)-2*H-*indazol-2-yl]-1,2,3,4-tetrahydroisoquinolinium trifluoroacetate;
3-[({4-[7-(Aminocarbonyl)-5-fluoro-2*H-*indazol-2-yl]phenyl}amino)carbonyl]azetidinium trifluoroacetate;
2-(4-{[(Azetidin-3-ylcarbonyl)(methyl)amino]methyl}phenyl)-2*H*-indazole-7-carboxamide;
3-[({4-[7-(Aminocarbonyl)-2*H-*indazol-2-yl]benzyl}amino)carbonyl]azetidinium trifluoroacetate; 2-(4-Bromophenyl)-5-fluoro-2H-indazole-7-carboxamide;
5-Fluoro-2-(4-pyridin-3-ylphenyl)-2H-indazole-7-carboxamide;
2-(4-Pyridin-3-ylphenyl)-2*H-*indazole-7-carboxamide;
4-{4-[7-(Aminocarbonyl)-2*H-*indazol-2-yl]phenyl}-1-methylpiperazin-1-ium trifluoroacetate;
4-{4-[7-(Aminocarbonyl)-2*H*-indazol-2-yl]phenyl}piperidinium trifluoroacetate;
2-{4-[7-(Aminocarbonyl)-2*H*-indazol-2-yl]phenyl}-*N*-methylethanaminium trifluoroacetate;
2-[({4-[7-(Aminocarbonyl)-2*H*-indazol-2-yl]phenyl}amino)carbonyl]azetidinium trifluoroacetate;
2-{5-[(Methylamino)methyl]pyridin-2-yl}-2*H*-indazole-7-carboxamide;
5-Fluoro-2-{3-fluoro-4-[(methylamino)methyl]phenyl}-2H-indazole-7-carboxamide;
5-Fluoro-2-{4-[(methylamino)methyl]phenyl}-2*H-*indazole-7-carboxamide trifluoroacetate;
2-{4-[7-(Aminocarbonyl)-2H-indazol-2-yl]phenyl}-N-methylpropan-2-aminium trifluoroacetate;
2-(6-Phenylpyridazin-3-yl)-2*H-*indazole-7-carboxamide;
{4-[7-(Aminocarbonyl)-5-fluoro-2*H*-indazol-2-yl]phenyl}-*N*-{[1-(hydroxymethyl)cyclohexyl] methyl}methanaminium trifluoroacetate;
5-Chloro-2-(4-formylphenyl)-2H-indazole-7-carboxamide;
2-{3-Methoxy-4-[(4-methylpiperazin-1-yl)methyl]phenyl}-2H-indazole-7-carboxamide;
2-{3-Methoxy-4-[(methylamino)methyl]phenyl}-2H-indazole-7-carboxamide;
5-Chloro-2-{4-[(4-methylpiperazin-1-yl)methyl]phenyl}-2H-indazole-7-carboxamide;
5-Chloro-2-{4-[(methylamino)methyl]phenyl}-2H-indazole-7-carboxamide;
{4-[7-(Aminocarbonyl)-4-fluoro-2H-indazol-2-yl]phenyl}-N-methylmethanaminium chloride;
{4-[7-(Aminocarbonyl)-5-fluoro-2H-indazol-2-yl]phenyl}-N-methylmethanaminium chloride;
1-{4-[7-(Aminocarbonyl)-4-fluoro-2H-indazol-2-yl]benzyl}-4-methylpiperazin-1-ium chloride;
1-{4-[7-(Aminocarbonyl)-5-fluoro-2H-indazol-2-yl]benzyl}-4-methylpiperazin-1-ium chloride;
1-{3-[7-(Aminocarbonyl)-2H-indazol-2-yl]benzyl}-4-methylpiperazinediiumbis(trifluoroacetate);
2-[4-(1-Hydroxy-1-methylethyl)phenyl]-2H-indazole-7-carboxamide;
2-(4-Acetylphenyl)-2H-indazole-7-carboxamide;
3-{[{4-[7-(Aminocarbonyl)-2H-indazol-2-yl]benzyl}(methyl)amino]carbonyl}-1-methylpiperidinium trifluoroacetate;
2-{4-[1-(Formylamino)-1-methylethyl]phenyl}-2H-indazole-7-carboxamide;
2-[3-(1,4-Diazepan-1-ylcarbonyl)phenyl]-2H-indazole-7-carboxamide;
3-[({4-[7-(Aminocarbonyl)-2H-indazol-2-yl]benzyl}amino)carbonyl]-1-methylpiperidinium trifluoroacetate;
(2S)-2-[({4-[7-(Aminocarbonyl)-2H-indazol-2-yl]phenyl}amino)carbonyl]pyrrolidinium trifluoroacetate;
3-[({4-[7-(Aminocarbonyl)-2H-indazol-2-yl]phenyl}amino)carbonyl]pyrrolidinium trifluoroacetate;
(2R)-2-[({4-[7-(Aminocarbonyl)-2H-indazol-2-yl]phenyl}amino)carbonyl]pyrrolidinium trifluoroacetate;
3-[({4-[7-(Aminocarbonyl)-2H-indazol-2-yl]phenyl}amino)carbonyl]piperidinium trifluoroacetate;
(3R)-3-[({4-[7-(Aminocarbonyl)-2H-indazol-2-yl]phenyl}amino)carbonyl]-1-methylpyrrolidinium trifluoroacetate;
2-[({4-[7-(Aminocarbonyl)-2H-indazol-2-yl]phenyl}amino)carbonyl]-1-methylpiperidinium trifluoroacetate;
4-Chloro-2-(4-formylphenyl)-2H-indazole-7-carboxamide;
(3S)-3-[({4-[7-(Aminocarbonyl)-2H-indazol-2-yl]phenyl}amino)carbonyl]-1-methylpyrrolidinium trifluoroacetate;
(R)-1-{4-[7-(Aminocarbonyl)-2H-indazol-2-yl]phenyl}-N-methylethanaminium chloride;
(S)-1-{4-[7-(Aminocarbonyl)-2H-indazol-2-yl]phenyl}-N-methylethanaminium chloride;
2-{3-Fluoro-4-[(methylamino)methyl]phenyl}-2H-indazole-7-carboxamide;
{4-[7-(Aminocarbonyl)-2H-indazol-2-yl]-2-fluorophenyl}-N-methanamium trifluoroacetate;
2-{4-[1-Methyl-1-(methylamino)ethyl]phenyl}-2H-indazole-7-carboxamide;
1-{4-[7-(Aminocarbonyl)-2H-indazol-2-yl]-2-hydroxybenzyl}-4-methylpiperazin-1-ium trifluoroacetate;
(3R)-3-[({4-[7-(Aminocarbonyl)-2H-indazol-2-yl]phenyl}amino)carbonyl]-1-methylpiperidinium chloride;
(3S)-3-[({4-[7-(Aminocarbonyl)-2H-indazol-2-yl]phenyl}amino)carbonyl]-1-methylpiperidinium chloride;
1-(2-{4-[7-(Aminocarbonyl)-2H-indazol-2-yl]phenyl}ethyl)-4-methylpiperazinediium bis(trifluoroacetate);
{4-[7-(Aminocarbonyl)-4-hydroxy-2H-indazol-2-yl]phenyl}-N-methylmethanaminium trifluoroacetate;
2-[({4-[7-(Aminocarbonyl)-2H-indazol-2-yl]phenyl}amino)carbonyl]-4-phenylpyrrolidinium trifluoroacetate;
(1R,3S)-3-[({4-[7-(Aminocarbonyl)-2H-indazol-2-yl]phenyl}amino)carbonyl]cyclopentanaminium trifluoroacetate;
(1R,3R)-3-[({4-[7-(Aminocarbonyl)-2H-indazol-2-yl]phenyl}amino)carbonyl]cyclopentanaminium trifluoroacetate;
(1S,3R)-3-[({4-[7-(Aminocarbonyl)-2H-indazol-2-yl]phenyl}amino)carbonyl]cyclopentanaminium trifluoroacetate;
2-[({4-[7-(Aminocarbonyl)-2H-indazol-2-yl]phenyl}amino)carbonyl]-2-methylazetidinium trifluoroacetate;
4-[2-({4-[7-(Aminocarbonyl)-2H-indazol-2-yl]phenyl}amino)-2-oxoethyl]-1-methylpiperidinium trifluoroacetate;
9-[2-({4-[7-(Aminocarbonyl)-2H-indazol-2-yl]phenyl}amino)-2-oxoethyl]-3-azoniaspiro[5.5]undecane trifluoroacetate;
4-[2-({4-[7-(Aminocarbonyl)-2H-indazol-2-yl]phenyl}amino)-2-oxoethyl]-4-phenylpiperidinium trifluoroacetate;
2-[({4-[7-(Aminocarbonyl)-2H-indazol-2-yl]phenyl}amino)carbonyl]pyridinium trifluoroacetate;
4-{3-[({4-[7-(Aminocarbonyl)-2H-indazol-2-yl]phenyl}amino)carbonyl]pyridin-2-yl}piperazin-1-ium trifluoroacetate;
3-[({4-[7-(Aminocarbonyl)-2H-indazol-2-yl]phenyl}amino)carbonyl]pyridinium trifluoroacetate;
4-[({4-[7-(Aminocarbonyl)-2H-indazol-2-yl]phenyl}amino)carbonyl]pyridinium trifluoroacetate;
4-[({4-[7-(Aminocarbonyl)-2H-indazol-2-yl]phenyl}amino)carbonyl]quinolinium trifluoroacetate;
4-[({4-[7-(Aminocarbonyl)-2H-indazol-2-yl]phenyl}amino)carbonyl]isoquinolinium trifluoroacetate;
2-[({4-[7-(Aminocarbonyl)-2H-indazol-2-yl]phenyl}amino)carbonyl]-1-methylazepanium trifluoroacetate;
3-[({4-[7-(Aminocarbonyl)-2H-indazol-2-yl]phenyl}amino)carbonyl]-2-methyl-1,2,3,4-tetrahydroisoquinolinium trifluoroacetate;
2-{4-[({4-[7-(Aminocarbonyl)-2H-indazol-2-yl]phenyl}amino)carbonyl]piperidin-1-yl}pyrimidin-1-ium trifluoroacetate;
1-{4-[7-(Aminocarbonyl)-5-fluoro-2H-indazol-2-yl]benzyl}-4-methylpiperazin-1-ium chloride;
5-[({4-[7-(Aminocarbonyl)-2H-indazol-2-yl]phenyl}amino)carbonyl]-3-oxooctahydroindolizin-2-aminium trifluoroacetate;
2-{3-[({4-[7-(Aminocarbonyl)-2H-indazol-2-yl]phenyl}amino)carbonyl]piperidin-1-yl}pyridinium trifluoroacetate;
2-[({4-[7-(Aminocarbonyl)-2H-indazol-2-yl]phenyl}amino)carbonyl]-4-methylmorpholin-4-ium trifluoroacetate;
(1R,4R)-N-{4-[7-(Aminocarbonyl)-2H-indazol-2-yl]phenyl}-1'-(methylsulfonyl)-1',2'-dihydrospiro[cyclohexane-1,3'-indole]-4-carboxamide;
1-[({4-[7-(Aminocarbonyl)-2H-indazol-2-yl]phenyl}amino)carbonyl]octahydro-1H-isoindolium trifluoroacetate;
2-[({4-[7-(Aminocarbonyl)-2H-indazol-2-yl]phenyl}amino)carbonyl]-4-benzylmorpholin-4-ium trifluoroacetate;
(3S,4R)-3-[({4-[7-(Aminocarbonyl)-2H-indazol-2-yl]phenyl}amino)carbonyl]-4-(methoxycarbonyl)pyrrolidinium trifluoroacetate;
4-{(2S)-2-[({4-[7-(Aminocarbonyl)-2H-indazol-2-yl]phenyl}amino)carbonyl]pyrrolidinium-1-yl}piperidinium bis(trifluoroacetate);
(1S,3S)-3-[({4-[7-(Aminocarbonyl)-2H-indazol-2-yl]phenyl}amino)carbonyl]cyclopentanaminium trifluoroacetate;
3-[({4-[7-(Aminocarbonyl)-2H-indazol-2-yl]phenyl}amino)carbonyl]-1-methylpyrrolidinium trifluoroacetate;
2-{4-[({4-[7-(Aminocarbonyl)-2H-indazol-2-yl]phenyl}amino)carbonyl]piperidin-1-yl}pyrimidin-1-ium trifluoroacetate;
2-(1-{4-[7-(Aminocarbonyl)-2H-indazol-2-yl]benzyl}pyrrolidinium-3-yl)pyridinium bis(trifluoroacetate);
3-(1-{4-[7-(Aminocarbonyl)-2H-indazol-2-yl]benzyl}pyrrolidinium-3-yl)pyridinium bis(trifluoroacetate);
(3S,4S)-1-{4-[7-(Aminocarbonyl)-2H-indazol-2-yl]benzyl}-3,4-difluoropyrrolidinium trifluoroacetate;
3-{4-[7-(Aminocarbonyl)-2H-indazol-2-yl]benzyl}-6-ammonio-3-azoniabicyclo[3.1.0]hexane bis(trifluoroacetate);
2-{4-[7-(Aminocarbonyl)-2H-indazol-2-yl]benzyl}-7-methyl-2,7-diazoniaspiro[4.4]nonane bis(trifluoroacetate);
1-{4-[7-(Aminocarbonyl)-2H-indazol-2-yl]benzyl}-3-[4-(dimethylammonio)phenyl]pyrrolidinium bis(trifluoroacetate);
5-{4-[7-(Aminocarbonyl)-2H-indazol-2-yl]benzyl}-1-methyl-1,2,4,5,6,6a-hexahydropyrrolo[3,4-b]pyrrolediium bis(trifluoroacetate);
3-{[{4-[7-(Aminocarbonyl)-2H-indazol-2-yl]benzyl}(methyl)ammonio]methyl}-1-methylpiperidinium bis(trifluoroacetate);
(1R,4S)-5-{4-[7-(Aminocarbonyl)-2H-indazol-2-yl]benzyl}-2-oxa-5-azoniabicyclo[2.2.1]heptane trifluoroacetate;
N-{4-[7-(Aminocarbonyl)-2H-indazol-2-yl]benzyl}-2-hydroxy-2-methylpropan-1-aminium trifluoroacetate;
N-{4-[7-(Aminocarbonyl)-2H-indazol-2-yl]benzyl}-3,3-difluorocyclobutanaminium trifluoroacetate;
4-{4-[7-(Aminocarbonyl)-2H-indazol-2-yl]benzyl}-6-fluoro-1,4-diazepan-1-ium trifluoroacetate;
1-{4-[7-(Aminocarbonyl)-2H-indazol-2-yl]benzyl}-4-pyrimidin-1-ium-2-yl-1,4-diazepan-1-ium bis(trifluoroacetate);
3-({4-[7-(Aminocarbonyl)-2H-indazol-2-yl]benzyl}ammonio)-1-benzylpyrrolidinium bis(trifluoroacetate);
3-[({4-[7-(Aminocarbonyl)-2H-indazol-2-yl]benzyl}ammonio)methyl]-1-methylpyrrolidinium bis(trifluoroacetate);
3-[({4-[7-(Aminocarbonyl)-2H-indazol-2-yl]benzyl}ammonio)methyl]-1-benzylpyrrolidinium bis(trifluoroacetate);
2-{4-[7-(Aminocarbonyl)-2H-indazol-2-yl]benzyl}-7-benzyl-2,7-diazoniaspiro[4.4]nonane bis(trifluoroacetate);
2-{4-[7-(Aminocarbonyl)-2H-indazol-2-yl]benzyl}-8-benzyl-2,8-diazoniaspiro[5.5]undecane bis(trifluoroacetate);
2-{4-[7-(Aminocarbonyl)-2H-indazol-2-yl]benzyl}-2,6-diazoniaspiro[3.3]heptane bis(trifluoroacetate);
7-{4-[7-(Aminocarbonyl)-2H-indazol-2-yl]benzyl}-2,7-diazoniaspiro[3.5]nonane bis(trifluoroacetate);
2-{4-[7-(Aminocarbonyl)-2H-indazol-2-yl]benzyl}-2,6-diazoniaspiro[3.5]nonane bis(trifluoroacetate);
2-{4-[7-(Aminocarbonyl)-2H-indazol-2-yl]benzyl}-2,8-diazoniaspiro[5.5]undecane bis(trifluoroacetate);
2-{4-[7-(Aminocarbonyl)-2H-indazol-2-yl]benzyl}-2,8-diazoniaspiro[4.5]decane bis(trifluoroacetate);
2-{4-[7-(Aminocarbonyl)-2H-indazol-2-yl]benzyl}-2,7-diazoniaspiro[4.5]decane bis(trifluoroacetate);
8-{4-[7-(Aminocarbonyl)-2H-indazol-2-yl]benzyl}-2,8-diazoniaspiro[4.5]decane bis(trifluoroacetate);
3-{4-[7-(Aminocarbonyl)-2H-indazol-2-yl]benzyl}-3,9-diazoniaspiro[5.5]undecane bis(trifluoroacetate);
2-{4-[7-(Aminocarbonyl)-2H-indazol-2-yl]benzyl}octahydropyrrolo[3,4-c]pyrrolediium bis(trifluoroacetate);
5-{4-[7-(Aminocarbonyl)-2H-indazol-2-yl]benzyl}octahydropyrrolo[3,4-b]pyrrolediium bis(trifluoroacetate);
4-({4-[7-(Aminocarbonyl)-2H-indazol-2-yl]benzyl}ammonio)octahydrocyclopenta[c]pyrrolium bis(trifluoroacetate);
N²-{4-[7-(Aminocarbonyl)-2H-indazol-2-yl]benzyl}-N¹,N¹-dimethyl-1-pyridin-2-ylethane-1,2-diaminium bis(trifluoroacetate);
7-(Aminocarbonyl)-2-[4-({[2-(2,3-dihydro-1H-indol-1-yl)ethyl]ammonio}methyl)phenyl]-2H-indazol-1-ium bis(trifluoroacetate);
(3S,4S)-1-[2-({4-[7-(Aminocarbonyl)-2H-indazol-2-yl]benzyl}ammonio)ethyl]-3,4-difluoropyrrolidinium bis(trifluoroacetate);
5-({4-[7-(Aminocarbonyl)-2H-indazol-2-yl]benzyl}amino)-1,3-benzothiazol-3-ium trifluoroacetate;
1-({4-[7-(Aminocarbonyl)-2H-indazol-2-yl]benzyl}ammonio)-8-azoniaspiro[4.5]decane bis(trifluoroacetate);
4-[({4-[7-(Aminocarbonyl)-2H-indazol-2-yl]benzyl}ammonio)methyl]-1-methylpiperidinium bis(trifluoroacetate);
N-{4-[7-(Aminocarbonyl)-2H-indazol-2-yl]benzyl}-2-hydroxyethanaminium trifluoroacetate;
7-[7-(Aminocarbonyl)-2H-indazol-2-yl]-1,2,3,4-tetrahydroisoquinolinium trifluoroacetate;
3-[2-({4-[7-(Aminocarbonyl)-2H-indazol-2-yl]phenyl}amino)-2-oxoethyl]azetidinium trifluoroacetate;
4-({4-[7-(Aminocarbonyl)-2H-indazol-2-yl]benzyl}ammonio)piperidinium bis(trifluoroacetate);
(3R,4R)-4-({4-[7-(Aminocarbonyl)-2H-indazol-2-yl]benzyl}ammonio)-3-fluoropiperidinium bis(trifluoroacetate);
(3S,4R)-4-({4-[7-(Aminocarbonyl)-2H-indazol-2-yl]benzyl}ammonio)-3-benzyl-1-methylpiperidinium bis(trifluoroacetate);
N-{4-[7-(Aminocarbonyl)-2H-indazol-2-yl]benzyl}-1-isobutyrylpiperidin-4-aminium trifluoroacetate;
2-[4-({4-[7-(Aminocarbonyl)-2H-indazol-2-yl]benzyl}ammonio)piperidin-1-yl]-3-methylpyridinium bis(trifluoroacetate);
3-({4-[7-(Aminocarbonyl)-2H-indazol-2-yl]benzyl}ammonio)piperidinium bis(trifluoroacetate);
3-(4-[7-(Aminocarbonyl)-2H-indazol-2-yl]benzyl}ammonio)-1-benzylpiperidinium bis(trifluoroacetate);
5-{4-[7-(Aminocarbonyl)-2H-indazol-2-yl]benzyl}-5-aza-2-azoniabicyclo[2.2.2]octane trifluoroacetate;
(1S,4S)-2-{4-[7-(Aminocarbonyl)-2H-indazol-2-yl]benzyl}-5-methyl-2,5-diazoniabicyclo[2.2.1]heptane bis(trifluoroacetate);
1-{4-[7-(Aminocarbonyl)-2H-indazol-2-yl]benzyl}-4-(pyridin-2-ylmethyl)piperazinediium bis(trifluoroacetate);
5-{4-[7-(Aminocarbonyl)-2H-indazol-2-yl]benzyl}-2-benzyl-5-aza-2-azoniabicyclo[2.2.2]octane trifluoroacetate;
8-{4-[7-(Aminocarbonyl)-2H-indazol-2-yl]benzyl}-3-benzyl-8-aza-3-azoniabicyclo[3.2.1]octane trifluoroacetate;
(1S,4S)-5-{4-[7-(Aminocarbonyl)-2H-indazol-2-yl]benzyl}-2-benzyl-5-aza-2-azoniabicyclo[2.2.1]heptane trifluoroacetate;
3-({4-[7-(Aminocarbonyl)-2H-indazol-2-yl]benzyl}ammonio)pyrrolidinium bis(trifluoroacetate;
6-({4-[7-(Aminocarbonyl)-2H-indazol-2-yl]benzyl}ammonio)-3-azoniabicyclo[3.1.0]hexane bis(trifluoroacetate);
(3S,4S)-N-{4-[7-(Aminocarbonyl)-2H-indazol-2-yl]benzyl}-4-hydroxytetrahydrothiophen-3-aminium 1,1-dioxide trifluoroacetate;
4-[({4-[7-(Aminocarbonyl)-2H-indazol-2-yl]benzyl}ammonio)methyl]-4-hydroxy-1-methylpiperidinium bis(trifluoroacetate);
N-{4-[7-(Aminocarbonyl)-2H-indazol-2-yl]benzyl}-1-cyclopropyl-2-hydroxyethanaminium trifluoroacetate;
{4-[7-(Aminocarbonyl)-2H-indazol-2-yl]phenyl}-N-{[1-(hydroxymethyl)cyclopentyl] methyl}methanaminium trifluoroacetate;
2-{4-[7-(Aminocarbonyl)-2H-indazol-2-yl]benzyl}-1,2,3,4-tetrahydro-2,7-naphthyridinediium bis(trifluoroacetate);
1-{4-[7-(Aminocarbonyl)-2H-indazol-2-yl]benzyl}-3-[(dimethylammonio)methyl]piperidinium bis(trifluoroacetate);
4-(1-{4-[7-(Aminocarbonyl)-2H-indazol-2-yl]benzyl}piperidinium-4-yl)thiomorpholin-4-ium bis(trifluoroacetate);
1-{4-[7-(Aminocarbonyl)-2H-indazol-2-yl]benzyl}-4-[(methylsulfonyl)amino]piperidinium trifluoroacetate;
1-{4-[7-(Aminocarbonyl)-2H-indazol-2-yl]benzyl}-4-(1H-imidazol-3-ium-1-ylmethyl)piperidinium bis(trifluoroacetate);
7-{4-[7-(Aminocarbonyl)-2H-indazol-2-yl]benzyl}-1-oxa-7-azoniaspiro[4.5]decane trifluoroacetate;
1-{4-[7-(Aminocarbonyl)-2H-indazol-2-yl]benzyl}-4-(1-hydroxy-1-methylethyl)piperidinium trifluoroacetate;
2-[({4-[7-(Aminocarbonyl)-2H-indazol-2-yl]phenyl}amino)carbonyl]-1-benzylpiperidinium trifluoroacetate;
2-[({4-[7-(Aminocarbonyl)-2H-indazol-2-yl]phenyl}amino)carbonyl]-1-ethylpiperidinium trifluoroacetate;
3-[({4-[7-(Aminocarbonyl)-2H-indazol-2-yl]phenyl}amino)carbonyl]-1-ethylpiperidinium trifluoroacetate;
2-[3-(1,4-Diazepan-1-ylcarbonyl)-4-fluorophenyl]-2H-indazole-7-carboxamide trifluoroacetate; tert-Butyl {4-[7-(aminocarbonyl)-4-chloro-2H-indazol-2-yl]benzyl}methylcarbamate;
6-[7-(Aminocarbonyl)-2H-indazol-2-yl]-1,2,3,4-tetrahydroisoquinolinium trifluoroacetate;
2-{4-[7-(Aminocarbonyl)-2H-indazol-2-yl]phenyl}pyrrolidiniumtrifluoroacetate;
6-Fluoro-2-{4-[(methylamino)methyl]phenyl}-2H-indazole-7-carboxamide;
5-Fluoro-2-{2-fluoro-4-[(methylamino)methyl]phenyl}-2H-indazole-7-carboxamide;
2-{3-Hydroxy-4-[(methylamino)methyl]phenyl}-2H-indazole-7-carboxamide trifluoroacetate;
2-(4-{[Formyl(methyl)amino]methyl}-3-hydroxyphenyl)-2H-indazole-7-carboxamide;
2-{2-Chloro-4-[(methylamino)methyl]phenyl}-5-fluoro-2H-indazole-7-carboxamide;
5-Fluoro-2-{3-fluoro-4-[(methylamino)methyl]phenyl}-2H-indazole-7-carboxamide trifluoroacetate;
2-{2,5-Difluoro-4-[(methylamino)methyl]phenyl}-5-fluoro-2H-indazole-7-carboxamide trifluoroacetate;
2-(4-Bromophenyl)-2H-indazole-7-carboxamide;
(3R)-3-[({4-[7-(Aminocarbonyl)-5-fluoro-2H-indazol-2-yl]phenyl}amino)carbonyl]-1-methylpiperidinium chloride;
(3R)-3-[({4-[7-(Aminocarbonyl)-5-fluoro-2H-indazol-2-yl]phenyl}amino)carbonyl]-1-methylpiperidinium trifluoroacetate;
2-(1,2,3,4-Tetrahydroisoquinolin-7-yl)-2H-indazole-7-carboxamide;
(R)-2-[4-({3-[(Dimethylamino)methyl]piperidin-1-yl}methyl)phenyl]-2H-indazole-7-carboxamide;
(S)-2-[4-({3-[(Dimethylamino)methyl]piperidin-1-yl}methyl)phenyl]-2H-indazole-7-carboxamide;
3-({4-[7-(Aminocarbonyl)-5-fluoro-2H-indazol-2-yl]phenyl}amino)-2-(chloromethyl)-3-oxopropan-1-aminium trifluoroacetate;
5-Fluoro-2-{3-fluoro-4-[(methylamino)methyl]phenyl}-2H-indazole-7-carboxamide hydrochloride;
2-{4-[(Dimethylamino)methyl]-3-fluorophenyl}-5-fluoro-2H-indazole-7-carboxamide trifluoroacetate;
2-{4-[(Azetidin-3-ylcarbonyl)amino]phenyl}-5-fluoro-2H-indazole-7-carboxamide;
2-[4-(2,7-Diazaspiro[4.5]dec-2-ylmethyl)phenyl]-2H-indazole-7-carboxamide;
(1S,4S)-5-{4-[7-(Aminocarbonyl)-2H-indazol-2-yl]benzyl}-2-(4-chlorobenzyl)-5-aza-2-azoniabicyclo[2.2.1]heptane trifluoroacetate;
(1S,4S)-5-{4-[7-(Aminocarbonyl)-2H-indazol-2-yl]benzyl}-2-(3-chlorobenzyl)-5-aza-2-azoniabicyclo[2.2.1]heptane trifluoroacetate;
1-{4-[7-(Aminocarbonyl)-2H-indazol-2-yl]benzyl}-4-[(methylamino)carbonyl]piperazin-1-ium trifluoroacetate;
N-{4-[7-(Aminocarbonyl)-2H-indazol-2-yl]benzyl}-2-hydroxy-2-pyridin-3-ylethanaminium trifluoroacetate;
N-{4-[7-(Aminocarbonyl)-2H-indazol-2-yl]benzyl}-2-cyclohexyl-2-hydroxyethanaminium trifluoroacetate;
4-{4-[7-(Aminocarbonyl)-2H-indazol-2-yl]benzyl}-6-(hydroxymethyl)-1,4-oxazepan-4-ium trifluoroacetate;
{4-[7-(Aminocarbonyl)-2H-indazol-2-yl]phenyl}-N-{[1-(hydroxymethyl)cyclobutyl] methyl} methanaminium trifluoroacetate;
{4-[7-(Aminocarbonyl)-2H-indazol-2-yl]phenyl}-N-{[1-(hydroxymethyl)cyclohexyl] methyl}methanaminium trifluoroacetate;
1-{4-[7-(Aminocarbonyl)-2H-indazol-2-yl]benzyl}-4-(5-methyl-1H-benzimidazol-2-yl)piperidinium trifluoroacetate;
2-(1-{4-[7-(Aminocarbonyl)-2H-indazol-2-yl]benzyl}-4-hydroxypiperidinium-4-yl)pyridinium bis(trifluoroacetate);
1-{4-[7-(Aminocarbonyl)-2H-indazol-2-yl]benzyl}-3,3-difluoropyrrolidinium trifluoroacetate;
2-(4-{[(2R)-2-(Fluoromethyl)pyrrolidin-1-yl]methyl}phenyl)-2H-indazole-7-carboxamide;
N-{4-[7-(Aminocarbonyl)-2H-indazol-2-yl]benzyl}-2-oxopyrrolidin-3-aminium trifluoroacetate;
5-Fluoro-2-(4-formylphenyl)-2H-indazole-7-carboxamide;
3-[({4-[7-(Aminocarbonyl)-5-fluoro-2H-indazol-2-yl]phenyl}amino)carbonyl]-1-methylazetidinium trifluoroacetate;
1-{4-[7-(Aminocarbonyl)-5-fluoro-2H-indazol-2-yl]benzyl}-3-[(dimethylammonio)methyl]piperidinium bis(trifluoroacetate);
3-[({4-[7-(Aminocarbonyl)-5-fluoro-2H-indazol-2-yl]-2-fluorophenyl}amino)carbonyl]azetidinium trifluoroacetate;
2-{4-[7-(Aminocarbonyl)-5-fluoro-2H-indazol-2-yl]benzyl}-2,7-diazoniaspiro[4.5]decane bis(trifluoroacetate);
4,5-Difluoro-2-{4-[(methylamino)methyl]phenyl}-2H-indazole-7-carboxamide trifluoroacetate;
5-Fluoro-2-(3-fluoro-4-{[(1-methylazetidin-3-yl)carbonyl]amino}phenyl)-2H-indazole-7-carboxamide trifluoroacetate;
5-Fluoro-2-(3-fluoro-4-formylphenyl)-2H-indazole-7-carboxamide;
5-Fluoro-2-(5-fluoro-2-formylphenyl)-2H-indazole-7-carboxamide;
{4-[7-(Aminocarbonyl)-5-fluoro-2H-indazol-2-yl]-2-fluorophenyl}-N-{[1-(hydroxymethyl) cyclopentyl]methyl}methanaminium trifluoroacetate;
5-Fluoro-2-[3-fluoro-4-({[(3R)-1-methylpiperidin-3-yl]carbonyl}amino)phenyl]-2H-indazole-7-carboxamide trifluoroacetate;
1-{4-[7-(Aminocarbonyl)-5-fluoro-2H-indazol-2-yl]-2-fluorobenzyl}-4-methylpiperazinediium bis(trifluoroacetate);
4-[({4-[7-(Aminocarbonyl)-5-fluoro-2H-indazol-2-yl]benzyl}ammonio)methyl]-1-methylpiperidinium bis(trifluoroacetate);
4-[({4-[7-(Aminocarbonyl)-5-fluoro-2H-indazol-2-yl]-2-fluorobenzyl}ammonio)methyl]-1-methylpiperidinium bis(trifluoroacetate);
and pharmaceutically acceptable salts or tautomers thereof.

Further particular compounds within the scope of the present invention are:
7-[7-(Aminocarbonyl)-2*H-*indazol-2-yl]-1-methyl-1,2,3,4-tetrahydroisoquinolinium trifluoroacetate;
3-{4-[7-(Aminocarbonyl)-5-fluoro-2*H*-indazol-2-yl]phenyl}-1-ethylpiperidinium trifluoroacetate;
2-(4-Cyanophenyl)-5-fluoro-2H-indazole-7-carboxamide;
5-fluoro-2-[4-(1H-tetrazol-5-yl)phenyl]-2H-indazole-7-carboxamide;
2-(4-Aminophenyl)-5-fluoro-2H-indazole-7-carboxamide hydrochloride;
*tert*-Butyl 3-[7-(aminocarbonyl)-5-fluoro-2*H*-indazol-2-yl]pyrrolidine-1-carboxylate;
3-[7-(Aminocarbonyl)-5-fluoro-2*H*-indazol-2-yl]pyrrolidinium trifluoroacetate;
3-[7-(Aminocarbonyl)-5-fluoro-2*H*-indazol-2-yl]-1-methylpyrrolidinium trifluoroacetate;
3-[7-(Aminocarbonyl)-5-fluoro-2*H*-indazol-2-yl]-1-ethylpyrrolidinium trifluoroacetate;
3-[7-(Aminocarbonyl)-5-fluoro-2*H*-indazol-2-yl]-1-propylpyrrolidinium trifluoroacetate;
3-[7-(Aminocarbonyl)-5-fluoro-2*H*-indazol-2-yl]-1-isopropylpyrrolidinium trifluoroacetate;
3-[7-(Aminocarbonyl)-5-fluoro-2*H*-indazol-2-yl]-1-cyclohexylpyrrolidinium trifluoroacetate;
3-[7-(Aminocarbonyl)-5-fluoro-2*H*-indazol-2-yl]-1-cyclobutylpyrrolidinium trifluoroacetate;
*tert-*Butyl 4-[7-(aminocarbonyl)-2*H-*indazol-2-yl]-4-methylpiperidine-1-carboxylate;
4-[7-(Aminocarbonyl)-2*H-*indazol-2-yl]-4-methylpiperidinium trifluoroacetate;
2-{4-[7-(Aminocarbonyl)-5-fluoro-2H-indazol-2-yl]phenyl}pyrrolidinium trifluoroacetate;
2-[4-(4,5-Dihydro-1H-imidazol-2-yl)phenyl]-5-fluoro-2H-indazole-7-carboxamide trifluoroacetate;
6-[7-(Aminocarbonyl)-5-fluoro-2H-indazol-2-yl]-1,2,3,4-tetrahydroisoquinolinium chloride;
2-{4-[7-(Aminocarbonyl)-5-fluoro-2H-indazol-2-yl]phenyl}piperidinium chloride;
5-Fluoro-2-[4-(1H-pyrazol-1-yl)phenyl]-2H-indazole-7-carboxamide;
5-Fluoro-2-(3-piperidin-3-ylphenyl)-2H-indazole-7-carboxamide;
2-[4-(Aminosulfonyl)phenyl]-5-fluoro-2H-indazole-7-carboxamide;
5-Fluoro-2-(5,6,7,8-tetrahydro-1,7-naphthyridin-3-yl)-2H-indazole-7-carboxamide;
5-Fluoro-2-(4-piperazin-2-ylphenyl)-2H-indazole-7-carboxamide;
Methyl 4-[7-(aminocarbonyl)-5-fluoro-2H-indazol-2-yl]benzoate;
5-Fluoro-2-(1-methylpiperidin-3-yl)-2*H-*indazole-7-carboxamide;
5-Fluoro-2-(1-ethylpiperidin-3-yl)-2*H-*indazole-7-carboxamide;
5-Fluoro-2-(1-propylpiperidin-3-yl)-2*H-*indazole-7-carboxamide;
5-Fluoro-2-(1-isopropylpipcridin-3-yl)-2*H*-indazole-7-carboxamide;
2-(1-Cyclohexylpiperidin-3-yl)-5-fluoro-2*H*-indazole-7-carboxamide;
5-Fluoro-2-(1-methylpiperidin-4-yl)-2*H-*indazole-7-carboxamide;
5-Fluoro-2-(1-ethylpiperidin-4-yl)-2*H*-indazole-7-carboxamide;
5-Fluoro-2-(1-propylpiperidin-4-yl)-2*H*-indazole-7-carboxamide;
5-Fluoro-2-(1-isopropylpiperidin-4-yl)-2*H*-indazole-7-carboxamide;
2-(1-Cyclohexylpiperidin-4-yl)-5-fluoro-2*H*-indazole-7-carboxamide;
2-(1-Cyclobutylpiperidin-4-yl)-5-fluoro-2*H-*indazole-7-carboxamide;
2-(1-Cyclobutylpiperidin-3-yl)-2*H*-indazole-7-carboxamide;
2-[7-(Aminocarbonyl)-5-fluoro-2*H*-indazol-2-yl]-*N,N-*dimethylethanaminium trifluoroacetate;
2-[7-(Aminocarbonyl)-5-fluoro-2*H*-indazol-2-yl]-*N,N-*diethylethanaminium trifluoroacetate;
*N*-{2-[7-(Aminocarbonyl)-5-fluoro-2*H*-indazol-2-yl]ethyl}propan-2-aminium trifluoroacetate;
*N-*2-[7-(Aminocarbonyl)-5-fluoro-2*H-*indazol-2-yl]ethyl}cyclohexanaminium trifluoroacetate;
2-[2-(Dicyclobutylamino)ethyl]-5-fluoro-2*H-*indazole-7-carboxamide;
tert-Butyl 3-[7-(aminocarbonyl)-5-fluoro-2H-indazol-2-yl]piperidine-1-carboxylate;
tert-Butyl 4-[7-(aminocarbonyl)-5-fluoro-2H-indazol-2-yl]piperidine-1-carboxylate;
3-[7-(Aminocarbonyl)-5-fluoro-2H-indazol-2-yl]piperidinium trifluoroacetate;
4-[7-(Aminocarbonyl)-5-fluoro-2H-indazol-2-yl]piperidinium trifluoroacetate;
tert-Butyl 3-[7-(aminocarbonyl)-2H-indazol-2-yl]piperidine-1-carboxylate;
*tert*-Butyl {2-[7-(aminocarbonyl)-5-fluoro-2*H*-indazol-2-yl]ethyl}carbamate;
2-[7-(Aminocarbonyl)-5-fluoro-2*H*-indazol-2-yl]ethanaminium trifluoroacetate;
3-[7-(Aminocarbonyl)- 2H-indazol-2-yl]piperidinium trifluoroacetate;
3-[7-(Aminocarbonyl)-2H-indazol-2-yl]-1-methylpiperidinium trifluoroacetate;
3-[7-(Aminocarbonyl)-2H-indazol-2-yl]-1-ethylpiperidinium trifluoroacetate;
3-[7-(Aminocarbonyl)-2H-indazol-2-yl]-1-propylpiperidinium trifluoroacetate;
3-[7-(Aminocarbonyl)-2H-indazol-2-yl]-1-isopropylpiperidinium trifluoroacetate;
3-[7-(Aminocarbonyl)-2H-indazol-2-yl]-1-cyclohexylpiperidinium trifluoroacetate;
3-[7-(Aminocarbonyl)-5-fluoro-2H-indazol-2-yl]-1-cyclobutylpiperidinium trifluoroacetate;
*N*-{2-[7-(Aminocarbonyl)-5-fluoro-2*H*-indazol-2-yl]ethyl}-*N*-propylpropan-1-aminium trifluoroacetate;
and pharmaceutically acceptable salts or tautomers thereof.

Included in the instant invention is the free base of compounds of Formula I, as well as the pharmaceutically acceptable salts and stereoisomers thereof. The compounds of the present invention can be protonated at the N atom(s) of an amine and/or N containing heterocycle moiety to form a salt. The term "free base" refers to the amine compounds in non-salt form. The encompassed pharmaceutically acceptable salts not only include the salts exemplified for the specific compounds described herein, but also all the typical pharmaceutically acceptable salts of the free form of compounds of Formula I. The free form of the specific salt compounds described may be isolated using techniques known in the art. For example, the free form may be regenerated by treating the salt with a suitable dilute aqueous base solution such as dilute aqueous NaOH, potassium carbonate, ammonia and sodium bicarbonate. The free forms may differ from their respective salt forms somewhat in certain physical properties, such as solubility in polar solvents, but the acid and base salts are otherwise pharmaceutically equivalent to their respective free forms for purposes of the invention.

The pharmaceutically acceptable salts of the instant compounds can be synthesized from the compounds of this invention which contain a basic or acidic moiety by conventional chemical methods. Generally, the salts of the basic compounds are prepared either by ion exchange chromatography or by reacting the free base with stoichiometric amounts or with an excess of the desired salt-forming inorganic or organic acid in a suitable solvent or various combinations of solvents. Similarly, the salts of the acidic compounds are formed by reactions with the appropriate inorganic or organic base.

Thus, pharmaceutically acceptable salts of the compounds of this invention include the conventional non-toxic salts of the compounds of this invention as formed by reacting a basic instant compound with an inorganic, organic acid or polymeric acid. For example, conventional non-toxic salts include those derived from inorganic acids such as hydrochloric, hydrobromic, hydroiodic, sulfuric, sulfurous, sulfamic, phosphoric, phosphorous, nitric, as well as salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxy-benzoic, fumaric, toluenesulfonic, methanesulfonic, ethane disulfonic, oxalic, isethionic, palmitic, gluconic, ascorbic, phenylacetic, aspartic, cinnamic, pyruvic, ethanesulfonic, ethane, disulfonic, valeric and trifluoroacetic. Examples of suitable polymeric salts include those derived from the polymeric acids such as tannic acid, carboxymethyl cellulose. Preferably, a pharmaceutically acceptable salt of this invention contains 1 equivalent of a compound of formula (I) and 1, 2 or 3 equivalent of an inorganic or organic acid. More particularly, pharmaceutically acceptable salts of this invention are the trifluoroacetate or the chloride salts. In an embodiment the salt is trifluoroacetate. In another embodiment the salt is chloride.

When the compound of the present invention is acidic, suitable "pharmaceutically acceptable salts" refers to salts prepared form pharmaceutically acceptable non-toxic bases including inorganic bases and organic bases. Salts derived from inorganic bases include aluminum, ammonium, calcium, copper, ferric, ferrous, lithium, magnesium, manganic salts, manganous, potassium, sodium and zinc. Particularly preferred are the ammonium, calcium, magnesium, potassium and sodium salts. Salts derived from pharmaceutically acceptable organic non-toxic bases include salts of primary, secondary and tertiary amines, substituted amines including naturally occurring substituted amines, cyclic amines and basic ion exchange resins, such as arginine, lysine, betaine caffeine, choline, N,N¹-dibenzylethylenediamine, ethylamine, diethylamine, 2-diethylaminoethanol, 2-dimethylaminoethanol, ethanolamine, diethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine tripropylamine, tromethamine, dicyclohexylamine, butylamine, benzylamine, phenylbenzylamine, and tromethamine.

The preparation of the pharmaceutically acceptable salts described above and other typical pharmaceutically acceptable salts is more fully described by Berg et al (1977) J. Pharm. Sci., 'Pharmaceutical Salts', 66:1-19.

It will also be noted that the compounds of the present invention are potentially internal salts or zwitterions, since under physiological conditions a deprotonated acidic moiety in the compound, such as a carboxyl group, may be anionic, and this electronic charge might then be balanced off internally against the cationic charge of a protonated or alkylated basic moiety, such as a quaternary nitrogen atom.

The compounds of the invention can be used in a method of treatment of the human or animal body by therapy.

The invention provides compounds for use in the treatment or prevention of conditions which can be ameliorated by the inhibition of poly(ADP-ribose)polymerase (PARP) (see, for example, Nature Review Drug Discovery (2005) 4:421- 440).

Thus, the present invention provides a compound of formula I for use in the manufacture of a medicament for the treatment or prevention of conditions which can be ameliorated by the inhibition of poly(ADP-ribose)polymerase (PARP).

The PARP inhibitors of the present invention are useful for the treatment of the diseases specified in WO 2005/082368.

The compounds of the invention are useful for the treatment of inflammatory diseases, including conditions resulting from organ transplant rejection, such as; chronic inflammatory diseases of the joints, including arthritis, rheumatoid arthritis, osteoarthritis and bone diseases associated with increased bone resorption; inflammatory bowel diseases such as ileitis, ulcerative colitis, Barrett's syndrome, and Crohn's disease; inflammatory lung diseases such as asthma, adult respiratory distress syndrome, and chronic obstructive airway disease; inflammatory diseases of the eye including corneal dystrophy, trachoma, onchocerciasis, uveitis, sympatheticophthalmitis and endophthalmitis; chronic inflammatory diseases of the gum, including gingivitis and periodontitis; tuberculosis; leprosy; inflammatory diseases of the kidney including uremic complications, glomerulonephritis and nephrosis; inflammatory diseases of the skin including sclerodermatitis, psoriasis and eczema; inflammatory diseases of the central nervous system, including chronic demyelinating diseases of the nervous system, multiple sclerosis, AIDS-related neurodegeneration and Alzheimer's disease, infectious meningitis, encephalomyelitis, Parkinson's disease, Huntington's disease, amyotrophic lateral sclerosis and viral or autoimmune encephalitis; diabetic complications, including, but not limited to, immune-complex vasculitis, systemic lupus erythematosus (SLE); inflammatory diseases of the heart such as cardiomyopathy, ischemic heart disease,hypercholesterolemia, and atherosclerosis; as well as various other diseases that can have significant inflammatory components, including preeclampsia, chronic liver failure, brain and spinal cord trauma and multiple organ dysfunction syndrome (MODS) (multiple organ failure (MOF)). The inflammatory disease can also be a systemic inflammation of the body, exemplified by gram-positive or gram negative shock, hemorrhagic or anaphylactic shock, or shock induced by cancer chemotherapy in response to pro-inflammatory cytokines, e. g., shock associated with pro-inflammatory cytokines. Such shock can be induced, e. g. by a chemotherapeutic agent that is administered as a treatment for cancer.

Thus, the present invention provides a compound of formula I for use in the manufacture of a medicament for treating or preventing inflammatory diseases.

The compounds of the instant invention may also be useful in the treatment or prevention of reperfusion injuries, resulting from naturally occurring episodes and during a surgical procedure, such as intestinal reperfusion injury; myocardial reperfusion injury; reperfusion injury resulting from cardiopulmonary bypass surgery, aortic aneurysm repair surgery, carotid endarterectomy surgery, or hemorrhagic shock; and reoxygenation injury resulting from transplantation of organs such as heart, lung, liver, kidney, pancreas, intestine, and cornea.

Thus, the present invention provides a compound of formula I for use in the manufacture of a medicament for the treatment or prevention of reperfusion injuries.

The compounds of the instant invention may also be useful in the treatment or prevention of ischemic conditions, including those resulting from organ transplantation, such as stable angina, unstable angina, myocardial ischemia, hepatic ischemia, mesenteric artery ischemia, intestinal ischemia, critical limb ischemia, chronic critical limb ischemia, cerebral ischemia, acute cardiac ischemia, ischemia kidney disease, ischemic liver disease, ischemic retinal disorder, septic shock, and an ischemic disease of the central nervous system, such as stroke or cerebral ischemia.

Thus, the present invention provides a compound of formula I for use in the manufacture of a medicament for the treatment or prevention of ischemic conditions.

The present invention provides a compound of formula I for use in the manufacture of a medicament for the treatment or prevention of stroke.

The compounds of the instant invention may also be useful for the treatment or prevention of chronic or acute renal failure.

Thus, the present invention provides a compound of formula I for use in the manufacture of a medicament for the treatment or prevention of renal failure.

The compounds of the instant invention may also be useful for the treatment or prevention of vascular diseases other than cardiovascular diseases, such as peripheral arterial occlusion, thromboangitis obliterans, Reynaud's disease and phenomenon, acrocyanosis, erythromelalgia, venous thrombosis, varicose veins, arteriovenous fistula, lymphedema and lipedema.

Thus, the present invention provides a compound of formula I for use in the manufacture of a medicament for the treatment or prevention of vascular diseases other than cardiovascular diseases.

The compounds of the instant invention may also be useful for the treatment or prevention of cardiovascular diseases such as chronic heart failure, atherosclerosis, congestive heart failure, circulatory shock, cardiomyopathy, cardiac transplant, myocardialinfarction, and a cardiac arrhythmia, such as atrial fibrillation, supraventricular tachycardia, atrial flutter, and paroxysmal atrial tachycardia.

Thus, the present invention provides a compound of formula I for use in the manufacture of a medicament for the treatment or prevention of cardiovascular diseases.

The compounds of this invention may also be useful for the treatment and prevention of diabetes mellitus, including Type I diabetes (Insulin Dependent Diabetes Mellitus), Type II diabetes (Non-Insulin Dependent Diabetes Mellitus), gestational diabetes,autoimmune diabetes, insulinopathies, diabetes due to pancreatic disease, diabetes associated with other endocrine diseases (such as Cushing's Syndrome, acromegaly, pheochromocytoma, glucagonoma, primary aldosteronism or somatostatinoma), Type A insulin resistance syndrome, Type B insulin resistance syndrome, lipatrophic diabetes, and diabetes induced by(3-cell toxins. The compounds of this invention may also be useful for the treatment or prevention of diabetic complications, such as diabetic cataract, glaucoma, retinopathy, nephropathy, (such asmicroaluminuria and progressive diabetic nephropathy), polyneuropathy, gangrene of the feet, atherosclerotic coronary arterial disease, peripheral arterial disease, nonketotic hyperglycemic-hyperosmolar coma, mononeuropathies, autonomic neuropathy, foot ulcers, joint problems, and a skin or mucous membrane complication (such as an infection, a shin spot, a candidal infection or necrobiosis lipoidica diabeticorumobesity), hyperlipidemia, hypertension, syndrome of insulin resistance, coronary artery disease, retinopathy, diabetic neuropathy, polyneuropathy, mononeuropathies, autonomic neuropathy, a foot ulcer, a joint problem, a fungal infection, a bacterial infection, and cardiomyopathy.

Thus, the present invention provides a compound of formula I for use in the manufacture of a medicament for the treatment or prevention of diabetes.

The compounds of this invention may also be useful for the treatment or prevention of cancer including solid tumors such as fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon cancer, colorectal cancer, kidney cancer, pancreatic cancer, bone cancer, breast cancer, ovarian cancer, prostate cancer, esophageal cancer, stomach cancer, oral cancer, nasal cancer, throat cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms'tumor, cervical cancer, uterine cancer, testicular cancer, small cell lung carcinoma, bladder carcinoma, lung cancer, epithelial carcinoma, skin cancer, melanoma, neuroblastoma and retinoblastoma; blood-borne cancers such as acute lymphoblastic leukemia("ALL"), acute lymphoblastic B-cell leukemia, acute lymphoblastic T-cell leukemia, acute myeloblastic leukemia ("AML"), acute promyelocytic leukemia("APL"), acute monoblastic leukemia, acute erythroleukemic leukemia, acute megakaryoblastic leukemia, acute myelomonocytic leukemia, acute nonlymphocyctic leukemia, acute undifferentiated leukemia, chronic myelocytic leukemia("CML"), chronic lymphocytic leukemia("CLL"), hairy cell leukemia and multiple myeloma; acute and chronic leukemias such as lymphoblastic, myelogenous, lymphocytic, myelocytic leukemias; Lymphomas such as Hodgkin's disease, non-Hodgkin's Lymphoma, Multiple myeloma, Waldenstrom's macroglobulinemia, Heavy chain disease and Polycythemia vera; CNS and brain cancers such as glioma, pilocytic astrocytoma, astrocytoma, anaplastic astrocytoma, glioblastoma multiforme, medulloblastoma, craniopharyngioma, ependymoma, pinealoma, hemangioblastoma, acoustic neuroma, oligodendroglioma, meningioma, vestibular schwannoma, adenoma, metastatic brain tumor, meningioma, spinal tumor and medulloblastoma.

Thus, the present invention provides a compound of formula I for use in the manufacture of a medicament for the treatment or prevention of cancer.

The compounds of the present invention may also be used for the treatment of cancer which is deficient in Homologous Recombination (HR) dependent DNA DSB repair activity (see WO 2006/021801).

The HR dependent DNA DSB repair pathway repairs double-strand breaks (DSBs) in DNA via homologous mechanisms to reform a continuous DNA helix (Nat. Genet. (2001) 27(3):247-254). The components of the HR dependent DNA DSB repair pathway include, but are not limited to, ATM (NM-000051), RAD51 (NM-002875), RAD51 L1 (NM-002877), RAD51 C (NM-002876), RAD51L3 (NM-002878), DMC1 (NM-007068), XRCC2 (NM7005431), XRCC3 (NM-005432), RAD52 (NM-002879), RAD54L (NM-003579), RAD54B (NM-012415), BRCA-1 (NM-007295), BRCA-2 (NM-000059), RAD50 (NM-005732), MREI 1A (NM-005590), NBS1 (NM-002485), ADPRT (PARP-1), ADPRTL2, (PARP02) CTPS, RPA, RPA1, RPA2, RPA3, XPD,ERCC1, XPF,MMS19, RAD51, RAD51p, RAD51C, RAD51D,DMC1, XRCCR, XRCC3, BRCA1, BRCA2, RAD52, RAD54, RAD50,MRE11, NB51, WRN, BLMKU70, RU80, ATM, ATRCHK1, CHK2, FANCA, FANCB, FANCC, FANCD1, FANCD2, FANCE, FANCF, FANCG, FANCC, FANCD1, FANCD2, FANCE, FANCF, FANCG, RAD1 and RAD9. Other proteins involved in the HR dependent DNA DSB repair pathway include regulatory factors such as EMSY (Cell (2003) 115:523-535).

A cancer which is deficient in HR dependent DNA DSB repair may comprise or consist of one or more cancer cells which have a reduced or abrogated ability to repair DNA DSBs through that pathway, relative to normal cells i.e. the activity of the HR dependent DNA DSB repair pathway may be reduced or abolished in the one or more cancer cells.

The activity of one or more components of the HR dependent DNA DSB repair pathway may be abolished in the one or more cancer cells of an individual having a cancer which is deficient in HR dependent DNA DSB repair. Components of the HR dependent DNA DSB repair pathway are well characterized in the art (see for example, Science (2001) 291:1284-1289) and include the components listed above.

The present invention provides a compound of formula I for use in the manufacture of a medicament for the treatment or prevention of a cancer which is deficient in HR dependent DNA DSB repair activity.

In an embodiment the cancer cells are deficient in the HR dependent DNA DSB repair activity of one or more phenotypes selected from ATM (NM-000051), RAD51 (NM-002875), RAD51 L1 (NM-002877), RAD51 C (NM-002876), RAD51L3 (NM-002878), DMC1 (NM-007068), XRCC2 (NM7005431), XRCC3 (NM-005432), RAD52 (NM-002879), RAD54L (NM-003579), RAD54B (NM-012415), BRCA-1 (NM-007295), BRCA-2 (NM-000059), RAD50 (NM-005732), MREI 1A (NM-005590), NBS1 (NM-002485)), ADPRT (PARP-1), ADPRTL2, (PARP02) CTPS, RPA, RPA1, RPA2, RPA3, XPD,ERCC1, XPF,MMS19, RAD51, RAD51p, RAD51C, RAD51D,DMC1, XRCCR, XRCC3, BRCA1, BRCA2, RAD52, RAD54, RAD50,MRE11, NB51, WRN, BLMKU70, RU80, ATM, ATRCHK1, CHK2, FANCA, FANCB, FANCC, FANCD1, FANCD2, FANCE, FANCF, FANCG, FANCC, FANCD1, FANCD2, FANCE, FANCF, FANCG, RAD1 and RAD9.

In another embodiment, the cancer cells have a BRCA1 and/or a BRCA2 deficient phenotype. Cancer cells with this phenotype may be deficient in BRCA1 and/or BRCA2, i. e. expression and/or activity of BRCA1 and/or BRCA2 may be reduced or abolished in the cancer cells, for example by means of mutation or polymorphism in the encoding nucleic acid, or by means of amplification, mutation or polymorphism in a gene encoding a regulatory factor, for example the EMSY gene which encodes a BRCA2 regulatory factor (Cell (2003) 115:523-535).

BRCA-1 and BRCA-2 are known tumor suppressors whose wild-type alleles are frequently lost in tumors of heterozygous carriers (Oncogene, (2002) 21(58):8981-93; Trends Mol Med., (2002) 8(12):571-6). The association of BRCA-1 and/or BRCA-2 mutations with breast cancer has been well-characterized (Exp Clin Cancer Res., (2002) 21 (3 Suppl):9-12). Amplification of the EMSY gene, which encodes a BRCA-2 binding factor, is also known to be associated with breast and ovarian cancer. Carriers of mutations in BRCA-1 and/or BRCA-2 are also at elevated risk of cancer of the ovary, prostate and pancreas. The detection of variation in BRCA-1 and BRCA-2 is well-known in the art and is described, for example in EP 699 754, EP 705 903, Genet. Test (1992) 1:75-83; Cancer Treat Res (2002) 107:29-59; Neoplasm (2003) 50(4):246-50; Ceskn Gynekol (2003) 68(1):11-16). Determination of amplification of the BRCA-2 binding factor EMSY is described in Cell 115:523-535. PARP inhibitors have been demonstrated as being useful for the specific killing of BRCA-1 and BRCA-2 deficient tumors (Nature (2005) 434:913-916 and 917-920).

Thus, the present invention provides a compound of formula I for use in the manufacture of a medicament for the treatment or prevention of BRCA-1 or BRCA-2 deficient tumors.

In an embodiment, the PARP inhibitors of the present can be used in prophylactic therapy for elimination of BRCA2-deficient cells (see, Cancer Res. (2005) 65:10145).

The compounds of this invention may be useful for the treatment or prevention of neurodegenerative diseases, including, polyglutamine-expansion-related neurodegeneration, Huntington's disease, Kennedy's disease, spinocerebellar ataxia, dentatorubral-pallidoluysian atrophy (DRPLA), protein-aggregation-related neurodegeneration, Machado-Joseph's disease, Alzheimer's disease, Parkinson's disease, amyotrophic lateral sclerosis, spongiform encephalopathy, a prion-related disease and multiple sclerosis (MS).

Thus, the present invention provides a compound of formula I for use in the manufacture of a medicament for treating or preventing neurodegenerative diseases.

The compounds of the present invention may also be useful for the treatment or prevention of retroviral infection (US 5652260), retinal damage (Curr. Eye Res. (2004), 29:403), skin senescence and UV-induced skin damage (US5589483 and Biochem. Pharmacol (2002) 63:921).

The compounds of the invention are useful for the treatment or prevention of premature aging and postponing the onset of age-related cellular dysfunction (Pharmacological Research (2005) 52:93-99).

The compounds of this invention may be administered to mammals, preferably humans, either alone or in combination with pharmaceutically acceptable carriers, excipients, diluents, adjuvants, fillers, buffers, stabilisers, preservatives, lubricants, in a pharmaceutical composition, according to standard pharmaceutical practice.

The compounds of this invention may be administered to a subject by any convenient route of administration, whether systemically/peripherally or at the site of desired action, including but not limited to, oral (e.g. by ingestion); topical (including e.g. transdermal, intranasal, ocular, buccal, and sublingual); pulmonary (e.g. by inhalation or insufflation therapy using, e.g. an aerosol, e.g. through mouth or nose); rectal; vaginal; parenteral, (e.g. by injection, including subcutaneous, intradermal, intramuscular, intravenous, intraarterial, intracardiac, intrathecal, intraspinal, intracapsular, subcapsular, intraorbital, intraperitoneal, intratracheal, subcuticular, intraarticular, subarachnoid, and intrasternal); and by implant of a depot (e.g. subcutaneously or intramuscularly).

The subject may be a eukaryote, an animal, a vertebrate animal, a mammal, a rodent (e.g. a guinea pig, a hamster, a rat, a mouse), murine (e.g. a mouse), canine (e.g. a dog), feline (e.g. a cat), equine (e.g. a horse), a primate, simian (e.g. a monkey or ape), a monkey (e.g. marmoset, baboon), an ape (e.g. gorilla, chimpanzee, orangutang, gibbon), or a human.

The invention also provides pharmaceutical compositions comprising one or more compounds of this invention and a pharmaceutically acceptable carrier. The pharmaceutical compositions containing the active ingredient may be in a form suitable for oral use, for example, as tablets, troches, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparations. Tablets contain the active ingredient in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example, microcrystalline cellulose, sodium crosscarmellose, corn starch, or alginic acid; binding agents, for example starch, gelatin, polyvinyl-pyrrolidone or acacia, and lubricating agents, for example, magnesium stearate, stearic acid or talc. The tablets may be uncoated or they may be coated by known techniques to mask the unpleasant taste of the drug or delay disintegration and absorption in the gastrointestinal tract and thereby provide a sustained action over a longer period. For example, a water soluble taste masking material such as hydroxypropyl-methylcellulose or hydroxypropylcellulose, or a time delay material such as ethyl cellulose, cellulose acetate butyrate may be employed.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water soluble carrier such as polyethyleneglycol or an oil medium, for example peanut oil, liquid paraffin, or olive oil.

Aqueous suspensions contain the active material in admixture with excipients suitable for the manufacture of aqueous suspensions. Such excipients are suspending agents, for example sodium carboxymethylcellulose, methylcellulose, hydroxypropylmethyl-cellulose, sodium alginate, polyvinyl-pyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl p-hydroxybenzoate, one or more coloring agents, one or more flavoring agents, and one or more sweetening agents, such as sucrose, saccharin or aspartame.

Oily suspensions may be formulated by suspending the active ingredient in a vegetable oil, for example arachis oil, olive oil, sesame oil or coconut oil, or in mineral oil such as liquid paraffin. The oily suspensions may contain a thickening agent, for example beeswax, hard paraffin or cetyl alcohol. Sweetening agents such as those set forth above, and flavoring agents may be added to provide a palatable oral preparation. These compositions may be preserved by the addition of an anti-oxidant such as butylated hydroxyanisol or alpha-tocopherol.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present. These compositions may be preserved by the addition of an anti-oxidant such as ascorbic acid.

The pharmaceutical compositions of the invention may also be in the form of an oil-in-water emulsions. The oily phase may be a vegetable oil, for example olive oil or arachis oil, or a mineral oil, for example liquid paraffin or mixtures of these. Suitable emulsifying agents may be naturally occurring phosphatides, for example soy bean lecithin, and esters or partial esters derived from fatty acids and hexitol anhydrides, for example sorbitan monooleate, and condensation products of the said partial esters with ethylene oxide, for example polyoxyethylene sorbitan monooleate. The emulsions may also contain sweetening, flavoring agents, preservatives and antioxidants.

Syrups and elixirs may be formulated with sweetening agents, for example glycerol, propylene glycol, sorbitol or sucrose. Such formulations may also contain a demulcent, a preservative, flavoring and coloring agents and antioxidant.

The pharmaceutical compositions may be in the form of a sterile injectable aqueous solutions. Among the acceptable vehicles and solvents that may be employed are water, Ringer's solution and isotonic sodium chloride solution.

The sterile injectable preparation may also be a sterile injectable oil-in-water microemulsion where the active ingredient is dissolved in the oily phase. For example, the active ingredient may be first dissolved in a mixture of soybean oil and lecithin. The oil solution then introduced into a water and glycerol mixture and processed to form a microemulation.

The injectable solutions or microemulsions may be introduced into a patient's blood stream by local bolus injection. Alternatively, it may be advantageous to administer the solution or microemulsion in such a way as to maintain a constant circulating concentration of the instant compound. In order to maintain such a constant concentration, a continuous intravenous delivery device may be utilized. An example of such a device is the Deltec CADD-PLUS™ model 5400 intravenous pump.

The pharmaceutical compositions may be in the form of a sterile injectable aqueous or oleagenous suspension for intramuscular and subcutaneous administration. This suspension may be formulated according to the known art using those suitable dispersing or wetting agents and suspending agents which have been mentioned above. The sterile injectable preparation may also be a sterile injectable solution or suspension in a non-toxic parenterally acceptable diluent or solvent, for example as a solution in 1,3-butanediol. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

Compounds of Formula I may also be administered in the form of suppositories for rectal administration of the drug. These compositions can be prepared by mixing the drug with a suitable nonirritating excipient which is solid at ordinary temperatures but liquid at the rectal temperature and will therefore melt in the rectum to release the drug. Such materials include cocoa butter, glycerinated gelatin, hydrogenated vegetable oils, mixtures of polyethylene glycols of various molecular weights and fatty acid esters of polyethylene glycol.

For topical use, creams, ointments, jellies, solutions or suspensions, etc., containing the compound of Formula I are employed. (For purposes of this application, topical application shall include mouth washes and gargles.)

The compounds for the present invention can be administered in intranasal form via topical use of suitable intranasal vehicles and delivery devices, or via transdermal routes, using those forms of transdermal skin patches well known to those of ordinary skill in the art. To be administered in the form of a transdermal delivery system, the dosage administration will, of course, be continuous rather than intermittent throughout the dosage regimen. Compounds of the present invention may also be delivered as a suppository employing bases such as cocoa butter, glycerinated gelatin, hydrogenated vegetable oils, mixtures of polyethylene glycols of various molecular weights and fatty acid esters of polyethylene glycol.

When a compound according to this invention is administered into a subject, the selected dosage level will depend on a variety of factors including, but not limited to, the activity of the particular compound, the severity of the individuals symptoms, the route of administration, the time of administration, the rate of excretion of the compound, the duration of the treatment, other drugs, compounds, and/or materials used in combination, and the age, sex, weight, condition, general health, and prior medical history of the patient. The amount of compound and route of administration will ultimately be at the discretion of the physician, although generally the dosage will be to achieve local concentrations at the site of action which achieve the desired effect without causing substantial harmful or deleterious side-effects.

*Administration in vivo* can be effected in one dose, continuously or intermittently (e.g. in divided doses at appropriate intervals) throughout the course of treatment. Methods of determining the most effective means and dosage of administration are well known to those of skill in the art and will vary with the formulation used for therapy, the purpose of the therapy, the target cell being treated, and the subject being treated. Single or multiple administrations can be carried out with the dose level and pattern being selected by the treating physician.

In general, a suitable dose of the active compound is in the range of about 100 µg to about 250 mg per kilogram body weight of the subject per day. Where the active compound is a salt, an ester, prodrug, the amount administered is calculated on the basis of the parent compound and so the actual weight to be used is increased proportionately.

The instant compounds are also useful in combination with anti-cancer agents or chemotherapeutic agents.

The compounds of this invention may be useful as chemo- and radiosensitizers for cancer treatment. They are useful for the treatment of mammals who have previously undergone or are presently undergoing treatment for cancer. Such previous treatments include prior chemotherapy, radiation therapy, surgery or immunotherapy, such as cancer vaccines.

Thus, the present invention provides a combination of a compound of formula I and an anti-cancer agent for simultaneous, separate or sequential administration.

The present invention also provides a compound of formula I for use in the manufacture of a medicament for use as an adjunct in cancer therapy or for potentiating tumor cells for treatment with ionizing radiation or chemotherapeutic agents.

The present invention also provides a method of chemotherapy or radiotherapy, which method comprises administration to a patient in need thereof of an effective amount of a compound of formula I or a composition comprising a compound of formula I in combination with ionizing radiation or chemotherapeutic agents.

In combination therapy, the compounds of this invention can be administered prior to (e. g., 5 minutes, 15 minutes, 30 minutes, 45 minutes, 1 hour, 2 hours, 4 hours, 6 hours, 12 hours, 24 hours, 48, hours, 72 hours, 96 hours, 1 week, 2 weeks, 3 weeks, 4 weeks, 5 weeks, 6 weeks, 8 weeks, or 12 weeks before), concurrently with, or subsequent to (e. g., 5 minutes, 15 minutes, 30 minutes, 45 minutes,1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks after) the administration of the other anticancer agent to a subject in need thereof. In various embodiments the instant compounds and another anticancer agent are administered 1 minute apart, 10 minutes apart, 30 minutes apart, less than 1 hour apart, 1 hour to 2 hours apart, 2 hours to 3 hours apart, 3 hours to 4 hours apart, 4 hours to 5 hours apart, 5 hours to 6 hours apart, 6 hours to 7 hours apart, 7 hours to 8 hours apart, 8 hours to 9 hours apart, 9 hours to 10 hours apart, 10 hours to 11 hours apart, 11 hours to 12 hours apart, no more than 24 hours apart, or no more than 48 hours apart.

The compounds of this invention and the other anticancer agent can act additively or synergistically. A synergistic combination of the present compounds and another anticancer agent might allow the use of lower dosages of one or both of these agents and/or less frequent dosages of one or both of the instant compounds and other anticancer agents and/or to administer the agents less frequently can reduce any toxicity associated with the administration of the agents to a subject without reducing the efficacy of the agents in the treatment of cancer. In addition, a synergistic effect might result in the improved efficacy of these agents in the treatment of cancer and/or the reduction of any adverse or unwanted side effects associated with the use of either agent alone.

Examples of cancer agents or chemotherapeutic agents for use in combination with the compounds of the present invention can be found in Cancer Principle and Practise of Oncology by V.T. Devita and S. Hellman (editors), 6th edition (February 15, 2001), Lippincott Williams & Wilkins Publishers. A person of ordinary skill in the art would be able to discern which combinations of agents would be useful based on the particular characteristics of the drugs and the cancer involved. Such anti-cancer agents include, but are not limited to, the following: HDAC inhibitors, estrogen receptor modulators, androgen receptor modulators, retinoid receptor modulators, cytotoxic/cytostatic agents, antiproliferative agents, prenyl-protein transferase inhibitors, HMG-COA reductase inhibitors, HIV protease inhibitors, reverse transcriptase inhibitors and other angiogenesis inhibitors, inhibitors of cell proliferation and survival signaling, apoptosis inducing agents and agents that interfere with cell cycle checkpoints. The instant compounds are particularly useful when co-administered with radiation therapy.

Examples of "HDAC inhibitors" include suberoylanilide hydroxamic acid (SAHA), LAQ824, LBH589, PXD101, MS275, FK228, valproic acid, butyric acid and CI-994.

"Estrogen receptor modulators" refers to compounds that interfere with or inhibit the binding of estrogen to the receptor, regardless of mechanism. Examples of estrogen receptor modulators include, but are not limited to, tamoxifen, raloxifene, idoxifene, LY353381, LY117081, toremifene, fulvestrant, 4-[7-(2,2-dimethyl-1-oxopropoxy-4-methyl-2-[4-[2-(1-piperidinyl)ethoxy]phenyl]-2*H-*1-benzopyran-3-yl]-phenyl-2,2-dimethylpropanoate, 4,4'-dihydroxybenzophenone-2,4-dinitrophenyl-hydrazone, and SH646.

"Androgen receptor modulators" refers to compounds which interfere or inhibit the binding of androgens to the receptor, regardless of mechanism. Examples of androgen receptor modulators include finasteride and other 5a-reductase inhibitors, nilutamide, flutamide, bicalutamide, liarozole, and abiraterone acetate.

"Retinoid receptor modulators" refers to compounds which interfere or inhibit the binding of retinoids to the receptor, regardless of mechanism. Examples of such retinoid receptor modulators include bexarotene, tretinoin, 13-cis-retinoic acid, 9-cis-retinoic acid, a-difluoromethylomithine, ILX23-7553, trans-*N-*(4'-hydroxyphenyl) retinamide, and *N*-4-carboxyphenyl retinamide.

"Cytotoxic/cytostatic agents" refer to compounds which cause cell death or inhibit cell proliferation primarily by interfering directly with the cell's functioning or inhibit or interfere with cell mytosis, including alkylating agents, tumor necrosis factors, intercalators, hypoxia activatable compounds, microtubule inhibitors/microtubule-stabilizing agents, inhibitors of mitotic kinesins, inhibitors of kinases involved in mitotic progression, antimetabolites, biological response modifiers; hormonal/anti-hormonal therapeutic agents, haematopoietic growth factors, monoclonal antibody targeted therapeutic agents, topoisomerase inhibitors, proteasome inhibitors and ubiquitin ligase inhibitors.

Examples of cytotoxic agents include, but are not limited to, cyclophosphamide, chlorambucil carmustine (BCNU), lomustine (CCNU), busulfan, treosulfan, sertenef, cachectin, ifosfamide, tasonermin, lonidamine, carboplatin, altretamine, prednimustine, dibromodulcitol, ranimustine, fotemustine, nedaplatin, aroplatin, oxaliplatin, temozolomide, methyl methanesulfonate, procarbazine , dacarbazine, heptaplatin, estramustine, improsulfan tosilate, trofosfamide, nimustine, dibrospidium chloride, pumitepa, lobaplatin, satraplatin, profiromycin, cisplatin, irofulven, dexifosfamide, cis-aminedichloro(2-methyl-pyridine)platinum, benzylguanine, glufosfamide, GPX100, (trans, trans, trans)-bis-mu-(hexane-1,6-diamine)-mu-[diamine-platinum(II)]bis[diamine(chloro)platinum (II)]tetrachloride, diarizidinylspermine, arsenic trioxide, 1-(11-dodecylamino-10-hydroxyundecyl)-3,7-dimethylxanthine, zorubicin, idarubicin, daunorubicin, bisantrene, mitoxantrone, pirarubicin, pinafide, valrubicin, amrubicin, doxorubicin, epirubicin, pirarubicin, antineoplaston, 3'-deamino-3'-morpholino-13-deoxo-10-hydroxycarminomycin, annamycin, galarubicin, elinafide, MEN10755 and 4-demethoxy-3-deamino-3-aziridinyl-4-methylsulphonyl-daunorubicin (see WO 00/50032).

In an embodiment the compounds of this invention can be used in combination with alkylating agents.

Examples of alkylating agents include but are not limited to, nitrogen mustards: cyclophosphamide, ifosfamide, trofosfamide and chlorambucil; nitrosoureas: carmustine (BCNU) and lomustine (CCNU); alkylsulphonates: busulfan and treosulfan; triazenes: dacarbazine, procarbazine and temozolomide; platinum containing complexes: cisplatin, carboplatin, aroplatin and oxaliplatin.

In an embodiment, the alkylating agent is dacarbazine. Dacarbazine can be administered to a subject at dosages ranging from about 150 mg/m2 (of a subject's body surface area) to about 250 mg/m2. In another embodiment, dacarbazine is administered intravenously to a subject once per day for five consecutive days at a dose ranging from about 150 mg/m2 to about 250 mg/m2.

In an embodiment, the alkylating agent is procarbazine. Procarbazine can be administered to a subject at dosages ranging from about 50 mg/m2 (of a subject's body surface area) to about 100mg/m2. In another embodiment, procarbazine is administered intravenously to a subject once per day for five consecutive days at a dose ranging from about 50 mg/m2 to about 100 mg/m2.

In an embodiment, the alkylating agent is temozoloamide. Temozolomide can be administered to a subject at dosages ranging from about about 150 mg/m2 (of a subject's body surface area) to about 200 mg/m2. In another embodiment, temozolomide is administered orally to an animal once per day for five consecutive days at a dose ranging from about 150 mg/m2 to about 200 mg/m2.

Examples of anti-mitotic agents include: allocolchicine, halichondrin B, colchicine, colchicine derivative, dolstatin 10, maytansine, rhizoxin, thiocolchicine and trityl cysteine.

An example of a hypoxia activatable compound is tirapazamine.

Examples of proteasome inhibitors include but are not limited to lactacystin, bortezomib, epoxomicin and peptide aldehydes such as MG 132, MG 115 and PSI.

Examples of microtubule inhibitors/microtubule-stabilising agents include paclitaxel, vindesine sulfate, vincristine, vinblastine, vinorelbine, 3',4'-didehydro-4'-deoxy-8'-norvincaleukoblastine, docetaxol, rhizoxin, dolastatin, mivobulin isethionate, auristatin, cemadotin, RPR109881, BMS184476, vinflunine, cryptophycin, 2,3,4,5,6-pentafluoro-*N-*(3-fluoro-4-methoxyphenyl) benzene sulfonamide, anhydrovinblastine, *N*,*N-*dimethyl-L-valyl-L-valyl-N-methyl-L-valyl-L-prolyl-L-proline-t-butylamide, TDX258, the epothilones (see for example U.S. Pat. Nos. 6,284,781 and 6,288,237) and BMS188797.

Some examples of topoisomerase inhibitors are topotecan, hycaptamine, irinotecan, rubitecan, exatecan, gimetecan, diflomotecan, silyl-camptothecins, 9-aminocamptothecin, camptothecin, crisnatol, mitomycin C, 6-ethoxypropionyl-3',4'-O-exo-benzylidene-chartreusin, 9-methoxy-*N*,*N*-dimethyl-5-nitropyrazolo[3,4,5-kl]acridine-2-(6*H*) propanamine, 1-amino-9-ethyl-5-fluoro-2,3-dihydro-9-hydroxy-4-methyl-1*H*,12*H*-benzo[de]pyrano[3',4':b,7]-indolizino[1,2b]quinoline-10,13(9*H*,15*H*)dione, lurtotecan, 7-[2-(*N*-isopropylamino)ethyl]-(20S)camptothecin, BNP1350, BNPI1100, BN80915, BN80942, etoposide phosphate, teniposide, sobuzoxane, 2'-dimethylamino-2'-deoxy-etoposide, GL331, *N*-[2-(dimethylamino)ethyl]-9-hydroxy-5,6-dimethyl-6*H-*pyrido[4,3-b]carbazole-1-carboxamide, asulacrine, (5a, 5aB, 8aa,9b)-9-[2-[*N-*[2-(dimethylamino)ethyl]-*N*-methylamino]ethyl]-5-[4-hydroxy-3,5-dimethoxyphenyl]-5,5a,6,8,8a,9-hexohydrofuro(3',4':6,7)naphtho(2,3-d)-1,3-dioxol-6-one, 2,3-(methylenedioxy)-5-methyl-7-hydroxy-8-methoxybenzo[c]-phenanthridinium, 6,9-bis[(2-aminoethyl)amino]benzo[g]isoguinoline-5,10-dione, 5-(3-aminopropylamino)-7,10-dihydroxy-2-(2-hydroxyethylaminomethyl)-6*H-*pyrazolo[4,5,1-de]acridin-6-one, *N*-[1-[2(diethylamino)ethylamino]-7-methoxy-9-oxo-9H-thioxanthen-4-ylmethyl]formamide, *N*-(2-(dimethylamino)ethyl)acridine-4-carboxamide, 6-[[2-(dimethylamino)ethyl]amino]-3-hydroxy-7*H*-indeno[2,1-c]quinolin-7-one, and dimesna; non-camptothecin topoisomerase-1 inhibitors such as indolocarbazoles; and dual topoisomerase-1 and II inhibitors such as benzophenazines, XR 20 115761MLN 576 and benzopyridoindoles.

In an embodiment, the topoisomerase inhibitor is irinotecan. Irinotecan can be administered to a subject at dosages ranging from about about 50 mg/m2 (of a subject's body surface area) to about 150 mg/m2. In another embodiment, irinotecan is administered intravenously to a subject once per day for five consecutive days at a dose ranging from about 50mg/m2 to about 150mg/m2 on days 1-5, then again intravenously once per day for five consecutive days on days 28-32 at a dose ranging from about 50mg/m2 to about 150mg/m2, then again intravenously once per day for five consecutive days on days 55-59 at a dose ranging from about 50mg/m2 to about 150mg/m2.

Examples of inhibitors of mitotic kinesins, and in particular the human mitotic kinesin KSP, are described in PCT Publications WO 01/30768, WO 01/98278, WO 02/056880, WO 03/050,064, WO 03/050,122, WO 03/049,527, WO 03/049,679, WO 03/049,678, WO 03/039460 , WO 03/079973, WO 03/099211, WO 2004/039774, WO 03/105855, WO 03/106417, WO 2004/087050, WO 2004/058700, WO 2004/058148 and WO 2004/037171 and US applications US 2004/132830 and US 2004/132719. In an embodiment inhibitors of mitotic kinesins include, but are not limited to inhibitors of KSP, inhibitors of MKLP1, inhibitors of CENP-E, inhibitors of MCAK, inhibitors of Kif14, inhibitors of Mphosphl and inhibitors of Rab6-KIFL.

"Inhibitors of kinases involved in mitotic progression" include, but are not limited to, inhibitors of aurora kinase, inhibitors of Polo-like kinases (PLK) (in particular inhibitors of PLK-1), inhibitors of bub-1 and inhibitors of bub-R1.

"Antiproliferative agents" includes antisense RNA and DNA oligonucleotides such as G3139, ODN698, RVASKRAS, GEM231, and INX3001, and antimetabolites such as enocitabine, carmofur, tegafur, pentostatin, doxifluridine, trimetrexate, fludarabine, capecitabine, galocitabine, cytarabine ocfosfate, fosteabine sodium hydrate, raltitrexed, paltitrexid, emitefur, tiazofurin, decitabine, nolatrexed, pemetrexed, nelzarabine, 2'-deoxy-2'-methylidenecytidine, 2'-fluoromethylene-2'-deoxycytidine, N-[5-(2,3-dihydro-benzofuryl)sulfonyl]-*N*'-(3,4-dichlorophenyl)urea, *N*6-[4-deoxy-4-[*N*2-[2(E),4(E)-tetradecadienoyl]glycylamino]-L-glycero-B-L-manno-heptopyranosyl]adenine, aplidine, ecteinascidin, troxacitabine, 4-[2-amino-4-oxo-4,6,7,8-tetrahydro-3*H-*pyrimidino[5,4-b][1,4]thiazin-6-yl-(S)-ethyl]-2,5-thienoyl-L-glutamic acid, aminopterin, 5-flurouracil, alanosine, 11-acetyl-8-(carbamoyloxymethyl)-4-formyl-6-methoxy-14-oxa-1,11-diazatetracyclo(7.4.1.0.0)-tetradeca-2,4,6-trien-9-yl acetic acid ester, swainsonine, lometrexol, dexrazoxane, methioninase, 2'-cyano-2'-deoxy-*N*-palmitoyl-1-B-D-arabino furanosyl cytosine and 3-aminopyridine-2-carboxaldehyde thiosemicarbazone.

Examples of monoclonal antibody targeted therapeutic agents include those therapeutic agents which have cytotoxic agents or radioisotopes attached to a cancer cell specific or target cell specific monoclonal antibody. Examples include Bexxar.

"HMG-CoA reductase inhibitors" refers to inhibitors of 3-hydroxy-3-methylglutaryl-CoA reductase. Examples of HMG-CoA reductase inhibitors that may be used include but are not limited to lovastatin (MEVACOR^{®}; see U.S. Pat. Nos. 4,231,938, 4,294,926 and 4,319,039), simvastatin (ZOCOR^{®}; see U.S. Pat. Nos. 4,444,784, 4,820,850 and 4,916,239), pravastatin (PRAVACHOL^{®}; see U.S. Pat. Nos. 4,346,227, 4,537,859, 4,410,629, 5,030,447 and 5,180,589), fluvastatin (LESCOL^{®}; see U.S. Pat. Nos. 5,354,772, 4,911,165, 4,929,437, 5,189,164, 5,118,853, 5,290,946 and 5,356,896) and atorvastatin (LIPITOR^{®}; see U.S. Pat. Nos. 5,273,995, 4,681,893, 5,489,691 and 5,342,952). The structural formulas of these and additional HMG-CoA reductase inhibitors that may be used in the instant methods are described at page 87 of M. Yalpani, "Cholesterol Lowering Drugs", Chemistry & Industry, pp. 85-89 (5 February 1996) and US Patent Nos. 4,782,084 and 4,885,314. The term HMG-CoA reductase inhibitor as used herein includes all pharmaceutically acceptable lactone and open-acid forms (i.e., where the lactone ring is opened to form the free acid) as well as salt and ester forms of compounds which have HMG-CoA reductase inhibitory activity, and therefore the use of such salts, esters, open-acid and lactone forms is included within the scope of this invention.

"Prenyl-protein transferase inhibitor" refers to a compound which inhibits any one or any combination of the prenyl-protein transferase enzymes, including farnesyl-protein transferase (FPTase), geranylgeranyl-protein transferase type I (GGPTase-I), and geranylgeranyl-protein transferase type-11 (GGPTase-II, also called Rab GGPTase).

Examples of prenyl-protein transferase inhibitors can be found in the following publications and patents: WO 96/30343, WO 97/18813, WO 97/21701, WO 97/23478, WO 97/38665, WO 98/28980, WO 98/29119, WO 95/32987, U.S. Pat. No. 5,420,245, U.S. Pat. No. 5,523,430, U.S. Pat. No. 5,532,359, U.S. Pat. No. 5,510,510, U.S. Pat. No. 5,589,485, U.S. Pat. No. 5,602,098, European Patent Publ. 0 618 221, European Patent Publ. 0 675 112, European Patent Publ. 0 604 181, European Patent Publ. 0 696 593, WO 94/19357, WO 95/08542, WO 95/11917, WO 95/12612, WO 95/12572, WO 95/10514, U.S. Pat. No. 5,661,152, WO 95/10515, WO 95/10516, WO 95/24612, WO 95/34535, WO 95/25086, WO 96/05529, WO 96/06138, WO 96/06193, WO 96/16443, WO 96/21701, WO 96/21456, WO 96/22278, WO 96/24611, WO 96/24612, WO 96/05168, WO 96/05169, WO 96/00736, U.S. Pat. No. 5,571,792, WO 96/17861, WO 96/33159, WO 96/34850, WO 96/34851, WO 96/30017, WO 96/30018, WO 96/30362, WO 96/30363, WO 96/31111, WO 96/31477, WO 96/31478, WO 96/31501, WO 97/00252, WO 97/03047, WO 97/03050, WO 97/04785, WO 97/02920, WO 97/17070, WO 97/23478, WO 97/26246, WO 97/30053, WO 97/44350, WO 98/02436, and U.S. Pat. No. 5,532,359. For an example of the role of a prenyl-protein transferase inhibitor on angiogenesis see European J. of Cancer (1999), 35(9):1394-1401.

"Angiogenesis inhibitors" refers to compounds that inhibit the formation of new blood vessels, regardless of mechanism. Examples of angiogenesis inhibitors include, but are not limited to, tyrosine kinase inhibitors, such as inhibitors of the tyrosine kinase receptors Flt-1 (VEGFR1) and Flk-1/KDR (VEGFR2), inhibitors of epidermal-derived, fibroblast-derived, or platelet derived growth factors, MMP (matrix metalloprotease) inhibitors, integrin blockers, interferon-α, interleukin-12, pentosan polysulfate, cyclooxygenase inhibitors, including nonsteroidal anti-inflammatories (NSAIDs) like aspirin and ibuprofen as well as selective cyclooxy-genase-2 inhibitors like celecoxib and rofecoxib (PNAS (1992) 89:7384; JNCI (1982) 69:475; Arch. Opthalmol. (1990) 108:573; Anat. Rec. (1994) 238:68; FEBS Letters (1995) 372:83; Clin, Orthop.(1995) 313:76; J. Mol. Endocrinol. (1996) 16:107; Jpn. J. Pharmacol. (1997) 75:105; Cancer- Res.(1997) 57:1625 (1997); Cell (1998) 93:705; Intl. J. Mol. Med. (1998) 2:715; J. Biol. Chem. (1999) 274:9116)), steroidal anti-inflammatories (such as corticosteroids, mineralocorticoids, dexamethasone, prednisone, prednisolone, methylpred, betamethasone), carboxyamidotriazole, combretastatin A-4, squalamine, 6-O-chloroacetyl-carbonyl)-fumagillol, thalidomide, angiostatin, troponin-1, angiotensin II antagonists (see J. Lab. Clin. Med. (1985) 105:141-145), and antibodies to VEGF (see Nature Biotechnology (1999) 17:963-968; Kim et al (1993) Nature 362:841-844; WO 00/44777; and WO 00/61186).

Other therapeutic agents that modulate or inhibit angiogenesis and may also be used in combination with the compounds of the instant invention include agents that modulate or inhibit the coagulation and fibrinolysis systems (see review in Clin. Chem. La. Med. (2000) 38:679-692). Examples of such agents that modulate or inhibit the coagulation and fibrinolysis pathways include, but are not limited to, heparin (see Throb. Haemost. (1998) 80:10-23), low molecular weight heparins and carboxypeptidase U inhibitors (also known as inhibitors of active thrombin activatable fibrinolysis inhibitor [TAFIa]) (see Thrombosis Res. (2001) 101:329-354). TAFIa inhibitors have been described in PCT Publication WO 03/013,526 and U,S, Ser. No. 60/349,925 (filed January 18, 2002).

"Agents that interfere with cell cycle checkpoints" refer to compounds that inhibit protein kinases that transduce cell cycle checkpoint signals, thereby sensitizing the cancer cell to DNA damaging agents. Such agents include inhibitors of ATR, ATM, the Chk1 and Chk2 kinases and cdk and cdc kinase inhibitors and are specifically exemplified by 7-hydroxystaurosporin, staurosporin, flavopiridol, CYC202 (Cyclacel) and BMS-387032.

"Inhibitors of cell proliferation and survival signaling pathway" refer to pharmaceutical agents that inhibit cell surface receptors and signal transduction cascades downstream of those surface receptors. Such agents include inhibitors of inhibitors of EGFR (for example gefitinib and erlotinib), inhibitors of ERB-2 (for example trastuzumab), inhibitors of IGFR (for example those disclosed in WO 03/059951), inhibitors of cytokine receptors, inhibitors of MET, inhibitors of PI3K (for example LY294002), serine/threonine kinases (including but not limited to inhibitors of Akt such as described in (WO 03/086404, WO 03/086403, WO 03/086394, WO 03/086279, WO 02/083675, WO 02/083139, WO 02/083140 and WO 02/083138), inhibitors of Raf kinase (for example BAY-43-9006), inhibitors of MEK (for example CI-1040 and PD-098059) and inhibitors of mTOR (for example Wyeth CCI-779 and Ariad AP23573). Such agents include small molecule inhibitor compounds and antibody antagonists.

"Apoptosis inducing agents" include activators of TNF receptor family members (including the TRAIL receptors).

The invention also encompasses combinations with NSAID's which are selective COX-2 inhibitors. For purposes of this specification NSAID's which are selective inhibitors of COX-2 are defined as those which possess a specificity for inhibiting COX-2 over COX-1 of at least 100 fold as measured by the ratio of IC₅₀ for COX-2 over IC₅₀ for COX-1 evaluated by cell or microsomal assays. Such compounds include, but are not limited to those disclosed in U.S. Pat. 5,474,995, U.S. Pat. 5,861,419, U.S. Pat. 6,001,843, U.S. Pat. 6,020,343, U.S. Pat. 5,409,944, U.S. Pat. 5,436,265, U.S. Pat. 5,536,752, U.S. Pat. 5,550,142, U.S. Pat. 5,604,260, U.S. 5,698,584, U.S. Pat. 5,710,140, WO 94/15932, U.S. Pat. 5,344,991, U.S. Pat. 5,134,142, U.S. Pat. 5,380,738, U.S. Pat. 5,393,790, U.S. Pat. 5,466,823, U.S. Pat. 5,633,272, and U.S. Pat. 5,932,598, all of which are hereby incorporated by reference.

Inhibitors of COX-2 that are particularly useful in the instant method of treatment are 5-chloro-3-(4-methylsulfonyl)phenyl-2-(2-methyl-5-pyridinyl)pyridine; or a pharmaceutically acceptable salt thereof.

Compounds that have been described as specific inhibitors of COX-2 and are therefore useful in the present invention include, but are not limited to: parecoxib, CELEBREX^{®} and B EXTRA^{®} or a pharmaceutically acceptable salt thereof.

Other examples of angiogenesis inhibitors include, but are not limited to, endostatin, ukrain, ranpirnase, IM862, 5-methoxy-4-[2-methyl-3-(3-methyl-2-butenyl)oxiranyl]-1-oxaspiro[2,5]oct-6-yl(chloroacetyl)carbamate, acetyldinanaline, 5-amino-1-[[3,5-dichloro-4-(4-chlorobenzoyl)phenyl]methyl]-1*H*-1,2,3-triazole-4-carboxamide,CM101, squalamine, combretastatin, RPI4610, NX31838, sulfated mannopentaose phosphate, 7,7-(carbonyl-bis[imino-*N-*methyl-4,2-pyrrolocarbonylimino[*N-*methyl-4,2-pyrrole]-carbonylimino]-bis-(1,3-naphthalene disulfonate), and 3-[(2,4-dimethylpyrrol-5-yl)methylene]-2-indolinone (SU5416).

As used above, "integrin blockers" refers to compounds which selectively antagonize, inhibit or counteract binding of a physiological ligand to the αᵥβ₃ integrin, to compounds which selectively antagonize, inhibit or counteract binding of a physiological ligand to the αvβ₅ integrin, to compounds which antagonize, inhibit or counteract binding of a physiological ligand to both the αᵥβ₃ integrin and the αᵥβ₅ integrin, and to compounds which antagonize, inhibit or counteract the activity of the particular integrin(s) expressed on capillary endothelial cells. The term also refers to antagonists of the αᵥβ₆ αᵥβ₈, α₁β₁, α₁β₁, α₅β₁, α₆β₁ and α₆β₄ integrins. The term also refers to antagonists of any combination of αᵥβ₃, αᵥβ₅, αᵥβ₆, αᵥβ₈, α₁β₁, α₂β₁, β5α₁, α₆β₁ and α₆β₄ integrins.

Some specific examples of tyrosine kinase inhibitors include *N*-(trifluoromethylphenyl)-5-methylisoxazol-4-carboxamide, 3-[(2,4-dimethylpyrrol-5-yl)methylidenyl)indolin-2-one, 17-(allylamino)-17-demethoxygeldanamycin, 4-(3-chloro-4-fluorophenylamino)-7-methoxy-6-[3-(4-morpholinyl)propoxyl]quinazoline, *N*-(3-ethynylphenyl)-6,7-bis(2-methoxyethoxy)-4-quinazolinamine, BIBX1382, 2,3,9,10,11,12-hexahydro-10-(hydroxymethyl)-10-hydroxy-9-methyl-9,12-epoxy-1*H-*diindolo[l,2,3-fg:3',2',r-kl]pyrrolo[3,4-i][l,6]benzodiazocin-l-one, SH268, genistein, STI571, CEP2563, 4-(3-chlorophenylamino)-5,6-dimethyl-7*H*-pyrrolo[2,3-d]pyrimidinemethane sulfonate, 4-(3-bromo-4-hydroxyphenyl)amino-6,7-dimethoxyquinazoline, 4-(4'-hydroxyphenyl)amino-6,7-dimethoxyquinazoline, SU6668, STI571A, *N*-4-chlorophenyl-4-(4-pyridylmethyl)-1-phthalazinamine, and EMD121974.

In an embodiment, the compounds of the present invention are useful for the treatment or prevention of the appearance of necrosis induced by selective *N*3-adenine methylating agents such as MeOSO₂(CH₂)-lexitropsin (Me-Lex).

Combinations with compounds other than anti-cancer compounds are also encompassed in the instant methods. For example, combinations of the instantly claimed compounds with PPAR-γ (i.e., PPAR-gamma) agonists and PPAR-δ (i.e., PPAR-delta) agonists are useful in the treatment of certain malingnancies. PPAR-γ and PPAR-δ are the nuclear peroxisome proliferator-activated receptors γ and δ. The expression of PPAR-γ on endothelial cells and its involvement in angiogenesis has been reported in the literature (see J. Cardiovasc. Pharmacol. (1998) 31:909-913; J. Biol. Chem. (1999) 274:9116-9121; Invest. Ophthalmol Vis. Sci. (2000) 41:2309-2317). More recently, PPAR-γ agonists have been shown to inhibit the angiogenic response to VEGF in vitro; both troglitazone and rosiglitazone maleate inhibit the development of retinal neovascularization in mice. *(*Arch. Ophthamol. (2001) 119:709-717). Examples of PPAR-γ agonists and PPAR- γ/α agonists include, but are not limited to, thiazolidinediones (such as DRF2725, CS-011, troglitazone, rosiglitazone, and pioglitazone), fenofibrate, gemfibrozil, clofibrate, GW2570, SB219994, AR-H039242, JTT-501, MCC-555, GW2331, GW409544, NN2344, KRP297, NP0110, DRF4158, NN622, GI262570, PNU182716, DRF552926, 2-[(5,7-dipropyl-3-trifluoromethyl-1,2-benzisoxazol-6-yl)oxy]-2-methylpropionic acid (disclosed in USSN 09/782,856), and 2(R)-7-(3-(2-chloro-4-(4-fluorophenoxy) phenoxy)propoxy)-2-ethylchromane-2-carboxylic acid (disclosed in USSN 60/235,708 and 60/244,697).

Another embodiment of the instant invention is the use of the presently disclosed compounds in combination with anti-viral agents (such as nucleoside analogs including ganciclovir for the treatment of cancer. See WO 98/04290.

Another embodiment of the instant invention is the use of the presently disclosed compounds in combination with gene therapy for the treatment of cancer. For an overview of genetic strategies to treating cancer see Hall et al (Am J Hum Genet (1997) 61:785-789) and Kufe et al (Cancer Medicine, 5th Ed, pp 876-889, BC Decker, Hamilton 2000). Gene therapy can be used to deliver any tumor suppressing gene. Examples of such genes include, but are not limited to, p53, which can be delivered via recombinant virus-mediated gene transfer (see U.S. Pat. No. 6,069,134, for example), a uPA/uPAR antagonist ("Adenovirus-Mediated Delivery of a uPA/uPAR Antagonist Suppresses Angiogenesis-Dependent Tumor Growth and Dissemination in Mice," Gene Therapy, August (1998) 5(8):1105-13), and interferon gamma (J Immunol (2000) 164:217-222).

The compounds of the instant invention may also be administered in combination with an inhibitor of inherent multidrug resistance (MDR), in particular MDR associated with high levels of expression of transporter proteins. Such MDR inhibitors include inhibitors of p-glycoprotein (P-gp), such as LY335979, XR9576, OC144-093, R101922, VX853, verapamil and PSC833 (valspodar).

A compound of the present invention may be employed in conjunction with anti-emetic agents to treat nausea or emesis, including acute, delayed, late-phase, and anticipatory emesis, which may result from the use of a compound of the present invention, alone or with radiation therapy. For the prevention or treatment of emesis, a compound of the present invention may be used in conjunction with other anti-emetic agents, especially neurokinin-1 receptor antagonists, 5HT3 receptor antagonists, such as ondansetron, granisetron, tropisetron, and zatisetron, GABA_{B} receptor agonists, such as baclofen, a corticosteroid such as Decadron (dexamethasone), Kenalog, Aristocort, Nasalide, Preferid, Benecorten or others such as disclosed in U.S.Patent Nos. 2,789,118, 2,990,401, 3,048,581, 3,126,375, 3,929,768, 3,996,359, 3,928,326 and 3,749,712, an antidopaminergic, such as the phenothiazines (for example prochlorperazine, fluphenazine, thioridazine and mesoridazine), metoclopramide or dronabinol. In an embodiment, an anti-emesis agent selected from a neurokinin-1 receptor antagonist, a 5HT3 receptor antagonist and a corticosteroid is administered as an adjuvant for the treatment or prevention of emesis that may result upon administration of the instant compounds.

Neurokinin-1 receptor antagonists of use in conjunction with the compounds of the present invention are fully described, for example, in U.S. Pat. Nos. 5,162,339, 5,232,929, 5,242,930, 5,373,003, 5,387,595, 5,459,270, 5,494,926, 5,496,833, 5,637,699, 5,719,147; European Patent Publication Nos. EP 0 360 390, 0 394 989, 0 428 434, 0 429 366, 0 430 771, 0 436 334, 0 443 132, 0 482 539, 0 498 069, 0 499 313, 0 512 901, 0 512 902, 0 514 273, 0 514 274, 0 514 275, 0 514 276, 0 515 681, 0 517 589, 0 520 555, 0 522 808, 0 528 495, 0 532 456, 0 533 280, 0 536 817, 0 545 478, 0 558 156, 0 577 394, 0 585 913,0 590 152, 0 599 538, 0 610 793, 0 634 402, 0 686 629, 0 693 489, 0 694 535, 0 699 655, 0 699 674, 0 707 006, 0 708 101, 0 709 375, 0 709 376, 0 714 891, 0 723 959, 0 733 632 and 0 776 893; PCT International Patent Publication Nos. WO 90/05525, 90/05729, 91/09844, 91/18899, 92/01688, 92/06079, 92/12151, 92/15585, 92/17449, 92/20661, 92/20676, 92/21677, 92/22569, 93/00330, 93/00331, 93/01159, 93/01165, 93/01169, 93/01170, 93/06099, 93/09116, 93/10073, 93/14084, 93/14113, 93/18023, 93/19064, 93/21155, 93/21181, 93/23380, 93/24465, 94/00440, 94/01402, 94/02461, 94/02595, 94/03429, 94/03445, 94/04494, 94/04496, 94/05625, 94/07843, 94/08997, 94/10165, 94/10167, 94/10168, 94/10170, 94/11368, 94/13639, 94/13663, 94/14767, 94/15903, 94/19320, 94/19323, 94/20500, 94/26735, 94/26740, 94/29309, 95/02595, 95/04040, 95/04042, 95/06645, 95/07886, 95/07908, 95/08549, 95/11880, 95/14017, 95/15311, 95/16679, 95/17382, 95/18124, 95/18129, 95/19344, 95/20575, 95/21819, 95/22525, 95/23798, 95/26338, 95/28418, 95/30674, 95/30687, 95/33744, 96/05181, 96/05193, 96/05203, 96/06094, 96/07649, 96/10562, 96/16939, 96/18643, 96/20197, 96/21661, 96/29304, 96/29317, 96/29326, 96/29328, 96/31214, 96/32385, 96/37489, 97/01553, 97/01554, 97/03066, 97/08144, 97/14671, 97/17362, 97/18206, 97/19084, 97/19942 and 97/21702; and in British Patent Publication Nos. 2 266 529, 2 268 931, 2 269 170, 2 269 590, 2 271 774, 2 292 144, 2 293 168, 2 293 169, and 2 302 689. The preparation of such compounds is fully described in the aforementioned patents and publications, which are incorporated herein by reference.

In an embodiment, the neurokinin-1 receptor antagonist for use in conjunction with the compounds of the present invention is selected from: 2-(*R*)-(1-(*R*)-(3,5-bis(trifluoromethyl)phenyl)ethoxy)-3-(S)-(4-fluorophenyl)-4-(3-(5-oxo-1*H*,4*H*-1,2,4-triazolo)methyl)morpholine, or a pharmaceutically acceptable salt thereof, which is described in U.S. Pat. No. 5,719,147.

A compound of the instant invention may also be administered with an agent useful in the treatment of anemia. Such an anemia treatment agent is, for example, a continuous eythropoiesis receptor activator (such as epoetin alfa).

A compound of the instant invention may also be administered with an agent useful in the treatment of neutropenia. Such a neutropenia treatment agent is, for example, a hematopoietic growth factor which regulates the production and function of neutrophils such as a human granulocyte colony stimulating factor, (G-CSF). Examples of a G-CSF include filgrastim.

A compound of the instant invention may also be administered with an immunologic-enhancing drug, such as levamisole, isoprinosine and Zadaxin.

A compound of the instant invention may also be useful for treating or preventing cancer, including bone cancer, in combination with bisphosphonates (understood to include bisphosphonates, diphosphonates, bisphosphonic acids and diphosphonic acids). Examples of bisphosphonates include but are not limited to: etidronate (Didronel), pamidronate (Aredia), alendronate (Fosamax), risedronate (Actonel), zoledronate (Zometa), ibandronate (Boniva), incadronate or cimadronate, clodronate, EB-1053, minodronate, neridronate, piridronate and tiludronate including any and all pharmaceutically acceptable salts, derivatives, hydrates and mixtures thereof.

Thus, the scope of the instant invention encompasses the use of the instantly claimed compounds in combination with ionizing radiation and/or in combination with a second compound selected from: HDAC inhibitors, an estrogen receptor modulator, an androgen receptor modulator, retinoid receptor modulator, a cytotoxic/cytostatic agent, an antiproliferative agent, a prenyl-protein transferase inhibitor, an HMG-CoA reductase inhibitor, an angiogenesis inhibitor, a PPAR-γ agonist, a PPAR-δ agonist, an anti-viral agent, an inhibitor of inherent multidrug resistance, an anti-emetic agent, an agent useful in the treatment of anemia, an agent useful in the treatment of neutropenia, an immunologic-enhancing drug, an inhibitor of cell proliferation and survival signaling, an agent that interfers with a cell cycle checkpoint, an apoptosis inducing agent and a bisphosphonate.

The term "administration" and variants thereof (e.g., "administering" a compound) in reference to a compound of the invention means introducing the compound or a prodrug of the compound into the system of the animal in need of treatment. When a compound of the invention or prodrug thereof is provided in combination with one or more other active agents (e.g., a cytotoxic agent, etc.), "administration" and its variants are each understood to include concurrent and sequential introduction of the compound or prodrug thereof and other agents.

As used herein, the term "composition" is intended to encompass a product comprising the specified ingredients in the specified amounts, as well as any product which results, directly or indirectly, from combination of the specified ingredients in the specified amounts.

The term "therapeutically effective amount" as used herein means that amount of active compound or pharmaceutical agent that elicits the biological or medicinal response in a tissue, system, animal or human that is being sought by a researcher, veterinarian, medical doctor or other clinician.

The term "treatment" refers to the treatment of a mammal afflicted with a pathological condition and refers to an effect that alleviates the condition by killing the cancerous cells, but also to an effect that results in the inhibition of the progress of the condition, and includes a reduction in the rate of progress, a halt in the rate of progress, amelioration of the condition, and cure of the condition. Treatment as a prophylactic measure (i.e. prophylaxis) is also included.

The term "pharmaceutically acceptable" as used herein pertains to compounds, materials, compositions, and/or dosage forms which are, within the scope of sound medical judgement, suitable for use in contact with the tissues of a subject (e.g. human) without excessive toxicity, irritation, allergic response, or other problem or complication, commensurate with a reasonable benefit/risk ratio. Each carrier, excipient, etc. must also be "acceptable" in the sense of being compatible with the other ingredients of the formulation.

The term "adjunct" refers to the use of compounds in conjunction with known therapeutic means. Such means include cytotoxic regimes of drugs and/or ionising radiation as used in the treatment of different cancer types. In particular, the active compounds are known to potentiate the actions of a number of cancer chemotherapy treatments, which include the topoisomerase class of poisons (e. g. topotecan, irinotecan, rubitecan), most of the known alkylating agents (e. g. DTIC, temozolamide) and platinum based drugs (e. g. carboplatin, cisplatin) used in treating cancer.

Also described is a method of treating cancer that comprises administering a therapeutically effective amount of a compound of Formula I in combination with radiation therapy and/or in combination with a compound selected from: HDAC inhibitors, an estrogen receptor modulator, an androgen receptor modulator, retinoid receptor modulator, a cytotoxic/cytostatic agent, an antiproliferative agent, a prenyl-protein transferase inhibitor, an HMG-CoA reductase inhibitor, an angiogenesis inhibitor, a PPAR-γ agonist, a PAR-δ agonist, an anti-viral agent, an inhibitor of inherent multidrug resistance, an anti-emetic agent, an agent useful in the treatment of anemia, an agent useful in the treatment of neutropenia, an immunologic-enhancing drug, an inhibitor of cell proliferation and survival signaling, an agent that interfers with a cell cycle checkpoint, an apoptosis inducing agent and a bisphosphonate.

These and other aspects of the invention will be apparent from the teachings contained herein.

### Abbreviations used in the description of the chemistry and in the Examples that follow are:

AcC1 (acetyl chloride); (BzO)₂ (benzoyl peroxide); Cbz-C1 (benzylchloroformate); DCM (dichloromethane); DMF (dimethylformamide); DMSO (dimethyl sulfoxide); eq. (equivalent); ES (electrospray); EtOAc (ethyl acetate); EtOH (ethanol); mol. sieves (molecular sieves); HATU [*O*-(7-azabenzotriazol-1-yl)-*N*,*N,N*',*N*'-tetramethyluronium hexafluoro-phosphate]; IBX (1-hydroxy-1 λ³,2-benziodoxol-3(1*H*)-one 1-oxide); MeCN (acetonitrile); MeOH (methanol); MS (mass spectrometry); MW (microwave); NBS (*N*-bromosuccinimide); NMMO (*N*-ethylmorpholine-*N-*oxide); NMR (nuclear magnetic resonance); Pcol (column pressure); iPrOH (isopropanol); RT (room temperature); sat. aq. (saturated aqueous); SiO₂ (silica gel); and THF (tetrahydrofuran). Ac₂O (acetic acid); t-BuOH (tert-butanol); DIPEA (di-iso-propylethylamine); KOAc (potassium acetate); MW microwave; IST ISOLUTE^{®} SPE column SCX (International Sorbent Technology ISOLUTE^{®} Solid Phase Extraction column cationic exchange resin); SFC (supercritical fluid chromatography); TBTU *O*-(1*H-*benzotriazol-1-yl)-*N*,*N*,*N*',*N*'-tetramethyluronium tetrafluoroborate and Tcol (column temperature).

Compounds of formula I can be prepared by reacting a compound of formula IA with a primary amine: 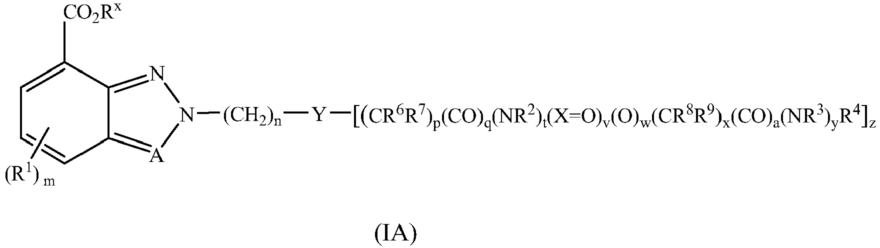 wherein a, m, n, p, q, t, v, w, x, y, z, A, R¹, R², R³, R⁴, R⁶, R⁷, R⁸, R⁹, X and Y are as defined above and R^{x} is C₁₋₆alkyl, such as methyl. The reaction is generally carried out using an aqueous solution of NH₃ in a solvent such as THF at about 70°C, in a sealed reaction vessel (with caution). Alternatively, a base such as NaOH may be added to hydrolyse the ester to the corresponding carboxylic acid (R^{x} is hydrogen), followed by the addition of NH₃ in the presence of coupling agents such as HATU and DIPEA in a solvent such as DMF, the reaction being carried out at about room temperature. Alternatively, ammonia in a solvent such as MeOH can be used at about 120°C, for example in a MW.

Compounds of formula IA wherein A is CH can be prepared by reacting a compound of formula IB with an azide: 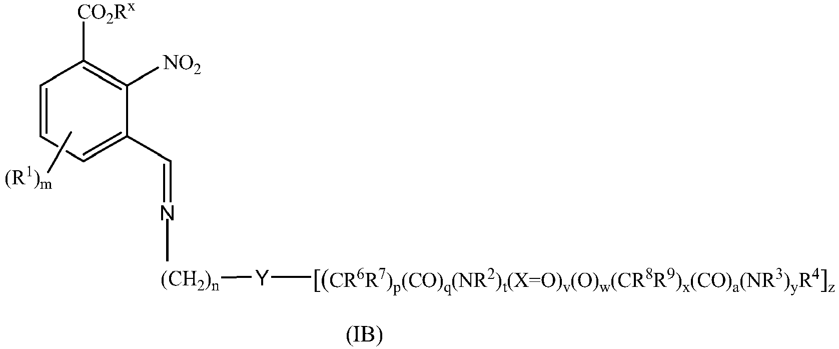 wherein a, m, n, p, q, t, v, w, x, y, z, R¹, R², R³, R⁴, R⁶, R⁷, R⁸, R⁹, X, Y and R^{x} are as defined above. An azide such as NaN₃ can be used, generally in a solvent such as DMF at about 90°C to 140°C. The reaction may be carried out under a nitrogen atmosphere.

Compounds of formula IB can be prepared by the condensation of a compound of formula IC with a compound of formula ID: 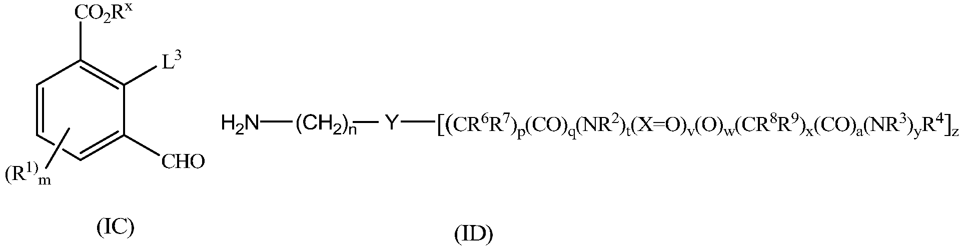 wherein a, m, n, p, q, t, v, w, x, y, z, R¹, R², R³, R⁴, R⁶, R⁷, R⁸, R⁹, X, Y and R^{x} are as defined above and L³ is a leaving group such as nitro or halogen, for example fluorine. In an embodiment L³ is nitro. Methods include condensation in the presence of a dehydrating agent such as MgS0₄ or molecular sieves or heating in an alcohol solvent such as ethanol at reflux. The reaction may be carried out under a nitrogen atmosphere.

Compounds of formula IC can be prepared by oxidizing a compound of formula IE with an oxidizing agent such as NMMO: 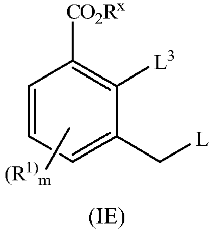 wherein m, R¹, R^{x} and L³ are as defined above and L is a leaving group such as halogen, for example bromine, generally in a solvent such as MeCN at about room temperature. The reaction may be carried out under a nitrogen atmosphere.

Compounds of formula IA wherein Y is an aromatic ring and (CR⁶R⁷)ₚ(CO)_{q}(NR²)ₜ(X=O)ᵥ(O)_{w}(CR⁸R⁹)ₓ(CO)ₐ(NR³)_{y}R⁴ is an electron withdrawing group can alternatively be prepared by the condensation of a compound of formula IF with a compound of formula IG:

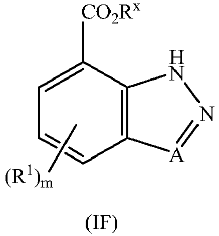

L¹-(CH₂)ₙ-Y-[(CR⁶R⁷)ₚ(CO)_{q}(NR²)ₜ(X=O)ᵥ(O)_{w}(CR⁸R⁹)ₓ(CO)ₐ(NR³)_{y}R⁴]_{z} (IG)

wherein a, m, n, p, q, t, v, w, y, z, A, R¹, R², R³, R⁴, R⁶, R⁷, R⁸, R⁹, X and R^{x} are as defined above and L¹ is a leaving group such as halogen, for example fluorine. The reaction is generally carried out in the presence of a base such as K₂C0₃, a solvent such as DMF at about 200°C. The reaction may be accelerated by using microwave heating. A base such as Cs₂C0₃ may also be used.

Compounds of formula IF can be prepared using the method described in WO 2004/029050.

Compounds of formula I wherein t is 1, v is 1 and z is 1 can be prepared by the condensation of a compound of formula IH with a compound of formula IJ: 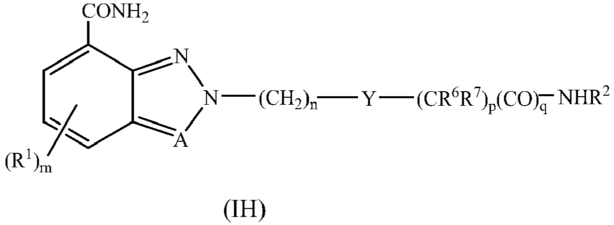 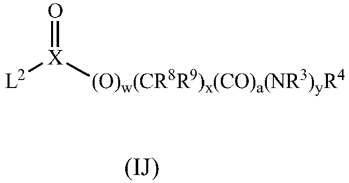 wherein a, m, n, p, q, w, x, y, A, R¹, R², R³, R⁴, R⁶, R⁷, R⁸, R⁹, X and Y are as defined above and L² is a leaving group such as hydroxy. The reaction is generally carried out in the presence of a coupling agent such at HATU and a base such as Et₃N in solvents such as DMF and DCM at about room temperature. The coupling agents TBTU or TEA may also be used. Other amide or sulfomide moieties in compounds of formula I can be prepared by an analogous method using appropriate variations of the compounds of formula IH and IJ.

Compounds of formula IH wherein R² is hydrogen can be prepared by hydrogenation of a compound of formula IK: 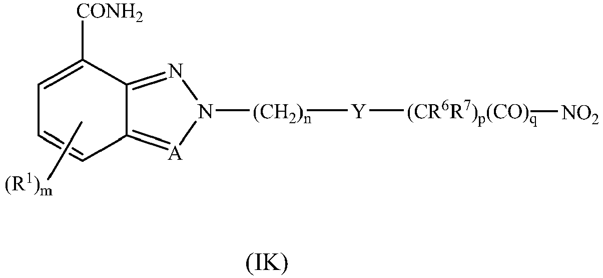 wherein m, n, p, q, A, R¹, R⁶, R⁷ and Y are as defined above. Standard hydrogenation conditions can be used, such as treating with a catalyst such as Pd/C, in a solvent such as methanol in a hydrogen atmosphere at about room temperature.

Compounds of formula IK can be prepared by reacting a compound of formula IL with ammonia: 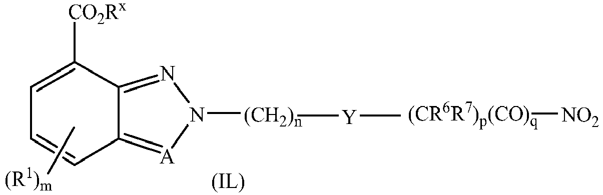 wherein m, n, p, q, A, R¹, R⁶, R⁷, R^{x} and Y are as defined above. The reaction is generally carried out using NH₃ in a solvent such as MeOH at about 50°C, in a sealed vessel (with caution).

Compounds of formula IF wherein A is CH can be prepared by cyclisation of a compound of formula IM: 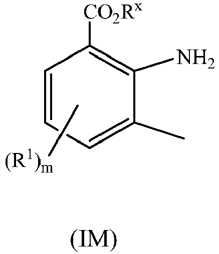 wherein m, R¹ and R^{x} are as defined above, in the presence of a nitrite such as isoamyl nitrite, with acetic anhydride and potassium acetate. The reaction is carried out in a solvent like chloroform. Alternatively, the compound of formula IM can initially be acylated, generally using acetyl chloride in a solvent such as 1,2-dichloroethane at about 55°C, and then cyclised using an inorganic nitrite such as sodium nitrite in the presence of a mineral acid such as HC1 at about 0°C.

Compounds of formula IF wherein A is N can be prepared by cyclisation of a compound of formula IN: 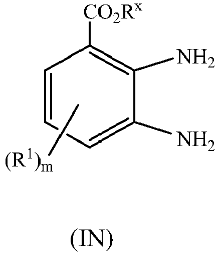 wherein m, R¹ and R^{x} are as defined above, in the presence of a nitrite such as sodim nitrite and a weak acid such as acetic acid.

Compounds of formula IA wherein A is N can be prepared by cyclisation of a compound of formula IO: 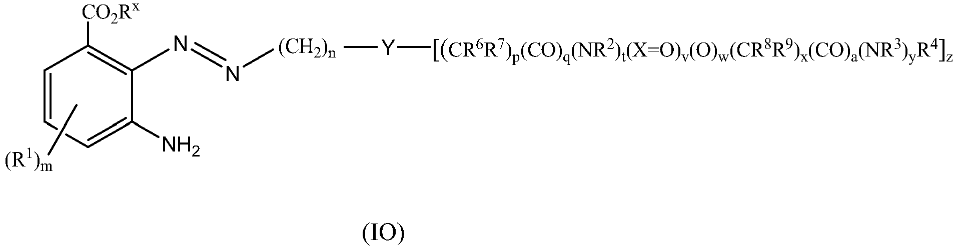 wherein a, m, n, p, q, t, v, w, x, y, z, R¹, R², R³, R⁴, R⁶, R⁷, R⁸, R⁹, X, Y and R^{x} are as defined above. The reaction is generally carried out in the presence of a cyclising agent such as Cu(OAc)₂ in a solvent such as DMF under an oxygen atmosphere at about 80°C.

Compounds of formula IO can be prepared by reacting a compound of formula IN with a compound of formula IP:

O=N-(CH₂)ₙ-Y-[(CR⁶R⁷)ₚ(CO)_{q}(NR²)ₜ(X=O)ᵥ(O)_{w}(CR⁸R⁹)ₓ(CO)ₐ(NR³)_{y}R⁴]_{z} (IP)

wherein a, n, p, q, t, v, w, x, y, z, R², R³, R⁴, R⁶, R⁷, R⁸, R⁹, X and Y are as defined above, generally in an acidic solvent such as AcOH at about room temperature.

Compounds of formula I wherein p is 1 to 6, q is 0, t is 1 and z is 1 can be prepared by reductive amination of a compound of formula IQ with an amine of formula IR:

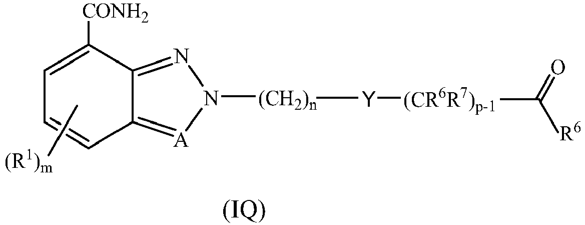

H(NR²)(O)_{w}(CR⁸R⁹)ₓ(CO)ₐ(NR³)_{y}R⁴ (IR)

wherein a, m, n, w, x, y, A, R¹, R², R³, R⁴, R⁶, R⁷, R⁸, R⁹ and Y are as defined above. The reaction is generally carried out in the presence of a reducing agent such as NaBH₃(CN) or MP-triacetoxyborohydride, in a solvent such as MeOH or DMF at about room temperature to 80°C. A catalyst such as ZnCl₂ may also be used.

Alternatively, compounds of formula I wherein q is 1, t is 1 and z is 1 can be prepared by a coupling reaction of a compound of formula IS with an amine of formula IT: 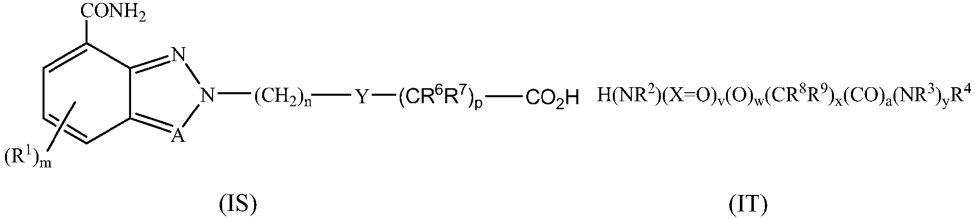 wherein a, m, n, p, v, w, x, y, A, R¹, R², R³, R⁴, R⁶, R⁷, R⁸, R⁹, X and Y are as defined above. The reaction is generally carried out in the presence of coupling agents such as HATU and a base such as Et₃N in a solvent such as DMF at about room temperature.

Compounds of formula IH wherein p is 1 to 6 and q is 0 can be prepared by hydrogenation of a compound of formula IU: 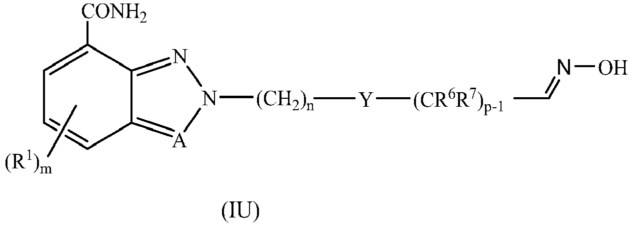 wherein m, n, R¹, R⁶, R⁷, A and Y are as defined above. Standard hydrogenation conditions such as those described above can be used.

Compounds of formula IU can be prepared by condensation with a compound of formula IQ in which R^{y} is hydrogen with a hydroxyamine such as NH₂OH.HCl, in the presence of a base such as Et₃N and a solvent such as MeCN at reflux.

Compounds of formula I wherein a, p, q, t, v, w, x and y are each 0, z is 1 and Y is a ring can be prepared by cross-coupling a compound of formula IW with a compound of formula IX:

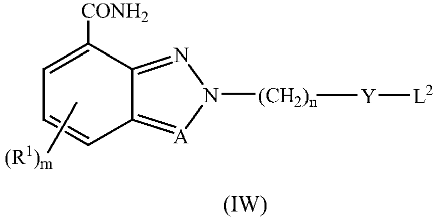

(HO)₂BR⁴ (IX)

wherein m, n, A, R¹, R², R³, R⁴, R⁶, R⁷, R⁸, R⁹ and X are as defined above and L² is a leaving group such as halogen, for example bromine. The reaction is generally carried out under Suzuki coupling conditions such as using catalysts such as Pd₂(dba)₃ and tri(tert-butyl)phosphine together with a base such as sodium carbonate and solvents such as DMF and water at about 90°C.

Compounds of formula IW can be prepared by condensation of a compound of formula IY with a compound of formula IZ:

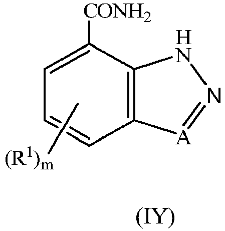

L⁴-(CH₂₎ₙ-Y-L² (IZ)

wherein m, n, R¹, A, Y and L² are as defined above and L⁴ is a leaving group such as halogen, for example fluorine, generally in a solvent such as DMF at about 180°C.

Compounds of formula IY can be prepared by reacting a compound of formula IF with a base such as KOH or NaOH at about room temperature to hydrolyse the ester to the corresponding carboxylic acid (R^{x} is hydrogen), followed by the addition of NH₃ in the presence of coupling agents such as HATU, DIPEA and TBTU in a solvent such as DMF, the reaction being carried out at about room temperature.

Compounds of formula IE can be prepared by oxidizing a compound of formula IAA with a brominating agent such as NBS in the presence of a radical initiator such as benzoyl peroxide: 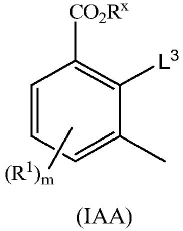 wherein m, L³, R¹ and R^{X} are as defined above, generally in a solvent such as CC1₄ at reflux.

Compounds of the formula IAA wherein L³ is fluorine can be prepared by diazonitisation of a compound of formula IM followed by decomposition of the intermediate diazonium salt. For example, the diazonitisation can be carried out using nitrosium tetrafluoroborate in a solvent such as DCM at about 0°C. The corresponding diazonium tetrafluoroborate salt can then be isolated and subsequently decomposed at elevated temperatures to the corresponding fluorobenzene derivative (Caution), such as by heating to 160°C in a solvent such as dichlorobenzene.

Compounds of formula IAA wherein L³ is nitro can be prepared by nitration of a compound of formula IBB followed by esterification: 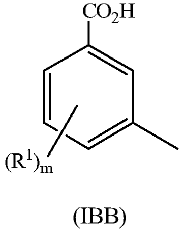 wherein m and R¹ are as defined above. The nitration reaction can be carried out in the presence of a nitrate such as potassium nitrate and an acid such as sulfuric acid at about room temperature. The esterification step can be carried out under standard conditions, such as by reacting with an alkyl halide of formula R^{x}-X wherein X is a halogen such as iodine, in the presence of a base such as cesium carbonate and in a solvent such as DMF at about room temperature. An alcohol of formula R^{x}-OH can also be used together with an acid catalyst, such as HCl generated in situ from AcCL/MeOH, at reflux.

Where the synthesis of intermediates and starting materials is not described, these compounds are commercially available or can be made from commercially available compounds by standard methods or by extension of the synthesis above, schemes and Examples herein.

Compounds of formula I may be converted to other compounds of formula I by known methods or by methods described in the synthesis above, schemes and Examples herein. For example, pyridine derivatives can be reduced to the corresponding piperidine derivatives by a Fowler reaction using an acyl chloride such as Cbz-Cl, a reducing agent such as NaBH₄ and in a solvent such as methanol at about -65°C to RT, followed by hydrogenation. Alternatively, alkylation with reactive alkyl halides, such as ethyl iodide in a solvent such as acetonitrile gives rise to a pyridinium salt that can be reduced with sodium borohydride in an alcohol solvent such as MeOH. This can then be hydrogenated to the corresponding 1-alkyl piperidine using hydrogen and palladium on carbon.

During any of the synthetic sequences described herein it may be necessary and/or desirable to protect sensitive or reactive groups on any of the molecules concerned. This may be achieved by means of conventional protecting groups, such as those described in Protesting Groups in Organic Synthesis, 3rd Edition, Greene, T. W. and Wuts, P. G. M.; Wiley Interscience, 1999 and Kocienski, P. J. Protecting Group, Thieme, 1994. The protecting groups may be removed at a convenient subsequent stage using methods known from the art. For example, when the Boc (*tert*-butoxycarbonyl) or benzylcarbonyl protecting group is present, it may be removed by the addition of solvents such as TFA, DCM and/or MeCN at about room temperature. The compound may also be hydrogenated using standard methods, such as treating with a catalyst such as Pd/C, in a solvent such as methanol under a hydrogen atmosphere. EtOAc in the presence of HCl and 1,4-dioxane may also be added to remove the Boc or benzylcarbonyl protecting group, at about room temperature.

The compounds of this invention were prepared according to the following schemes. All variables within the formulae are as defined above.

When the compounds of the present invention have chiral centres, the enantiomers may be separated from the racemic mixtures by standard separating methods such as using SFC.

### Scheme 1

A procedure to synthesize derivatives of those compounds of this invention wherein A = CH is shown in **scheme 1,** whereby the substituted 2*H-*indazoles are prepared using a synthetic route similar to that described in WO 2005/066136. Following initial conversion of the 2-nitro-3-methyl-benzoic acid derivative into the corresponding ester, radical bromination of the methyl group using reagents like N-bromosuccinimide and benzoyl peroxide yields the key benzyl bromide derivative. Oxidation of this benzylic bromide to the corresponding benzaldehyde can be accomplished for instance using *N-*methylmorpholine-*N-*oxide and molecular sieves. Following the condensation of the aldehyde with an amine, ring closure can be accomplished by treating the key intermediate with sodium azide at elevated temperature to introduce the final nitrogen atom and the resultant extrusion of nitrogen to furnish the indazole ring. Final conversion of the ester to the primary amide yields the desired derivatives.

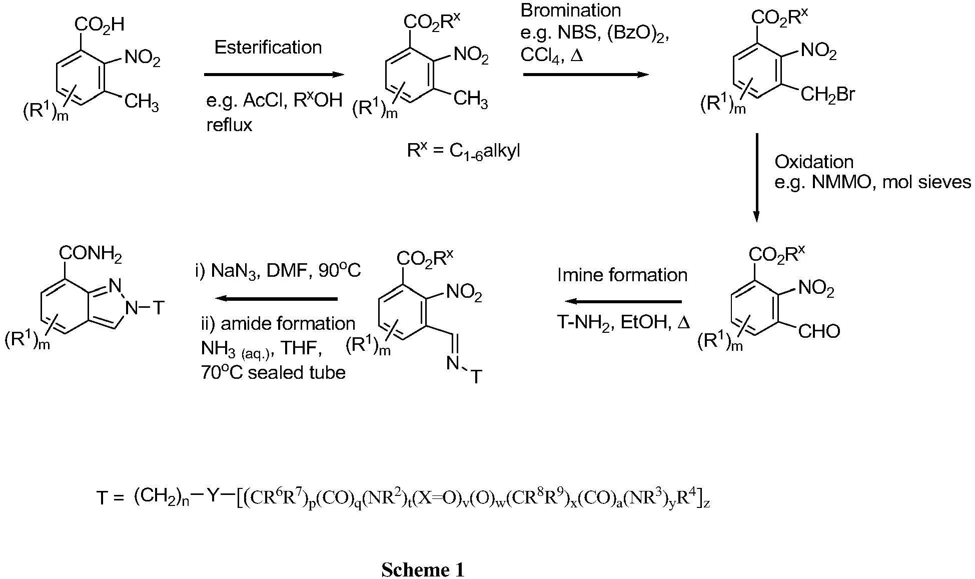

### Scheme 2

Alternatively, compounds of the present invention can be prepared by a microwave assisted S_{N}Ar on the methyl-2H-indazole-7-carboxylate (for the preparation see WO2004/029050) using the appropriate substituted activated haloaromatic or haloheteroaromatic derivative in a polar solvent, like DMF, in presence of a base, such as K₂C0₃. The functional groups on the (hetero)aromatic groups can then be further manipulated for instance performing reductive amination of an aldehyde functionality such as using ZnCl₂ and NaBH₃(CN) together with the appropriate amines. Conversion to the desired carboxamide can be achieved by treatment an aqueous solution of NH₃ in THF at reflux (Scheme 2).

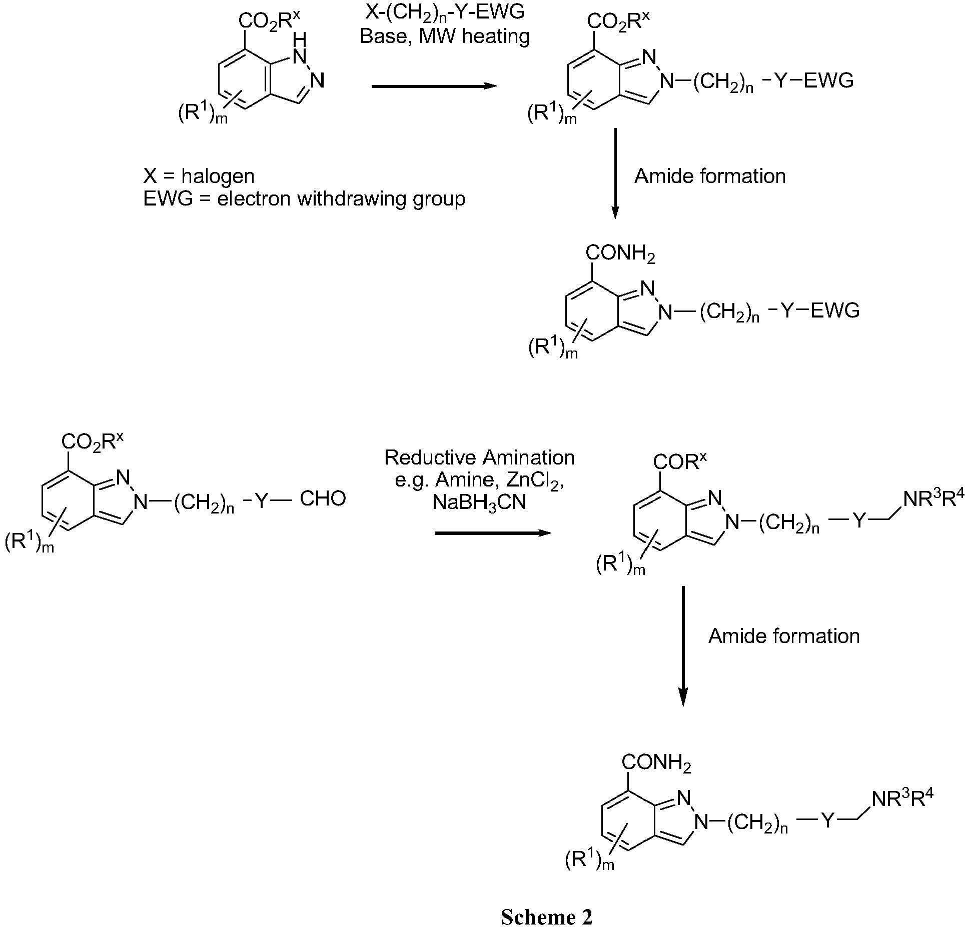

### Scheme 3

A modification of the procedure in **scheme 2** is where the displacement is carried out with a fluoronitrobenzene, to yield the corresponding *N-*nitrophenyl indazole analogs, which after conversion to the corresponding amide, the nitro group can be reduced, for instance by hydrogenation using palladium on carbon as catalyst. The resulting anilines can be coupled by standard methods to yield the desired inhibitors.

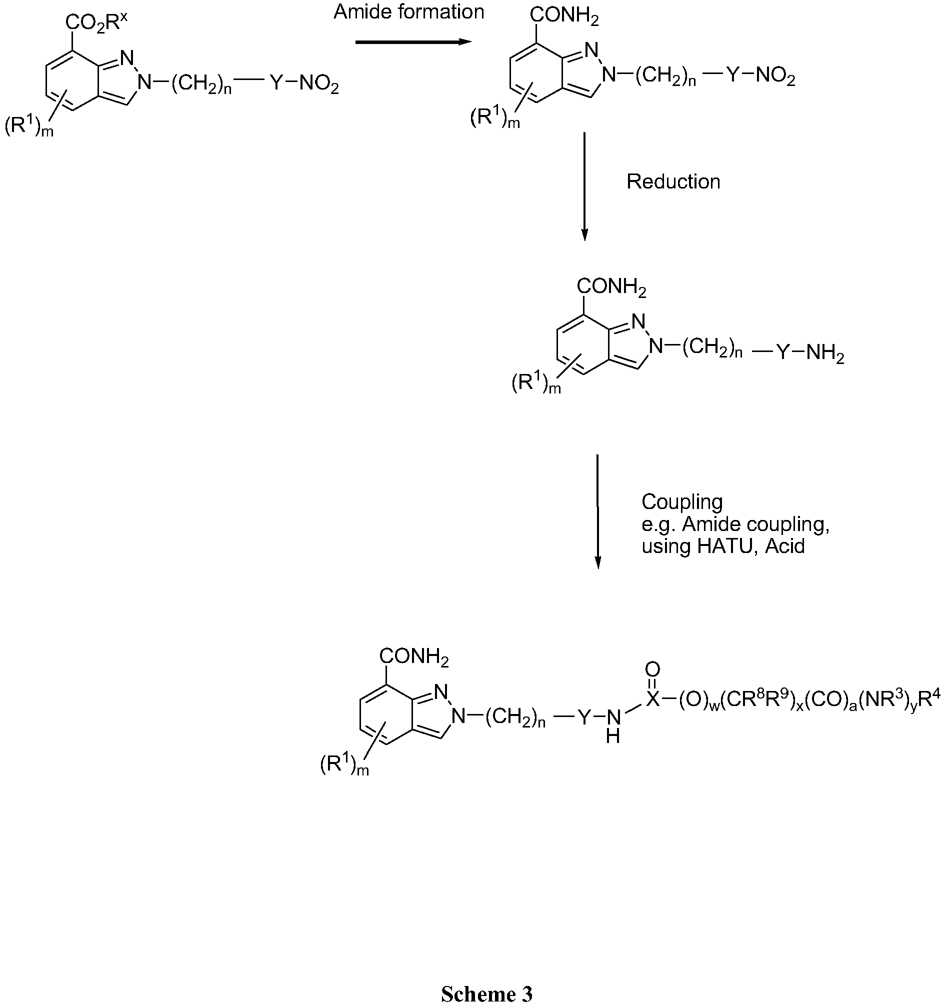

### Scheme 4

A modification of the procedure in **scheme 2** is shown below in **scheme 4** whereby the displacement is carried out with a fluoroacetophenone or related ketone derivative, in this case subsequent reductive amination yields the corresponding amine derivative with a branch at the *alpha*-position. The two enantiomers can be subsequently resolved by chiral separation.

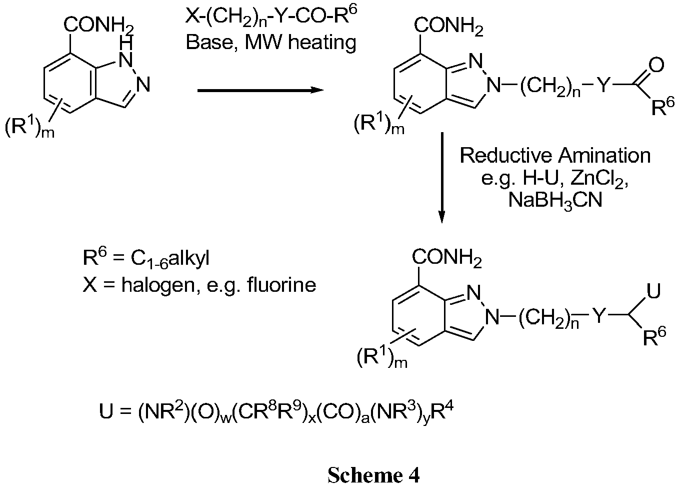

### Scheme 5

Further derivatives can be prepared by late stage modification of these compounds, for instance if an aldehyde has been carried through the earlier procedures it can be oxidized to the corresponding carboxylic acid using reagents such as NaClO₂/NaH₂PO₄ in the presence of a scavenger such as methylbutene. The resulting acid can be coupled with a variety of amines using coupling reagents such as HATU in the presence of a base such as Et₃N.

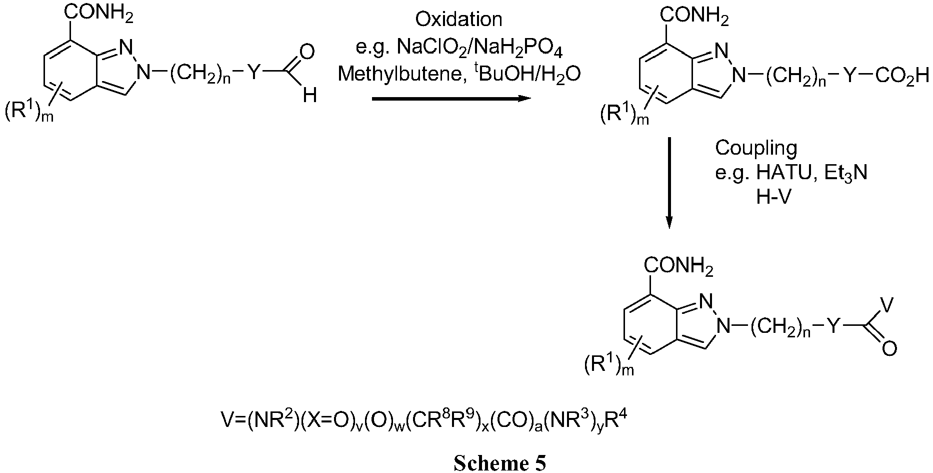

### Scheme 6

Alternatively benzotriazoles can be prepared as described in **scheme 6.** A functionalised *ortho-*nitroaniline can be reduced to the corresponding 1,2-diaminobenzene using conditions such as hydrogenation over palladium on carbon. In turn, this 1,2-diaminobenzene can be treated with sodium nitrite in acetic acid to give rise to the corresponding benzotriazole as described in Heterocycles (1985) 23(9):2225-2228 and J. Med. Chem.; (1989) 32(7):1566-1571. The resulting heterocycle can then be alkylated under basic conditions to given an isomeric mixture of benzotriazoles from which can be isolated the desired 2-substituted isomer. Conversion of the ester to the corresponding primary amide gives the desired inhibitors.

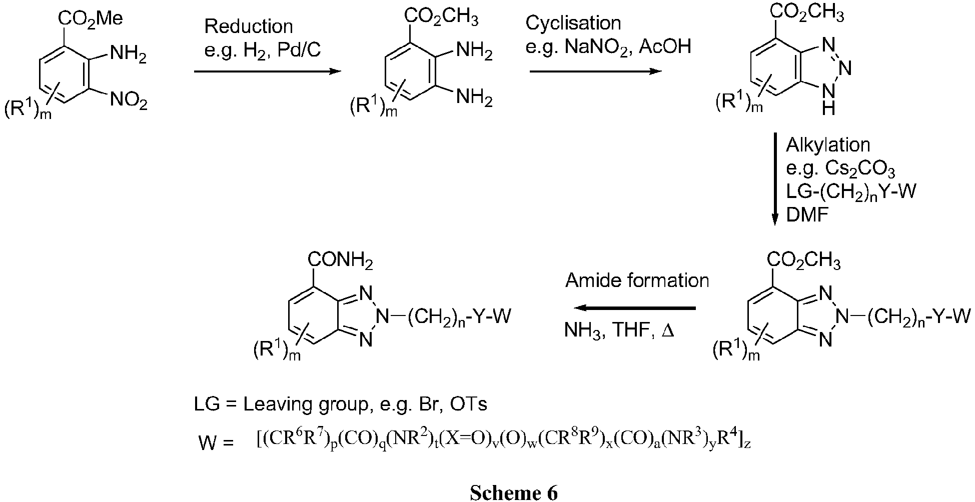

### Scheme 7

In a related manner, the 1,2-diaminobenzene can be reacted with a nitroso derivatives at RT in a solvent like AcOH to form a diazo compound. This diazo compound can then be cyclised to the corresponding benzotriazole by treatment with copper acetate in DMF at 80°C under an oxygen atmosphere. Manipulation of the ester to the amide as previously, or by hydrolysis to the carboxylic acid and coupling to ammonia, yields the desired compounds.

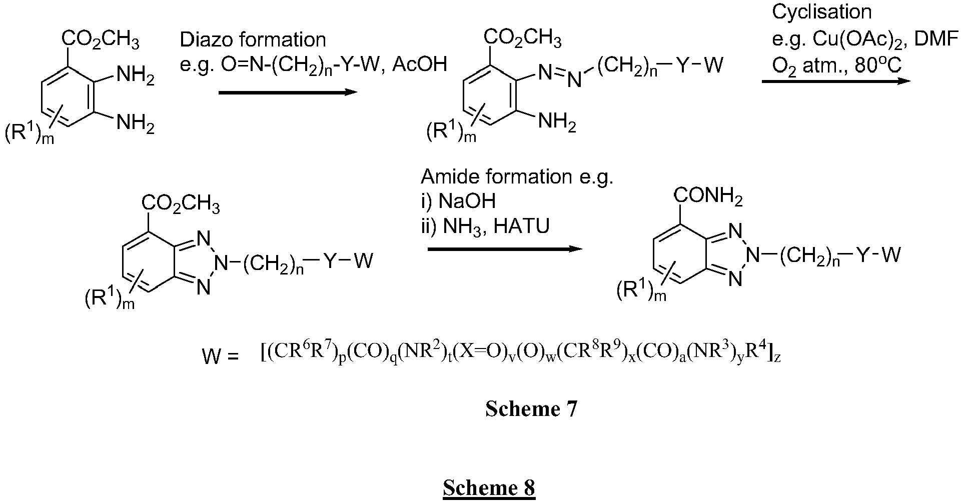

An alternative preparation of the indazole scaffold is shown in scheme **8** whereby a substituted alkyl 2-amino-3-methylbenzoate can be treated with isoamyl nitrite and potassium acetate in the presence of acetic anhydride as reported in Bioorganic & Medicinal Chemistry, 2004, 12(9), 2115-2137 and the desired functionalised indazole can be isolated. In turn this indazole can be derivatised as previously descibed.

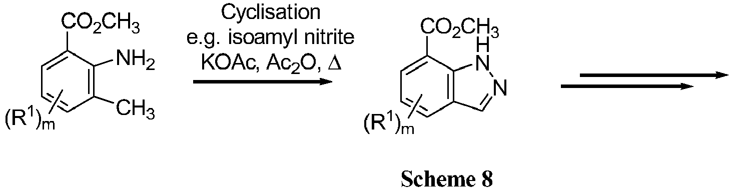

### Scheme 9

Further derivativisation to allow the preparation of compounds with a gem-dialkyl substituent can be achieved as described in **scheme 9.** Here a ketone derivative can be treated with an appropriate Grignard reagent to yield the corresponding tertiary alcohol, This alcohol can, in turn, be treated with an inorganic cyanide in concentrated acid to yield the corresponding formamide by Ritter rearrangement reaction (Ritter, J. J.; Kalish J.; Org. Synth. Col. Vol. V, 1973, 471). This formamide can be reduced to give (hetero)benzylic amines.

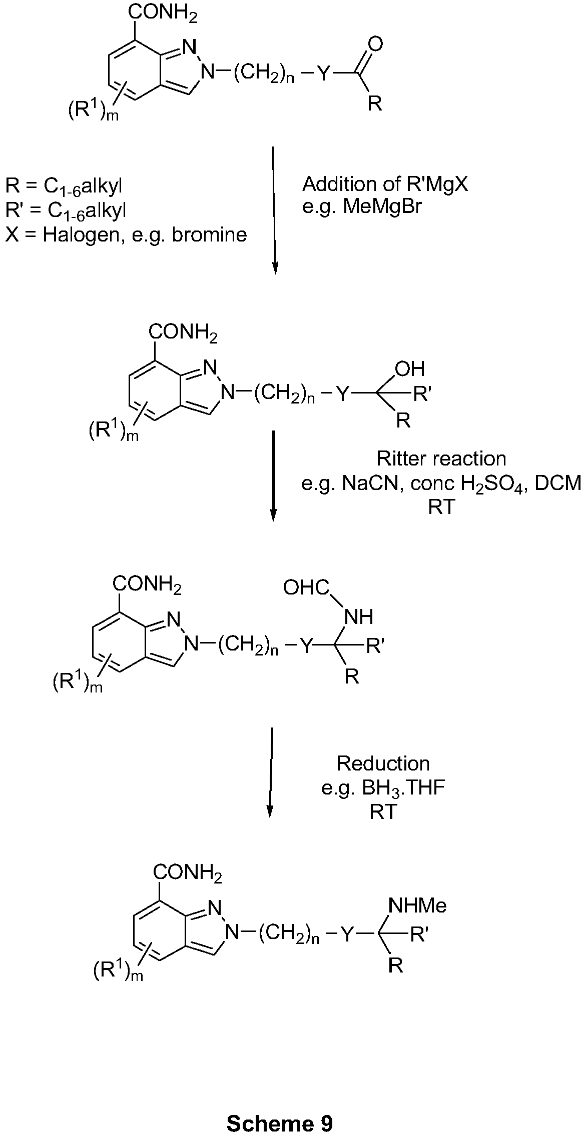

### Scheme 10

Late stage modifications of these derivatives also allow the preparation of other derivatives and further examples are shown in **scheme 10.** Here nucleophilic aromatic substitution of the appropriate fluoro(hetero)aromatic bromide allows the preparation of a bromide derivative that can be cross coupled under Suzuki coupling conditions, for instance using tri(tert-butyl)phosphine and Pd₂(dba)₃ as catalysts in the presence of a base, such as sodium carbonate. If a pyridine derivative has been prepared it can then be reduced in a Fowler reaction using an acyl chloride and a reducing agent such as NaBH₄. Final hydrogenation reaction can yield the corresponding piperidine derivatives. Alternatively quaternisation of the corresponding pyridine derivative followed by reduction and hydrogenation gives alkylated piperidine derivatives.

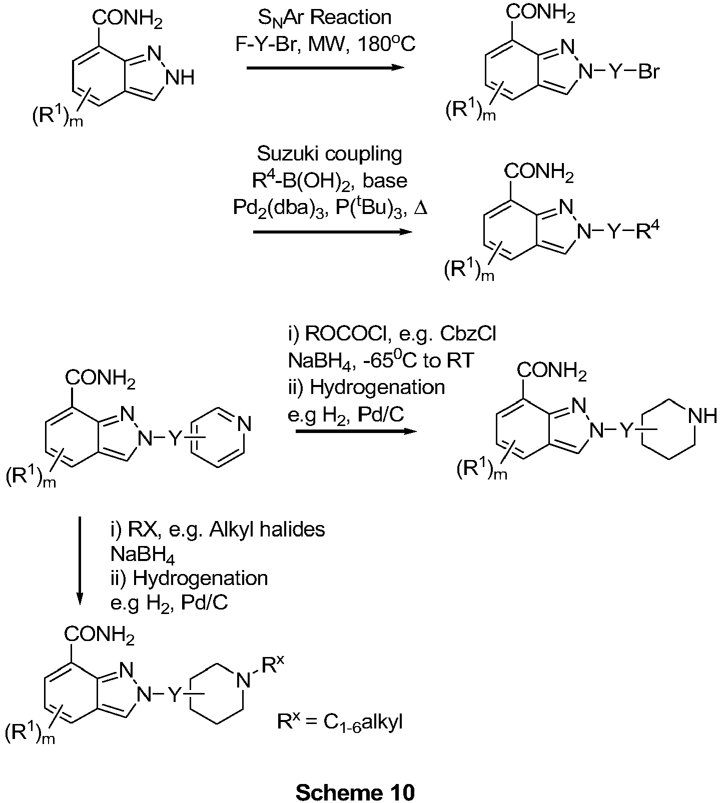

### Scheme 11

Homologation reactions of advanced intermediates are also possible as shown in **scheme 11,** for instance treating an aldehyde with methoxymethyltriphenylphosphonium chloride and a base such as potassium *tert*-butoxide followed by mild acid treatment to allow a one-carbon homologation reaction. The aldehyde can then be elaborated as described above to introduce amino functionalities. Furthermore, the aldehyde can also be condensed with hydroxylamine and hydrogenated to yield the corresponding primary amines that can in turn be manipulated further into the desired derivatives.

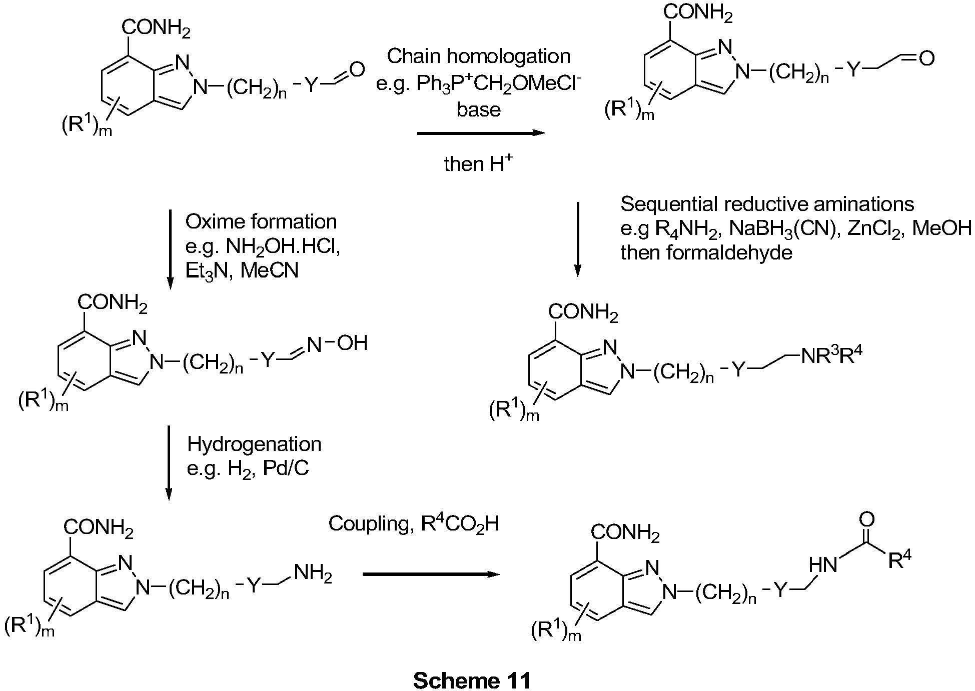

### Scheme 12

A variation of **schemes 1 and 8** is shown below in **scheme 12** and allows the introduction of substituents onto the indazole cores. When the required nitrobenzoic acid derivatives are not commercial available they can be prepared through nitration of the corresponding benzoic acid derivatives, for instance using potassium nitrate in concentrated sulphuric acid. Synthetic manipulations as decribed above allow the formation of the corresponding aniline which can either be cyclised to the indazole by firstly acetylation of the indazole and cyclisation with sodium nitrite in concentrated HCl acid at 0°C. Alternatively, the aniline can be diazonitised with nitrosium tetrafluoroborate and the corresponding diazonium tetrafluoroborate salt decomposed at elevated temperatures to the corresponding dilfluorobenzene derivative by a Schiemann reaction (Caution). Following the synthetic sequence as described in **scheme 1** allows oxidation of the benzylic methyl group to the corresponding aldehyde and elaboration of the desired indazole derivatives by coupling with a (hetero)anilide and cyclisation with sodium azide.

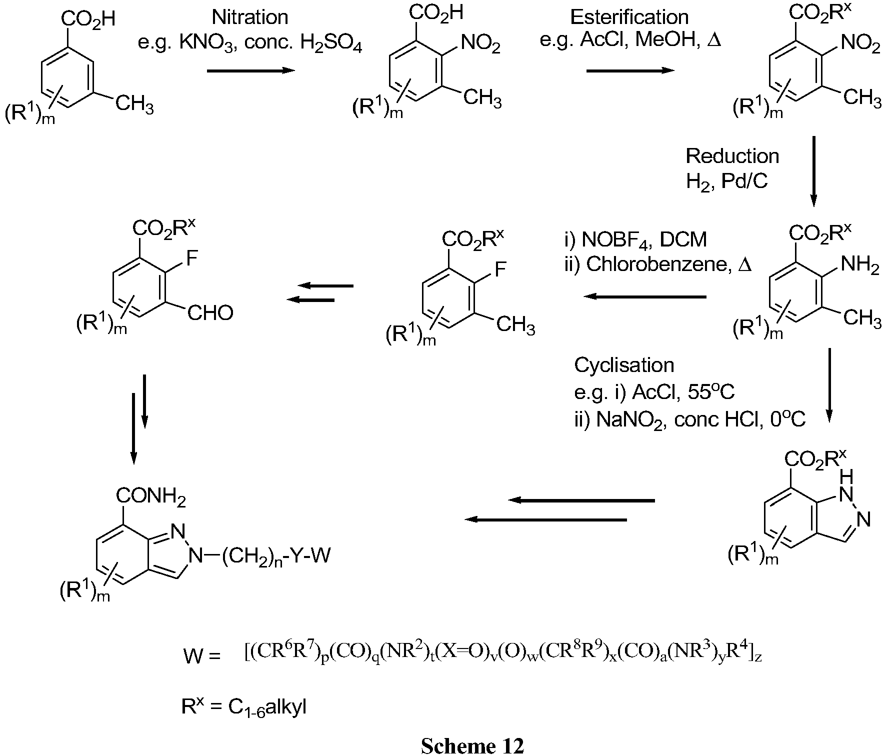

The exemplified compounds described herein were tested by the assays described below and were found to have an IC₅₀ value of less than 5µM.

### PARP-1 SPA assay

### Working Reagents

**Assay buffer:** 100 mM Tris pH 8, 4 mM MgCL₂, 4 mM Spermine, 200 mM KCI, 0.04% Nonidet P-40. **Enzyme Mix:** Assay buffer (12.5 ul), 100 mM DTT (0.5 ul), PARP-1 (5 nM, Trevigen 4668-500-01), H₂O (to 35 ul).

**Nicotinamide-adenine dinucleotide (NAD)/ DNA Mix:** [³H-NAD] (250 uCi/ml, 0.4 ul, Perkin-Elmer NET-443H), NAD (1.5 mM, 0.05 ul, SIGMA N-1511), Biotinylated-NAD (250 uM, 0.03 ul, Trevigen 4670-500-01), Activated calf thymus (Img/ml, 0.05ul, Amersham Biosciences 27-4575), H₂O (to 10µl). **Developing Mix:** Streptavidin SPA beads (5mg/ml, Amersham Biosciences RPNQ 0007) dissolved in 500 mM EDTA.

### Experimental Design

The reaction is performed in 96-well microplate with a final volume of 50 uL/well. Add 5µl 5%DMSO/compound solution, add enzyme mix (35ul), start the reaction by adding NAD/DNA mix (10 uL) and incubate for 2 hrs at RT. Stop the reaction by adding developing mix (25 ul) and incubate 15 min at RT. Measure using a Packard TOP COUNT instrument.

### Proliferation Assay in BRCA-1 silenced HeLa cells_{.}

### Abbreviations:

IMDM (Iscove's Modified Dulbecco's Media); RPMI (Roswell Park Memorial Institute Media); MOI (multiplicity of infection); GFP (green fluorescent protein); PBS (Phosphate Buffered Saline); FCS (fetal calf serum); and DMEM (Dulbecco's Modified Eagle's Medium).

Compounds were also tested in an anti-proliferative assay in matched pair BRCAlwt and BRCA1-(shRNA) HeLa cells. The assay shows that PARP inhibitors are able to show selectivity with growth inhibition of the BRCA deficient cells. Certain compounds showed CC₅₀'s less than 10 µM in BRCA1 deficient cells and a greater than 10 fold selectivity over the BRCA proficient cells.

The assay is based on the ability of living cells to convert a redox dye (resazurin) into a fluorescent end product (resofurin). The amount of resofurin produced is directly proportional to the cell number.

### Cell lines:

***HeLa shBRCA1-GFP -*** These are HeLa cells transduced at an MOI of 100 with a Lentivirus containing a shRNA against BRCA-1 and an expression cassette for GFP. BRCA-1 silencing is more than 80 % as assessed by Taqman analysis and the cells stably express GFP.

***HeLa THM-GFP-*** These are HeLa cells transduced at an MOI of 100 with a control vector not expressing any shRNA.

### Protocol

- Seed 300 cell/well in 96 wells viewplate black in 90µl culture Medium*:
- Incubate 4 hours at 37°C, 5 % CO₂
- Add 10µl/well of 10X compound (5 % DMSO in H₂O)
- Incubate for 168 hours at 37°C, 5 % CO₂
- Add 10µl of Celltiter Blue solution (Promega, G8081) pre-diluted 1:1 in PBS1x
- Incubate the mixture for 45' at 37°C, 5 % CO₂
   - Incubate 15' at RT in the dark
- Read plate at fluorimeter ex: 550 nm; em: 590 nm

*Culture Medium: DMEM (GIBCO, 41966-029), 10% FCS (GIBCO, 10106-169), 0.1mg/ml Penicillin-Streptomycin (GIBCO, 15140-114), 2mM L-Glutamine (GIBCO, 3042190)

### Proliferation Assay in naturally BRCA deficent cells lines_{.}

Certain compounds of the present invention were also demonstrated to inhibit the proliferation of naturally BRCA-1 (MDA-MB-436) and BRCA-2 (CAPAN-1) deficient cell lines with CC₅₀'s less than 5 micromolar.

### Proliferation Assay

Cells are seeded in a 96-well plate at 700 cells/well in 100ul of the appropriate medium/well.*
The following day, serial dilutions of the compound are added in a final volume of 200 µl/well. Each dilution is assayed in triplicates.
Six days later, cell viability is estimated using CellTiter-Blue Cell Viability Assay according to the manufacturer instructions (Promega). Plates are read at the Fusion Alpha microplate reader (Packard Bioscience).

For low-proliferating cell lines (i.e. CAPAN-1), proliferation is assayed 14 days after adding the compounds and changing the medium once at day 7 (170 µl of medium per well are aspirated and replaced with 170 µl fresh medium containing the compounds).

### * Culture Medium:

MDA-MB-436: RPMI (GIBCO), 10 % FBS (5% CO₂)
CAPAN-1: IMDM (GIBCO), 20 % FBS (5% CO₂)

### EXAMPLE 1 2-Phenyl-2H-indazole-7-carboxamide (A6)

### Step 1: Methyl 3-methyl-2-nitrobenzoate (A1)

To a suspension of 3-methyl-2-nitro-benzoic acid (1.0 eq.) in MeOH (0.4 M) at 0°C was added dropwise AcC1 (3.0 eq.). The reaction mixture was stirred for 20 hr at reflux. The solvent was reduced in vacuo and the residue was dissolved in EtOAc and washed several times with sat. aq. NaHC0₃ solution, brine and dried (Na₂SO₄). Evaporation of the solvent gave (**A1**) as a white solid which was used in the next step without further purification.¹H NMR (400MHz, CDCl₃, 300K) δ 7.86 (1H, d, J = 7.5 Hz), 7.53-7.42 (2H, m), 3.89 (3H, s), 2.36 (3H, s). MS (ES) C₉H₉NO₄ requires: 195, found: 218 (M+Na)⁺.

### Step 2: Methyl 3-(bromomethyl)-2-nitrobenzoate (A2)

A mixture of (**A1**) (1.0 eq.), (BzO)₂ (0.06 eq.) and NBS (1.18 eq.) in CC1₄ (0.2 M) was heated at reflux under N₂ atmosphere for 12 hr. The mixture was cooled to RT, diluted with DCM, concentrated under reduced pressure whilst dry loading onto SiO₂. The residue was purified by flash column chromatography on SiO₂ using 10:90 EtOAc/Petroleum ether to yield the desired (A2) as a white solid. ¹H NMR (400MHz, CDCl₃, 300K) δ 7.93 (1H, d, J = 7.7 Hz), 7.72 (1H, d, J = 7.7 Hz), 7.57 (1H, t, J = 7.7 Hz), 4.43 (2H, s), 3.88 (3H, s). MS (ES) C₉H₈BrNO₄ requires: 273:275, found: 242:244 (M-MeO)⁺, 227:229 (M-NO₂)⁺.

### Step 3: Methyl 3-formyl-2-nitrobenzoate (A3)

To a mixture **of (A2)** and 4Å mol. sieves (15 g) in MeCN (0.2M) at RT was added NMMO (2.0 eq.) and the reaction mixture was stirred for 1.5 hr under N₂ atmosphere. Then, the mixture was diluted with EtOAc, filtered and the filtrate was washed with H₂O, 1N HCl, brine and dried (Na₂SO₄). Evaporation of the solvent gave **(A3)** as a white solid which was used in the next step without further purification. ¹H NMR (400MHz, CDCl₃, 300K) δ 9.96 (1H, s), 8.26 (1H, d, J= 7.9 Hz), 8.18 (1H, d, J = 7.9 Hz), 7.77 (1H, t, J = 7.9 Hz), 3.93 (3H, s). MS (ES) C₉H₇NO₅ requires: 209, found: 208 (M-H)-.

### Step 4: Methyl 2-nitro-3-[(phenylimino)methyl]benzoate (A4)

A mixture of (A3) (1.0 eq.) and aniline (1.05 eq.) in EtOH (0.2 M) was stirred at reflux under N₂ atmosphere for 2 hr until TLC revealed completation of the reaction (Hexane/EtOAc = 75:25). Evaporation of the solvent gave (**A4**) as a white solid which was used in the next step without further purification. ¹H NMR (400MHz, CDCl₃, 300K) δ 8.51 (1H, d, J = 7.3 Hz), 8.41 (1H, s), 8.11 (1H, d, J = 7.8 Hz), 7.67 (1H, t, J = 7.8 Hz), 7.43 (2H, t, J = 7.8 Hz), 7.31 (1H, t, J = 7.3 Hz), 7.16 (2H, d, J = 7.8 Hz), 3.94 (3H, s).

### Step 5: Methyl 2-phenyl-2H-indazole-7-carboxylate (A5)

A mixture **of (A4)** (1.0 eq.) and NaN₃ (1.05 eq.) in dry DMF (0.3 M) was stirred at 90 °C overnight under N₂ atmosphere. The crude was reduced in vacuo and the residue purified by flash column chromatography on silica using a gradient of EtOAc/Petroleum ether from 10:90 to 40:60 to yield the desired (A5) as a brown oil. ¹H NMR (400MHz, CDCl₃, 300K) δ 8.50 (1H, s), 8.12 (1H, d, J = 7.0 Hz), 7.96-7.90 (3H, m), 7.49 (2H, t, J = 7.6 Hz), 7.38 (1H, t, J = 7.4 Hz), 7.15 (1H, t, J = 7.4 Hz), 4.03 (3H, s). MS (ES) C₁₅H₁₂N₂O₂ requires: 252, found: 253 (M+H)⁺.

### Step 6: 2-Phenyl-2H-indazole-7-carboxamide (A6)

The ester (**A5**) was heated in a mixture of THF and 32% aq. NH₃ solution at 70 °C overnight in a sealed tube. The solvents were reduced in vacuo and the residue purified by flash column chromatography on silica using a gradient of EtOAc/Petroleum ether from 30:70 to 50:50 to yield the desired (A6) as white solid. ¹H NMR (400MHz, DMSO, 300K) δ 9.33 (1H, s), 8.56 (1H, bs), 8.16 (2H, d, J = 7.9 Hz), 8.08-8.00 (2H, m), 7.88 (1H, bs), 7.63 (2H, t, J = 7.7 Hz), 7.50 (1H, t, 7.4 Hz), 7.27 (1H, t, J = 7.9 Hz). MS (ES) C₁₄H₁₁N₃O requires: 237, found: 238 (M+H)⁺.

### EXAMPLE 2 2-(3-Chlorophenyl)-2H-indazole-7-carboxamide (B2)

### Step 1: Methyl 2-(3-chlorophenyl)-2H-indazole-7-carboxylate (B1)

**(B1)** was prepared following the general procedure reported in Example 1 step 4 using methyl 3-formyl-2-nitrobenzoate and 3-chloroaniline and was used in the next step without further purification. ¹H NMR (300MHz, CDCl₃, 300K) δ 8.49 (1H, s), 8.13 (1H, d, J = 8.2 Hz), 8.01 (1H, s), 7.92 (1H, d, J = 8.2 Hz), 7.83 (1H, d, J = 7.5 Hz), 7.37 (1H, t, J = 7.5 Hz), 7.18 (1H, d, J = 7.5 Hz), 7.03 (1H, t, J = 8.2 Hz), 4.03 (3H, s). MS (ES) C₁₅H₁₁ClN₂O₂ requires: 286:288, found: 287:289 (M+H)⁺.

### Step 2: 2-(3-Chlorophenyl)-2H-indazole-7-carboxamide (B2)

**(B2)** was prepared from **(B1)** following the general procedure reported for Example 1 step 5 and the crude was purified by reverse phase HPLC and the desired fractions were freeze dried to yield the desired product **(B2)** as a white solid. ¹H NMR (400MHz, CDCl₃, 300K) δ 9.02 (1H, bs), 8.54 (1H, s), 8.32 (1H, d, J = 7.8 Hz), 7.95 (1H, s), 7.92 (1H, d, J = 8.0 Hz), 7.79 (1H, d, J = 7.8 Hz), 7.50 (1H, t, 8.0 Hz), 7.44 (1H, d, J = 8.0 Hz), 7.26 (1H, t, J = 7.8 Hz), 6.34 (1H, bs). MS (ES) C₁₅H₁₀ClN₃O requires: 271:273, found: 272:274 (M+H)⁺.

### EXAMPLE 3 2-{4-[(Dimethylamino)methyl]phenyl}-2H-indazole-7-carboxamide(C3)

### Step 1: Methyl 2-(4-formylphenyl)-2H-indazole-7-carboxylate (C1)

To a solution of methyl 2*H*-indazole-7-carboxylate(1.0 eq., see WO2004/029050) in DMF (0.8 M) were added K₂CO₃ (1.1 eq.) and 4-fluorobenzaldehyde (1.3 eq), and the reaction mixture was heated under MW conditions at 200 °C for 10 min. The reaction mixture was cooled to RT and diluted with EtOAc. The organic phase was washed with brine; dried (Na₂SO₄). Evaporation of the solvent gave **(C1)** which was purified by flash column chromatography from EtOAc: Petroleum Ether = 30:70 to 40:60. ¹H NMR (400MHz, CDCl₃, 300K) δ 10.08 (1H, s), 8.64 (1H, s), 8.20 (2H, d, J = 8.6 Hz), 8.16 (1H, d, J = 7.2 Hz), 8.05 (2H, d, J = 8.6 Hz), 7.96 (1H, d, J = 7.2 Hz), 7.21 (1H, t, J = 7.2 Hz), 4.06 (3H, s). MS (ES) C₁₆H₁₂N₂O₃ requires: 280, found: 281 (M+H)⁺.

### Step 2: Methyl 2-{4-[(Dimethylamino)methyl]phenyl}-2H-indazole-7-carboxylate (C2)

**(C1)** was suspended in MeOH (0.1 M). Me₂NH (8 eq., 2.0 M in MeOH) was added upon which the starting material dissolved. To this solution was added a solution of NaBH₃ (CN) (1.1 eq.) and ZnCl₂ (0.5 eq.) in MeOH (0.5 mL). The pH was adjusted to 6 with 1.25 M HCl in MeOH and the mixture stirred at RT for 3h. 6N HC1 (0.1 mL) was added and the solvent was reduced in vacuo. Added sat. aq. NaHCO₃ solution (5 mL) and the product extracted with DCM; dried (Na₂SO₄). Evaporation of the solvent gave **(C2)** which was used in the next step without further purification. MS (ES) C₁₈H₁₉N₃O₂ requires: 309, found: 310 (M+H)⁺.

### Step 3: 2-{4-[(Dimethylamino)methyl]phenyl}-2H-indazole-7-carboxamide(C3)

**(C2)** was heated in 7N NH₃ in MeOH (0.1 M) in a sealed tube overnight at 50°C. The solvents were reduced in vacuo and the residue was purified by flash column chromatography (DCM : MeOH = 90:10). ¹H NMR (300MHz, CD₃CN, 300K) δ 8.85 (1H, s), 8.78 (1H, bs), 8.17 (1H, d, J = 8.0 Hz), 8.05-8.00 (3H, m), 7.56 (2H, d, J = 8.4 Hz), 7.28 (1H, t, J = 8.0 Hz), 6.36 (1H, bs), 3.56 (2H, s), 2.28 (6H, s). MS (ES) C₁₇H₁₈N₄O requires: 294, found: 295 (M+H)⁺.

### EXAMPLE 4 2-{4-[(N,N-Dimethylglycyl)amino]phenyl}-2H-indazole-7-carboxamide (D4)

### Step 1: Methyl 2-(4-nitrophenyl)-2H-indazole-7-carboxylate (D1)

**(D1)** was prepared following the general procedure reported in Example 3 step 1 using 4-fluoronitrobenzene and the crude was purified by flash column chromatography from EtOAc: Petroleum Ether = 10:90 to 50:50. ¹H NMR (400MHz, CDCl₃, 300K) δ 8.65 (1H, s), 8.44 (2H, d, J = 8.6 Hz), 8.23 (1H, d, J = 8.6 Hz), 8.19 (2H, d, J = 7.2 Hz), 7.98 (1H, d, J = 8.4 Hz), 7.24 (1H, m overlapped to solvent signal), 4.08 (3H, s). MS (ES) C₁₅H₁₁N₃O₄ requires: 297, found: 298 (M+H)⁺.

### Step 2: 2-(4-Nitrophenyl)-2H-indazole-7-carboxamide (D2)

**(D2)** was prepared following the general procedure reported in Example 3 step 3 and was used in the next step without further purification. ¹H NMR (400MHz, DMSO-d6, 300K) δ 9.52 (1H, s), 8.52-8.45 (5H, m), 8.10 (1H, d, J = 6.9 Hz), 8.05 (1H, d, J = 8.2 Hz), 7.94 (1H, bs), 7.30 (1H, dd, J = 8.2, 6.9 Hz). MS (ES) C₁₄H₁₀N₄O₃ requires: 282, found: 283 (M+H)⁺.

### Step 3: 4-[7-(Aminocarbonyl)-2H-indazol-2-yl]benzenaminium chloride (D3)

To a suspension of **(D2)** in MeOH (0.1M) Pd/C (10% w/w) and 6N HCl solution (1.0 eq.) were added. The reaction mixture was stirred under hydrogen atmosphere at RT for 8 h. To the suspension 1.25 M HCl in MeOH was added and then the catalyst was filtered off through a pad of celite. The solvent was reduced in vacuo and the crude used in the next step without further purification. ¹H NMR (400MHz, DMSO-d6, 300K) δ 9.22 (1H, s), 8.56 (1H, bs), 8.08 (2H, d, J = 8.6 Hz), 8.05 (1H, d, J = 6.9 Hz), 8.01 (1H, d, J = 8.2 Hz), 7.87 (1H, bs), 7.30-7.23 (3H, m). MS (ES) C₁₄H₁₃ClN₄O requires: 252, found: 253 (M+H)⁺.

### Step 4: 2-{4-[(N,N-Dimethylglycyl)amino]phenyl}-2H-indazole-7-carboxamide (D4)

To a solution of N,N-dimethylglycine (1.5 eq.) in DMF (0.1M) HATU (1.5 eq.) and Et₃N (1.5 eq.) were added and stirring was continued at RT for 30 min. Then, a solution of **(D3)** (1.0 eq.) and Et₃N (1.0 eq.) in DMF (0.1M) was added and the reaction mixture was stirred for overnight at RT. Solvent was reduced in vacuo, the residue portioned between EtOAc and sat. aq. NaHCO₃ solution. Organic phase was washed with brine, dried (Na₂SO₄) and concentrated under reduced pressure. Crude was purified by flash column chromatography (DCM : MeOH = 95:5). ¹H NMR (400MHz, DMSO-d6,300K) δ 10.01 (1H, bs), 9.25 (1H, s), 8.57 (1H, bs), 8.10 (2H, d, J = 9.0 Hz), 8.05 (1H, d, J 6.9 = Hz), 8.01 (1H, d, J = 8.2 Hz), 7.92 (2H, d, J = 9.0 Hz), 7.86 (1H, bs), 7.26 (1H, dd, J = 8.2, 6.9 Hz), 3.12 (2H, s), 2.30 (6H, s). MS (ES) C₁₈H₁₉N₅O₂ requires: 337, found: 338 (M+H)⁺.
The following Examples were prepared according to the methods of Examples 1-4.

| **Example** | **Name** | **MWt** | **(M+H)⁺** | **Procedure of Example** |
|---|---|---|---|---|
| 5 | 2-benzyl-2*H-*indazole-7-carboxamide | 251 | 252 | 1 |
| 6 | 2-(4-Chlorophenyl)-2H-indazole-7-carboxamide | 271,273 | 272,274 | 2 |
| 7 | 2-(2-Chlorophenyl)-2H-indazole-7-carboxamide | 271,273 | 272,274 | 2 |
| 8 | {4-[7-(Aminocarbonyl)-2H-indazol-2-yl]phenyl}-N,N-dimethylmethanaminium chloride | 294 | 295 | 3 |
| 9 | 2-{4-[(4-Methylpiperazin-1-yl)methyl]phenyl}-2H-indazole-7-carboxamide | 349 | 350 | 3 |
| 10 | 2-[4-(Morpholin-4-ylmethyl)phenyl]-2H-indazole-7-carboxamide | 336 | 337 | 3 |
| 11 | 2-{4-[(Methylamino)methyl]phenyl}-2H-indazole-7-carboxamide | 280 | 281 | 3 |
| 12 | 2-[4-(pyrrolidin-1-ylmethyl)phenyl]-2H-indazole-7-carboxamide | 320 | 321 | 3 |
| 13 | 2-[4-(piperidin-1-ylmethyl)phenyl]-2H-indazole-7-carboxamide | 334 | 335 | 3 |

### EXAMPLE 14 4-[({4-[7-(aminocarbonyl)-2H-indazol-2-yl]benzoyl}amino)methyl]pyridinium trifluoroacetate (E3)

### Step 1: 2-(4-Formylphenyl)-2H-indazole-7-carboxamide(E1)

(**E1**) was prepared following the general procedure reported in Example 3 step 1 using *2H-*indazole-7-carboxamide and 4-fluorobenzaldehyde and the crude was purified by precipitation from MeOH/EtOAc.¹H NMR (400 MHz, DMSO-d₆, 300K) δ 10.10 (s, 1H), 9.50 (s, 1H), 8.52 (br. s, 1H), 8.45 (d, J = 8.6 Hz, 2H), 8.15 (d, J = 8.6 Hz, 2H), 8.08 (d, J = 7.2 Hz, 1H), 8.04 (d, J = 7.2 Hz, 1H), 7.92 (br. s, 1H), 7.30 (t, J = 7.0 Hz, 1H). MS (ES⁺) C₁₅H₁₁N₃O₂ requires: 265, found: 266 (M+H)⁺.

### Step 2: 4-[7-(Aminocarbonyl)-2H-indazol-2-yl]benzoic acid (E2)

To a solution **of (E1)** (1.0 eq.) in t-BuOH/H₂O (5:1, 0.2 M) was added 2-methyl-2-butene (10 eq., 2.0 M in THF) in THF, then NaH₂PO₄ (7.0 eq.) followed by the addition of NaClO₂ (7.0 eq.) and the resulting mixture was stirred at RT O/N. Solvents were reduced under reduced pressure and **(E2)** was isolated by precipitation from 3N HCl.¹H NMR (400 MHz, DMSO-d₆, 300K) δ 9.45 (s, 1H), 8.52 (br. s, 1H), 8.32 (d, J = 8.6 Hz, 2H), 8.16 (d, J = 8.6 Hz, 2H), 8.08 (d, J = 7.7 Hz, 1H), 8.03 (d, J = 7.7 Hz, 1H), 7.92 (br. s, 1H), 7.30 (t, J = 7.0 Hz, 1H). MS (ES⁻) C₁₅H₁₁N₃O₃ requires: 281, found: 280 (M-H)⁻.

### Step 3: 4-[({4-[7-(aminocarbonyl)-2H-indazol-2-yl]benzoyl}amino)methyl]pyridinium trifluoroacetate (E3)

**(E2)** (1.0 eq.), HATU (1.5 eq.), Et₃N (1.5 eq.) were dissolved in DMF and the mixture was left 30 min under stirring at RT. Then, 1-pyridin-4-ylmethanamine (1.5 eq.) was added and the mixture left under stirring O/N. Evaporation of the solvent gave a residue which was purified by reverse phase HPLC (column: C18), using H₂O (0.1% TFA) and MeCN (+0.1% TFA) as eluents, the desired fractions were lyophilized to afford the titled compound **(E3).** ¹H NMR (400 MHz, CDCl₃) δ 9.45 (s, 1H), 9.42 (t, J = 5.8 Hz, 1H), 8.71 (d, J = 5.6 Hz, 2H), 8.54 (br. s, 1H), 8.34 (d, J = 8.6 Hz, 2H), 8.16 (d, J = 8.6 Hz, 2H), 8.08 (d, J= 6.8 Hz, 1H), 8.04 (d, J= 6.8 Hz, 1H), 7.91 (br. s, 1H), 7.71 (d, J = 5.3 Hz, 2H), 7.29 (t, J = 7.6 Hz, 1H), 4.68 (d, J = 5.5 Hz, 2H).MS (ES) C₂₁H₁₇N₅O₂ requires: 371, found: 372 (M+H)⁺.

### EXAMPLE 15 2-{4-[1-(Methylamino)ethyl]phenyl}-2H-indazole-7-carboxamide (F2)

### Step 1: 2-(4-Acetylphenyl)-2H-indazole-7-carboxamide (F1)

**(F1)** was prepared following the general procedure reported in Example 3 step 1 using 2*H-*indazole-7-carboxamide and 1-(4-fluorophenyl)ethanone and was used in the next step without further purification. ¹H NMR (400 MHz, DMSO-d₆, 300K) δ 9.48 (s, 1H), 8.53 (br. s, 1H), 8.36 (d, J = 8.8 Hz, 1H), 8.20 (d, J = 8.8 Hz, 1H), 8.09 (dd, J = 6.8, 0.8 Hz, 1H), 8.05 (dd, J = 8.4, 0.8 Hz, 1H), 7.92 (br. s, 1H), 7.30 (dd, J = 8.4, 7.2 Hz, 1H), 2.66 (s, 3H). MS (ES⁺) C₁₆H₁₃N₃O₂ requires: 279, found: 280 (M+H)⁺.

### Step 2: 2-{4-[1-(Methylamino)ethyl]phenyl}-2H-indazole-7-carboxamide (F2)

**(F2)** was prepared following the general procedure reported in Example 3 step 2 using MeNH₂.HCl and the crude was purified using an IST ISOLUTE^{®} SPE column SCX, loading the reaction mixture as a MeOH solution and then eluting the desired compound with 3N NH₃ in MeOH. The titled compound was isolated after evaporation of the solvent under reduced pressure. ¹H NMR (400 MHz, CDCl₃, 300K) δ 9.05 (br. s, 1H), 8.49 (s, 1H), 8.27 (d, J = 7.7 Hz, 1H), 7.87 (d, J = 7.7 Hz, 1H), 7.83 (d, J = 8.4 Hz, 2H), 7.50 (d, J = 8.4 Hz, 2H), 7.23 (t, J = 8.0 Hz, 1H), 6.46 (br. s, 1H), 3.77 (m, 1H), 2.56 (br. s, 1H), 2.35 (s, 3H), 1.41 (d, J = 6.6 Hz, 3H). MS (ES⁺) C₁₇H₁₈N₄O requires: 294, found: 295 (M+H)⁺.

### EXAMPLE 16 N-{4-[7-(aminocarbonyl)-2H-indazol-2-yl]benzyl}cyclohexanaminium trifluoroacetate (G1)

To MP-Triacetoxyborohydride (5 eq, loading 2.5 mmol/g) a solution of Example 14, **(E1)** (1 eq) in DMF was added followed by cyclohexanamine (1.03 eq.) in DMF. The mixture was heated in the MW (10 min, 80 °C) and then purified by RP-HPLC (column Atlantis dC18 5µm, gradient A: H₂O + 0.1% TFA; B: MeCN +0.1% TFA) and the desired fractions were evaporated under reduced pressure to yield the desired compound. ¹H NMR (400 MHz, DMSOd₆, 300K) δ 9.35 (s, 1H), 8.77 (br. s, 2H), 8.53 (br. s, 1H), 8.26 (d, J = 8.6 Hz, 2H), 8.06 (d, J = 7.1 Hz, 1H), 8.01 (d, J = 8.1 Hz, 1H), 7.88 (bs, 1H), 7.73 (d, J = 8.6 Hz, 2H), 7.28 (t, J = 7.1 Hz, 1H), 4.30-4.24 (m, 2H), 3.04 (br. s, 1H), 2.15-2.08 (m, 2H), 1.82-1.74 (m, 2H), 1.65-1.57 (m, 1H) 1.40-1.05 (m, 5H). MS (ES⁺) C₂₁H₂₄N₄O requires: 348, found: 349 (M+H)⁺.

### EXAMPLE 17 {4-[7-(aminocarbonyl)-4-chloro-2H-indazol-2-yl]phenyl}-N-methylmethanaminium trifluoroacetate (H5)

### Step 1: Methyl 2-amino-4-chloro-3-methylbenzoate (H1)

To a solution of 2-amino-4-chloro-3-methylbenzoic acid (1.0 eq., from Butt Park Ltd.) and cesium carbonate (1.5 eq.) in DMF (0.25 M) at RT was added methyl iodide (1.0 eq.). After the mixture was stirred for 18 h, brine was added and the mixture was extracted with EtOAc. The organic phase was dried (Na₂SO₄) and concentrated under reduced pressure. The yellow solid **(H1)** was used in the next step without further purification. ¹H NMR (400MHz, DMSO, 300K) δ 7.29 (1H, d, J = 8.6 Hz), 6.50 (2H, brs), 6.32 (1H, d, J = 8.6 Hz), 3.78 (3H, s), 2.17 (3H, s). MS (ES) C₉H₁₀ClNO₂ requires: 199, found: 168 (M-OCH₃)⁺.

### Step 2: Methyl 4-chloro-1H-indazole-7-carboxylate (H2)

To a solution of the ester **(H1)** (1.0 eq.) in chloroform (0.02 M) was added Ac₂O (2.3 eq.) and the mixture was stirred for two hours at RT. Then KOAc (0.3 eq.) and isoamyl nitrite (2.2 eq.) were added and the solution was heated at reflux for 18 h. The mixture was cooled to RT and diluted with DCM. The organic phase was washed carefully with sat. aq. NaHCO₃ solution and dried (Na₂SO₄). Evaporation of the solvent gave **(H2)** as a brown solid which was used in the next step without purification. MS (ES) C₉H₇ClN₂O₂ requires: 210, found: 211 (M+H)⁺.

### Step 3: 4-Chloro-1H-indazole-7-carboxamide (H3)

A mixture of **(H2)** (1.0 eq.) and KOH (1.3 eq.) in dioxane/water (0.1 M) was stirred for 12 h at RT. Solvents were removed under reduced pressure. The carboxylic acid was dissolved in DMF (0.1 M) and TBTU (1.5 eq.) was added. After 15 min DIPEA (2.0 eq.) and ammonia (3.0 eq., 0.5 M in THF) were added and the solution was stirred for 36 h. The mixture was diluted with EtOAc and then the organic phase was washed with sat. aq. NaHCO₃ solution and brine. Evaporation of the solvent gave **(H3)** as a yellow solid which was used in the next step without purification. ¹H NMR (300MHz, DMSO, 300K) δ 13.38 (1H, br. s), 8.19 (2H, br. s), 7.89 (1H, d, J = 7.9 Hz), 7.56 (1H, br. s), 7.27 (1H, d, J = 7.9 Hz). MS (ES) C₈H₆ClN₃O requires: 195, found: 196 (M+H)⁺.

### Step 4: 4-Chloro-2-(4-formylphenyl)-2H-indazole-7-carboxamide (H4)

**(H4)** was prepared following the procedure reported in Example 3 step 1. The reaction mixture was poured in water and the precipitation was filtered, washed with water and dried in vacuo. The yellow solid was used in the next step without purification. ¹H NMR (300MHz, DMSO, 300K) δ 10.09 (1H, s), 9.61 (1H, s), 8.51 (2H, d, J = 8.3 Hz), 8.37 (1H, br. s), 8.13 (2H, d, J = 8.3 Hz), 8.02 (1H, d, J = 7.5 Hz), 7.98 (1H, br. s), 7.38 (1H, d, J = 7.5 Hz). MS (ES) C₁₅H₁₀ClN₃O₂ requires: 299, found: 300 (M+H)⁺.

### Step 5: {4-[7-(aminocarbonyl)-4-chloro-2H-indazol-2-yl]phenyl}-N-methylmethanaminium trifluoroacetate (H5)

**(H5)** was prepared following the procedure reported in Example 3 step 2. After purification by preparative RP-HPLC (column: C18), using H₂O (0.1% TFA) and MeCN (+0.1% TFA) as eluents, the desired fractions were lyophilized to afford the titled compound **(H5)** as a yellow solid. ¹H NMR (300MHz, DMSO, 300K) δ 9.51 (1H, s), 8.87 (2H, brs), 8.39 (1H, brs), 8.35 (2H, d, J = 8.6 Hz), 8.02 (1H, d, J = 7.6 Hz), 7.98 (1H, brs), 7.71 (2H, d, J = 8.6 Hz), 7.38 (1H, d, J = 7.6 Hz), 4.23 (2H, s), 2.16 (3H, s). MS (ES) C₁₆H₁₅ClN₄O requires: 314, found: 315 (M+H)⁺.

### EXAMPLE 18 2-Phenyl-2H-1,2,3-benzotriazole-4-carboxamide (I4)

### Step 1: Methyl 2,3-diaminobenzoate (I1)

A mixture of 2-amino-3-nitro-benzoic acid methyl ester (1.0 eq., from CHESS GmbH) and Pd/C (10% w/w) in MeOH (0.25 M) was stirred for 3 d at RT under H₂ atmosphere (1 atm). The mixture was filtered through Celite® and then the solvent was evaporated under reduced pressure. The red solid **(I1)** was used in the next step without further purification. ¹H NMR (300MHz, CDCl₃, 300K) δ 7.46 (1H, dd, J = 8.1 Hz, 1.4 Hz), 6.84 (1H, dd, J = 7.5 Hz, 1.4 Hz), 6.59 (1H, dd, J = 8.1 Hz, 7.5 Hz), 3.87 (3H, s). MS (ES) C₈H₁₀N₂O₂ requires: 166, found: 167 (M+H)⁺.

### Step 2: Methyl 3-amino-2-[phenyldiazenyl]benzoate (I2)

A mixture of **(I1)** (1.0 eq.) and nitrosobenzene (1.1 eq.) in AcOH (0.1 M) was stirred for 3 h at RT. Evaporation of the solvent gave **(I2)** as a yellow solid which was used in the next step without purification. MS (ES) C₁₄H₁₃N₃O₂ requires: 255, found: 256 (M+H)⁺.

### Step 3: Methyl 2-phenyl-2H-1,2,3-benzotriazole-4-carboxylate (I3)

A mixture of **(I2)** (1.0 eq.) and copper (II) acetate (1.0 eq.) in DMF (0.4 M) was stirred for 90 min at 80°C while oxygen was bubbled through this solution. The mixture was cooled to RT and diluted with EtOAc. The organic phase was washed with brine and dried (Na₂SO₄). Evaporation of the solvent gave **(I3)** as a yellow oil which was used in the next step without purification. MS (ES) C₁₄H₁₁N₃O₂ requires: 253, found: 276 (M+Na)⁺.

### Step 4: 2-Phenyl-2H-1,2,3-benzotriazole-4-carboxamide (I4)

A mixture of **(I3)** (1.0 eq.) and NaOH (1.5 eq.) in dioxane/water (0.1 M) was stirred for 5 h at RT. Solvents were removed under reduced pressure. The carboxylic acid was dissolved in DMF (0.1 M) and HATU (1.5 eq.) was added. After 15 min DIEA (2.0 eq.) and ammonia (3.0 eq., 0.5 M in THF) were added and the mixture was stirred for 36 h. The mixture was diluted with EtOAc and then the organic phase was washed with sat. aq. NaHCO₃ solution and brine. Evaporation of the solvent gave a crude which was purified by preparative RP-HPLC (column: C18), using H₂O (0.1% TFA) and MeCN (+0.1% TFA) as eluents. The desired fractions were lyophilized to afford the titled compound **(I4)** as a yellow solid. ¹H NMR (300MHz, DMSO, 300K) δ 8.44 (2H, d, J = 8.0 Hz), 8.25 (1H, d, J = 8.5 Hz), 8.12 (2H, d, J = 7.1 Hz), 8.03 (1H, br. s), 7.72-7.56 (4H, m). MS (ES) C₁₃H₁₀N₄O requires: 238, found: 239 (M+H)⁺.

### EXAMPLE 19 2-Benzyl-2H-1,2,3-benzotriazole-4-carboxamide (J3)

### Step 1: Methyl 1H-1,2,3-benzotriazole-7-carboxylate (J1)

To a solution of Example 18, **(I1)** (1.0 eq.) in AcOH (0.4 M) at 0°C was slowly added NaNO₂ (1.1 eq.) in water (6 M). After 1 h the suspension was filtered and the precipitation was washed with water. The white solid **(J1)** was dried in vacuo and was used in the next step without further purification. ¹H NMR (300MHz, DMSO, 300K) δ 15.91 (1H, br. s), 8.36 (1H, d, J = 7.9 Hz), 8.10 (1H, d, J = 6.9 Hz), 7.50 (1H, dd, J = 7.9 Hz, 6.9 Hz), 3.92 (3H, s). MS (ES) C₈H₇N₃O₂, requires: 177, found: 178 (M+H)⁺.

### Step 2: Methyl 2-benzyl-2H-1,2,3-benzotriazole-4-carboxylate (J2)

To a solution of **(J1)** and Cs₂CO₃ (3.0 eq.) in DMF (0.1 M) was added benzyl bromide (1.2 eq.) and the mixture was stirred for 1 h at 80°C. The reaction mixture was cooled to RT and diluted with EtOAc. The organic phase was washed with brine and dried (Na₂SO₄). The solvents were removed under reduced pressure and the residue purified by flash column chromatography on silica using a gradient of EtOAc/Petroleum ether from 0:100 to 60:40 to yield the desired triazole **(J2)** as a white solid. MS (ES) C₁₅H₁₃N₃O₂ requires: 267, found: 268 (M+H)⁺.

### Step 3: 2-Benzyl-2H-1,2,3-benzotriazole-4-carboxamide (J3)

**(J3)** was prepared from **(J2)** following the general procedure reported for Example 1 step 6. After purification by preparative RP-HPLC (column: C18), using H₂O (0.1 % TFA) and MeCN (+0.1 % TFA) as eluents, the desired fractions were lyophilized to afford the titled compound **(J3)** as a white solid. ¹H NMR (400MHz, DMSO, 300K) δ 8.14 (1H, d, J = 8.6 Hz), 8.04 (1H, d, J = 7.0 Hz), 7.97 (1H, br. s), 7.94 (1H, br. s), 7.56 (1H, dd, J = 8.6 Hz, 7.0 Hz), 7.46-7.31 (5H, m), 6.05 (2H, s). MS (ES) C₁₄H₁₂N₄O requires: 252, found: 253 (M+H)⁺.

### EXAMPLE 20 2-{3-[(methylamino)methyl]phenyl}-2H-indazole-7-carboxamide (K5)

### Step 1: methyl 3-((E)-{[3-(1,3-dioxolan-2-yl)phenyl]imino}methyl)-2-nitrobenzoate (K1)

**(K1)** was prepared following the general procedure reported for Example 1 step 4 using methyl 3-formyl-2-nitrobenzoate (**A3**) and 3-aminobenzaldehyde ethylene acetal until TLC revealed completation of the reaction (Petroleum ether:EtOAc = 85:15) and was used in the next step without further purification.

### Step 2: methyl 2-[3-(1,3-dioxolan-2-yl)phenyl]-2H-indazole-7-carboxylate (K2)

**(K2)** was prepared following the general procedure reported for Example 1 step 5 and the crude was purified by flash column cromatography on silica using a gradient of 10-20% EtOAc/Petroleum ether to yield the desired **(K2)** as an orange oil. MS (ES) C₁₈H₁₆N₂O₄ requires: 324, found: 325 (M+H)⁺.

### Step 3: methyl 2-(3-formylphenyl)-2H-indazole-7-carboxylate (K3)

(**K2**) was stirred in a mixture of DCM:TFA:H₂O (8:1:1, 0.1M) for 1 hr at RT. Evaporation of the solvent under reduced pressure yielded the desired **(K3)** as brown oil. ¹H NMR (400 MHz, CDCl₃, 300K) δ 10.13 (1H, s), 8.63 (1H, s), 8.45 (1H, s), 8.34 (1H, d, J = 8.1 Hz), 8.16 (1H, d, J = 6.8 Hz), 7.98 (1H, d, J = 8.3 Hz), 7.96 (1H, d, J = 7.6 Hz), 7.73 (1H, t, J = 7.8 Hz), 7.30 (1H, dd, J = 8.3, 7.3 Hz), 4.06 (3H, s). MS (ES) C₁₆H₁₂N₂O₃ requires: 280, found: 281 (M+H)⁺.

### Step 4: methyl 2-{3-[(methylamino)methyl]phenyl}-2H-indazole-7-carboxylate (K4)

**(K4)** was prepared as described in Example 3 step 2 and the crude product was used in the next step without further purification. MS (ES) C₁₇H₁₇N₃O₂ requires: 295, found: 296 (M+H)⁺.

### Step 5: 2-{3-[(methylamino)methyl]phenyl}-2H-indazole-7-carboxamide (K5)

**(K5)** was prepared as described in Example 3 step 3 and the crude product was purified by preparative RP-HPLC (column: C18), using H₂O (0.1% TFA) and MeCN (+0.1% TFA) as eluents. The desired fractions were lyophilized to afford the titled compound (**K5**) as a yellow solid. ¹H NMR (400 MHz, DMSO-d6, 300K) δ 9.32 (1H, s), 8.96 (1H, br. s), 8.54 (1H, br. s), 8.33 (1H, s), 8.20 (1H, d, J = 8.0 Hz), 8.09 (1H, d, J = 7.1 Hz), 8.05 (1H, d, J = 8.2 Hz), 7.98 (1H, br. s), 7.71 (1H, t, J = 8.0 Hz), 7.59 (1H, d, J = 8.0 Hz), 7.30 (1H, dd, J = 8.2, 7.1 Hz), 4.30 (2H, s), 2.64 (3H, s). MS (ES) C₁₆H₁₆N₄O requires: 280, found: 281 (M+H)⁺.

### EXAMPLE 21 4-({3-[7-(aminocarbonyl)-2H-indazol-2-yl]benzoyl}amino)piperidinium trifluoroacetate

### Step 1: methyl 3-((E)-{[3-(tert-butoxycarbonyl)phenyl]imino}methyl)-2-nitrobenzoate (L1)

**(L1)** was prepared following the general procedure reported for Example 1 step 4 using methyl 3-formyl-2-nitrobenzoate (**A3**) and *tert*-butyl 3-aminobenzoate until TLC revealed completation of the reaction (Petroleum ether:EtOAc = 85:15) and was used in the next step without further purification.

### Step 2: methyl 2-[3-(tert-butoxycarbonyl)phenyl]-2H-indazole-7-carboxylate (L2)

**(L2)** was prepared following the general procedure reported for Example 1 step 5 and the crude was purified by flash column cromatography on silica using a gradient of 10-20% EtOAc/Petroleum ether to yield the desired **(L2)** as an orange solid. ¹H NMR (400 MHz, CDCl₃, 300K) δ 8.61 (1H, s), 8.49 (1H, s), 8.29 (1H, d, J = 8.1 Hz), 8.15 (1H, d, J = 6.8 Hz), 8.04 (1H, d, J = 7.8 Hz), 7.97 (1H, d, J = 8.3 Hz), 7.60 (1H, t, J = 7.9 Hz), 7.21 (1H, t, J = 7.7 Hz), 4.06 (3H, s), 1.64 (9H, s). MS (ES) C₂₀H₂₀N₂O₄ requires: 352, found: 353 (M+H)⁺.

### Step 3: tert-butyl 3-[7-(aminocarbonyl)-2H-indazol-2-yl]benzoate (L3)

(L3) was prepared as described in Example 3 step 3 and the crude product was used in the next step without further purification. ¹H NMR (400 MHz, CDCl₃, 300K) δ 9.10 (1H, br. s), 8.61 (1H, s), 8.47 (1H, s), 8.32 (1H, d, J = 7.1 Hz), 8.13 (1H, d, J = 8.1 Hz), 8.07 (1H, d, J = 7.6 Hz), 7.93 (1H, d, J = 8.3 Hz), 7.63 (1H, t, J = 7.9 Hz), 7.28 (1H, dd, J = 8.1, 7.3 Hz), 6.26 (1H, br. s), 1.65 (9H, s). MS (ES) C₁₉H₁₉N₃O₃ requires: 337, found: 338 (M+H)⁺.

### Step 4: 3-[7-(aminocarbonyl)-2H-indazol-2-yl]benzoic acid (L4)

(L3) was stirred in a mixture of DCM:TFA (9:1, 0.1M) at RT overnight. The solvent was evaporated under reduced pressure, the residue taken up with EtOAc, washed with brine, dried (Na₂SO₄) and concentrated under reduced pressure affording **(L4)** as a pale brown solid which was used in the next step without further purification. ¹H NMR (400 MHz, DMSO-d6,300K) δ 9.45 (1H, s), 8.62 (1H, s), 8.53 (1H, br. s), 8.44 (1H, d, J = 8.1 Hz), 8.10-8.00 (3H, m), 7.91 (1H, br. s), 7.77 (1H, t, J = 7.9 Hz), 7.29 (1H, dd, J = 8.1, 7.1 Hz). MS (ES) C₁₅H₁₁N₃O₃ requires: 295, found: 296 (M+H)⁺.

### Step 5: tert-butyl 4-({3-[7-(aminocarbonyl)-2H-indazol-2-yl]benzoyl}amino) piperidine-1-carboxylate (L5)

(**L5**) was prepared as described in Example 4, step 4 and the crude product was purified by preparative RP-HPLC (column: C18), using H₂O (0.1% TFA) and MeCN (+0.1% TFA) as eluents. The desired fractions were lyophilized to afford the titled compound (**L5**) as a white solid. MS (ES) C₂₅H₂₉N₅O₄ requires: 463, found: 464 (M+H)⁺.

### Step 6: 4-({3-[7-(aminocarbonyl)-2H-indazol-2-yl]benzoyl}amino)piperidinium trifluoroacetate (L6)

**(L5)** was stirred in a mixture of MeCN:TFA = 9:1 (0.1M) for 24 hr at RT. Solvent was evaporated under reduced pressure, the residue dissolved in a mixture of MeCN:H₂O = 1:1 and lyophilized to afford the title compound **(L6)** as a white solid. ¹H NMR (400 MHz, DMSO-d6, 300K) δ 9.38 (1H, s), 8.69 (1H, d, J = 7.3 Hz), 8.60 (1H, br. s), 8.57-8.51 (2H, m), 8.40 (1H, br. s), 8.34 (1H, d, J = 8.1 Hz), 8.09 (1H, d, J = Hz), 8.05 (1H, d, J = 7.1 Hz), 7.97 (1H, d, J = 8.3 Hz), 7.92 (1H, br. s), 7.73 (1H, t, J = 7.9 Hz), 7.29 (1H, dd, J = 8.3, 7.2 Hz), 4.18-4.06 (1H, m), 3.40-3.28 (2H, m overlapped to H₂O signal), 3.14-2.98 (2H, m), 2.09-1.99 (2H, m), 1.83-1.69 (2H, m). MS (ES) C₂₀H₂₁N₅O₂ requires: 363, found: 364 (M+H)⁺.

The following examples were prepared according to the methods of the previous examples.

| **Example** | **Name** | **MWt** | **(M+H)⁺** | **Procedure of Example** |
|---|---|---|---|---|
| 22 | {4-[7-(Aminocarbonyl)-2H-indazol-2-yl]phenyl}-N-methylmethanaminium chloride | 280 | 281 | 3 |
| 23 | 2-{3-Chloro-4-[(dimethylamino)methyl]phenyl}-2H-indazole-7-carboxamide | 328 | 329 | 3 |
| 24 | 1-[2-({4-[7-(Aminocarbonyl)-2H-indazol-2-yl]phenyl}amino)-2-oxoethyl]-4-methylpiperazin-1-ium trifluoroacetate | 392 | 393 | 4 |
| 25 | 2-(4-{[(4-Pyrrolidin-1-ylpiperidin-1-yl)acetyl]amino}phenyl)-2H-indazole-7-carboxamide | 446 | 447 | 4 |
| 26 | 2-{4-[(Pyrrolidin-1-ylacetyl)amino]phenyl}-2H-indazole-7-carboxamide | 363 | 364 | 4 |
| 27 | 2- {4-[(Piperidin-1-ylacetyl)amino]phenyl}-2H-indazole-7-carboxamide | 377 | 378 | 4 |
| 28 | 2-{4-[(Morpholin-4-ylacetyl)amino]phenyl}-2H-indazole-7-carboxamide | 379 | 380 | 4 |
| 29 | 4-[2-({4-[7-(Aminocarbonyl)-2H-indazol-2-yl]phenyl}amino)-2-oxoethyl]morpholin-4-ium chloride | 379 | 380 | 4 |
| 30 | 2-{4-[(Ethylamino)methyl]phenyl}-2H-indazole-7-carboxamide | 294 | 295 | 3 |
| 31 | 2-{4-[(Isopropylamino)methyl]phenyl}-2H-indazole-7-carboxamide | 308 | 309 | 3 |
| 32 | N-{4-[7-(Aminocarbonyl)-2H-indazol-2-yl]benzyl}propan-2-aminium chloride | 308 | 309 | 3 |
| 33 | 2-(4-{[(2-Fluoroethyl)amino]methyl}phenyl)-2H-indazole-7-carboxamide | 312 | 313 | 3 |
| 34 | 2-(4-{[(2,2-Difluoroethyl)amino]methyl}phenyl)-2H-indazole-7-carboxamide | 330 | 331 | 3 |
| 35 | 2-{4-[(Cyclopropylamino)methyl]phenyl}-2H-indazole-7-carboxamide | 306 | 307 | 3 |
| 36 | 4-{4-[7-(Aminocarbonyl)-2H-indazol-2-yl]benzoyl}-1,4-diazepan-1-ium trifluoroacetate | 363 | 364 | 5 |
| 37 | 2-({4-[7-(Aminocarbonyl)-2H-indazol-2-yl]benzoyl}amino)-N,N-dimethylethanaminium trifluoroacetate | 351 | 352 | 5 |
| 38 | 4-[({4-[7-(Aminocarbonyl)-2H-indazol-2-yl]benzoyl}amino)methyl]piperidinium trifluoroacetate | 377 | 378 | 5 |
| 39 | N-{4-[7-(Aminocarbonyl)-2H-indazol-2-yl]benzyl}-N,N',N'-trimethylethane-1,2-diaminium dichloride | 351 | 352 | 3 |
| 40 | 4-{4-[7-(Aminocarbonyl)-2H-indazol-2-yl]benzoyl}-1-methylpiperazin-1-ium trifluoroacetate | 363 | 364 | 5 |
| 41 | 3-[({4-[7-(Aminocarbonyl)-2H-indazol-2-yl]phenyl}amino)carbonyl]azetidinium trifluoroacetate | 335 | 336 | 4 |
| 42 | (2S)-2-[({4-[7-(Aminocarbonyl)-2H-indazol-2-yl]phenyl}amino)carbonyl]-1-methylpyrrolidinium trifluoroacetate | 363 | 364 | 4 |
| 43 | 3-[({4-[7-(Aminocarbonyl)-2H-indazol-2-yl]phenyl}amino)carbonyl]-1-methylpiperidinium trifluoroacetate | 377 | 378 | 4 |
| 44 | 4-[({4-[7-(Aminocarbonyl)-2H-indazol-2-yl]phenyl}amino)carbonyl]-1-methylpiperidinium trifluoroacetate | 377 | 378 | 4 |
| 45 | 4-({4-[7-(Aminocarbonyl)-2H-indazol-2-yl]benzoyl}amino)-1-benzylpiperidinium trifluoroacetate | 453 | 454 | 5 |
| 46 | 2-{4-[(Pyridin-4-ylamino)carbonyl]phenyl}-2H-indazole-7-carboxamide | 357 | 358 | 5 |
| 47 | 2-{4-[(4-Phenylpiperazin-1-yl)carbonyl]phenyl}-2H-indazole-7-carboxamide | 425 | 426 | 5 |
| 48 | 2-(4-{[Methyl(quinoxalin-6-ylmethyl)amino]carbonyl}phenyl)-2H-indazole-7-carboxamide | 436 | 437 | 5 |
| 49 | 2-(4-Formylphenyl)-2H-indazole-7-carboxamide | 265 | 266 | 5 |
| 50 | 1-[2-({4-[7-(Aminocarbonyl)-2H-indazol-2-yl]benzoyl}amino)ethyl]pyrrolidinium trifluoroacetate | 377 | 378 | 5 |
| 51 | 1-[2-({4-[7-(Aminocarbonyl)-2H-indazol-2-yl]benzoyl}amino)ethyl]piperidinium trifluoroacetate | 391 | 392 | 5 |
| 52 | 4-[2-({4-[7-(Aminocarbonyl)-2H-indazol-2-yl]benzoyl}amino)ethyl]morpholin-4-ium trifluoroacetate | 393 | 394 | 5 |
| 53 | 4-({4-[7-(Aminocarbonyl)-2H-indazol-2-yl]benzoyl}amino)-1-methylpiperidinium trifluoroacetate | 377 | 378 | 5 |
| 54 | 2-[4-[(4-methylpiperazin-1-yl)methyl]-3-(trifluoromethyl)phenyl]-2H-indazole-7-carboxamide | 417 | 418 | 3 |
| 55 | 2-[4-[(methylamino)methyl]-3-(trifluoromethyl)phenyl]-2H-indazole-7-carboxamide | 348 | 349 | 3 |
| 56 | 1-{4-[7-(aminocarbonyl)-2*H*-indazol-2-yl]phenyl}-*N*-methylethanaminium chloride | 294 | 295 | 6 |
| 57 | 2-[4-(pyrrolidin-1-ylmethyl)-3-(trifluoromethyl)phenyl]-2H-indazole-7-carboxamide | 388 | 389 | 1 3 |
| 58 | 2-[4-(piperidin-1-ylmethyl)-3-(trifluoromethyl)phenyl]-2H-indazole-7-carboxamide | 402 | 403 | 3 |
| 59 | 2-[4-[(ethylamino)methyl]-3-(trifluoromethyl)phenyl]-2H-indazole-7-carboxamide | 362 | 363 | 3 |
| 60 | 4-{4-[7-(Aminocarbonyl)-4-chloro-2H-indazol-2-yl]benzyl}-1-methylpiperazin-1-ium trifluoroacetate | 383 | 384 | 8 |
| 61 | 1-[2-({4-[7-(aminocarbonyl)-2*H-*indazol-2-yl]benzyl}ammonio)ethyl]piperidinium bis(trifluoroacetate) | 377 | 378 | 7 |
| 62 | 4-[2-({4-[7-(aminocarbonyl)-2*H-*indazol-2-yl]benzyl}ammonio)ethyl]morpholin-4-ium bis(trifluoroacetate) | 379 | 380 | 7 |
| 63 | 1-[2-({4-[7-(aminocarbonyl)-2*H*-indazol-2-yl]benzyl}ammonio)ethyl]pyrrolidinium bis(trifluoroacetate) | 363 | 364 | 7 |
| 64 | 4-({4-[7-(aminocarbonyl)-2*H*-indazol-2-yl]benzyl}ammonio)-1-methylpiperidimum bis(trifluoroacetate) | 363 | 364 | 7 |
| 65 | 4-({4-[7-(aminocarbonyl)-2*H*-indazol-2-yl]benzyl}ammonio)-1-benzylpiperidinium bis(trifluoroacetate) | 439 | 440 | 7 |
| 66 | 1-{4-[7-(aminocarbonyl)-2*H*-indazol-2-yl]benzyl}-4-phenylpiperazinediium bis(trifluoroacetate) | 411 | 412 | 7 |
| 67 | *N-*{4-[7-(aminocarbonyl)-2*H*-indazol-2-yl]benzyl}-2-(dimethylamino)-2-oxoethanaminium trifluoroacetate | 351 | 352 | 7 |
| 68 | 2-[({4-[7-(aminocarbonyl)-2*H*-indazol-2-yl]benzyl}ammonio)methyl]pyridinium bis(trifluoroacetate) | 357 | 358 | 7 |
| 69 | 4-[({4-[7-(aminocarbonyl)-2*H*-indazol-2-yl]benzyl}ammonio)methyl]pyridinium bis(trifluoroacetate) | 357 | 358 | 7 |
| 70 | *N-*{4-[7-(aminocarbonyl)-2*H*-indazol-2-yl]benzyl}-2-methylpropan-2-aminium trifluoroacetate | 322 | 323 | 7 |
| 71 | *N*'-{4-[7-(aminocarbonyl)-2*H*-indazol-2-yl]benzyl}-*N*,*N*-dimethylethane-1,2-diaminium bis(trifluoroacetate) | 337 | 338 | 7 |
| 72 | {4-[7-(aminocarbonyl)-2*H*-indazol-2-yl]phenyl}-*N*-(1,3-oxazol-2-ylmethyl)methanaminium trifluoroacetate | 347 | 348 | 7 |

### EXAMPLE 73 7-[7-(Aminocarbonyl)-2H-indazol-2-yl]-1,2,3,4-tetrahydroisoquinolinium chloride (M1)

**(M1)** was obtained following the general procedure reported in Example 1. ¹H NMR (400 MHz, DMSO-d6, 300K) δ 9.54 (2H, br. s), 9.30 (1H, s), 8.43 (1H, br. s), 8.05-7.98 (4H, m), 7.90 (1H, br. s), 7.47 (1H, d, J = 8.3 Hz), 7.27 (1H, dd, J = 8.2, 7.2 Hz), 4.45-4.35 (2H, m), 3.47-3.37 (2H, m), 3.14-3.04 (2H, m). MS (ES) C₁₇H₁₇ClN₄O requires: 292, found: 293 (M+H)⁺.

### EXAMPLE 74 6-[7-(Aminocarbonyl)-2H-indazol-2-yl]-1,2,3,4-tetrahydroisoquinolinium chloride (N1)

**(N1)** was obtained following the general procedure reported in Example 1. ¹H NMR (400 MHz, DMSO-d6,300K) δ 9.43-9.28 (3H, m), 8.53 (1H, br. s), 8.12-8.00 (4H, m), 7.89 (1H, br. s), 7.48 (1H, d, J = 8.3 Hz), 7.28 (1H, dd, J = 8.2, 7.2 Hz), 4.40-4.33 (2H, m), 3.55-3.35 (2H, m overlapped to the H₂O signal), 3.20-3.12 (2H, m). MS (ES) C₁₇H₁₇ClN₄O requires: 292, found: 293 (M+H)⁺.

### EXAMPLE 75 5-[7-(Aminocarbonyl)-2H-indazol-2-yl]-1,2,3,4-tetrahydroisoquinolinium trifluoroacetate (01)

**(O1)** was obtained following the general procedure reported in Example 1. The crude product was purified by preparative RP-HPLC (column: C18), using H₂O (0.1% TFA) and MeCN (+0.1% TFA) as eluents. The desired fractions were lyophilized to afford the titled compound **(O1)** as a white powder. ¹H NMR (400 MHz, DMSO-d6, 300K) δ 9.30-9.10 (2H, m), 8.90 (1H, s), 8.38 (1H, br. s), 8.13-8.03 (2H, m), 7.90 (1H, br. s), 7.64-7.48 (3H, m), 7.32 (1H, dd, J = 8.2, 7.2 Hz), 4.48-4.38 (2H, m), 3.40-3.28 (2H, m partially overlapped to the H₂O signal), 2.90-2.80 (2H, m). MS (ES) C₁₉H₁₇F₃N₄O₃ requires: 292, found: 293 (M+H)⁺.

### EXAMPLE 76 3-[({4-[7-(Aminocarbonyl)-5-fluoro-2H-indazol-2-yl]phenyl}amino)carbonyl]azetidinium trifluoroacetate (P4)

### Step 1: 5-Fluoro-2-(4-nitrophenyl)-2H-indazole-7-carboxamide (P1)

**(P1)** was prepared following the general procedure reported for Example 4 step 1 using Example 88, **(DD2)** and 4-fluoronitrobenzene. Solvent was evaporated under reduced pressure, the residue was portioned between EtOAc and H₂O and the precipitate filtered off giving the titled compound **(P1)** as yellow solid. ¹H NMR (400MHz, DMSO-d6, 300K) δ 9.47 (1H, s), 8.53-8.42 (5H, m), 8.12 (1H, br. s), 7.90-7.80 (2H, m). MS (ES) C₁₄H₉FN₄O₃ requires: 300, found: 301 (M+H)⁺.

### Step 2: 4-[7-(Aminocarbonyl)-5-fluoro-2H-indazol-2-yl]benzenaminium chloride (P2)

**(P2)** was prepared from **(P1)** following the general procedure reported for Example 4 step 3 and the crude product was used in the next step without further purification. ¹H NMR (400 MHz, DMSO-d6, 300K) δ 9.12 (1H, s), 8.57 (1H, br. s), 8.04 (1H, br. s), 7.95 (2H, d, J = 8.4 Hz), 7.83-7.73 (2H, m), 7.05 (2H, d, J = 8.4 Hz). MS (ES) C₁₄H₁₂ClFN₄O requires: 270, found: 271 (M+H)⁺.

### Step 3: tert-Butyl 3-[({4-[7-(aminocarbonyl)-5-fluoro-2H-indazol-2-yl]phenyl}amino) carbonyl]azetidine-1-carboxylate (P3)

A solution of 1-(*tert*-butoxycarbonyl)azetidine-3-carboxylic acid (1.3 eq) and HATU (1.3 eq) in DMF (0.2M) was stirred at RT for 30 min, then a solution of **(P2)** (1.0 eq) and DMAP (1.0 eq) in DMF (0.2 M) was added and stirring was continued at RT for O/N. Solvent was evaporated under reduced pressure, the residue was portioned between EtOAc and H₂O and the precipitate filtered off giving the titled compound **(P3)** as pale brown solid. ¹H NMR (400 MHz, DMSO-d6, 300K) δ 10.33 (1H, br. s), 9.20 (1H, s), 8.56 (1H, br. s), 8.11 (2H, d, J = 9.0 Hz), 8.05 (1H, br. s), 7.87-7.76 (4H, m), 4.10-3.90 (5H, m), 1.39 (9H, s). MS (ES) C₂₃H₂₄FN₅O₄ requires: 453, found: 454 (M+H)⁺.

### Step 4: 3-[({4-[7-(Aminocarbonyl)-5-fluoro-2H-indazol-2-yl]phenyl}amino)carbonyl] azetidinium trifluoroacetate (P4)

To a stirred solution **of (P3)** (1.0 eq) in DCM (0.1 M) TFA (10.0 eq) was added and the reaction mixture was stirred at RT for 2h. Solvent was evaporated under reduced pressure and the crude product was purified by preparative RP-HPLC (column: C18), using H₂O (0.1% TFA) and MeCN (+0.1% TFA) as eluents. The desired fractions were lyophilized to afford the titled compound **(P4)** as a yellow powder. ¹H NMR (400 MHz, DMSO-d6, 300K) δ 10.44 (1H, br. s), 9.23 (1H, s), 8.75 (2H, br. s), 8.54 (1H, br. s), 8.15 (2H, d, J = 9.0 Hz), 8.09 (1H, br. s), 7.89-7.77 (4H, m), 4.20-4.05 (4H, m), 3.85-3.73 (1H, m). MS (ES) C₂₀H₁₇F₄N₅O₄ requires: 353, found: 354 (M+H)⁺.

### EXAMPLE 77 2-(4-{[(Azetidin-3-ylcarbonyl)(methyl)amino]methyl}phenyl)-2H-indazole-7-carboxamide (Q2)

### Step 1: tert-Butyl 3-{[{4-[7-(aminocarbonyl)-2H-indazol-2-yl]benzyl}(methyl) amino]carbonyl}azetidine-1-carboxylate (Q1)

A solution of 1-(*tert*-butoxycarbonyl)azetidine-3-carboxylic acid (1.3 eq) and TBTU (1.3 eq) in DMF (0.2M) was stirred at RT for 30 min, then a solution of 2-{4-[(methylamino)methyl]phenyl}-2H-indazole-7-carboxamide (prepared following the general procedure reported in Example 3 for (C3)) (1.0 eq) and Et₃N (1.3 eq) in DMF (0.2M) was added and stirring was continued at RT for O/N. Solvent was reduced *in vacuo,* the residue dissolved in EtOAc, washed with saturated NaHCO₃ solution, brine, dried (Na₂SO₄) and concentrated under reduced pressure. Crude product was purified by preparative RP-HPLC (column: C18), using H₂O (0.1% TFA) and MeCN (+0.1% TFA) as eluents. The desired fractions were lyophilized to afford the titled compound **(Q1)** as a yellow powder. MS (ES) C₂₅H₂₉N₅O₄ requires: 463, found: 464 (M+H)⁺.

### Step 2: 2-(4-{[(Azetidin-3-ylcarbonyl)(methyl)amino]methyl}phenyl)-2H-indazole-7-carboxamide (Q2)

(**Q2**) was prepared following the general procedure reported for Example 21 step 6 and the crude product was purified by ISOLUTE® HM-N SCX cartridge to yield the titled compound (**Q2**) as a yellow solid. ¹H NMR (400 MHz, DMSO-d6, 300K) δ 9.30 (1H, s), 8.55 (1H, br. s), 8.11 (2H, d, J = 8.5 Hz), 8.06 (1H, d, J = 7.2 Hz), 8.02 (1H, d, J = 8.2 Hz), 7.90 (1H, br. s), 7.47 (1H, d, J = 8.5 Hz), 7.27 (1H, dd, J = 8.2, 7.2 Hz), 7.01 (1H, br. s), 4.65-4.57 (2H, m), 4.25-3.95 (5H, m), 2.82 (3H, s). MS (ES) C₂₀H₂₁N₅O₂ requires: 363, found: 364 (M+H)⁺.

### EXAMPLE 78 3-[({4-[7-(Aminocarbonyl)-2H-indazol-2-yl]benzyl}amino)carbonyl]azetidinium trifluoroacetate (R4)

### Step 1: 2-{4-[(Hydroxyimino)methyl]phenyl}-2H-indazole-7-carboxamide (R1)

To a stirred suspension of Example 14, **E1** (1.0 eq) and hydroxylamine hydrochloride (4.0 eq) in dry CH₃CN (0.035 M) was added Et₃N (4.0 eq). The reaction mixture was stirred at reflux for 3h, and then cooled to RT. The titled compound (**R1**) was isolated by precipitation from the reaction mixture and used in the next step without further purification. ¹H NMR (400 MHz, DMSO-d6, 300K) δ 11.45 (1H, s), 9.37 (1H, s), 8.55 (1H, br. s), 8.30-8.18 (3H, m), 8.06 (1H, d, J = 6.8 Hz), 8.02 (1H, d, J = 8.3 Hz), 7.89 (1H, br. s), 7.83 (2H, d, J = 8.2 Hz), 7.27 (1H, t, J = 7.5 Hz). MS (ES) C₁₅H₁₂N₄O₂, requires: 280, found: 281 (M+H)⁺.

### Step 2: {4-[7-(Aminocarbonyl)-2H-indazol-2-yl]phenyl}methanaminium chloride (R2)

To a suspension of (**R1**) in MeOH (0.1M) Pd/C (10% w/w) and 6N HCl solution (1.0 eq.) were added. The reaction mixture was stirred at RT under hydrogen atmosphere O/N. To the suspension 1.25 M HCl in MeOH was added and then the catalyst was filtered off through a pad of Celite. The solvent was reduced *in vacuo* and the crude product used in the next step without further purification. ¹H NMR (400 MHz, DMSO-d6, 300K) 9.37 (1H, s), 8.64 (3H, br. s), 8.55 (1H, br. s), 8.23 (2H, d, J = 8.5 Hz), 8.06 (1H, d, J = 6.8 Hz), 8.02 (1H, d, J = 8.2 Hz), 7.87 (1H, br. s), 7.76 (2H, d, J = 8.2 Hz), 7.28 (1H, dd, J = 6.8, 8.1 Hz), 4.15-4.08 (2H, m). MS (ES) C₁₅H₁₄N₄O requires: 267, found: 268 (M+H)⁺.

### Step 3: tert-Butyl 3-[({4-[7-(aminocarbonyl)-2H-indazol-2-yl]benzyl}amino)carbonyl] azetidine-1-carboxylate (R3)

(R3) was prepared following the general procedure reported for Example 77 step 1 and the crude product was purified by preparative RP-HPLC (column: C18), using H₂O (0.1% TFA) and MeCN (+0.1% TFA) as eluents The desired fractions were lyophilized to afford the titled compound **(R3)** as a yellow powder. MS (ES) C₂₄H₂₇N₅O₄ requires: 449, found: 450 (M+H)⁺.

### Step 4: 3-[({4-[7-(Aminocarbonyl)-2H-indazol-2-yl]benzyl}amino)carbonyl]azetidinium trifluoroacetate (R4)

(**R4**) was prepared following the general procedure reported for Example 21 step 6. ¹H NMR (400 MHz, DMSO-d6, 300K) δ 9.30 (1H, s), 8.77-8.64 (3H, m), 8.55 (1H, br. s), 8.11 (2H, d, J = 8.5 Hz), 8.06 (1H, d, J = 7.1 Hz), 8.02 (1H, d, J = 8.2 Hz), 7.90 (1H, br. s), 7.51 (2H, d, J = 8.5 Hz), 7.27 (1H, dd, J = 8.2, 7.1 Hz), 4.45-4.38 (2H, m), 4.15-3.98 (5H, m). MS (ES) C₂₁H₂₀F₃N₅O₄ requires: 349, found: 350 (M+H)⁺.

### EXAMPLE 79 2-(4-Bromophenyl)-5-fluoro-2H-indazole-7-carboxamide (U1)

To a solution of Example 88, **DD2** (1.0 eq) in DMF (0.2 M) K₂CO₃ (1.3 eq) and 4-bromofluorobenzene (10.0 eq) were added and the reaction mixture was heated under MW conditions at 180 °C for 20 min. The reaction mixture was cooled to RT and diluted with EtOAc. The organic phase was washed with brine; dried (Na₂SO₄). Evaporation of the solvent gave (**U1**) which was purified by chromatography on silica gel eluting with 50-70% EtOAc/Petroleum ether to obtain the title compound as a yellow powder. ¹H NMR (400 MHz, DMSO-d₆, 300K) δ 9.34 (1H, s), 8.50 (1H, br. s), 8.17 (2H, d, J = 9.0 Hz), 8.03 (1H, br. s), 7.90-7.80 (4H, m). MS (ES⁺) C₁₄H₉BrFN₃O requires: 334/336, found: 335/337 (M+H)⁺.

### EXAMPLE 80 5-Fluoro-2-(4-pyridin-3-ylphenyl)-2H-indazole-7-carboxamide (U2)

A mixture of **(U1)** from Example 79 (1.0 eq) and pyridine-3-boronic acid (1.3 eq) in DMF (1.0 M) together with 2N Na₂CO₃ solution (2.0 eq) was degassed with a stream of Ar for 30 min. ^{t}Bu₃PH⁺ BF₄⁻ (0.05 eq) and Pd₂(dba)₃ (0.05 eq) were added and the reaction mixture was heated at 90°C for 48 h. The mixture was cooled to RT, DCM was added and the organic phase was washed with sat. aq. NaHCO₃ solution, brine, dried (Na₂SO₄). The solution was concentrated under reduced pressure and the residue was purified by chromatography on silica gel eluting with 50-90% EtOAc/Petroleum ether then 10%MeOH/DCM to obtain the title compound as a yellow powder. ¹H NMR (400 MHz, DMSO-d₆, 300K) δ 9.40 (1H, s), 9.01 (1H, d, J = 1.6 Hz), 8.63 (1H, dd, J = 4.8, 1.6 Hz), 8.57 (1H, br. s), 8.32 (2H, d, J = 8.8 Hz), 8.20 (1H, d, J = 7.8 Hz), 8.10 (1H, br. s), 8.01 (2H, d, J = 8.8 Hz), 7.88-7.82 (2H, m), 7.54 (1H, dd, J = 7.8, 4.8 Hz). MS (ES) C₁₉H₁₃FN₄O requires: 332, found: 333 (M+H⁺).

### EXAMPLE 81 2-(4-Pyridin-3-ylphenyl)-2H-indazole-7-carboxamide (V2)

### Step 1: 2-(4-Bromophenyl)-2H-indazole-7-carboxamide (V1)

**(V1)** was prepared following the general procedure reported in Example 3 step 1 using 2*H-*indazole-7-carboxamide and 4-bromofluorobenzene and the crude was purified by chromatography on silica gel eluting with 80-90% EtOAc/Petroleum ether to obtain the title compound as a yellow powder. MS (ES⁺) C₁₄H₁₀BrN₃O requires: 315/317, found: 316/318 (M+H)⁺.

### Step 2: 2-(4-Pyridin-3-ylphenyl)-2H-indazole-7-carboxamide (V2)

**(V2)** was prepared following the general procedure reported in Example 80 using **(V1)** and pyridine-3-boronic acid and the crude was purified by chromatography on silica gel eluting with 90% EtOAc/Petroleum ether then 10%MeOH/DCM to obtain the title compound as a yellow powder. ¹H NMR (400 MHz, DMSO-d₆, 300K) δ 9.42 (1H, s), 9.01 (1H, d, J = 2.0 Hz), 8.63 (1H, dd, J = 4.8, 1.5 Hz), 8.58 (1H, br. s), 8.31 (2H, d, J = 8.6 Hz), 8.19 (1H, d, J = 7.8 Hz), 8.10-8.03 (2H, m), 8.01 (2H, d, J = 8.6 Hz), 7.89 (1H, br. s), 7.56-7.50 (1H, m), 7.35-7.25 (1H, m). MS (ES) C₁₉H₁₄N₄O requires: 314 found: 315 (M+H⁺).

### EXAMPLE 82 4-{4-[7-(Aminocarbonyl)-2H-indazol-2-yl]phenyl-1-methylpiperazin-1-ium trifluoroacetate (W1)

**(W1)** was prepared following the general procedure reported in Example 1 using methyl 3-formyl-2-nitrobenzoate and 4-(4-methylpiperazin-1-yl)aniline and the product was isolated by reverse phase RP-HPLC (column: C18), using H₂O (0.1% TFA) and MeCN (0.1% TFA) as eluents, the desired fractions were lyophilized to afford the titled compound (**W1**) as a white powder. ¹H NMR (400MHz, DMSO, 300K) δ 10.11 (1H, br. s), 9.22 (1H, s), 8.58 (1H, s), 8.10-7.90 (4H, m), 7.86 (1H, br. s), 7.30-7.20 (3H, m), 4.10-3.90 (2H, m), 3.60-3.50 (2H, m), 3.30-3.00 (4H, m), 2.88 (3H, s). MS (ES) C₂₀H₂₂N₄O₂ requires: 335, found: 336 (M+H⁺).

### EXAMPLE 83 4-{4-[7-(Aminocarbonyl)-2H-indazol-2-yl]phenyl}piperidinium trifluoroacetate (X1)

**(X1)** was prepared following the general procedure reported in Example 1 using methyl 3-formyl-2-nitrobenzoate and *tert*-butyl 4-(4-aminophenyl)piperidine-1-carboxylate and the product was isolated by reverse phase RP-HPLC (column: C18), using H₂O (0.1% TFA) and MeCN (0.1% TFA) as eluents, the desired fractions were lyophilized to afford the titled compound (**X1**) as a white powder. ¹H NMR (400MHz, DMSO, 300K) δ 9.30 (1H, s), 8.60-8.50 (2H, br. s), 8.40-8.30 (1H, br. s), 8.12 (2H, d, J = 8.6 Hz), 8.10-8.00 (2H, m), 7.91 (1H, br. s), 7.48 (2H, d, J = 8.6 Hz), 7.30-7.25 (1H, m), 3.50-3.40 (2H, m), 3.10-2.90 (2H, m), 2.10-2.00 (2H, m), 1.90-1.80 (2H, m). MS (ES) C₁₉H₂₀N₄O requires: 320, found: 321 (M+H⁺).

### EXAMPLE 84 2-{4-[7-(Aminocarbonyl)-2H-indazol-2-vl]phenyl}-N-methylethanaminium trifluoroacetate (Y3)

### Step 1: 2-[4-(2-Oxoethyl)phenyl]-2H-indazole-7-carboxamide (Y1)

To a suspension of (methoxymethyl)triphenylphosphonium chloride (2.0 eq) in dry Et₂O:THF (1:1, 0.2 M) under Ar at -78 °C was added t-BuOK (2.0 eq). The reaction mixture was stirred for 30 min at - 78 °C, then 30 min at 0 °C, cooled again to -78 °C and Example 14, (**E1**) (1.0 eq) was added and the resulting solution was stirred O/N at RT. The reaction mixture was diluted with EtOAc, washed with H₂O, dried (Na₂SO₄). Evaporation of the solvent gave the intermediate enol ether which was used in the next step without further purification. MS (ES) C₁₇H₁₅N₃O₂ requires: 293, found: 294 (M+H⁺).

The enol ether (1.0 eq) was dissolved in THF:Et₂O (1:1, 0.1 M) and HCl (3.0 eq, 2.0 M in Et₂O) was added and the solution was stirred at RT for 3h. The reaction mixture was quenched by the addition of sat. aq. NaHCO₃ solution until pH ∼5 and stirred at RT for 5 min. Organic layer was separated, washed with brine and dried (Na₂SO₄). Evaporation of the solvent gave the aldehyde intermediate (**Y1**) which was used in the next step without further purification. MS (ES) C₁₆H₁₃N₃O₂ requires: 279, found 280 (M+H)⁺.

### Step 2: 2-(4-{2-[Benzyl(methyl)amino]ethyl}phenyl)-2H-indazole-7-carboxamide (Y2)

**(Y1)** was dissolved in MeOH (0.1 M) and benzaldehyde (1.0 eq) was added followed by a solution of NaBH₃CN (1.1 eq) and ZnCl₂ (0.5 eq) in MeOH (0.5 mL). The pH was adjusted to ∼6 with 1.25 M HCl in MeOH and the mixture stirred at RT for 1h after which the formation off 2-{4-[2-(benzylamino)ethyl]phenyl}-2*H*-indazole-7-carboxamide was observed (MS (ES) C₂₃H₂₂N₄O requires: 370, found: 371 (M+H)⁺). Then, formaldehyde (1.0 eq) was added and the reaction mixture stirred for additional 1h after which the formation of **(Y2)** was observed (MS (ES) C₂₄H₂₄N₄O requires: 384, found: 385 (M+H)⁺). 6N HCl (0.1 mL) was added and the solvent was removed under reduced pressure. The reaction mixture was diluted with DCM, washed with sat. aq. NaHCO₃ solution, then brine and dried (Na₂SO₄). Evaporation of the solvent under reduced pressure gave **(Y2)** which was used in the next step without further purification.

### Step 3: 2-{4-[7-(Aminocarbonyl)-2H-indazol-2-yl]phenyl}-N-methylethanaminium trifluoroacetate (Y3)

To a solution of **(Y2)** (1.0 eq) in MeOH (0.1M) were added Pd/C (10% w/w) and 6N HCl solution (1.0 eq). The reaction mixture was stirred under H₂ atmosphere (1 atm) O/N. The catalyst was filtered through a pad of Celite and solvent was removed under vacuum and the crude was purified by reverse phase RP-HPLC (column: C18), using H₂O (0.1% TFA) and MeCN (0.1% TFA) as eluents, the desired fractions were lyophilized to afford the titled compound as a white powder. ¹H NMR (400MHz, DMSO, 330K) δ 9.25 (1H, s), 8.51 (2H, br. s), 8.10 (2H, d, J = 8.6 Hz), 8.05 (2H, m), 7.94 (1H, br. s), 7.53 (2H, d, J = 8.6 Hz), 7.30-7.25 (1H, m, overlapped with TFA signal), 3.00-2.80 (4H, m), 2.59 (3H, s). MS (ES) C₁₇H₁₈N₄O requires: 294, found: 295 (M+H⁺).

### EXAMPLE 85 2-[({4-[7-(Aminocarbonyl)-2H-indazol-2-yl]phenyl}amino)carbonyl}azetidinium trifluoroacetate (Z1)

To a solution of 1-(*tert*-butoxycarbonyl)azetidine-3-carboxylic acid (1 eq) in DMF/DCM (3:2, 0.05 M)) was added PL-Mukaiyama resin (1.9 eq), 2-(4-aminophenyl)-2*H*-indazole-7-carboxamide (free base of Example 4, **D3**) (0.83 eq), TEA (4 eq) and PS-DMAP (0.19 eq). The mixture was stirred at RT for 24 hours, filtered through a silica-carbonate cartridge (2 g) and evaporated. Purification by RP-HPLC (column XBridge C18 5µm, 19X100mm, gradient A: H₂O + 0.1% TFA; B: MeCN + 0.1% TFA) and evaporation of the desired fractions yield the desired compound. ¹H NMR (300 MHz, DMSOd₆ + TFA, 300K) δ 10.70 (1H, s), 9.26 (1H, s), 9.14 (1H, br. s), 8.93 (1H, br. s), 8.54 (1H, br. s), 8.18 (2H, d, J = 8.8 Hz), 8.20 (2H, dd, J₁ = 7.1 Hz , J₂ = 8.2 Hz), 7.93-7.78 (3H, m), 7.25 (1H, t, J = 8.0 Hz), 5.18-5.02 (1H, m), 4.10-3.75 (2H, m), 2.85-2.50 (2H, m). MS (ES⁺) C₁₈H₁₇N₅O₂ requires: 335, found: 336 (M+H)⁺.

### EXAMPLE 86 2-{5-[(Methylamino)methyl]pyridin-2-yl}-2H-indazole-7-carboxamide (AA2)

### Step 1: 2-(5-Formylpyridin-2-yl)-2H-indazole-7-carboxamide (AA1)

**(AA1)** was prepared following the general procedure reported in Example 3 step 1 using 6-bromonicotinaldehyde and 2H-indazole-7-carboxamide. The crude was purified by precipitation by adding water to the reaction mixture followed by filtration. ¹H NMR (300 MHz, DMSOd₆, 300K) δ 10.18 (1H, s), 9.55 (1H, s), 9.13 (1H, br. s), 8.66 (1H, d, J= 8.4 Hz), 8.55 (1H, dd, J₁ = 2.0 Hz , J₂ = 8.4 Hz), 8.49 (1H, s), 8.13-8.05 (2H, m), 7.94 (1H, s), 7.25 (1H, dd, , J₁ = 7.0 Hz , J₂ = 8.4 Hz), MS (ES⁺) C₁₄H₁₀N₄O₂ requires: 266, found: 268 (M+2H)⁺.

### Step 2: 2-{5-[(Methylamino)methyl]pyridin-2-yl}-2H-indazole-7-carboxamide (AA2)

**(AA2)** was prepared from **AA1** following the general procedure reported in Example 3 step 2 and the crude was purified by RP- HPLC (column: C18), using H₂O (+0.1% TFA) and MeCN (+0.1% TFA) as eluents, and the desired fractions were lyophilized to afford the titled compound. ¹H NMR (300 MHz, DMSOd₆, 300K) δ 9.43 (1H, s), 8.53 (2H, br. s), 8.38 (1H, d, J = 8.2 Hz), 8.09 - 8.03 (3H, m), 7.89 (1H, s), 7.30-7.25 (1H, m), 3.76 (2H, s), 2.30 (3H, s), MS (ES⁺) C₁₅H₁₅N₅O requires: 281, found: 282 (M+H)⁺.

### EXAMPLE 87 5-Fluoro-2-{3-fluoro-4-[(methylamino)methyl]phenyl}-2H-indazole-7-carboxamide (BB2)

### Step 1: 5-Fluoro-2-(3-fluoro-4-formylphenyl)-2H-indazole-7-carboxamide (BB1)

**(BB1)** was prepared following the general procedure reported in Example 3 step 1 using 2,4-difluorobenzaldehyde and Example 88, **(DD2).** The crude was purified by precipitation by adding water to the reaction mixture followed by filtration. The crude solid was taken forward without further purification. MS (ES⁺) C₁₅H₉F₂N₃O₂ requires: 301, found: 302 (M+H)⁺.

### Step 2: 5-Fluoro-2-{3-fluoro-4-[(methylamino)methyl]phenyl}-2H-indazole-7-carboxamide (BB2)

**(BB2)** was prepared from **BB1** following the general procedure reported in Example 3 step 2 and the crude was purified by RP- HPLC (column: C18), using H₂O (0.1% TFA) and MeCN (0.1% TFA) as eluents, and the desired fractions were lyophilized to afford the titled compound.. ¹H NMR (300 MHz, DMSOd₆, 300K) δ 9.40 (1H, s), 9.02 (1H, br. s), 8.50 (1H, s), 8.34 (1H, dd, J₁ = 2.0 Hz , J₂ = 10.8 Hz), 8.20 (1H, dd, J₁ = 2.0 Hz , J₂ = 8.3 Hz), 8.07 (1H, s), 7.90-7.80 (3H, m), 4.28 (2H, s), 2.60 (3H, s), MS (ES⁺) C₁₆H₁₄F₂N₄O requires: 316, found: 317 (M+H)⁺.

### PREPARATIVE EXAMPLE A

### Step 1: 5-Fluoro-3-methyl-2-nitrobenzoic acid (CC1)

To a solution of 3-fluoro-5-methylbenzoic acid (1.0 eq.) in conc. H₂SO₄ was added slowly KNO₃ (1.1 eq.) at 0°C. The mixture was stirred at RT for 1 h and then slowly poured into iced water. After stirring to until the ice has completely melted, the white precipitation was filtered, washed with cold water and dried under reduced pressure. The white solid was used without further purification for the next step. ¹H NMR (400 MHz, DMSO, 300K) δ 14.08 (1H, br. s), 7.65 (2H, m), 2.30 (3H, s).

### Step 2: Methyl 5-fluoro-3-methyl-2-nitrobenzoate (CC2)

**(CC2)** was prepared following the general procedure reported in Example 17 step 1. The yellow solid was used in the next step without purification. ¹H NMR (400 MHz, DMSO, 300K) δ 7.63 (2H, m), 3.83 (3H, s), 2.29 (3H, s).

### Step 3: Methyl 2-amino-5-fluoro-3-methylbenzoate (CC3)

**(CC3)** was prepared following the general procedure reported in Example 18 step 1. The white solid was used without further purification in the subsequent step. ¹H NMR (400 MHz, DMSO, 300K) δ 7.29 (1H, dd, J = 9.5 Hz, J = 3.0 Hz), 7.12 (1H, dd, J = 9.5 Hz, J = 3.0 Hz), 6.36 (2H, br. s), 3.78 (3H, s), 2.11 (3H, s).

### Step 4: Methyl 2,5-difluoro-3-methylbenzoate (CC4)

To a solution of **(CC3)** (1.0 eq.) in dry DCM (0.4 M) at 0°C was added nitrosonium tetrafluoroborate (1.3 eq.) portionwise. After 1 h at 0°C dry dichlorobenzene (120 eq.) was added and the reaction was slowly heated to 160°C while DCM was distilled off. After 3 hrs, the mixture was cooled to RT, EtOAc was added and the organic phase was washed with brine (2x). After drying over MgSO₄ the solvents were removed under reduced pressure. The crude was purified by flash chromatography 1-10% EtOAc/petroleum ether to yield **(CC4)** as a yellow oil. ¹H NMR (400 MHz, CDCl₃, 300K) δ 7.42 (1H, m), 7.06 (1H, m), 3.92 (3H, s), 2.30 (3H, d, J = 2.3 Hz).

### Step 5: Methyl 2,5-difluoro-3-formylbenzoate (CC5)

(**CC5**) was prepared from **CC4** following the general procedure reported in Example 1 step 2 and 3. The crude was purified by flash chromatography 1-20% EtOAc/petroleum ether to yield a white solid. ¹H NMR (300 MHz, DMSO, 300K) δ 10.19 (1H, d, J = 2.4 Hz), 7.98 (1H, m), 7.86 (1H, m), 3.89 (3H, s). MS (ES⁺) C₉H₆F₂O₃ requires: 200, found: 201 (M+H)⁺.

### EXAMPLE 88 5-Fluoro-2-{4-[(methylamino)methyl]phenyl}-2H-indazole-7-carboxamide trifluoroacetate (DD3)

### Step 1: Methyl 5-fluoro-1H-indazole-7-carboxylate (DD1)

To a solution of Preparative Example A, CC3 (1.0 eq.) in 1,2-dichloroethane (0.1 M) was added AcCl (5 eq.) and heated at 55°C for 2h. Afterwards the solvent was removed under reduced pressure.

The white solid was dissolved in toluene/water (5/1, 0.1 M). The solution was cooled to 0°C and HCl (10 eq., 37%) was added. Then slowly and in portions NaNO₂ (10 eq.) was added and the mixture was stirred for 3h at 0°C. The organic phase was washed with water (3x), dried over MgSO₄ and the solvent was removed under reduced pressure.

The yellow solution in toluene (0.1 M) was then heated for 2 h at 90°C. Evaporation of toluene yielded the desired product as a red solid. ¹H NMR (400 MHz, DMSO, 300K) δ 13.37 (1H, s), 8.23 (1H, s), 7.63 (1H, dd, J = 8.6 Hz, J = 2.5 Hz), 7.48 (1H, dd, J = 8.6 Hz, J = 2.5 Hz), 3.66 (3H, s). MS (ES⁺) C₉H₇FN₂O₂ requires: 194, found: 195 (M+H)⁺.

### Step 2: 5-Fluoro-1H-indazole-7-carboxamide (DD2)

**(DD1)** was solved in dioxane/water (1/1, 0.1 M) and KOH (1.5 eq.) was added. After stirring 12 h at RT the solvents were removed under reduced pressure. The white solid was used without purification for the coupling.

The carboxylic acid was dissolved in DMF (0.1 M) and TBTU (1.5 eq.) was added at 0°C. After 15 min DIEA (2.0 eq.) and ammonia (3.0 eq., 0.5 M in dioxane) were added and the mixture was stirred 36h at RT. EtOAc was added and the organic phase was washed with sat. aq. NaHCO₃ solution (3x) and brine (2x). The organic phase was dried and evaporated under reduced pressure. The crude was purified by flash chromatography 1-20% MeOH/DCM to yield **(DD2)** as a white solid. MS (ES⁺) C₈H₆FN₃O requires: 179, found: 180 (M+H)⁺.

### Step 3: 5-Fluoro-2-{4-[(methylamino)methyl]phenyl}-2H-indazole-7-carboxamide trifluoroacetate (DD3)

**(DD3)** was prepared from **DD2** following the general procedure reported in Example 3 step 1 and 2 and the crude was purified by RP- HPLC (column: C18), using H₂O (0.1% TFA) and MeCN (0.1% TFA) as eluents, and the desired fractions were lyophilized to afford the titled compound. ¹H NMR (400 MHz, DMSO, 300K) δ 9.43 (2H, s), 9.33 (1H, s), 8.52 (1H, s), 8.24 (2H, dd, J = 8.6 Hz), 8.08 (1H, s), 7.88-7.71 (4H, m), 4.20 (2H, t, J = 5.6 Hz), 2.55 (3H, t, J = 5.6 Hz). MS (ES⁺) C₁₆H₁₅FN₄O requires: 299, found: 300 (M+H)⁺.

### EXAMPLE 89 2-{4-[7-(Aminocarbonyl)-2H-indazol-2-yl]phenyl-N-methylpropan-2-aminium trifluoroacetate (EE4)

### Step 1: 2-(4-Acetylphenyl)-2H-indazole-7-carboxamide (EE1)

(**EE1**) was prepared following the general procedure reported in Example 3 step 1 using 1*H-*indazole-7-carboxamide and 1-(4-fluorophenyl)ethanone. The product was obtained as a white solid by precipitation from the reaction mixture by adding MeOH/EtOAc (5/1). ¹H NMR (400 MHz, DMSO, 300K) δ 9.46 (1H, s), 8.51 (1H, s), 8.34 (2H, d, J = 8.8 Hz), 8.17 (2H, d, J = 8.8 Hz), 8.07 (1H, d, J = 7.2 Hz), 8.03 (1H, d, J = 7.2 Hz), 7.91 (1H, s), 7.28 (1H, dd, J = 8.3 Hz, J = 6.8 Hz), 2.65 (3H, s). MS (ES⁺) C₁₆H₁₃N₃O₂ requires: 279, found: 280 (M+H)⁺.

### Step 2: 2-[4-(1-Hydroxy-1-methylethyl)phenyl]-2H-indazole-7-carboxamide (EE2)

To a solution of (**EE1**) (1.0 eq.) in THF (0.05 M) at 0°C was added slowly MeMgBr (1.5 eq., 3 M in THF). The mixture was stirred overnight at RT. H₂O was added and then mixture was extracted with EtOAc. The combined organic phase was dried over MgSO₄ and evaporated under reduced pressure to yield the product **(EE2)** as a yellow solid. ¹H NMR (400 MHz, DMSO, 300K) δ 9.27 (1H, s), 8.57 (1H, s), 8.06 (4H, m), 7.87 (1H, s), 7.68 (2H, d, J = 8.6 Hz), 7.25 (1H, m), 1.47 (6H, s). MS (ES⁺) C₁₇H₁₇N₃O₂ requires: 295, found: 296 (M+H)⁺.

### Step 3: 2-{4-[1-(Formylamino)-1-methylethyl]phenyl}-2H-indazole-7-carboxamide (EE3)

To a solution of (**EE2**) (1.0 eq.) and NaCN (1.0 eq.) in DCM (0.12 M) was added conc. H₂SO₄ (5.0 eq.) and the mixture was stirred for 24h at RT. Sat. aq. NaHCO₃ solution was added and the mixture was extracted with EtOAc. The combined organic phase was dried over MgSO₄ and evaporated under reduced pressure. The residue was purified by reverse phase HPLC (column: C18) to afford the titled compound **(EE3)** a yellow solid. ¹H NMR (400 MHz, DMSO, 300K) δ 9.26 (1H, s), 8.56 (1H, s), 8.42 (1H, s), 8.05 (5H, m), 7.86 (1H, s), 7.56 (2H, d, J = 8.8 Hz), 7.26 (1H, dd, J = 8.4 Hz, J = 7.1 Hz), 1.61 (6H, s). MS (ES⁺) C₁₈H₁₈N₄O₂ requires: 322, found: 323 (M+H)⁺.

### Step 4: 2-{4-[7-(Aminocarbonyl)-2H-indazol-2-yl]phenyl}-N-methylpropan-2-aminium trifluoroacetate (EE4)

To a solution of (**EE3**) (1.0 eq.) in THF (0.03 M) was added BH₃-THF (1.5 eq., 1 M in THF) and the mixture was stirred for 24h at RT. Then sat. aq. NaHCO₃ solution was added and the mixture was extracted with EtOAc. The combined organic phase was dried over MgSO₄ and evaporated under reduced pressure. The residue was purified by reverse phase HPLC (column: C18) to afford the titled compound **(EE4)** a yellow solid. ¹H NMR (400 MHz, DMSO, 300K) δ 9.38 (1H, s), 9.14 (1H, s), 8.53 (1H, s), 8.27 (2H, d, J = 8.7 Hz), 8.04 (2H, s), 7.88 (1H, s), 7.79 (2H, d, J = 8.7 Hz), 7.28 (1H, dd, J = 8.2 Hz, J = 8.2 Hz), 2.36 (3H, s), 1.73 (6H, s). MS (ES⁺) C₁₈H₂₀N₄O requires: 308, found: 309 (M+H)⁺.

### EXAMPLE 90 2-(6-Phenylpyridazin-3-yl)-2H-indazole-7-carboxamide (FF1)

(**FF1**) was prepared following the general procedure reported in Example 3 step 1, using 3-chloro-6-phenylpyridazine and 1*H-*indazole-7-carboxamide as starting materials. The product was obtained as a white solid. ¹H NMR (400 MHz, DMSO, 300K) δ 9.69 (1H, s), 8.85 (1H, d, J = 9.2 Hz), 8.54 (1H, d, J = 9.2 Hz), 8.52 (1H, s), 8.24 (2H, d, J = 6.8 Hz), 8.11 (2H, m) 7.91 (1H, s), 7.59 (3H, m), 7.32 (1H, dd, J = 7.7 Hz, J = 7.7 Hz). MS (ES⁺) C₁₈H₁₃N₅O requires: 315, found: 316 (M+H)⁺.

### EXAMPLE 91 {4-[7-(Aminocarbonyl)-5-fluoro-2H-indazol-2-yl]phenyl}-N-{[1-(hydroxymethyl)cyclohexyl]methyl}methanaminium trifluoroacetate (GG1)

To MP-Triacetoxyborohydride (5 eq, loading 2.5 mmol/g) a solution of 5-fluoro-2-(4-formylphenyl)-2*H*-indazole-7-carboxamide (Intermediate in preparation of Example 88, **(DD3)** (1eq) and [1-(aminomethyl)cyclohexyl]methanol (1.2 eq.) in DMF (0.132 M) was added. The mixture was heated in the MW apparatus (10 min, 80 °C) and then purified by RP-HPLC (column Symmetry RP18 7µm, 19x300mm, gradient A: H₂O + 0.1% TFA; B: MeCN + 0.1% TFA) and the pooled fractions were evaporated under reduced pressure to yield the title compound. ¹H NMR (300 MHz, DMSOd₆, 300K) δ 9.30 (1H, s), 8.54 (3H, br. s), 8.25 (2H, d, J = 8.6 Hz), 8.05 (1H, br. s), 7.88-7.80 (1H, m), 7.75 (3H, d, J = 8.3 Hz), 4.28-4.20 (2H, m), 3.39 (2H, s), 2.90-2.83 (2H, m), 1.45-1.25 (10H, m). MS (ES⁺) C₂₃H₂₇N₄O₂ requires: 410, found: 411 (M+H)⁺.

The following examples were prepared according to the methods of the previous examples:

| **Example** | **Name** | **MW** | **M+H⁺** | **Procedure of Example** |
|---|---|---|---|---|
| 92 | 5-Chloro-2-(4-formylphenyl)-2H-indazole-7-carboxamide | 299/301 | 300/302 | 17 |
| 93 | 2-{3-Methoxy-4-[(4-methylpiperazin-1-yl)methyl]phenyl}-2H-indazole-7-carboxamide | 379 | 380 | 17 |
| 94 | 2-{3-Methoxy-4-[(methylamino)methyl]phenyl}-2H-indazole-7-carboxamide | 310 | 311 | 3 |
| 95 | 5-Chloro-2-{4-[(4-methylpiperazin-1-yl)methyl]phenyl}-2H-indazole-7-carboxamide | 383/385 | 384/386 | 17 |
| 96 | 5-Chloro-2-{4-[(methylamino)methyl]phenyl}-2H-indazole-7-carboxamide | 314/316 | 315/317 | 17 |
| 97 | {4-[7-(Aminocarbonyl)-4-fluoro-2H-indazol-2-yl]phenyl}-N-methylmethanaminium chloride | 298 | 299 | 17 |
| 98 | {4-[7-(Aminocarbonyl)-5-fluoro-2H-indazol-2-yl]phenyl}-N-methylmethanaminium chloride | 298 | 299 | 88 |
| 99 | 1-{4-[7-(Aminocarbonyl)-4-fluoro-2H-indazol-2-yl]benzyl}-4-methylpiperazin-1-ium chloride | 367 | 368 | 3 |
| 100 | 1-{4-[7-(Aminocarbonyl)-5-fluoro-2H-indazol-2-yl]benzyl}-4-methylpiperazin-1-ium chloride | 367 | 368 | 3 |
| 101 | 1-{3-[7-(Aminocarbonyl)-2H-indazol-2-yl]benzyl}-4-methylpiperazinediium bis(trifluoroacetate) | 349 | 350 | 20 |
| 102 | 2-[4-(1-Hydroxy-1-methylethyl)phenyl]-2H-indazole-7-carboxamide | 295 | 296 | 89 |
| 103 | 2-(4-Acetylphenyl)-2H-indazole-7-carboxamide | 279 | 280 | 15 |
| 104 | 3-}{4-[7-(Aminocarbonyl)-2H-indazol-2-yl]benzyl}(methyl)amino]carbonyl}-1-methylpiperidinium trifluoroacetate | 405 | 406 | 77 |
| 105 | 2-{4-[1-(Formylamino)-1-methylethyl]phenyl}-2H-indazole-7-carboxamide | 322 | 323 | 89 |
| 106 | 2-[3-(1,4-Diazepan-1-ylcarbonyl)phenyl]-2H-indazole-7-carboxamide | 363 | 364 | 21 |
| 107 | 3-[({4-[7-(Aminocarbonyl)-2H-indazol-2-yl]benzyl}amino)carbonyl]-1-methylpiperidinium trifluoroacetate | 391 | 392 | 78 |
| 108 | (2S)-2-[({4-[7-(Aminocarbonyl)-2H-indazol-2-yl]phenyl}amino)carbonyl]pyrrolidinium trifluoroacetate | 349 | 350 | 4 |
| 109 | 3-[({4-[7-(Aminocarbonyl)-2H-indazol-2-yl]phenyl}amino)carbonyl]pyrrolidinium trifluoroacetate | 349 | 350 | 4 |
| 110 | (2R)-2-[({4-[7-(Aminocarbonyl)-2H-indazol-2-yl]phenyl} amino)carbonyl]pyrrolidinium trifluoroacetate | 349 | 350 | 4 |
| 111 | 3-[({4-[7-(Aminocarbonyl)-2H-indazol-2-yl]phenyl}amino)carbonyl]piperidinium trifluoroacetate | 363 | 364 | 4 |
| 112 | (3R)-3-[({4-[7-(Aminocarbonyl)-2H-indazol-2-yl]phenyl}amino)carbonyl]-1-methylpyrrolidinium trifluoroacetate | 363 | 364 | 4 |
| 113 | 2-[({4-[7-(Aminocarbonyl)-2H-indazol-2-yl]phenyl}amino)carbonyl]-1-methylpiperidinium trifluoroacetate | 377 | 378 | 4 |
| 114 | 4-Chloro-2-(4-formylphenyl)-2H-indazole-7-carboxamide | 299/301 | 300/302 | 17 |
| 115 | (3S)-3-[({4-[7-(Aminocarbonyl)-2H-indazol-2-yl]phenyl}amino)carbonyl]-1-methylpyrrolidinium trifluoroacetate | 363 | 364 | 4 |
| 116 | (R)-1-{4-[7-(Aminocarbonyl)-2H-indazol-2-yl]phenyl}-N-methylethanaminium chloride | 294 | 295 | Separated by SFC |
| 117 | (S)-1-{4-[7-(Aminocarbonyl)-2H-indazol-2-yl]phenyl}-N-methylethanaminium chloride | 294 | 295 | Separated by SFC |
| 118 | 2-{3-Fluoro-4-[(methylamino)methyl]phenyl}-2H-indazole-7-carboxamide | 298 | 299 | 3 |
| 119 | {4-[7-(Aminocarbonyl)-2H-indazol-2-yl]-2-fluorophenyl} -N-methanamium trifluoroacetate | 298 | 299 | 3 |
| 120 | 2-{4-[1-Methyl-1-(methylamino)ethyl]phenyl}-2H-indazole-7-carboxamide | 308 | 309 | 89 |
| 121 | 1-{4-[7-(Aminocarbonyl)-2H-indazol-2-yl]-2-hydroxybenzyl}-4-methylpiperazin-1-ium trifluoroacetate | 365 | 366 | 3 |
| 122 | (3R)-3-[({4-[7-(Aminocarbonyl)-2H-indazol-2-yl]phenyl}amino)carbonyl]-1-methylpiperidinium chloride | 377 | 378 | 4 |
| 123 | (3S)-3-[({4-[7-(Aminocarbonyl)-2H-indazol-2-yl]phenyl} amino)carbonyl]-1-methylpiperidinium chloride | 377 | 378 | 4 |
| 124 | 1-(2-{4-[7-(Ammocarbonyl)-2H-indazol-2-yl]phenyl}ethyl)-4-methylpiperazinediium bis(trifluoroacetate) | 363 | 364 | 84 |
| 125 | {4-[7-(Aminocarbonyl)-4-hydroxy-2H-indazol-2-yl]phenyl}-N-methylmethanaminium trifluoroacetate | 296 | 297 | 3 |
| 126 | 2-[({4-[7-(Aminocarbonyl)-2H-indazol-2-yl]phenyl} amino)carbonyl]-4-phenylpyrrolidinium trifluoroacetate | 425 | 426 | 85 |
| 127 | (1R,3S)-3-[({4-[7-(Aminocarbonyl)-2H-indazol-2-yl]phenyl}amino)carbonyl]cyclopentanamini um trifluoroacetate | 363 | 364 | 85 |
| 128 | (1R,3R)-3-[({4-[7-(Aminocarbonyl)-2H-indazol-2-yl]phenyl}amino)carbonyl]cyclopentanamini um trifluoroacetate | 363 | 364 | 85 |
| 129 | (1S,3R)-3-[({4-[7-(Aminocarbonyl)-2H-indazol-2-yl]phenyl} amino)carbonyl]cyclopentanamini um trifluoroacetate | 363 | 364 | 85 |
| 130 | 2-[({4-[7-(Aminocarbonyl)-2H-indazol-2-yl]phenyl} amino)carbonyl]-2-methylazetidinium trifluoroacetate | 349 | 350 | 85 |
| 131 | 4-[2-({4-[7-(Aminocarbonyl)-2H-indazol-2-yl]phenyl}amino)-2-oxoethyl]-1-methylpiperidinium trifluoroacetate | 391 | 392 | 85 |
| 132 | 9-[2-({4-[7-(Aminocarbonyl)-2H-indazol-2-yl]phenyl}amino)-2-oxoethyl]-3-azoniaspiro[5.5]undecane trifluoroacetate | 445 | 446 | 85 |
| 133 | 4-[2-({4-[7-(Ammocarbonyl)-2H-indazol-2-yl]phenyl}amino)-2-oxoethyl]-4-phenylpiperidinium trifluoroacetate | 453 | 454 | 85 |
| 134 | 2-[({4-[7-(Aminocarbonyl)-2H-indazol-2-yl]phenyl}amino)carbonyl]pyridinium trifluoroacetate | 357 | 358 | 85 |
| 135 | 4-{3-[({4-[7-(Aminocarbonyl)-2H-indazol-2-yl]phenyl}amino)carbonyl]pyridin-2-yl}piperazin-1-ium trifluoroacetate | 441 | 442 | 85 |
| 136 | 3-[({4-[7-(Aminocarbonyl)-2H-indazol-2-yl]phenyl}amino)carbonyl]pyridinium trifluoroacetate | 357 | 358 | 85 |
| 137 | 4-[({4-[7-(Aminocarbonyl)-2H-indazol-2-yl]phenyl}amino)carbonyl]pyridinium trifluoroacetate | 357 | 358 | 85 |
| 138 | 4-[({4-[7-(Aminocarbonyl)-2H-indazol-2-yl]phenyl}amino)carbonyl]quinolinium trifluoroacetate | 407 | 408 | 85 |
| 139 | 4-[({4-[7-(Aminocarbonyl)-2H-indazol-2-yl]phenyl}amino)carbonyl]isoquinolinium trifluoroacetate | 407 | 408 | 85 |
| 140 | 2-[({4-[7-(Aminocarbonyl)-2H-indazol-2-yl]phenyl}amino)carbonyl]-1-methylazepanium trifluoroacetate | 391 | 392 | 85 |
| 141 | 3-[({4-[7-(Aminocarbonyl)-2H-indazol-2-yl]phenyl}amino)carbonyl]-2-methyl-1,2,3,4-tetrahydroisoquinolinium trifluoroacetate | 425 | 426 | 85 |
| 142 | 2-{4-[({4-[7-(Aminocarbonyl)-2H-indazol-2-yl]phenyl}amino)carbonyl]piperidin-1-yl}pyrimidin-1-ium trifluoroacetate | 441 | 442 | 85 |
| 143 | 1-{4-[7-(Aminocarbonyl)-5-fluoro-2H-indazol-2-yl]benzyl}-4-methylpiperazin-1-ium chloride | 367 | 368 | 85 |
| 144 | 5-[({4-[7-(Aminocarbonyl)-2H-indazol-2-yl]phenyl}amino)carbonyl]-3-oxooctahydroindolizin-2-aminium trifluoroacetate | 432 | 433 | 85 |
| 145 | 2-{3-[({4-[7-(Aminocarbonyl)-2H-indazol-2-yl]phenyl}amino)carbonyl]piperidin-1-yl}pyridinium trifluoroacetate | 440 | 441 | 85 |
| 146 | 2-[({4-[7-(Aminocarbonyl)-2H-indazol-2-yl]phenyl} amino)carbonyl]-4-methylmorpholin-4-ium trifluoroacetate | 379 | 380 | 85 |
| 147 | (1R,4R)-N-{4-[7-(Aminocarbonyl)-2H-indazol-2-yl]phenyl}-1'-(methylsulfonyl)-1',2'-dihydrospiro[cyclohexane-1,3'-indole]-4-carboxamide | 543 | 544 | 85 |
| 148 | 1-[({4-[7-(Aminocarbonyl)-2H-indazol-2-yl]phenyl}amino)carbonyl]octahydro-1H-isoindolium trifluoroacetate | 403 | 404 | 85 |
| 149 | 2-[({4-[7-(Aminocarbonyl)-2H-indazol-2-yl]phenyl} amino)carbonyl]-4-benzylmorpholin-4-ium trifluoroacetate | 455 | 456 | 85 |
| 150 | (3S,4R)-3-[({4-[7-(Aminocarbonyl)-2H-indazol-2-yl]phenyl}amino)carbonyl]-4-(methoxycarbonyl)pyrrolidinium trifluoroacetate | 407 | 408 | 85 |
| 151 | 4-{(2S)-2-[({4-[7-(Aminocarbonyl)-2H-indazol-2-yl]phenyl}amino)carbonyl]pyrrolidinium-1-yl}piperidinium bis(trifluoroacetate) | 432 | 433 | 85 |
| 152 | (1S,3S)-3-[({4-[7-(Aminocarbonyl)-2H-indazol-2-yl]phenyl}amino)carbonyl]cyclopentanamini um trifluoroacetate | 363 | 364 | 85 |
| 153 | 3-[({4-[7-(Aminocarbonyl)-2H-indazol-2-yl]phenyl} amino)carbonyl]-1-methylpyrrolidinium trifluoroacetate | 363 | 364 | 85 |
| 154 | 2-{4-[({4-[7-(Aminocarbonyl)-2H-indazol-2-yl]phenyl}amino)carbonyl]piperidin-1-yl}pyrimidin-1-ium trifluoroacetate | 397 | 398 | 16 |
| 155 | 2-(1-{4-[7-(Aminocarbonyl)-2H-indazol-2-yl]benzyl}pyrrolidinium-3-yl)pyridinium bis(trifluoroacetate) | 397 | 398 | 16 |
| 156 | 3-(1-{4-[7-(Aminocarbonyl)-2H-indazol-2-yl]benzyl}pyrrolidinium-3-yl)pyridinium bis(trifluoroacetate) | 397 | 398 | 16 |
| 157 | (3S,4S)-1-{4-[7-(Aminocarbonyl)-2H-indazol-2-yl]benzyl}-3,4-difluoropyrrolidinium trifluoroacetate | 356 | 357 | 16 |
| 158 | 3-{4-[7-(Aminocarbonyl)-2H-indazol-2-yl]benzyl}-6-ammonio-3-azoniabicyclo[3.1.0]hexane bis(trifluoroacetate) | 347 | 348 | 16 |
| 159 | 2-{4-[7-(Aminocarbonyl)-2H-indazol-2-yl]benzyl}-7-methyl-2,7-diazoniaspiro[4.4]nonane bis(trifluoroacetate) | 389 | 390 | 16 |
| 160 | 1-{4-[7-(Aminocarbonyl)-2H-indazol-2-yl]benzyl}-3-[4-(dimethylammonio)phenyl]pyrrolidinium bis(trifluoroacetate) | 439 | 440 | 16 |
| 161 | 5-{4-[7-(Aminocarbonyl)-2H-indazol-2-yl]benzyl}-1-methyl-1,2,4,5,6,6a-hexahydropyrrolo[3,4-b]pyrrolediium bis(trifluoroacetate) | 373 | 374 | 16 |
| 162 | 3-{[{4-[7-(Aminocarbonyl)-2H-indazol-2-yl]benzyl}(methyl)ammonio]methyl}-1-methylpiperidinium bis(trifluoroacetate) | 391 | 392 | 16 |
| 163 | (1R,4S)-5-{4-[7-(Aminocarbonyl)-2H-indazol-2-yl]benzyl}-2-oxa-5-azoniabicyclo[2.2.1]heptane trifluoroacetate | 348 | 349 | 16 |
| 164 | N-{4-[7-(Aminocarbonyl)-2H-indazol-2-yl]benzyl}-2-hydroxy-2-methylpropan-1-aminium trifluoroacetate | 338 | 339 | 16 |
| 165 | N-{4-[7-(Aminocarbonyl)-2H-indazol-2-yl]benzyl}-3,3-difluorocyclobutanaminium trifluoroacetate | 356 | 357 | 16 |
| 166 | 4-{4-[7-(Aminocarbonyl)-2H-indazol-2-yl]benzyl}-6-fluoro-1,4-diazepan-1-ium trifluoroacetate | 367 | 368 | 16 |
| 167 | 1-{4-[7-(Aminocarbonyl)-2H-indazol-2-yl]benzyl}-4-pyrimidin-1-ium-2-yl-1,4-diazepan-1-ium bis(trifluoroacetate) | 427 | 428 | 16 |
| 168 | 3-({4-[7-(Aminocarbonyl)-2H-indazol-2-yl]benzyl} ammonio)-1-benzylpyrrolidinium bis(trifluoroacetate) | 425 | 426 | 16 |
| 169 | 3-[({4-[7-(Aminocarbonyl)-2H-indazol-2-yl]benzyl}ammonio)methyl]-1-methylpyrrolidinium bis(trifluoroacetate) | 363 | 364 | 16 |
| 170 | 3-[({4-[7-(Aminocarbonyl)-2H-indazol-2-yl]benzyl}ammonio)methyl]-1-benzylpyrrolidinium bis(trifluoroacetate) | 439 | 440 | 16 |
| 171 | 2-{4-[7-(Aminocarbonyl)-2H-indazol-2-yl]benzyl}-7-benzyl-2,7-diazoniaspiro[4.4]nonane bis(trifluoroacetate) | 465 | 466 | 16 |
| 172 | 2-{4-[7-(Aminocarbonyl)-2H-indazol-2-yl]benzyl}-8-benzyl-2,8-diazoniaspiro[5.5]undecane bis(trifluoroacetate) | 493 | 494 | 16 |
| 173 | 2-{4-[7-(Aminocarbonyl)-2H-indazol-2-yl]benzyl}-2,6-diazoniaspiro[3.3]heptane bis(trifluoroacetate) | 347 | 348 | 16 |
| 174 | 7-{4-[7-(Aminocarbonyl)-2H-indazol-2-yl]benzyl}-2,7-diazoniaspiro[3.5]nonane bis(trifluoroacetate) | 375 | 376 | 16 |
| 175 | 2-{4-[7-(Aminocarbonyl)-2H-indazol-2-yl]benzyl}-2,6-diazoniaspiro[3.5]nonane bis(trifluoroacetate) | 375 | 376 | 16 |
| 176 | 2-{4-[7-(Aminocarbonyl)-2H-indazol-2-yl]benzyl}-2,8-diazoniaspiro[5.5]undecane bis(trifluoroacetate) | 403 | 404 | 16 |
| 177 | 2-{4-[7-(Aminocarbonyl)-2H-indazol-2-yl]benzyl}-2,8-diazoniaspiro[4.5]decane bis(trifluoroacetate) | 389 | 390 | 16 |
| 178 | 2-{4-[7-(Aminocarbonyl)-2H-indazol-2-yl]benzyl}-2,7-diazoniaspiro[4.5]decane bis(trifluoroacetate) | 389 | 390 | 16 |
| 179 | 8-{4-[7-(Aminocarbonyl)-2H-indazol-2-yl]benzyl}-2,8-diazoniaspiro[4.5]decane bis(trifluoroacetate) | 389 | 390 | 16 |
| 180 | 3-{4-[7-(Aminocarbonyl)-2H-indazol-2-yl]benzyl}-3,9-diazoniaspiro[5.5]undecane bis(trifluoroacetate) | 403 | 404 | 16 |
| 181 | 2-{4-[7-(Aminocarbonyl)-2H-indazol-2-yl]benzyl}octahydropyrrolo[3,4-c]pyrrolediium bis(trifluoroacetate) | 361 | 362 | 16 |
| 182 | 5-{4-[7-(Aminocarbonyl)-2H-indazol-2-yl]benzyl}octahydropyrrolo[3,4-b]pyrrolediium bis(trifluoroacetate) | 361 | 362 | 16 |
| 183 | 4-({4-[7-(Aminocarbonyl)-2H-indazol-2-yl]benzyl} ammonio)octahydrocyclopenta[c]p yrrolium bis(trifluoroacetate) | 375 | 376 | 16 |
| 184 | N²-{4-[7-(Aminocarbonyl)-2H-indazol-2-yl]benzyl}-N¹,N¹-dimethyl-1-pyridin-2-ylethane-1,2-diaminium bis(trifluoroacetate) | 414 | 415 | 16 |
| 185 | 7-(Aminocarbonyl)-2-[4-({[2-(2,3-dihydro-1H-indol-1-yl)ethyl]ammonio}methyl)phenyl]-2H-indazol-1-ium bis(trifluoroacetate) | 411 | 412 | 16 |
| 186 | (3S,4S)-1-[2-({4-[7-(Aminocarbonyl)-2H-indazol-2-yl]benzyl}ammonio)ethyl]-3,4-difluoropyrrolidinium bis(trifluoroacetate) | 399 | 400 | 16 |
| 187 | 5-({4-[7-(Aminocarbonyl)-2H-indazol-2-yl]benzyl}amino)-1,3-benzothiazol-3-ium trifluoroacetate | 399 | 400 | 16 |
| 188 | 1-({4-[7-(Aminocarbonyl)-2H-indazol-2-yl]benzyl}ammonio)-8-azoniaspiro[4.5]decane bis(trifluoroacetate) | 403 | 404 | 16 |
| 189 | 4-[({4-[7-(Aminocarbonyl)-2H-indazol-2-yl]benzyl}ammonio)methyl]-1-methylpiperidinium bis(trifluoroacetate) | 377 | 378 | 16 |
| 190 | N-{4-[7-(Aminocarbonyl)-2H-indazol-2-yl]benzyl} -2-hydroxyethanaminium trifluoroacetate | 310 | 311 | 16 |
| 191 | 7-[7-(Aminocarbonyl)-2H-indazol-2-yl]-1,2,3,4-tetrahydroisoquinolinium trifluoroacetate | 292 | 293 | 73 |
| 192 | 3-[2-({4-[7-(Aminocarbonyl)-2H-indazol-2-yl]phenyl}amino)-2-oxoethyl]azetidinium trifluoroacetate | 349 | 350 | 4 |
| 193 | 4-({4-[7-(Aminocarbonyl)-2H-indazol-2-yl]benzyl} ammonio)piperidinium bis(trifluoroacetate) | 349 | 350 | 16 |
| 194 | (3R,4R)-4-({4-[7-(Aminocarbonyl)-2H-indazol-2-yl]benzyl}ammonio)-3-fluoropiperidinium bis(trifluoroacetate) | 367 | 368 | 16 |
| 195 | (3S,4R)-4-({4-[7-(Aminocarbonyl)-2H-indazol-2-yl]benzyl}ammonio)-3-benzyl-1-methylpiperidinium bis(trifluoroacetate) | 453 | 454 | 16 |
| 196 | N-{4-[7-(Aminocarbonyl)-2H-indazol-2-yl]benzyl}-1-isobutyrylpiperidin-4-aminium trifluoroacetate | 419 | 420 | 16 |
| 197 | 2-[4-({4-[7-(Aminocarbonyl)-2H-indazol-2-yl]benzyl}ammonio)piperidin-l-yl]-3-methylpyridinium bis(trifluoroacetate) | 440 | 441 | 16 |
| 198 | 3-({4-[7-(Aminocarbonyl)-2H-indazol-2-yl]benzyl} ammonio)piperidinium bis(trifluoroacetate) | 349 | 350 | 16 |
| 199 | 3-({4-[7-(Aminocarbonyl)-2H-indazol-2-yl]benzyl}ammonio)-1-benzylpiperidinium bis(trifluoroacetate) | 439 | 440 | 16 |
| 200 | 5-{4-[7-(Aminocarbonyl)-2H-indazol-2-yl]benzyl}-5-aza-2-azoniabicyclo[2.2.2]octane trifluoroacetate | 361 | 362 | 16 |
| 201 | (1S,4S)-2-{4-[7-(Aminocarbonyl)-2H-indazol-2-yl]benzyl}-5-methyl-2,5-diazoniabicyclo[2.2.1]heptane bis(trifluoroacetate) | 361 | 362 | 16 |
| 202 | 1-{4-[7-(Aminocarbonyl)-2H-indazol-2-yl]benzyl} -4-(pyridin-2-ylmethyl)piperazinediium bis(trifluoroacetate) | 426 | 427 | 16 |
| 203 | 5-{4-[7-(Aminocarbonyl)-2H-indazol-2-yl]benzyl}-2-benzyl-5-aza-2-azoniabicyclo[2.2.2]octane trifluoroacetate | 451 | 452 | 16 |
| 204 | 8-{4-[7-(Aminocarbonyl)-2H-indazol-2-yl]benzyl}-3-benzyl-8-aza-3-azoniabicyclo[3.2.1]octane trifluoroacetate | 451 | 452 | 16 |
| 205 | (1S,4S)-5-{4-[7-(Aminocarbonyl)-2H-indazol-2-yl]benzyl}-2-benzyl-5-aza-2-azoniabicyclo[2.2.1]heptane trifluoroacetate | 437 | 438 | 16 |
| 206 | 3-({4-[7-(Aminocarbonyl)-2H-indazol-2-yl]benzyl} ammonio)pyrrolidinium bis(trifluoroacetate) | 335 | 336 | 16 |
| 207 | 6-({4-[7-(Aminocarbonyl)-2H-indazol-2-yl]benzyl}ammonio)-3-azoniabicyclo[3.1.0]hexane bis(trifluoroacetate) | 347 | 348 | 16 |
| 208 | (3S,4S)-N-{4-[7-(Aminocarbonyl)-2H-indazol-2-yl]benzyl}-4-hydroxytetrahydrothiophen-3-aminium 1,1-dioxide trifluoroacetate | 400 | 401 | 16 |
| 209 | 4-[({4-[7-(Aminocarbonyl)-2H-indazol-2-yl]benzyl}ammonio)methyl]-4-hydroxy-1-methylpiperidinium bis(trifluoroacetate) | 393 | 394 | 16 |
| 210 | N-{4-[7-(Aminocarbonyl)-2H-indazol-2-yl]benzyl}-1-cyclopropyl-2-hydroxyethanaminium trifluoroacetate | 350 | 351 | 16 |
| 211 | {4-[7-(Aminocarbonyl)-2H-indazol-2-yl]phenyl}-N-{[1-(hydroxymethyl)cyclopentyl]methyl}methana minium trifluoroacetate | 378 | 379 | 16 |
| 212 | 2-{4-[7-(Aminocarbonyl)-2H-indazol-2-yl]benzyl}-1,2,3,4-tetrahydro-2,7-naphthyridinediium bis(trifluoroacetate) | 383 | 384 | 16 |
| 213 | 1-{4-[7-(Aminocarbonyl)-2H-indazol-2-yl]benzyl}-3-[(dimethylammonio)methyl]piperidinium bis(trifluoroacetate) | 391 | 392 | 16 |
| 214 | 4-(1-{4-[7-(Aminocarbonyl)-2H-indazol-2-yl]benzyl}piperidinium-4-yl)thiomorpholin-4-ium bis(trifluoroacetate) | 435 | 436 | 16 |
| 215 | 1-{4-[7-(Aminocarbonyl)-2H-indazol-2-yl]benzyl}-4-[(methylsulfonyl)amino]piperidinium trifluoroacetate | 427 | 428 | 16 |
| 216 | 1-4-[7-(Aminocarbonyl)-2H-indazol-2-yl]benzyl}-4-(1H-imidazol-3-ium-1-ylmethyl)piperidinium bis(trifluoroacetate) | 414 | 415 | 16 |
| 217 | 7-{4-[7-(Aminocarbonyl)-2H-indazol-2-yl]benzyl}-1-oxa-7-azoniaspiro[4.5]decane trifluoroacetate | 390 | 391 | 16 |
| 218 | 1-{4-[7-(Aminocarbonyl)-2H-indazol-2-yl]benzyl}-4-(1-hydroxy-1-methylethyl)piperidinium trifluoroacetate | 392 | 393 | 16 |
| 219 | 2-[({4-[7-(Aminocarbonyl)-2H-indazol-2-yl]phenyl}amino)carbonyl]-1-benzylpiperidinium trifluoroacetate | 453 | 454 | 4 |
| 220 | 2-[({4-[7-(Aminocarbonyl)-2H-indazol-2-yl]phenyl}amino)carbonyl]-1-ethylpiperidinium trifluoroacetate | 391 | 392 | 4 |
| 221 | 3-[({4-[7-(Aminocarbonyl)-2H-indazol-2-yl]phenyl}amino)carbonyl]-1-ethylpiperidinium trifluoroacetate | 391 | 392 | 4 |
| 222 | 2-[3-(1,4-Diazepan-1-ylcarbonyl)-4-fluorophenyl]-2H-indazole-7-carboxamide trifluoroacetate | 381 | 382 | 21 |
| 223 | tert-Butyl {4-[7-(aminocarbonyl)-4-chloro-2H-indazol-2-yl]benzyl}methylcarbamate | 414/416 | 415/417 | 3 |
| 224 | 6-[7-(Aminocarbonyl)-2H-indazol-2-yl]-1,2,3,4-tetrahydroisoquinolinium trifluoroacetate | 292 | 293 | 74 |
| 225 | 2-{4-[7-(Aminocarbonyl)-2H-indazol-2-yl]phenyl}pyrrolidinium trifluoroacetate | 306 | 307 | 1 |
| 226 | 6-Fluoro-2-{4-[(methylamino)methyl]phenyl}-2H-indazole-7-carboxamide | 298 | 299 | 17 |
| 227 | 5-Fluoro-2-{2-fluoro-4-[(methylamino)methyl]phenyl}-2H-indazole-7-carboxamide | 316 | 317 | 17 |
| 228 | 2-{3-Hydroxy-4-[(methylamino)methyl]phenyl}-2H-indazole-7-carboxamide trifluoroacetate | 296 | 297 | 3 |
| 229 | 2-(4-{[Formyl(methyl)amino]methyl}-3-hydroxyphenyl)-2H-indazole-7-carboxamide | 324 | 325 | 3 |
| 230 | 2-{2-chloro-4-[(methylamino)methyl]phenyl}-5-fluoro-2H-indazole-7-carboxamide | 332/334 | 333/335 | 17 |
| 231 | 5-Fluoro-2-{3-fluoro-4-[(methylamino)methyl]phenyl}-2H-indazole-7-carboxamide trifluoroacetate | 316 | 317 | 87 |
| 232 | 2-{2,5-Difluoro-4-[(methylamino)methyl]phenyl}-5-fluoro-2H-indazole-7-carboxamide trifluoroacetate | 334 | 335 | 17 |
| 233 | 2-(4-Bromophenyl)-2H-indazole-7-carboxamide | 315/317 | 316/318 | 81 |
| 234 | (3R)-3-[({4-[7-(Aminocarbonyl)-5-fluoro-2H-indazol-2-yl]phenyl}amino)carbonyl]-1-methylpiperidinium chloride | 395 | 396 | 76 |
| 235 | (3R)-3-[({4-[7-(Aminocarbonyl)-5-fluoro-2H-indazol-2-yl]phenyl}amino)carbonyl]-1-methylpiperidinium trifluoroacetate | 395 | 396 | 76 |
| 236 | 2-(1,2,3,4-Tetrahydroisoquinolin-7-yl)-2H-indazole-7-carboxamide | 292 | 293 | 73 |
| 237 | (R)-2-[4-({3-[(Dimethylamino)methyl]piperidin-1-yl}methyl)phenyl]-2H-indazole-7-carboxamide | 391 | 392 | Separated by SFC |
| 238 | (S)-2-[4-({3-[(Dimethylamino) methyl]piperidin-1-yl}methyl)phenyl]-2H-indazole-7-carboxamide | 391 | 392 | Separated by SFC |
| 239 | 3-({4-[7-(Aminocarbonyl)-5-fluoro-2H-indazol-2-yl]phenyl}amino)-2-(chloromethyl)-3-oxopropan-1-aminium trifluoroacetate | 389/391 | 390/392 | 76 |
| 240 | 5-Fluoro-2-}3-fluoro-4-[(methylamino)methyl]phenyl}-2H-indazole-7-carboxamide hydrochloride | 316 | 317 | 87 |
| 241 | 2-{4-[(Dimethylamino)methyl]-3-fluorophenyl}-5-fluoro-2H-indazole-7-carboxamide trifluoroacetate | 330 | 331 | 87 |
| 242 | 2-{4-[(Azetidin-3-ylcarbonyl)amino]phenyl}-5-fluoro-2H-indazole-7-carboxamide | 353 | 354 | 76 |
| 243 | 2-[4-(2,7-Diazaspiro[4.5]dec-2-ylmethyl) phenyl]-2H-indazole-7-carboxamide | 389 | 390 | 16 |
| 244 | (1S,4S)-5-{4-[7-(Aminocarbonyl)-2H-indazol-2-yl]benzyl}-2-(4-chlorobenzyl)-5-aza-2-azoniabicyclo[2.2.1]heptane trifluoroacetate | 471/473 | 472/474 | 16 |
| 245 | (1S,4S)-5-{4-[7-(Aminocarbonyl)-2H-indazol-2-yl]benzyl}-2-(3-chlorobenzyl)-5-aza-2-azoniabicyclo[2.2.1]heptane trifluoroacetate | 471/473 | 472/474 | 16 |
| 246 | 1-{4-[7-(Aminocarbonyl)-2H-indazol-2-yl]benzyl}-4-[(methylamino)carbonyl] piperazin-1-ium trifluoroacetate | 392 | 393 | 16 |
| 247 | N-{4-[7-(Aminocarbonyl)-2H-indazol-2-yl]benzyl}-2-hydroxy-2-pyridin-3-ylethanaminium trifluoroacetate | 387 | 388 | 16 |
| 248 | N-{4-[7-(Aminocarbonyl)-2H-indazol-2-yl]benzyl}-2-cyclohexyl-2-hydroxyethanaminium trifluoroacetate | 392 | 393 | 16 |
| 249 | 4-{4-[7-(Aminocarbonyl)-2H-indazol-2-yl]benzyl}-6-(hydroxymethyl)-1,4-oxazepan-4-ium trifluoroacetate | 380 | 381 | 16 |
| 250 | {4-[7-(Aminocarbonyl)-2H-indazol-2-yl]phenyl}-N-{[1-(hydroxymethyl)cyclobutyl]methyl}methana minium trifluoroacetate | 364 | 365 | 16 |
| 251 | {4-[7-(Aminocarbonyl)-2H-indazol-2-yl]phenyl}-N-{[1-(hydroxymethyl)cyclohexyl]methyl}methana minium trifluoroacetate | 392 | 393 | 16 |
| 252 | 1-{4-[7-(Aminocarbonyl)-2H-indazol-2-yl]benzyl}-4-(5-methyl-1H-benzimidazol-2-yl)piperidinium trifluoroacetate | 464 | 465 | 16 |
| 253 | 2-(1-{4-[7-(Aminocarbonyl)-2H-indazol-2-yl]benzyl}-4-hydroxypiperidinium-4-yl)pyridinium bis(trifluoroacetate) | 427 | 428 | 16 |
| 254 | 1-{4-[7-(Aminocarbonyl)-2H-indazol-2-yl]benzyl}-3,3-difluoropyrrolidinium trifluoroacetate | 356 | 357 | 16 |
| 255 | 2-(4-{[(2R)-2-(Fluoromethyl)pyrrolidin-1-yl]methyl}phenyl)-2H-indazole-7-carboxamide | 352 | 353 | 16 |
| 256 | N-{4-[7-(Aminocarbonyl)-2H-indazol-2-yl]benzyl}-2-oxopyrrolidin-3-aminium trifluoroacetate | 349 | 350 | 16 |
| 257 | 5-Fluoro-2-(4-formylphenyl)-2H-indazole-7-carboxamide | 283 | 284 | 88 |
| 258 | 3-[({4-[7-(Aminocarbonyl)-5-fluoro-2H-indazol-2-yl]phenyl}amino)carbonyl]-1-methylazetidinium trifluoroacetate | 367 | 368 | 76 |
| 259 | 1-{4-[7-(Aminocarbonyl)-5-fluoro-2H-indazol-2-yl]benzyl}-3-[(dimethylammonio)methyl]piperidinium bis(trifluoroacetate) | 409 | 410 | 16 |
| 260 | 3-[({4-[7-(Aminocarbonyl)-5-fluoro-2H-indazol-2-yl]-2-fluorophenyl}amino)carbonyl]azetidinium trifluoroacetate | 371 | 372 | 77 |
| 261 | 2-{4-[7-(Aminocarbonyl)-5-fluoro-2H-indazol-2-yl]benzyl}-2,7-diazoniaspiro[4.5]decane bis(trifluoroacetate) | 407 | 408 | 16 |
| 262 | 4,5-Difluoro-2-{4-[(methylamino)methyl]phenyl}-2H-indazole-7-carboxamide trifluoroacetate | 316 | 317 | 3 |
| 263 | 5-Fluoro-2-(3-fluoro-4-{[(1-methylazetidin-3-yl)carbonyl]amino}phenyl)-2H-indazole-7-carboxamide trifluoroacetate | 385 | 386 | 77 |
| 264 | 5-Fluoro-2-(3-fluoro-4-formylphenyl)-2H-indazole-7-carboxamide | 301 | 302 | 87 |
| 265 | 5-Fluoro-2-(5-fluoro-2-formylphenyl)-2H-indazole-7-carboxamide | 301 | 302 | 87 |
| 266 | {4-[7-(Aminocarbonyl)-5-fluoro-2H-indazol-2-yl]-2-fluorophenyl}-N-{[1-(hydroxymethyl)cyclopentyl]methyl}methana minium trifluoroacetate | 414 | 415 | 16 |
| 267 | 5-Fluoro-2-[3-fluoro-4-({[(3R)-1-methylpiperidin-3-yl]carbonyl}amino)phenyl]-2H-indazole-7-carboxamide trifluoroacetate | 413 | 414 | 77 |
| 268 | 1-{4-[7-(Aminocarbonyl)-5-fluoro-2H-indazol-2-yl]-2-fluorobenzyl}-4-methylpiperazinediium bis(trifluoroacetate) | 367 | 368 | 16 |
| 269 | 4-[({4-[7-(Aminocarbonyl)-5-fluoro-2H-indazol-2-yl]benzyl}ammonio)methyl]-1-methylpiperidinium bis(trifluoroacetate) | 395 | 396 | 16 |
| 270 | 4-[({4-[7-(Aminocarbonyl)-5-fluoro-2H-indazol-2-yl]-2-fluorobenzyl}ammonio)methyl]-1-methylpiperidinium bis(trifluoroacetate) | 413 | 414 | 16 |

### EXAMPLE 271 7-[7-(Aminocarbonyl)-2H-indazol-2-yl]-1-methyl-1,2,3,4-tetrahydroisoquinolinium trifluoroacetate (HH2)

### Step 1: 2-(3,4-Dihydroisoquinolin-7-yl)-2H-indazole-7-carboxamide (HH1)

To a stirred solution of Example 73, **(M1)** (1.0 eq) and Et₃N (1.0 eq) in DMSO (0.15M) was added IBX (1.1 eq) and reaction mixture was stirred at RT for 1h, then quenched with sat. aq. Na₂S₂O₃ solution. Reaction mixture was partitoned between EtOAc and sat. aq. NaHCO₃ solution, and separated. The aqueous phase was extracted with EtOAc several times. Recombined organic phases were washed with brine, dried (Na₂SO₄), filtered and concentrated. Crude product was purified by SCX cartridge to remove traces of DMSO affording a yellow solid which was used in the next step without further purification. ¹H NMR (400 MHz, DMSO-d₆,300K) δ 9.30 (1H, s), 8.58 (1H, br. s), 8.50 (1H, br. s), 8.24 (1H, s), 8.17 (1H, d, J = 8.1 Hz), 8.07 (1H, d, J = 6.8 Hz), 8.03 (1H, d, J = 8.3 Hz), 7.86 (1H, br. s), 7.48 (1H, d, J = 8.3 Hz), 7.28 (1H, dd, J = 8.1, 7.3 Hz), 4.05-3.96 (2H, m), 3.28-3.19 (2H, m). MS (ES) C₁₇H₁₄N₄O requires: 290, found: 291 (M+H)⁺.

### Step 2: 7-[7-(Aminocarbonyl)-2H-indazol-2-yl]-1-methyl-1,2,3,4-tetrahydro isoquinolinium trifluoroacetate (HH2)

**(HH1)** (1.0 eq) was dissolved in dry THF (0.05 M) under Argon atmosphere and cooled to -78°C. To this solution boron trifluoride etherate (2.0 eq) and methylmagnesium bromide (2.0 eq) were added. Reaction mixture was stirred overnight warming to RT and was then quenched by adding sat. aq. NH₄Cl solution. EtOAc was added and the layers were separated. The aqueous phase was extracted with EtOAc, and the recombined organic phases were washed with brine, dried (Na₂SO₄), filtered and concentrated. Crude product was purified by preparative RP-HPLC (column: C18), using H₂O (0.1 % TFA) and MeCN (+0.1 % TFA) as eluents. The desired fractions were lyophilized to afford the titled compound **(HH2)** as a white solid. ¹H NMR (400 MHz, CD₃CN,300K) δ 8.85 (1H, s), 8.76 (1H, br. s), 8.17 (1H, d, J = 6.8 Hz), 8.02 (1H, d, J = 8.3 Hz), 7.98-7.92 (2H, m), 7.44 (1H, d, J = 8.1 Hz), 7.27 (1H, dd, J = 8.1, 7.1 Hz), 6.42 (1H, br. s), 4.82-4.74 (1H, m), 3.63-3.53 (1H, m), 3.51-3.42 (1H, m), 3.29-3.09 (2H, m), 1.78 (3H, d, J = 6.8 Hz). MS (ES) C₂₀H₁₉F₃N₄O₃ requires: 306, found: 307 (M+H)⁺.

### EXAMPLE 272 3-{4-[7-(Aminocarbonyl)-5-fluoro-2H-indazol-2-yl]phenyl}-1-ethylpiperidinium trifluoroacetate (II3)

### Step 1: 3-{4-[7-(Aminocarbonyl)-5-fluoro-2H-indazol-2-yl]phenyl}-1-ethylpyridinium iodide (II1)

To a solution of Example 80, **(U2)** (1.0 eq) in dry CH₃CN (0.2 M) iodoethane (1.3 eq) was added dropwise at RT and the reaction mixture was refluxed for 3h. Then, the mixture was cooled to RT. Evaporation of the solvent gave **II1** which was used in the next step without further purification. MS (ES) C₂₁H₁₈FN₄OI requires: 361, found: 362 (M+H⁺).

### Step 2: 2-[4-(1-Ethyl-1,2,5,6-tetrahydropyridin-3-yl)phenyl]-5-fluoro-2H-indazole-7-carboxamide (II2)

To a stirred solution of **(II1)** in dry MeOH (0.2 M), NaBH₄ (3.0 eq) was added portionwise at RT and the reaction mixture was stirred at RT O/N. Then, the mixture was quenched with sat. aq. NH₄Cl solution. MeOH was concentrated under reduced pressure and EtOAc was added. The organic phase separated and was washed with sat. aq. NaHCO₃ solution, brine and dried (Na₂SO₄). Evaporation of the solvent gave **112** which was used in the next step without further purification. MS (ES) C₂₁H₂₁FN₄O requires: 364, found: 365 (M+H⁺).

### Step 3: 3-{4-[7-(Aminocarbonyl)-5-fluoro-2H-indazol-2-yl]phenyl}-1-ethylpiperidinium trifluoroacetate (II3)

To a solution of **(II2)** (1.0 eq) in MeOH (0.2 M) were added 10 % Pd/C (0.35 eq) and HCl (1.0 eq) and the reaction mixture was stirred under H₂ atmosphere (1 atm) for 48 h. Then, the mixture was filtered through Celite and solvent was removed under reduced pressure affording a residue which was purified by reverse phase RP-HPLC (column: C18), using H₂O (0.1% TFA) and MeCN (0.1% TFA) as eluents, the desired fractions were lyophilized to afford the titled compound **(II3)** as a white powder. ¹H NMR (400MHz, DMSOd₆, 300K) δ 9.30 (1H, s), 8.53 (1H, br. s), 8.16 (2H, d, J = 8.3 Hz), 8.11 (1H, br. s), 7.90-7.70 (2H, m), 7.55 (2H, d, J = 8.3 Hz), 3.60-3.40 (2H, m), 3.20-3.00 (4H, m),, 3.00-2.80 (1H, m), 2.00-1.90 (2H, m), 1.80-1.70 (2H, m), 1.30-1.20 (3H, m), MS (ES) C₂₁H₂₃FN₄O requires: 366, found: 367 (M+H⁺).

### EXAMPLE 273 2-(4-Cyanophenyl)-5-fluoro-2H-indazole-7-carboxamide (JJ1)

The desired compound was prepared following the general procedure reported in Example 3, step 1 using 5-fluoro-1H-indazole-7-carboxamide and 4-fluorobenzonitrile. The crude was purified by precipitation by adding water to the reaction mixture followed by filtration. ¹H NMR (300 MHz, DMSOd₆ , 300K) δ 9.42 (1H, s), 8.47 (3H, m), 8.11 (3H, d, J = 8.4 Hz), 7.82 (2H, m). MS (ES⁺) C₁₅H₉FN₄O requires: 280, found: 281 (M+H)⁺.

### EXAMPLE 274 5-fluoro-2-[4-(1H-tetrazol-5-yl)phenyl]-2H-indazole-7-carboxamide (KK1)

To a solution of Example 273, **JJ1** in DMF (0.8 M) were added sodium azide (12.0 eq.) and ammonium chloride (12.0 eq), and the reaction mixture was heated under MW conditions at 200 °C for 20 min. The crude was purified by precipitation by adding water to the reaction mixture followed by filtration.¹H NMR (300 MHz, DMSOd₆, 300K) δ 9.31 (1H, s), 8.8.61 (1H, br. s), 8.18 (4H, s), 8.07 (1H, br. s), 7.77-7.87 (2H, m), MS (ES⁺) C₁₅H₁₀FN₇O requires: 323, found: 324 (M+H)⁺.

### EXAMPLE 275 2-(4-Aminophenyl)-5-fluoro-2H-indazole-7-carboxamide hydrochloride (LL2)

### Step 1: 5-Fluoro-2-(4-nitrophenyl)-2H-indazole-7-carboxamide (LL1)

The desired compound prepared following the general procedure reported in Example 3 step 1 using 5-fluoro-1H-indazole-7-carboxamide and 1-fluoro-4-nitrobenzene. The crude was purified by precipitation by adding water to the reaction mixture followed by filtration. MS (ES⁺) C₁₄H₉FN₄O requires: 300, found: 301 (M+H)⁺.

**Step 2: 2-(4-Aminophenyl)-5-fluoro-2H-indazole-7-carboxamide hydrochloride (LL2) (LL1)** was dissolved in DMF (0.2 M) and PtO₂ (0.5 eq) and 1N HCl (1 eq) were added. The mixture was stirred under H₂ atmosphere for 24 hr. Then the mixture was filtered and solvent was removed under reduced pressure affording a residue which was crude was purified by RP- HPLC (column: C 18), using H₂O (0.1 % TFA) and MeCN (0.1 % TFA) as eluents, and the desired fractions were lyophilized to afford the titled compound.. ¹H NMR (300 MHz, DMSOd₆, 300K) δ 9.05 (1H, s), 8.58 (1H, br. s), 8.10 (1H, s), 7.73-7.83 (4H, m), 6.8 (2H, d, J= 8.8). MS (ES⁺) C₁₄H₁₁FN₄O requires: 270, found: 271 (M+H)⁺.

### EXAMPLE 276 tert-Butyl 3-[7-(aminocarbonyl)-5-fluoro-2H-indazol-2-yl]pyrrolidine-1-carboxylate (MM3)

### Step 1: tert-Butyl 3-{[2,5-difluoro-3-(methoxycarbonyl)phenyl]methylene}amino) pyrrolidine-1-carboxylate (MM1).

A mixture of methyl 2,5-difluoro-3-formylbenzoate (Preparative Example A, **CC5)** (1.0 eq.) and tert-butyl 3-aminopyrrolidine-1-carboxylate (1.05 eq.) in EtOH (0.2 M) was heated at reflux for 2 hr until TLC revealed reaction completion (25% EtOAc/Hexane). Evaporation of the solvent gave the title imine which was used in the next step without further purification. ¹H NMR (d6 DMSO, 300K, 300MHz) δ (ppm) 8.63 (1H, s), 7.88-7.73 (2H, m); 4.22-4.10 (1H, m); 3.87 (3H, s); 3.58-3.12 (6H, m); 2.20-2.03 (1H, m); 1.93-1.77 (1H, m); 1.40 (9H, s).

### Step 2: Methyl 2-[1-(tert-butoxycarbonyl)pyrrolidin-3-yl]-5-fluoro-2H-indazole-7-carboxylate (MM2).

A mixture of **(MM1)** (1.0 eq.) and NaN₃ (2 eq.) in dry DMF (0.6 M) was irradiated in a microwave oven in a sealed tube (110 °C 3h, then 140 °C 40min). The crude was filtered through a silica pad and reduced under reduced pressure. The residue was purified by flash column chromatography on silica using a gradient of 18-100% EtOAc/Petroleum ether to yield the desired compound as a brown oil. 1H NMR (D6 DMSO + 5% TFA, 300K, 300 MHz): δ (ppm) 8.58 (1H, s); 7.82-7.69 (2H, m); 5.40-5.29 (1H, m); 3.88 (3H, s); 3.86-3.79 (1H, m); 3.72-3.53 (2H, m); 3.51-3.39 (1H, m); 2.46-2.35 (2H, m, partially overlapped by DMSO); 1.39 (9H, s). ¹⁹F NMR (D6 DMSO + 5% TFA, 300K, 283 MHz): δ (ppm) -122.82. MS (ES) C₁₈H₂₂FN₃O₄ requires: 363, found: 364 (M+H)⁺.

### Step 3: tert-Butyl 3-[7-(aminocarbonyl)-5-fluoro-2H-indazol-2-yl]pyrrolidine-1-carboxylate (MM3).

A solution of **(MM2)** in 7N ammonia/MeOH (100 eq.) was irradiated at microwave in a sealed tube (120 °C, 1h 40min). After evaporation of solvent under reduced pressure, the residue was taken into EtOAc and filtered over a silica pad, eluting with EtOAc. After evaporation of the solvent, the title compound was isolated as a pale yellow foam. ¹H NMR (D6 DMSO+ 5%TFA, 300K, 300 MHz): δ (ppm) 8.64 (1H, s); 8.42 (1H, br. s); 8.00 (1H, br. s); 7.80-7.67 (2H, m); 5.43-5.32 (1H, m); 3.89-3.67 (2H, m); 3.63-3.39 (2H, m); 2.47-2.37 (2H, m, partially overlapped by DMSO); 1.39 & 1.37 (9H, 2s). ¹⁹F NMR (D6 DMSO + 5%TFA, 300K, 283 MHz): δ (ppm) -121.80. MS (ES) C₁₇H₂₁FN₄O₃ requires: 348, found: 349 (M+H)⁺.

### EXAMPLE 277 3-[7-(Aminocarbonyl)-5-fluoro-2H-indazol-2-yl]pyrrolidinium trifluoroacetate (NN1).

A solution of Example 276, **(MM3)** in TFA / DCM 1:1 (0.1 M) was stirred at room temperature for 20 min. After evaporation of solvent under reduced pressure, and treatment with Et₂O, the title compound was isolated as a yellowish solid. ¹H NMR (D6 DMSO, 300K, 600 MHz): δ (ppm) 9.32 (2H, br. s), 8.74 (1H, s), 8.36 (1H, s), 8.03 (1H, s), 7.80 (2H, dd, J₁=16Hz, J₂= 3Hz), 5.64-5.59 (1H, m), 3.79 (2H, d, J= 4Hz), 3.59-3.52 (1H, m), 3.48-3.41 (1H, m), 2.61-2.53 (1H, m), 2.48-2.41 (1H, m). ¹³C NMR (D6 DMSO, 300K, 150 MHz): δ (ppm) 164.31, 157.72, 156.15, 142.79, 126.22 (d, J= 8Hz), 123.65 (d, J= 8Hz), 121.76 (d, J= 11Hz), 118.99 (d, J= 30Hz), 107.94 (d, J= 24Hz), 60.84. 49.90, 44.85, 31.86. ¹⁹F NMR (D6 DMSO, 300K, 283 MHz): δ (ppm) -76.60, -123.17. MS (ES) C₁₂H₁₃FN₄O requires: 248, found: 249 (M+H)⁺.

### EXAMPLE 278 3-[7-(Aminocarbonyl)-5-fluoro-2H-indazol-2-yl]-1-methylpyrrolidinium trifluoroacetate (OO1)

To a solution of Example 277, **(NN1)** in MeOH (0.06 M), AcOH (5 eq.), formaldehyde (37% in water, 2.8 eq.) and Silica-supported cyanoborohydride (3 eq.) were added and the mixture was stirred at room temperature overnight. After filtration, the filtrate was passed through a SCX cartridge, eluting with MeOH then with 1N NH₃/MeOH. The solvent was evaporated, a mixture of DCM/TFA 1:1 was added and the title compound was isolated after evaporation of the solvents. ¹H NMR (D6 DMSO, 300K, 300 MHz): δ (ppm) 10.19 (1H, br. s), 8.72 (1H, s), 8.38 (1H, br. s), 8.03 (1H, br. s), 7.84-7.72 (2H, m), 5.73-5.57 (1H, m), 4.30-3.55 (4H, m, partially overlapped by water), 2.96 (3H, s), 2.88-2.61 (1H, m), 2.60-2.53 (1H, m, partially overlapped by DMSO). ¹⁹F NMR (D6 DMSO, 300K, 283 MHz): δ (ppm) -76.60, - 123.27. MS (ES) C₁₃H₁₅FN₄O requires: 262, found: 263 (M+H)⁺.

### EXAMPLE 279 3-[7-(Aminocarbonyl)-5-fluoro-2H-indazol-2-yl]-1-ethylpyrrolidinium trifluoroacetate PP1

The title compound was prepared according to the procedure for Example 278, substituting acetaldehyde for formaldehyde, and isolated by RP-HPLC. ¹⁹F NMR (D6 DMSO, 300K, 283 MHz): δ (ppm) -76.60, - 122.36. MS (ES) C₁₄H₁₇FN₄O requires: 276, found: 277 (M+H)⁺.

### EXAMPLE 280 3-[7-(Aminocarbonyl)-5-fluoro-2H-indazol-2-y]-1-propylpyrrolidinium trifluoroacetate (QQ1)

The title compound was prepared according to the procedure for Example 278, substituting propionaldehyde for formaldehyde. Compound at NMR was present as a mixture of 2 rotamers. ¹H NMR (D6 DMSO, 300K, 400 MHz): δ (ppm) 10.21 (0.4H, br. s), 10.04 (0.6H, br. s), 8.77-8.72 (1H, m), 8.41 (0.6H, br. s), 8.30 (0.4H, br. s), 8.06 (1H, br. s), 7.83-7.74 (2H, m), 5.71-5.54 (1H, m), 4.25-4.09 (1H, m), 3.91-3.68 (2H, m), 3.55-3.43 (0.4H, m), 3.35-3.14 (2.6H, m), 2.84-2.73 (0.6H, m), 2.62-2.51 (1.4H, m, partially overlapped under water), 1.74-1.62 (2H, m), 0.93 (3H, t, J= 7 Hz). ¹⁹F NMR (D6 DMSO, 300K, 376 MHz): δ (ppm) -76.60, -122.52, -122.55. MS (ES) C₁₅H₁₉FN₄O requires: 290, found: 291 (M+H)⁺.

### EXAMPLE 281 3-[7-(Aminocarbonyl)-5-fluoro-2H-indazol-2-yl]-1-isopropylpyrrolidinium trifluoroacetate (RR1)

The title compound was prepared according to the procedure for Example 278, substituting acetone for formaldehyde. Compound at NMR was present as a mixture of 2 rotamers. ¹H NMR (D6 DMSO, 300K, 400 MHz): δ (ppm) 10.15 (0.5 H, br. s), 10.02 (0.5H, br. s), 8.75 (1H, s), 8.42 (0.5H, br. s), 8.31 (0.5H, br. s), 8.09 (0.5H, br. s), 8.03 (0.5H, br. s), 7.83-7.75 (2H, m), 5.68-5.53 (1H, m), 4.22-4.12 (1H, m), 3.87-3.70 (2H, m), 3.68-3.33 (2H, m), 2.82-2.70 (0.6H, m), 2.62-2.52 (1.4H, m), 1.37-1.26 (6H, m). ¹⁹F NMR (D6 DMSO, 300K, 376 MHz): δ (ppm) -76.60, -122.66. MS (ES) C₁₅H₁₉FN₄O requires: 290, found: 291 (M+H)⁺.

### EXAMPLE 282 3-[7-(Aminocarbonyl)-5-fluoro-2H-indazol-2-yl]-1-cyclohexylpyrrolidinium trifluoroacetate (SS1)

The title compound was prepared according to the procedure for Example 278, substituting cyclohexanone for formaldehyde. MS (ES) C₁₈H₂₃FN₄O requires: 330, found: 331 (M+H)⁺.

### EXAMPLE 283 3-[7-(Aminocarbonyl)-5-fluoro-2H-indazol-2-yl]-1-cyclobutylpyrrolidinium trifluoroacetate (TT1)

The title compound was prepared according to the procedure for Example 278, substituting cyclobutanone for formaldehyde. MS (ES) C₁₆H₁₉FN₄O requires: 302, found: 303 (M+H)⁺.

### EXAMPLE 284 tert-Butyl 4-[7-(aminocarbonyl)-2H-indazol-2-yl]-4-methylpiperidine-1-carboxylate (UU3)

### Step 1: tert-Butyl 4-{[3-(methoxycarbonyl)-2-nitrobenzylidene]amino}-4-methylpiperidine-1-carboxylate (UU1).

A mixture of methyl 3-formyl-2-nitrobenzoate Example 1, **(A3)** (1.0 eq.) and tert-butyl 4-amino-4-methylpiperidine-1-carboxylate (WO 2005/101989)(1.05 eq.) in EtOH (0.2 M) was stirred at reflux for 24 hr. Evaporation of the solvent gave the title imine which was used in the next step without further purification.

### Step 2: Methyl 2-[1-(tert-butoxycarbonyl)pyrrolidin-3-yl]-5-fluoro-2H-indazole-7-carboxylate (UU2).

A mixture of **(UU1)** (1.0 eq.) and NaN₃ (2 eq.) in dry DMF (0.25 M) was irradiated in a microwave oven in a sealed tube (140 °C 40min). The title compound was isolated as described in Example 276, step 2 for **(MM2).** ¹H NMR (D6 DMSO + 5% TFA, 300K, 400 MHz): δ (ppm) 8.67 (1H, s); 7.97 (1H, d, J= 8 Hz); 7.90 (1H, d, J= 7 Hz); 7.12 (1H, t, J= 8 Hz); 3.88 (3H, s); 3.57-3-47 (2H, m); 3.34-3.25 (2H, m); 2.55.2.50 (2H, m, partially hidden under DMSO); 2.20-1.93 (2H, m); 1.61 (3H, s); 1.40 (9H, s). MS (ES) C₂₀H₂₇N₃O₄ requires: 373, found: 374 (M+H)⁺.

### Step 3: tert-Butyl 4-[7-(aminocarbonyl)-2H-indazol-2-yl]-4-methylpiperidine-1-carboxylate (UU3).

A solution of **(UU2)** in 7N ammonia/MeOH (100 eq.) was irradiated at microwave in a sealed tube (130 °C, 1h 14 h). Evaporation of the solvent provided the title compound. ¹H NMR (D6 DMSO + 5% TFA, 300K, 400 MHz): δ (ppm) 8.77 (1H, s); 8.53 (1H, br. s); 7.98 (1H, d, J= 7 Hz); 7.92 (1H, d, J= 8 Hz); 7.74 (1H, br. s); 7.22-7.14 (1H, m); 3.58-3.46 (2H, m); 3.32-3.19 (2H, m); 2.50-2.49 (2H, m, partially hidden under DMSO); 2.07-1.96 (2H, m); 1.60 (3H, s); 1.38 (9H, s). MS (ES) C₁₉H₂₆N₄O₃ requires: 358, found: 359 (M+H)⁺.

### EXAMPLE 285 4-[7-(Aminocarbonyl)-2H-indazol-2-yl]-4-methylpiperidinium trifluoroacetate (VV1)

A solution of Example 284, **(UU3)** in TFA / DCM 1:1 (0.1 M) was stirred at room temperature for 20 min. After evaporation of solvent under reduced pressure and treatment with Et₂O, the title compound was isolated as a yellowish solid. ¹H NMR (D6 DMSO, 300K, 600 MHz): δ (ppm) 8.85 (1H, s); 8.57 (1H, br. s); 8.45 (2H, br. s); 8.01 (1H, d, J= 6 Hz); 7.96 (1H, d, J= 8 Hz); 7.85 (1H, br. s); 7.25-7.19 (1H, m); 3.34-3.23 (2H, m); 3.07-2.94 (2H, m); 2.85-2.73 (2H, m); 2.26-2.15 (2H, m); 1.65 (3H, s). MS (ES) C₁₄H₁₈N₄O requires: 258, found: 259 (M+H)⁺.

The following examples were prepared according to the methods of the previous examples:

| **Example** | **Name** | **MWt** | **(M+H)⁺** | **Procedure of Example** |
|---|---|---|---|---|
| 286 | 2-{4-[7-(Aminocarbonyl)-5-fluoro-2H-indazol-2-yl]phenyl}pyrrolidinium trifluoroacetate | 324 | 325 | Example 1 using Preparative Example A, CC5 |
| 287 | 2-[4-(4,5-Dihydro-1H-imidazol-2-yl)phenyl]-5-fluoro-2H-indazole-7-carboxamide trifluoroacetate | 323 | 324 | Example 1 using Preparative Example A, CC5 |
| 288 | 6-[7-(Aminocarbonyl)-5-fluoro-2H-indazol-2-yl]-1,2,3,4-tetrahydroisoquinolinium chloride | 310 | 311 | Example 1 using Preparative Example A, CC5 |
| 289 | 2-{4-[7-(Aminocarbonyl)-5-fluoro-2H-indazol-2-yl]phenyl}piperidinium chloride | 338 | 339 | Example 1 using Preparative Example A, CC5 |
| 290 | 5-Fluoro-2-[4-(1H-pyrazol-1-yl)phenyl]-2H-indazole-7-carboxamide | 321 | 322 | Example 1 using Preparative Example A, CC5 |
| 291 | 2-[4-(Aminosulfonyl)phenyl]-5-fluoro-2H-indazole-7-carboxamide | 334 | 335 | Example 1 using Preparative Example A, CC5 |
| 292 | 5-Fluoro-2-(5,6,7,8-tetrahydro-1,7-naphthyridin-3-yl)-2H-indazole-7-carboxamide | 311 | 312 | Example 1 using Preparative Example A, CC5 |
| 293 | 5-Fluoro-2-(4-piperazin-2-ylphenyl)-2H-indazole-7-carboxamide | 339 | 339 | Example 1 using Preparative Example A, CC5 |
| 294 | Methyl 4-[7-(aminocarbonyl)-5-fluoro-2H-indazol-2-yl]benzoate | 313 | 313 | 273 |
| 295 | 5-Fluoro-2-(1-methylpiperidin-3-yl)-2*H*-indazole-7-carboxamide | 276 | 277 | 278 |
| 296 | 5-Fluoro-2-(1-ethylpiperidin-3-yl)-2*H*-indazole-7-carboxamide | 290 | 291 | 279 |
| 297 | 5-Fluoro-2-(1-propylpiperidin-3-yl)-2*H*-indazole-7-carboxamide | 304 | 305 | 280 |
| 298 | 5-Fluoro-2-(1-isopropylpiperidin-3-yl)-2*H*-indazole-7-carboxamide | 304 | 305 | 281 |
| 299 | 2-(1-Cyclohexylpiperidin-3-yl)-5-fluoro-2*H*-indazole-7-carboxamide | 344 | 345 | 282 |
| 300 | 5-Fluoro-2-(1-methylpiperidin-4-yl)-2*H*-indazole-7-carboxamide | 276 | 277 | 278 |
| 301 | 5-Fluoro-2-(1-ethylpiperidin-4-yl)-2*H*-indazole-7-carboxamide | 290 | 291 | 279 |
| 302 | 5-Fluoro-2-(1-propylpiperidin-4-yl)-2*H*-indazole-7-carboxamide | 304 | 305 | 280 |
| 303 | 5-Fluoro-2-(1-isopropylpiperidin-4-yl)-2*H*-indazole-7-carboxamide | 304 | 305 | 281 |
| 304 | 2-(1-Cyclohexylpiperidin-4-yl)-5-fluoro-2*H*-indazole-7-carboxamide | 344 | 345 | 282 |
| 305 | 2-(1-Cyclobutylpiperidin-4-yl)-5-fluoro-2*H*-indazole-7-carboxamide | 316 | 317 | 283 |
| 306 | 2-(1-Cyclobutylpiperidin-3-yl)-2*H*-indazole-7-carboxamide | 298 | 299 | 283 |
| 307 | 2-[7-(Aminocarbonyl)-5-fluoro-2*H*-indazol-2-yl]-*N,N-*dimethylethanaminium trifluoroacetate | 250 | 251 | 278 |
| 308 | 2-[7-(Aminocarbonyl)-5-fluoro-2*H*-indazol-2-yl]-*N,N-*diethylethanaminium trifluoroacetate | 278 | 279 | 279 |
| 309 | N-{2-[7-(Aminocarbonyl)-5-fluoro-2*H*-indazol-2-yl]ethyl}propan-2-aminium trifluoroacetate | 264 | 265 | 281 |
| 310 | *N*-{2-[7-(Aminocarbonyl)-5-fluoro-2*H*-indazol-2-yl]ethyl}cyclohexanaminium trifluoroacetate | 304 | 305 | 282 |
| 311 | 2-[2-(Dicyclobutylamino)ethyl]-5-fluoro-2*H*-indazole-7-carboxamide | 330 | 331 | 283 |
| 312 | tert-Butyl 3-[7-(aminocarbonyl)-5-fluoro-2H-indazol-2-yl]piperidine-1-carboxylate | 362 | 363 | 276 |
| 313 | tert-Butyl 4-[7-(aminocarbonyl)-5-fluoro-2H-indazol-2-yl]piperidine-1-carboxylate | 362 | 363 | 276 |
| 314 | 3-[7-(Aminocarbonyl)-5-fluoro-2H-indazol-2-yl]piperidinium trifluoroacetate | 262 | 263 | 277 |
| 315 | 4-[7-(Aminocarbonyl)-5-fluoro-2H-indazol-2-yl]piperidinium trifluoroacetate | 262 | 263 | 277 |
| 316 | tert-Butyl 3-[7-(aminocarbonyl)-2H-indazol-2-yl]piperidine-1-carboxylate | 344 | 345 | 276 |
| 317 | *tert*-Butyl {2-[7-(aminocarbonyl)-5-fluoro-2*H*-indazol-2-yl]ethyl}carbamate | 322 | 323 | 276 |
| 318 | 2-[7-(Aminocarbonyl)-5-fluoro-2*H*-indazol-2-yl]ethanaminium trifluoroacetate | 222 | 223 | 277 |
| 319 | 3-[7-(Aminocarbonyl)- 2H-indazol-2-yl]piperidinium trifluoroacetate | 244 | 245 | 277 |
| 320 | 3-[7-(Aminocarbonyl)-2H-indazol-2-yl]-1-methylpiperidinium trifluoroacetate | 258 | 259 | 278 |
| 321 | 3-[7-(Aminocarbonyl)-2H-indazol-2-yl]-1-ethylpiperidinium trifluoroacetate | 272 | 273 | 279 |
| 322 | 3-[7-(Aminocarbonyl)-2H-indazol-2-yl]-1-propylpiperidinium trifluoroacetate | 286 | 287 | 280 |
| 323 | 3-[7-(Aminocarbonyl)-2H-indazol-2-yl]-1-isopropylpiperidinium trifluoroacetate | 286 | 287 | 281 |
| 324 | 3-[7-(Aminocarbonyl)-2H-indazol-2-yl]-1-cyclohexylpiperidinium trifluoroacetate | 326 | 327 | 282 |
| 325 | 3-[7-(Aminocarbonyl)-5-fluoro-2H-indazol-2-yl]-1-cyclobutylpiperidinium trifluoroacetate | 316 | 317 | 283 |
| 326 | *N*-{2-[7-(Aminocarbonyl)-5-fluoro-2*H*-indazol-2-yl]ethyl}-*N*-propylpropan-1-aminium trifluoroacetate | 306 | 307 | 280 |

## Claims

1. A compound of formula I: 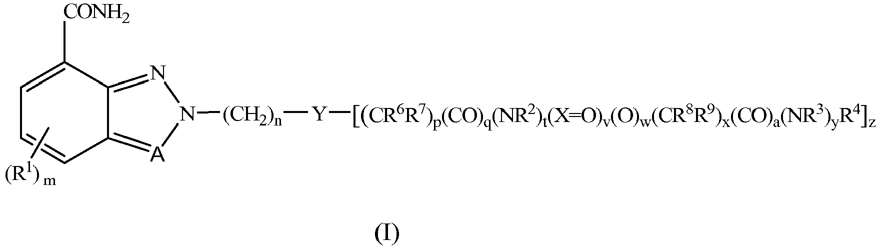 wherein:
a is 0 or 1;
m is 0, 1, 2 or 3;
n is 0, 1, 2, 3, 4, 5 or 6;
each p is independently 0, 1, 2, 3, 4, 5 or 6;
each q is independently 0 or 1;
each t is independently 0 or 1;
each v is independently 0 or 1;
each w is independently 0 or 1;
each x is independently 0, 1, 2, 3, 4, 5 or 6;
each y is independently 0 or 1;
zis 1, 2 or 3;
A is CH or N;
each R¹ is independently hydroxy, halogen, cyano, C₁₋₆alkyl, haloC₁₋₆alkyl, C₁₋₆alkoxy or haloC₁₋₆alkoxy;
Y is a direct bond or a ring which is: a C₃₋₅cycloalkyl, a 4 membered saturated heterocycle containing one N atom, a 5, 6 or 7 membered saturated or partially saturated heterocycle containing 1, 2 or 3 heteroatoms independently selected from N, O and S, a 5 membered unsaturated heterocycle containing 1, 2, 3 or 4 heteroatoms independently selected from O, N and S, but not more than one of which is O or S, a 6 membered unsaturated heterocycle containing 1, 2 or 3 nitrogen atoms, a 6-13 membered saturated, partially saturated or unsaturated hydrocarbon ring or a 8-13 membered unsaturated or partially saturated heterocyclic ring containing 1, 2, 3 or 4 heteroatoms independently selected from N, O and S;
each R² is independently hydrogen, C₁₋₆alkyl or C₃₋₁₀cycloalkyl;
each X is independently C or SO;
each R³ is independently hydrogen or C₁₋₆alkyl;
each R⁴ is independently hydrogen, hydroxy, cyano, halogen, C₁₋₆alkyl, C₂₋₁₀alkenyl, haloC₁₋₆alkyl, hydroxyC₁₋₆alkyl, C₁₋₆alkylcarbonyl, C₁₋₆alkoxy, haloC₁₋₆alkyl, C₁₋₆alkoxycarbonyl, carboxy, nitro or a ring which is: C₆₋₁₀aryl; C₆₋₁₀aryloxy; C₆₋₁₀arylcarbonyl; C₃₋₁₀cycloalkyl; a 4 membered saturated heterocyclic ring containing one N atom; a 5 or 6 membered saturated or partially saturated heterocyclic ring containing one, two or three atoms independently selected from N, O and S; a 5 membered heteroaromatic ring containing 1,2,3 or 4 heteroatoms independently selected from N, O and S, not more than one heteroatom of which is O or S; a 6 membered heteroaromatic ring containing 1, 2 or 3 nitrogen atoms; or a 7-15 membered unsaturated, partially saturated or saturated heterocyclic ring containing 1, 2, 3 or 4 heteroatoms independently selected from N, O and S; any of which rings being optionally substituted by one or more groups independently selected from (CH₂)_{b}R⁵;
each b is independently 0, 1, 2, 3, 4, 5 or 6;
each R⁵ is independently hydroxy, oxo, cyano, halogen, C₁₋₆alkyl, C₂₋₁₀alkenyl, haloC₁₋₆alkyl, C₁₋₆alkylcarbonyl, C₁₋₆alkoxy, haloC₁₋₆alkoxy, hydroxyC₁₋₆alkyl, C₁₋₆alkoxycarbonyl, carboxy, NR^{a}R^{b}, CONR^{a}R^{b}, S(O)ᵣR^{c} or a ring which is: C₁₋₁₀aryl; C₆₋₁₀arylC₁₋₆alkyl; a 4 membered saturated heterocyclic ring containing one N atom; a 5, 6 or 7 membered saturated or partially saturated heterocyclic ring containing one, two or three atoms independently selected from N, O and S; a 5 membered heteroaromatic ring containing 1, 2, 3 or 4 heteroatoms independently selected from N, O and S, not more than one heteroatom of which is O or S; a 6 membered heteroaromatic ring containing 1, 2 or 3 nitrogen atoms; or a 7-10 membered unsaturated or partially saturated heterocyclic ring containing 1, 2, 3 or 4 heteroatoms independently selected from N, O and S; any of which rings being optionally substituted by one or more groups independently selected from hydroxy, cyano, halogen, C₁₋₆alkyl, C₁₋₆alkoxy, C₂₋₁₀alkenyl, haloC₁₋₆alkyl, amino, C₁₋₆alkylamino and di(C₁₋₆alkyl)amino;
each of R^{a} and R^{b} is independently hydrogen, C₁₋₆alkyl, C₁₋₆alkylcarbonyl, haloC₁₋₆alkyl, hydroxyC₁₋₆alkyl, S(O)ᵣR^{c} or S(O)ᵣN(R^{d})₂; or
R^{a} and R^{b} together with the N atom to which they are attached form a 4 membered saturated heterocycle containing one N atom or a 5, 6 or 7 membered saturated or partially saturated heterocycle containing one, two or three N atoms and zero or one O atom, the ring being optionally substituted by one or more groups independently selected from hydroxy, cyano, halogen, C₁₋₆alkyl, C₁₋₆alkoxy, C₂₋₁₀alkenyl and haloC₁₋₆alkyl;
r is 0, 1 or 2;
R^{c} is C₁₋₆alkyl, C₆₋₁₀aryl, a 5 membered heteroaromatic ring containing 1, 2, 3 or 4 heteroatoms independently selected from N, O and S, not more than one heteroatom of which is O or S; a 6 membered heteroaromatic ring containing 1, 2 or 3 nitrogen atoms; or a 7-10 membered unsaturated or partially saturated heterocyclic ring containing 1, 2, 3 or 4 heteroatoms independently selected from N, O and S; any of which rings being optionally substituted by one or more groups independently selected from hydroxy, cyano, halogen, C₁₋₆alkyl, C₂₋₁₀alkenyl and haloC₁₋₆alkyl;
each R^{d} is independently hydrogen or C₁₋₆alkyl;
each of R⁶ and R⁷ is independently hydrogen or C₁₋₆alkyl;
each of R⁸ and R⁹ is independently hydrogen, C₁₋₆alkyl, hydroxy, haloC₁₋₆alkyl, hydroxyC₁₋₆alkyl, amino, C₁₋₆alkylamino or di(C₁₋₆alkyl)amino;
or a pharmaceutically acceptable salt, stereoisomer or tautomer thereof, with the proviso that the following compound is excluded:

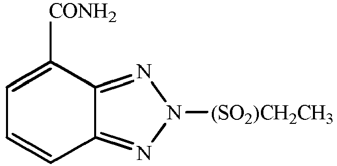

2. A compound of claim 1 wherein Y is a 6 membered unsaturated heterocycle containing 1, 2 or 3 nitrogen atoms, a 6-13 membered saturated, partially saturated or unsaturated hydrocarbon ring or a 8-13 membered unsaturated or partially saturated heterocyclic ring containing 1, 2, 3 or 4 heteroatoms independently selected from N, O and S.

3. A compound of claim 1 of formula II: 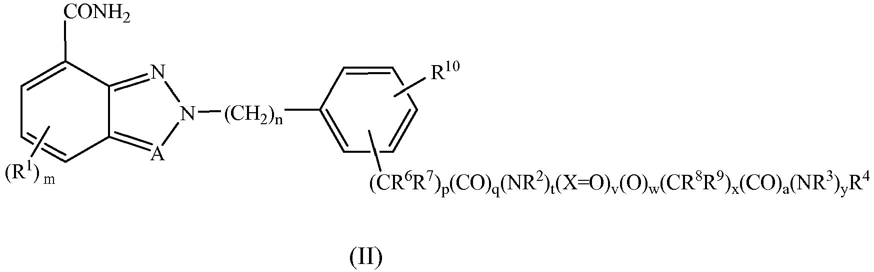 wherein:
a, m, n, p, q, t, v, w, x, y, A, R¹, R², R³, R⁴, R⁶, R⁷, R⁸and R⁹ are as defined in claim 1;
R¹⁰ is hydrogen, halogen, C₁₋₆alkyl or haloC₁₋₆alkyl;
or a pharmaceutically acceptable salt, stereoisomer or tautomer thereof.

4. A compound of claim 1 formula IV: 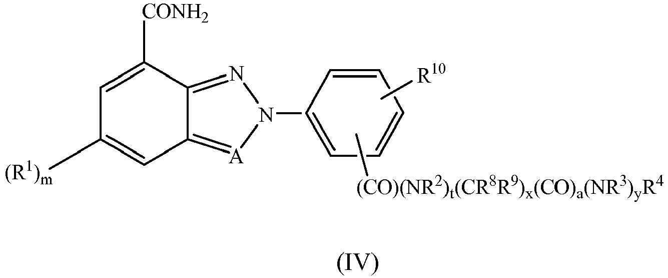 wherein:
m, A, R¹, R², R³, R⁴, R⁸and R⁹ are as defined in claim 1;
a is 0 or 1;
t is 0 or 1;
x is 0, 1 or 2;
y is 0 or 1;
R¹⁰ is hydrogen, halogen, C₁₋₆alkyl or haloC₁₋₆alkyl;
or a pharmaceutically acceptable salt, stereoisomer or tautomer thereof

5. A compound of claim 1 of formula V: 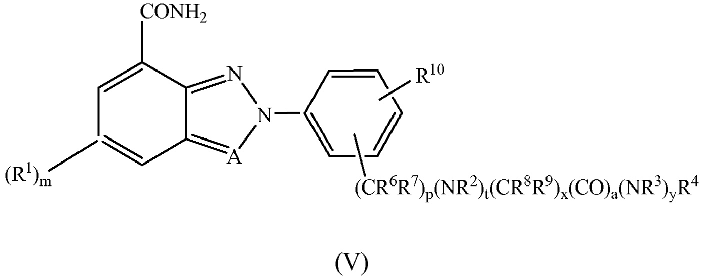 wherein:
a, m, t, y, A, R¹, R², R³, R⁴, R⁶, R⁷, R⁸ and R⁹ are as defined in claim 1;
p is 1 or 2;
x is 0, 1 or 2;
R¹⁰ is hydrogen, halogen, C₁₋₆alkyl or haloC₁₋₆alkyl;
or a pharmaceutically acceptable salt, stereoisomer or tautomer thereof

6. A compound of claim 1 of formula VI: 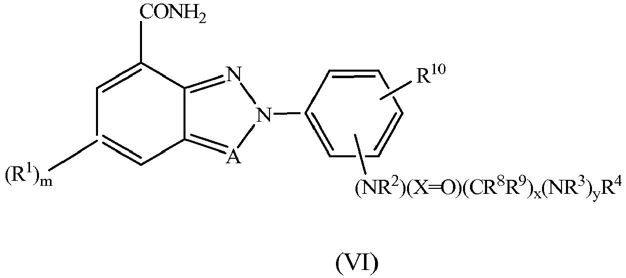 wherein:
m, y, A, R¹, R², R³, R⁴, R⁸, R⁹ and X are as defined in claim 1;
x is 0, 1 or 2;
R¹⁰ is hydrogen, halogen, C₁₋₆alkyl or haloC₁₋₆alkyl;
or a pharmaceutically acceptable salt, stereoisomer or tautomer thereof

7. A compound of claim 1 of formula VII: 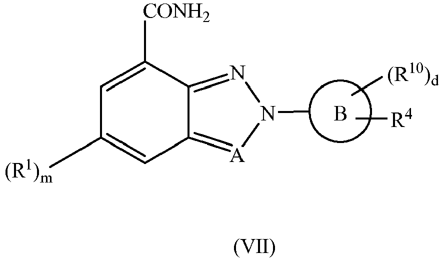 wherein:
m, A, R¹ and R⁴ are as defined in claim 1;
d is 0, 1 or 2;
B is a 6 membered unsaturated heterocycle containing 1, 2 or 3 nitrogen atoms, 6-13 membered saturated, partially saturated or unsaturated hydrocarbon ring or a 8-13 membered unsaturated or partially saturated heterocyclic ring containing 1, 2, 3 or 4 heteroatoms independently selected from N, O and S;
R¹⁰ is halogen or C₁₋₆alkyl;
or a pharmaceutically acceptable salt, stereoisomer or tautomer thereof.

8. A compound of claim 1 of formula VIII: 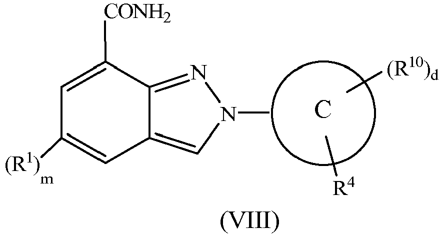
wherein m, R¹ and R⁴ are as defined in claim 1;
d is 0, 1 or 2;
C is a 4 membered saturated heterocycle containing one N atom or a 5, 6 or 7 membered saturated or partially saturated heterocycle containing 1, 2 or 3 heteroatoms independently selected from N, O and S;
R¹⁰ is halogen or C₁₋₆alkyl;
or a pharmaceutically acceptable salt, stereoisomer or tautomer thereof.

9. A compound of any previous claim wherein:
R⁴ is is hydrogen, hydroxy, halogen, C₁₋₆alkyl, haloC₁₋₄alkyl, C₁₋₄alkoxy, cyano or a ring selected from cyclopropyl, cyclobutyl, cyclopentyl, cyclohexyl, azetidinyl, pyrrolidinyl, piperidinyl, piperazinyl, diazepanyl, oxazolyl, pyridinyl, quinoxalinyl, phenyl, azoniaspiro[5.5]undecanyl, quinolinyl, isoquinolinyl, azepanyl, tetrahydroisoquinolinyl, octahydroindolizinyl, morpholinyl, 1'2'-dihydrospirocyclohexane-1,3'-indolyl, octahydroisoindolyl, azoniabicyclo[3.1.0]hexanyl, diazoniaspiro[4.4]nonanyl, hexahydropyrrolo[3,4-b]pyrrolyl, oxaazoniabicyclo[2.2.1]heptanyl, diazoniaspriro[5.5]undecanyl, diazoniaspiro[3.3]heptanyl, diazoniaspiro[3.5]nonanyl, diazoniaspiro[4.5]decanyl, octahydropyrrolo[3,4-c]pyrrolyl, octahydropyrrolo[3,4-b]pyrrolyl, octahydrocyclopenta[c]pyrrolyl, dihydroindolyl, benzothiazolyl, azoniaspiro[4.5]decanyl, diazoniabicyclo[2.2.2]octanyl, diazoniabicyclo[2.2.1]heptanyl, diazoniabicyclo[3.2.1]octanyl, diazoniabicyclo[2.2.1]heptanyl, azoniabicyclo[3.1.0]hexanyl, tetrahydrothiophenyl, tetrahydronaphthyridinyl, oxaazoniaspiro[4.5]decanyl, oxazepanyl, tetrazolyl, cyclobutyl, dihydroimidazolyl and pyrazolyl, the ring being optionally substituted by one or more independently selected (CH₂)_{b}R⁵ groups;
b is 0 or 1;
R⁵ is C₁₋₆alkyl, NR^{a}R^{b}, oxo, S(O)ᵣR^{c}, C₁₋₆alkoxycarbonyl, halogen, hydroxy, C₁₋₆alkylcarbonyl, hydroxyC₁₋₆alkyl, CONR^{a}R^{b}, haloC₁₋₆alkyl or an optionally substituted ring selected from C₆₋₁₀aryl, C₆₋₁₀arylC₁₋₆alkyl, pyrrolidinyl, piperazinyl, pyrimidinyl, pyridinyl, piperidinyl, thiomorpholinyl, imidazolyl and benzimidazolyl;
r is 1 or 2;
each of R^{a} and R^{b} is independently hydrogen, C₁₋₆alkyl or S(O)₂R^{c}; and
R^{c} is C₁₋₆alkyl.

10. A pharmaceutical composition comprising a compound of any previous claim, or a pharmaceutically acceptable salt, stereoisomer or tautomer thereof in association with a pharmaceutically acceptable carrier.

11. A compound of any one of claims 1 to 9, or a pharmaceutically acceptable salt, stereoisomer or tautomer thereof and an anti-cancer agent for use in simultaneous, separate or sequential administration.

12. A compound of any one of claims 1 to 9, or a pharmaceutically acceptable salt, stereoisomer or tautomer, for use in therapy.

13. The use of a compound of any one of claims 1 to 9, or a pharmaceutically acceptable salt, stereoisomer or tautomer thereof for the manufacture of a medicament for the treatment or prevention of conditions which can be ameliorated by the inhibition of poly(ADP-ribose) polymerase (PARP).

14. The use of a compound of any one of claims 1 to 9, or a pharmaceutically acceptable salt, stereoisomer or tautomer thereof for the manufacture of a medicament for the treatment or prevention of cancer, inflammatory diseases, reperfusion injuries, ischemic conditions, stroke, renal failure, cardiovascular diseases, vascular diseases other than cardiovascular diseases, diabetes, neurodegenerative diseases, retroviral infection, retinal damage or skin senescence and UV-induced skin damage.

15. A compound of any one of claims 1 to 9, or a pharmaceutically acceptable salt, stereoisomer or tautomer, for use as a chemo- or radiosensitizer for cancer treatment.

16. A compound of any one of claims 1 to 9, or a pharmaceutically acceptable salt, stereoisomer or tautomer thereof for use in treating or preventing cancer, inflammatory diseases, reperfusion injuries, ischemic conditions, stroke, renal failure, cardiovascular diseases, vascular diseases other than cardiovascular diseases, diabetes, neurodegenerative diseases, retroviral infection, retinal damage or skin senescence and UV-induced skin damage.

## Patentansprüche

1. Eine Verbindung der Formel I: 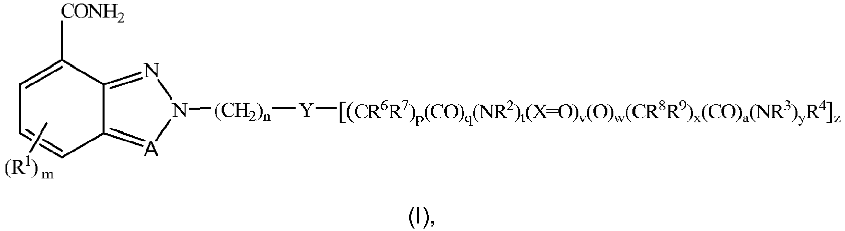 wobei:
a 0 oder 1 ist,
m 0, 1, 2 oder 3 ist,
n 0, 1, 2, 3, 4, 5 oder 6 ist,
jedes p unabhängig 0,1,2, 3, 4, 5 oder 6 ist,
jedes q unabhängig 0 oder 1 ist,
jedes t unabhängig 0 oder 1 ist,
jedes v unabhängig 0 oder 1 ist,
jedes w unabhängig 0 oder 1 ist,
jedes x unabhängig 0, 1, 2, 3, 4, 5 oder 6 ist,
jedes y unabhängig 0 oder 1 ist,
z 1, 2 oder 3 ist,
A CH oder N ist,
jedes R¹ unabhängig Hydroxy, Halogen, Cyano, C₁₋₆-Alkyl, Halogen-C₁₋₆-alkyl, C₁₋₆-Alkoxy oder Halogen-C₁₋₆-alkoxy ist,
Y eine direkte Bindung oder ein Ring ist, wobei der Ring ist: ein C₃₋₅-Cycloalkyl, ein 4-gliedriger gesättigter Heterocyclus, der ein N-Atom enthält, ein 5-, 6- oder 7-gliedriger gesättigter oder teilweise gesättigter Heterocyclus, der 1, 2 oder 3 Heteroatome, unabhängig ausgewählt aus N, O und S, enthält, ein 5-gliedriger ungesättigter Heterocyclus, der 1, 2, 3 oder 4 Heteroatome enthält, unabhängig ausgewählt aus O, N und S, wobei jedoch nicht mehr als eines davon O oder S ist, ein 6-gliedriger ungesättigter Heterocyclus, der 1, 2 oder 3 Stickstoffatome enthält, ein 6-13-gliedriger gesättigter, teilweise gesättigter oder ungesättigter Kohlenwasserstoffring, oder ein 8-13-gliedriger ungesättigter oder teilweise gesättigter heterocyclischer Ring, der 1, 2, 3 oder 4 Heteroatome enthält, unabhängig ausgewählt aus N, O und S,
jedes R² unabhängig Wasserstoff, C₁₋₆-Alkyl oder C₃₋₁₀-Cycloalkyl ist,
jedes X unabhängig C oder SO ist,
jedes R³ unabhängig Wasserstoff oder C₁₋₆-Alkyl ist,
jedes R⁴ unabhängig Wasserstoff, Hydroxy, Cyano, Halogen, C₁₋₆-Alkyl, C₂₋₁₀-Alkenyl, Halogen-C₁₋₆-alkyl, Hydroxy-C₁₋₆-alkyl, C₁₋₆-Alkylcarbonyl, C₁₋₆-Alkoxy, Halogen-C₁₋₆-alkoxy, C₁₋₆-Alkoxycarbonyl, Carboxy, Nitro oder ein Ring ist, wobei der Ring ist: C₆₋₁₀-Aryl, C₆₋₁₀-Aryloxy, C₆₋₁₀-Arylcarbonyl, C₃₋₁₀-Cycloalkyl, ein 4-gliedriger gesättigter heterocyclischer Ring, der ein N-Atom enthält, ein 5- oder 6-gliedriger gesättigter oder teilweise gesättigter heterocyclischer Ring, der ein, zwei oder drei Atome enthält, die unabhängig ausgewählt sind aus N, O und S, ein 5-gliedriger heteroaromatischer Ring, der 1, 2, 3 oder 4 Heteroatome enthält, unabhängig ausgewählt aus N, O und S, wobei nicht mehr als ein Heteroatom davon O oder S ist, ein 6-gliedriger heteroaromatischer Ring, der 1, 2 oder 3 Stickstoffatome enthält, oder ein 7-15 gliedriger ungesättigter, teilweise gesättigter oder gesättigter heterocyclischer Ring, der 1, 2, 3 oder 4 Heteroatome enthält, unabhängig ausgewählt aus N, O und S, wobei jeder dieser Ringe gegebenenfalls substituiert ist durch eine oder mehrere Gruppen, unabhängig ausgewählt aus (CH₂)_{b}R⁵,
jedes b unabhängig 0, 1, 2, 3, 4, 5 oder 6 ist,
jedes R⁵ unabhängig Hydroxy, Oxo, Cyano, Halogen, C₁₋₆-Alkyl, C₂₋₁₀-Alkenyl, Halogen-C₁₋₆-Alkyl, C₁₋₆-Alkylcarbonyl, C₁₋₆-Alkoxy, Halogen-C₁₋₆-alkoxy, Hydroxy-C₁₋₆-Alkyl, C₁₋₆-Alkoxycarbonyl, Carboxy, NR^{a}R^{b}, CONR^{a}R^{b}, S(O)ᵣR^{c} oder ein Ring ist, wobei der Ring ist: C₆₋₁₀-Aryl, C₆₋₁₀-Aryl-C₁₋₆-alkyl, ein 4-gliedriger gesättigter heterocyclischer Ring, der ein N-Atom enthält, ein 5-, 6- oder 7-gliedriger gesättigter oder teilweise gesättigter heterocyclischer Ring, der ein, zwei oder drei Atome enthält, die unabhängig ausgewählt sind aus N, O und S, ein 5-gliedriger heteroaromatischer Ring, der 1, 2, 3 oder 4 Heteroatome enthält, unabhängig ausgewählt aus N, O und S, wobei nicht mehr als ein Heteroatom davon O oder S ist, ein 6-gliedriger heteroaromatischer Ring, der 1, 2 oder 3 Stickstoffatome enthält, oder ein 7-10-gliedriger ungesättigter oder teilweise gesättigter heterocyclischer Ring, der 1, 2, 3 oder 4 Heteroatome enthält, unabhängig ausgewählt aus N, O und S, wobei jeder dieser Ringe gegebenenfalls substituiert ist durch eine oder mehrere Gruppen, unabhängig ausgewählt aus Hydroxy, Cyano, Halogen, C₁₋₆-Alkyl, C₁₋₆-Alkoxy, C₂₋₁₀-Alkenyl, Halogen-C₁₋₆-alkyl, Amino, C₁₋₆-Alkylamino und Di(C₁₋₆-alkyl)amino,
jedes R^{a} und R^{b} unabhängig Wasserstoff, C₁₋₆-Alkyl, C₁₋₆-Alkylcarbonyl, Halogen-C₁₋₆-alkyl, Hydroxy-C₁₋₆-alkyl, S(O)ᵣR^{c} oder S(O)ᵣN(R^{d})₂ ist, oder
R^{a} und R^{b} zusammen mit dem N-Atom, an das sie gebunden sind, einen 4-gliedrigen gesättigten Heterocyclus, der ein N-Atom enthält, oder einen 5-, 6- oder 7-gliedrigen gesättigten oder teilweise gesättigten Heterocyclus, der ein, zwei oder drei N-Atome und null oder ein O-Atom enthält, bilden, wobei der Ring gegebenenfalls durch eine oder mehrere Gruppen, unabhängig ausgewählt aus Hydroxy, Cyano, Halogen, C₁₋₆-Alkyl, C₁₋₆-Alkoxy, C₂₋₁₀-Alkenyl und Halogen-C₁₋₆-alkyl, substituiert ist,
r 0, 1 oder 2 ist,
R^{c} C₁₋₆-Alkyl, C₆₋₁₀-Aryl, ein 5-gliedriger heteroaromatischer Ring, der 1, 2, 3 oder 4 Heteroatome enthält, unabhängig ausgewählt aus N, O und S, wobei nicht mehr als ein Heteroatom davon O oder S ist, ein 6-gliedriger heteroaromatischer Ring, der 1, 2 oder 3 Stickstoffatome enthält, oder ein 7-10-gliedriger ungesättigter oder teilweise gesättigter heterocyclischer Ring, der 1, 2, 3 oder 4 Heteroatome enthält, unabhängig ausgewählt aus N, O und S, ist, wobei jeder dieser Ringe gegebenenfalls substituiert ist durch eine oder mehrere Gruppen, unabhängig ausgewählt aus Hydroxy, Cyano, Halogen, C₁₋₆-Alkyl, C₂₋₁₀-Alkenyl und Halogen-C₁₋₆-Alkyl,
jedes R^{d} unabhängig Wasserstoff oder C₁₋₆-Alkyl ist,
jedes R⁶ und R⁷ unabhängig Wasserstoff oder C₁₋₆-Alkyl ist,
jedes R⁸ und R⁹ unabhängig Wasserstoff, C₁₋₆-Alkyl, Hydroxy, Halogen-C₁₋₆-alkyl, Hydroxy-C₁₋₆-alkyl, Amino, C₁₋₆-Alkylamino oder Di(C₁₋₆-Alkyl)amino ist, oder ein pharmazeutisch annehmbares Salz, Stereoisomer oder Tautomer davon, mit der Maßgabe, dass die folgende Verbindung ausgeschlossen ist:

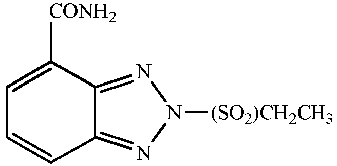

2. Eine Verbindung nach Anspruch 1, wobei Y ein 6-gliedriger ungesättigter Heterocyclus, der 1, 2, oder 3 Stickstoffatome enthält, ein 6-13-gliedriger gesättigter, teilweise gesättigter oder ungesättigter Kohlenwasserstoffring oder ein 8-13-gliedriger ungesättigter oder teilweise gesättigter heterocyclischer Ring, der 1, 2, 3 oder 4 Heteroatome enthält, unabhängig ausgewählt aus N, O und S, ist.

3. Eine Verbindung nach Anspruch 1 der Formel II: 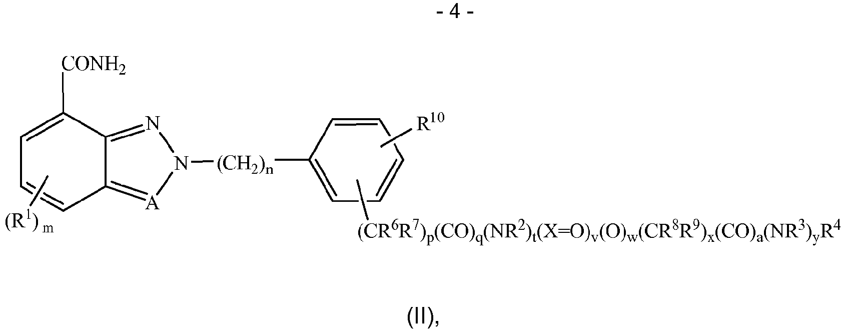 wobei:
a, m, n, p, q, t, v, w, x, y, A, R¹, R², R³, R⁴, R⁶, R⁷, R⁸ und R⁹ wie in Anspruch 1 definiert sind,
R¹⁰ Wasserstoff, Halogen, C₁₋₆-Alkyl oder Halogen-C₁₋₆-alkyl ist,
oder ein pharmazeutisch annehmbares Salz, Stereoisomer oder Tautomer davon.

4. Eine Verbindung nach Anspruch 1 der Formel IV: 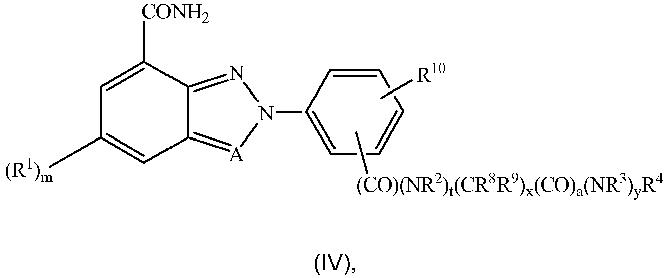 wobei:
m, A, R¹, R², R³, R⁴, R⁸ und R⁹ wie in Anspruch 1 definiert sind,
a 0 oder 1 ist,
t 0 oder 1 ist,
x 0, 1 oder 2 ist,
y 0, oder 1 ist,
R¹⁰ Wasserstoff, Halogen, C₁₋₆-Alky oder Halogen-C₁₋₆-Alkyl, ist,
oder ein pharmazeutisch annehmbares Salz, Stereoisomer oder Tautomer davon.

5. Eine Verbindung nach Anspruch 1 der Formel V: 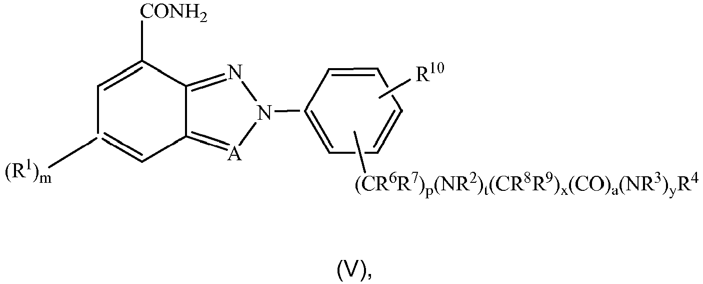 wobei:
a, m, t, y, A, R¹, R², R³, R⁴, R⁶, R⁷, R⁸ und R⁹ wie in Anspruch 1 definiert sind,
p 1 oder 2 ist,
x 0, 1 oder 2 ist,
R¹⁰ Wasserstoff, Halogen, C₁₋₆-Alkyl oder Halogen-C₁₋₆-alkyl ist,
oder ein pharmazeutisch annehmbares Salz, Stereoisomer oder Tautomer davon.

6. Eine Verbindung nach Anspruch 1 der Formel VI: 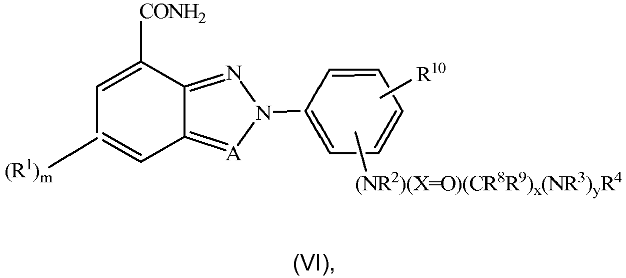 wobei:
m, y, A, R¹, R², R³, R⁴, R⁸, R⁹ und X wie in Anspruch 1 definiert sind,
x 0, 1 oder 2 ist,
R¹⁰ Wasserstoff, Halogen, C₁₋₆-Alkyl oder Halogen-C₁₋₆-alkyl ist,
oder ein pharmazeutisch annehmbares Salz, Stereoisomer oder Tautomer davon.

7. Eine Verbindung nach Anspruch 1 der Formel VII: 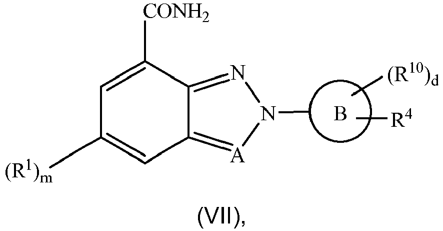 wobei:
m, A, R¹ und R⁴ wie in Anspruch 1 definiert sind,
d 0, 1 oder 2 ist,
B ein 6-gliedriger ungesättigter Heterocyclus, der 1, 2 oder 3 Stickstoffatome enthält, ein 6-13-gliedriger gesättigter, teilweise gesättigter oder ungesättigter Kohlenwasserstoffring oder ein 8-13-gliedriger ungesättigter oder teilweise gesättigter heterocyclischer Ring, der 1, 2, 3 oder 4 Heteroatome enthält, unabhängig ausgewählt aus N, O und S, ist,
R¹⁰ Halogen oder C₁₋₆-Alkyl ist,
oder ein pharmazeutisch annehmbares Salz, Stereoisomer oder Tautomer davon.

8. Eine Verbindung nach Anspruch 1 der Formel VIII: 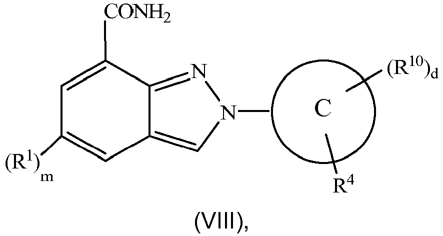
wobei m, R¹ und R⁴ wie in Anspruch 1 definiert sind,
d 0, 1 oder 2 ist,
C ein 4-gliedriger gesättigter Heterocyclus, der ein N-Atom enthält, oder ein 5-, 6- oder 7-gliedriger gesättigter oder teilweise gesättigter Heterocyclus, der 1, 2 oder 3 Heteroatome enthält, unabhängig ausgewählt aus N, O und S, ist,
R¹⁰ Halogen oder C₁₋₆-Alkyl ist,
oder ein pharmazeutisch annehmbares Salz, Stereoisomer oder Tautomer davon.

9. Eine Verbindung nach einem vorhergehenden Anspruch, wobei:
R⁴ Wasserstoff, Hydroxy, Halogen, C₁₋₄-Alkyl, Halogen-C₁₋₄-alkyl, C₁₋₄-Alkoxy, Cyano oder ein Ring ist, ausgewählt aus Cyclopropyl, Cyclobutyl, Cyclopentyl, Cyclohexyl, Azetidinyl, Pyrrolidinyl, Piperidinyl, Piperazinyl, Diazepanyl, Oxazolyl, Pyridinyl, Chinoxalinyl, Phenyl, Azoniumspiro[5.5]undecanyl, Chinolinyl, Isochinolinyl, Azepanyl, Tetrahydroisochinolinyl, Octahydroindolazinyl, Morpholinyl, 1 ',2'-Dihydrospirocyclohexan-1,3'-indolyl, Octahydroisoindolyl, Azoniumbicyclo[3.1.0]hexanyl, Diazoniumspiro[4.4]nonanyl, Hexahydropyrrolo[3,4-b]pyrrolyl, Oxaazoniumbicyclo[2.2.1]heptanyl, Diazoniumspiro[5.5]undecanyl, Diazoniumspiro[3.3]heptanyl, Diazoniumspiro[3.5]nonanyl, Diazoniumspiro[4.5]decanyl, Octahydropyrrolo[3,4-c]pyrrolyl, Octahydropyrrolo[3,4-b]pyrrolyl, Octahydrocyclopenta[c]pyrrolyl, Dihydroindolyl, Benzothiazolyl, Azoniumspiro[4.5]decanyl, Diazoniumbicyclo[2.2.2]octanyl, Diazoniumbicyclo[2.2.1]heptanyl, Diazoniumbicyclo[3.2.1]octanyl, Diazoniumbicyclo[2.2.1]heptanyl, Azoniumbicyclo[3.1.0]hexanyl, Tetrahydrothiophenyl, Tetrahydronaphthyridinyl, Oxaazoniumspiro[4.5]decanyl, Oxazepanyl, Tetrazolyl, Cyclobutyl, Dihydroimidazolyl und Pyrazolyl, wobei der Ring gegebenenfalls substituiert ist durch eine oder mehrere unabhängig ausgewählte (CH₂)_{b}R⁵-Gruppen,
b 0 oder 1 ist,
R⁵ C₁₋₆-Alkyl, NR^{a}R^{b}, Oxo, S(O)ᵣR^{c}, C₁₋₆-Alkoxycarbonyl, Halogen, Hydroxy, C₁-₆-Alkylcarbonyl, Hydroxy-C₁₋₆-alkyl, CONR^{a}R^{b}, Halogen-C₁₋₆-alkyl oder ein gegebenenfalls substituierter Ring, ausgewählt aus C₆₋₁₀-Aryl, C₆₋₁₀-Aryl-C₁₋₆-alkyl, Pyrrolidinyl, Piperazinyl, Pyrimidinyl, Pyridinyl, Piperidinyl, Thiomorpholinyl, Imidazolyl und Benzimidazolyl, ist,
r 1 oder 2 ist,
jedes R^{a} und R^{b} unabhängig Wasserstoff, C₁₋₆-Alkyl oder S(O)₂R^{c} ist und
R^{c} C₁₋₆-Alkyl ist.

10. Eine pharmazeutische Zusammensetzung, umfassend eine Verbindung nach einem vorhergehenden Anspruch oder ein pharmazeutisch annehmbares Salz, Stereoisomer oder Tautomer davon in Verbindung mit einem pharmazeutisch annehmbaren Träger.

11. Eine Verbindung nach einem der Ansprüche 1 bis 9 oder ein pharmazeutisch annehmbares Salz, Stereoisomer oder Tautomer davon und ein Mittel gegen Krebs zur Verwendung bei der gleichzeitigen, separaten oder sequentiellen Verabreichung.

12. Eine Verbindung nach einem der Ansprüche 1 bis 9 oder ein pharmazeutisch annehmbares Salz, Stereoisomer oder Tautomer zur Verwendung in der Therapie.

13. Die Verwendung einer Verbindung nach einem der Ansprüche 1 bis 9 oder eines pharmazeutisch annehmbaren Salzes, Stereoisomers oder Tautomers davon zur Herstellung eines Medikaments zur Behandlung oder Prävention von Zuständen, die durch die Inhibierung von Poly(ADP-ribose)-Polymerase (PARP) gebessert werden können.

14. Die Verwendung einer Verbindung nach einem der Ansprüche 1 bis 9 oder eines pharmazeutisch annehmbaren Salzes, Stereoisomers oder Tautomers davon zur Herstellung eines Medikaments zur Behandlung oder Prävention von Krebs, Entzündungserkrankungen, Reperfusionsverletzungen, ischämischen Zuständen, Schlaganfall, Niereninsuffizienz, kardiovaskulären Erkrankungen, Gefäßerkrankungen anders als kardiovaskuläre Erkrankungen, Diabetes, neurodegenerativen Erkrankungen, Retrovirusinfektion, Netzhautschädigung oder Hautalterung und UV-induzierter Hautschädigung.

15. Eine Verbindung nach einem der Ansprüche 1 bis 9 oder ein pharmazeutisch annehmbares Salz, Stereoisomer oder Tautomer davon zur Verwendung als Chemo- oder Radiosensibilisator für die Krebsbehandlung.

16. Eine Verbindung nach einem der Ansprüche 1 bis 9 oder ein pharmazeutisch annehmbares Salz, Stereoisomer oder Tautomer davon zur Verwendung bei der Behandlung von Krebs, Entzündungserkrankungen, Reperfusionsverletzungen, ischämischen Zuständen, Schlaganfall, Niereninsuffizienz, kardiovaskulären Erkrankungen, Gefäßerkrankungen anders als kardiovaskuläre Erkrankungen, Diabetes, neurodegenerativen Erkrankungen, Retrovirusinfektion, Netzhautschädigung oder Hautalterung und UV-induzierter Hautschädigung.

## Revendications

1. Composé de la formule I: 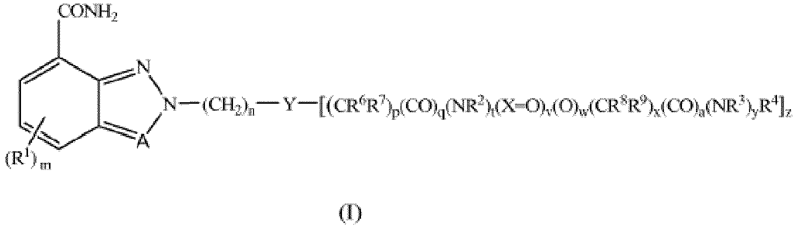 où:
a est 0 ou 1;
m est 0, 1,2 ou 3;
n est 0, 1, 2, 3, 4, 5 ou 6;
chaque p est indépendamment 0, 1, 2, 3, 4, 5 ou 6;
chaque q est indépendamment 0 ou 1;
chaque t est indépendamment 0 ou 1;
chaque v est indépendamment 0 ou 1;
chaque w est indépendamment 0 ou 1;
chaque x est indépendamment 0, 1, 2, 3, 4, 5 ou 6;
chaque y est indépendamment 0 ou 1;
z est 1, 2 ou 3;
A est CH ou N;
chaque R¹ est indépendamment un hydroxy, halogène, cyano, alkyle C₁₋₆, haloalkyle C₁₋₆, alcoxy C₁₋₆ ou haloalcoxy C₁₋₆;
Y est une liaison directe ou un cycle qui est: un cycloalkyle C₃₋₅, un hétérocycle saturé à 4 chaînons contenant un atome N, un hétérocycle saturé ou partiellement saturé à 5, 6 ou 7 chaînons contenant 1, 2 ou 3 hétéroatomes sélectionnés indépendamment parmi N, O et S, un hétérocycle insaturé à 5 chaînons contenant 1, 2, 3 ou 4 hétéroatomes sélectionnés indépendamment parmi O, N et S, dont pas plus d'un est O ou S, un hétérocycle insaturé à 6 chaînons contenant 1, 2 ou 3 atomes d'azote, un cycle hydrocarbure saturé, partiellement saturé ou insaturé à 6-13 chaînons ou un cycle hétérocyclique insaturé ou partiellement saturé à 8-13 chaînons contenant 1, 2, 3 ou 4 hétéroatomes sélectionnés indépendamment parmi N, O et S;
chaque R² est indépendamment un hydrogène, un alkyle C₁₋₆ ou un cycloalkyle C₃₋₁₀;
chaque X est indépendamment C ou SO;
chaque R³ est indépendamment un hydrogène ou un alkyle C₁₋₆;
chaque R⁴ est indépendamment un hydrogène, hydroxy, cyano, halogène, alkyle C₁₋₆, alcényle C₂₋₁₀, haloalkyle C₁₋₆, hydroxyalkyle C₁₋₆, alkyle C₁₋₆carbonyle, alcoxy C₁₋₆, haloalcoxy C₁₋₆, alcoxy C₁₋₆carbonyle, carboxy, nitro ou un cycle qui est: un aryle C_{6-10;} aryloxy C_{6-10;} aryle C₆₋₁₀carbonyle; cycloalkyle C₃₋₁₀; un cycle hétérocyclique saturé à 4 chaînons contenant un atome N; un cycle hétérocyclique saturé ou partiellement saturé à 5 ou 6 chaînons contenant un, deux ou trois atomes sélectionnés indépendamment parmi N, O et S; un cycle hétéroaromatique à 5 chaînons contenant 1, 2, 3 ou 4 hétéroatomes sélectionnés indépendamment parmi N, O et S, dont pas plus d'un hétéroatome est O ou S; un cycle hétéroatomatique à 6 chaînons contenant 1, 2 ou 3 atomes d'azote; ou un cycle hétérocyclique insaturé, partiellement saturé ou saturé à 7-15 chaînons contenant 1, 2, 3 ou 4 hétéroatomes sélectionnés indépendamment parmi N, O et S; l'un quelconque desquels cycles étant optionnellement substitué par un ou plusieurs groupes sélectionnés indépendamment parmi (CH₂)_{b}R⁵;
chaque b est indépendamment 0, 1, 2, 3, 4, 5 ou 6;
chaque R⁵ est indépendamment hydroxy, oxo, cyano, halogène, alkyle C₁₋₆, alcényle C₂₋₁₀, haloalkyle C₁₋₆, alkyle C₁₋₆carbonyle, alcoxy C₁₋₆, haloalcoxy C₁₋₆, hydroxyalkyle C₁₋₆, alcoxy C₁₋₆carbonyle, carboxy, NR^{a}R^{b}, CONR^{a}R^{b}, S(O)ᵣ-R^{c} ou un cycle qui est: un aryle C₆₋₁₀; aryle C₆₋₁₀alkyle C₁₋₆; un cycle hétérocyclique saturé à 4 chaînons contenant un atome N; un cycle hétérocyclique saturé ou partiellement saturé à 5, 6 ou 7 chaînons contenant un, deux ou trois atomes sélectionnés indépendamment parmi N, O et S; un cycle hétéroaromatique à 5 chaînons contenant 1, 2, 3 ou 4 hétéroatomes sélectionnés indépendamment parmi N, O et S, dont pas plus d'un hétéroatome est O ou S; un cycle hétéroaromatique à 6 chaînons contenant 1, 2 ou 3 atomes d'azote; ou un cycle hétérocyclique insaturé ou partiellement saturé à 7-10 chaînons contenant 1, 2, 3 ou 4 hétéroatomes sélectionnés indépendamment parmi N, O et S; l'un quelconque desquels cycles étant optionnellement substitué par un ou plusieurs groupes sélectionnés indépendamment parmi hydroxy, cyano, halogène, alkyle C₁₋₆, alcoxy C₁₋₆, alcényle C₂-₁₀, haloalkyle C₁₋₆, amino, alkyle C₁₋₆amino et di(alkyle C₁₋₆)amino;
chacun d'entre R^{a} et R^{b} est indépendamment un hydrogène, alkyle C₁₋₆, alkyle C₁₋₆carbonyle, haloalkyle C₁₋₆, hydroxyalkyle C₁₋₆, S(O)ᵣR^{c} ou S(O)ᵣN(R^{d})₂; ou bien
R^{a} et R^{b} ensemble avec l'atome N auquel ils sont attachés forment un hétérocycle saturé à 4 chaînons contenant un atome N ou un hétérocycle saturé ou partiellement saturé à 5, 6 ou 7 chaînons contenant un, deux ou trois atomes N et zéro ou un atome O, le cycle étant optionnellement substitué par un ou plusieurs groupes sélectionnés indépendamment parmi hydroxy, cyano, halogène, alkyle C₁₋₆, alcoxy C₁₋₆, alcényle C₂₋₁₀ et haloalkyle C₁₋₆;
r est 0, 1 ou 2;
R^{c} est un alkyle C₁₋₆, aryle C₆₋₁₀, un cycle hétéroaromatique à 5 chaînons contenant 1, 2, 3 ou 4 hétéroatomes sélectionnés indépendamment parmi N, O et S, dont pas plus d'un hétéroatome est O ou S; un cycle hétéroaromatique à 6 chaînons contenant 1, 2 ou 3 atomes d'azote; ou un cycle hétérocyclique insaturé ou partiellement saturé à 7-10 chaînons contenant 1, 2, 3 ou 4 hétéroatomes sélectionnés indépendamment parmi N, O et S; l'un quelconque desquels cycles étant optionnellement substitué par un ou plusieurs groupes sélectionnés indépendamment parmi hydroxy, cyano, halogène, alkyle C₁₋₆, alcényle C₂₋₁₀ et haloalkyle C₁₋₆;
chaque R^{d} est indépendamment un hydrogène ou un alkyle C₁₋₆;
chacun d'entre R⁶ et R⁷ est indépendamment un hydrogène ou un alkyle C₁₋₆;
chacun d'entre R⁸ et R⁹ est indépendamment un hydrogène, alkyle C₁₋₆, hydroxy, haloalkyle C₁₋₆, hydroxyalkyle C₁₋₆, amino, alkyle C₁₋₆amino ou di(alkyle C₁₋₆)amino;
ou un sel pharmaceutiquement acceptable, un stéréoisomère ou un tautomère de celui-ci, à condition que le composé suivant soit exclu:

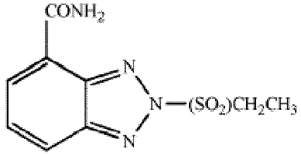

2. Composé selon la revendication 1, dans lequel Y est un hétérocycle insaturé à 6 chaînons contenant 1, 2 ou 3 atomes d'azote, un cycle hydrocarbure saturé, partiellement saturé ou insaturé à 6-13 chaînons ou un cycle hétérocyclique insaturé ou partiellement saturé à 8-13 chaînons contenant 1, 2, 3 ou 4 hétéroatomes sélectionnés indépendamment parmi N, O et S.

3. Composé selon la revendication 1 de la formule II: 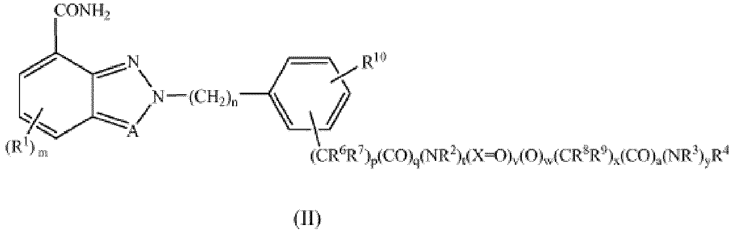 où:
a, m, n, p, q, t, v,w, x, y, A, R¹, R², R³, R⁴, R⁶, R⁷, R⁸ et R⁹ sont tels que définis dans la revendication 1;
R¹⁰ est un hydrogène, halogène, alkyle C₁₋₆ ou haloalkyle C₁₋₆;
ou un sel pharmaceutiquement acceptable, un stéréoisomère ou un tautomère de celui-ci.

4. Composé selon la revendication 1 de la formule IV: 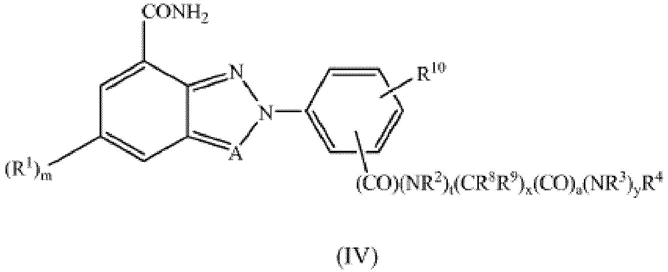 où:
m, A, R¹, R², R³, R⁴, R⁸ et R⁹ sont tels que définis dans la revendication 1;
a est 0 ou 1;
t est 0 ou 1;
x est 0, 1 ou 2;
y est 0 ou 1;
R¹⁰ est un hydrogène, halogène, alkyle C₁₋₆ ou haloalkyle C₁₋₆;
ou un sel pharmaceutiquement acceptable, un stéréoisomère ou un tautomère de celui-ci.

5. Composé selon la revendication 1 de la formule V: 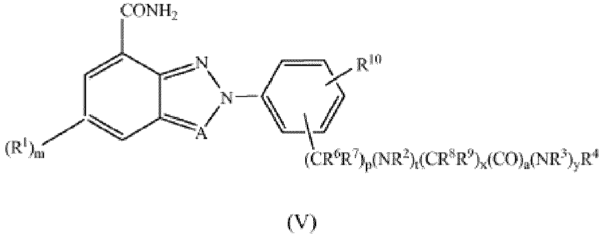 où:
a, m, t, y, A, R¹, R², R³, R⁴, R⁶, R⁷, R⁸ et R⁹ sont tels que définis dans la revendication 1;
p est 1 ou 2;
x est 0, 1 ou 2;
R¹⁰ est un hydrogène, halogène, alkyle C₁₋₆ ou haloalkyle C₁₋₆;
ou un sel pharmaceutiquement acceptable, un stéréoisomère ou un tautomère de celui-ci.

6. Composé selon la revendication 1 de la formule VI: 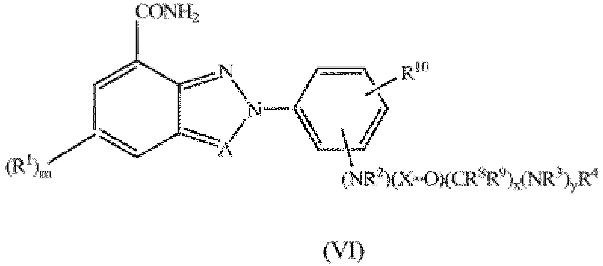 où:
m, y, A, R¹, R², R³, R⁴, R⁸ et R⁹ sont tels que définis dans la revendication 1;
x est 0, 1 ou 2;
R¹⁰ est un hydrogène, halogène, alkyle C₁₋₆ ou haloalkyle C₁₋₆;
ou un sel pharmaceutiquement acceptable, un stéréoisomère ou un tautomère de celui-ci.

7. Composé selon la revendication 1 de la formule VII: 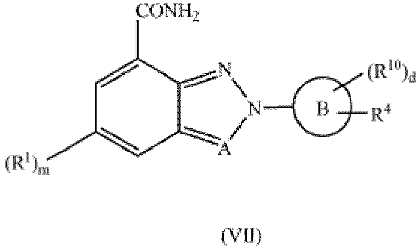 où:
m, A, R¹ et R⁴ sont tels que définis dans la revendication 1;
d est 0, 1 ou 2;
B est un hétérocycle insaturé à 6 chaînons contenant 1, 2 ou 3 atomes d'azote, un cycle hydrocarbure saturé, partiellement saturé ou insaturé à 6-13 chaînons ou un cycle hétérocyclique insaturé ou partiellement saturé à 8-13 chaînons contenant 1, 2, 3 ou 4 hétéroatomes sélectionnés indépendamment parmi N, O et S;
R¹⁰ est un halogène ou un alkyle C₁₋₆;
ou un sel pharmaceutiquement acceptable, un stéréoisomère ou un tautomère de celui-ci.

8. Composé selon la revendication 1 de la formule VIII: 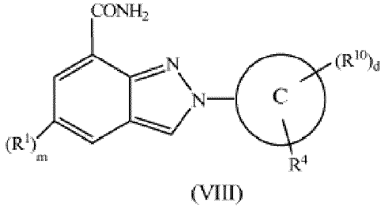
où m, R¹ et R⁴ sont tels que définis dans la revendication 1;
d est 0, 1 ou 2;
C est un hétérocycle saturé à 4 chaînons contenant un atome N ou un hétérocycle saturé ou partiellement saturé à 5, 6 ou 7 chaînons contenant 1, 2 ou 3 hétéroatomes sélectionnés indépendamment parmi N, O et S;
R¹⁰ est un halogène ou un alkyle C₁₋₆;
ou un sel pharmaceutiquement acceptable, un stéréoisomère ou un tautomère de celui-ci.

9. Composé selon l'une quelconque des revendications précédentes, dans lequel:
R⁴ est un hydrogène, hydroxy, halogène, alkyle C₁₋₄, haloalkyle C₁₋₄, alcoxy C₁₋₄, cyano ou un cycle sélectionné parmi cyclopropyle, cyclobutyle, cyclopentyle, cyclohexyle, azétidinyle, pyrrolidinyle, pipéridinyle, pipérazinyle, diazépanyle, oxazolyle, pyridinyle, quinoxazolyle, phényle, azoniaspiro[5.5]undécanyle, quinolinyle, isoquinolinyle, azépanyle, tétrahydroisoquinolinyle, octahydroindolizinyle, morpholinyle, 1'2'-dihydrospirocyclohexane-1,3'-indolyle, octahydroisoindolyle, azoniabicyclo[3.1.0]hexanyle, diazoniaspiro[4.4]nonanyle, hexahydropyrrolo[3,4-b]pyrrolyle, oxaazoniabicyclo[2.2.1]heptanyle, diazoniaspiro[5.5]undécanyle, diazoniaspiro[3.3]heptanyle, diazoniaspiro[3.5]nonanyle, diazoniaspiro[4.5]décanyle, octahydropyrrolo[3,4-c]pyrrolyle, octahydropyrrolo[3,4-b]pyrrolyle, octahydrocyclopenta[c]pyrrolyle, dihydroindolyle, benzothiazolyle, azoniaspiro[4.5]décanyle, diazoniabicyclo[2.2.2]octanyle, diazoniabicyclo[2.2.1]heptanyle, diazoniabicyclo[3.2.1]octanyle, diazoniabicyclo[2.2.1]heptanyle, azoniabicyclo[3.1.0]hexanyle, tétrahydrothiophényle, tétrahydronaphtyridinyle, oxaazoniaspiro[4.5]décanyle, oxazépanyle, tétrazolyle, cyclobutyle, dihydroimidazolyle et pyrazolyle, le cycle étant optionnellement substitué par un ou plusieurs groupes (CH₂)_{b}R⁵ sélectionnés indépendamment;
b est 0 ou 1;
R⁵ est un alkyle C₁₋₆, NR^{a}R^{b}, oxo, S(O)ᵣR^{c}, alcoxy C₁₋₆carbonyle, halogène, hydroxy, alkyle C₁₋₆carbonyle, hydroxyalkyle C₁₋₆, CONR^{a}R^{b}, haloalkyle C₁₋₆ ou un cycle optionnellement substitué sélectionné parmi aryle C₆₋₁₀, aryle C₆₋₁₀alkyle C₁₋₆, pyrrolidinyle, pipérazinyle, pyrimidinyle, pyridinyle, pipéridinyle, thiomorpholinyle, imidazolyle et benzimidazolyle;
r est 1 ou 2;
chacun d'entre R^{a} et R^{b} est indépendamment un hydrogène, alkyle C₁₋₆ ou S(O)₂R^{c}; et
R^{c} est un alkyle C₁₋₆.

10. Composition pharmaceutique comprenant un composé selon l'une quelconque des revendications précédentes, ou un sel pharmaceutiquement acceptable, un stéréoisomère ou un tautomère de celui-ci en association avec un véhicule pharmaceutiquement acceptable.

11. Composé selon l'une quelconque des revendications 1 à 9, ou un sel pharmaceutiquement acceptable, un stéréoisomère ou un tautomère de celui-ci et un agent anticancéreux à utiliser en administration simultanée, séparée ou séquentielle.

12. Composé selon l'une quelconque des revendications 1 à 9, ou un sel pharmaceutiquement acceptable, un stéréoisomère ou un tautomère, à utiliser en thérapie.

13. Utilisation d'un composé selon l'une quelconque des revendications 1 à 9, ou d'un sel pharmaceutiquement acceptable, d'un stéréoisomère ou d'un tautomère de celui-ci, dans la fabrication d'un médicament pour le traitement ou la prévention de conditions qui peuvent être améliorées par l'inhibition de la poly(ADP-ribose) polymérase (PARP).

14. Utilisation d'un composé selon l'une quelconque des revendications 1 à 9, ou d'un sel pharmaceutiquement acceptable, d'un stéréoisomère ou d'un tautomère de celui-ci, dans la fabrication d'un médicament pour le traitement ou la prévention du cancer, de maladies inflammatoires, lésions de reperfusion, conditions ischémiques, accident vasculaire cérébral, insuffisance rénale, maladies cardiovasculaires, maladies vasculaires autres que des maladies cardiovasculaires, diabète, maladies neurodégénératives, infection rétrovirale, lésion de la rétine ou sénescence cutanée et lésion cutanée induite par les UV.

15. Composé selon l'une quelconque des revendications 1-9, ou un sel pharmaceutiquement acceptable, un stéréoisomère ou tautomère, à utiliser en qualité de chimiosensibilisateur ou de radiosensibilisateur dans le traitement du cancer.

16. Composé selon l'une quelconque des revendications 1-9, ou un sel pharmaceutiquement acceptable, un stéréoisomère ou tautomère de celui-ci, à utiliser dans le traitement ou la prévention du cancer, de maladies inflammatoires, lésions de reperfusion, conditions ischémiques, accident vasculaire cérébral, insuffisance rénale, maladies cardiovasculaires, maladies vasculaires autres que des maladies cardiovasculaires, diabète, maladies neurodégénératives, infection rétrovirale, lésion de la rétine ou sénescence cutanée et lésion cutanée induite par les UV.
